(19) Europäisches Patentamt
European Patent Office
Office européen des brevets

(11) EP 3 632 461 A1

(12) EUROPEAN PATENT APPLICATION

(43) Date of publication:
**08.04.2020 Bulletin 2020/15**

(51) Int Cl.:
***A61K 39/00*** *(2006.01)*

(21) Application number: **18198850.2**

(22) Date of filing: **05.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **St. Anna Kinderkrebsforschung
1090 Vienna (AT)**
- **Universität für Bodenkultur Wien
1180 Wien (AT)**

(72) Inventors:
- **SALZER, Benjamin
3744 Stockern (AT)**
- **LEHNER, Manfred
1090 Vienna (AT)**
- **TRAXLMAYR, Michael
1180 Vienna (AT)**

(74) Representative: **Sonn & Partner Patentanwälte
Riemergasse 14
1010 Wien (AT)**

(54) **A GROUP OF CHIMERIC ANTIGEN RECEPTORS (CARS)**

(57) Disclosed is a group of chimeric antigen receptors (CARs) consisting of two, three or four CAR molecules,
wherein the members of the group of CARs can be different or identical in their amino acid sequences to one another, and
wherein each of the CAR molecules of the group comprise at least a transmembrane domain and an ectodomain comprising either an antigen binding moiety or a binding site to which another polypeptide is able to bind, wherein the another polypeptide comprises an antigen binding moiety;
wherein each CAR molecule of the group comprises at least one dimerization domain, wherein this dimerization of a pair of dimerization domains is either induced by a regulating molecule and optionally reduced by another regulating molecule, or occurs in the absence of a regulating molecule and is reduced by a regulating molecule, and
wherein the ectodomain of each CAR molecule of the group in its prevalent conformation is free of cysteine amino acid moieties which are able to form intermolecular disulphide bonds with other CAR molecules of the group, respectively, and
wherein the antigen binding moieties of the CAR molecules of the group and of the other polypeptides being able to bind to the CAR molecules of the group are either specific for one target antigen or for a non-covalent or a covalent complex of different target antigens.

EP 3 632 461 A1

## Description

The invention relates to a group of chimeric antigen receptors (CARs) consisting of two, three or four CAR molecules.

## BACKGROUND OF THE INVENTION

Immunotherapy with CAR T cells, i.e., T cells modified to express chimeric antigen receptors (CARs), is one of the most promising approaches in cancer therapy. To date, the high potential of this therapeutic strategy has been demonstrated by impressive clinical responses in patients with B cell malignancies. Further translation of this success to other tumours, however, is currently prevented by several hurdles (Lim and June, Cell. 2017;168(4):724). For example, CAR T cells are living drugs that replicate after administration and whose activity cannot be controlled sufficiently in a reversible manner. To date, several strategies for conditional CARs have been developed (Lim and June, Cell. 2017;168(4):724). These strategies enable reversible regulation by administration of either small molecule drugs or by infusion of bispecific proteins binding to the target antigen and the CAR (Wu et al. Science. 2015;350(6258):aab4077; Juillerat et al. Sci Rep. 2016;6:18950; Ma et al. Proc Natl Acad Sci U S A. 2016;113(4):E450; Urbanska et al. J Transl Med. 2014;12:347; Urbanska et al. Cancer Res. 2012;72(7):1844; Cartellieri et al. Blood Cancer J. 2016;6(8):e458). The present invention provides a novel strategy for controlling the function of CARs which is based on regulating the avidity, i.e. the synergistic amplification of the affinities of individual antigen binding moieties of a group of CARs and/or of individual antigen binding moieties of other polypeptides being able to bind to CAR molecules of the group. Specifically, the novel CARs should be applicable *in vivo*, especially for the treatment of human patients, without the risk of adverse reactions or at least with reduced adverse reactions. It is a further object to provide means for tumour treatment, especially immunotherapy concepts for the treatment of tumours.

## SUMMARY OF THE INVENTION

The present invention provides a system which is based on a group of chimeric antigen receptors (CARs) consisting of two, three or four CAR molecules,
wherein the members of the group of CARs can be different or identical in their amino acid sequences to one another, and wherein each of the CAR molecules of the group comprise at least a transmembrane domain and an ectodomain comprising either an antigen binding moiety or a binding site to which another polypeptide is able to bind, wherein the another polypeptide comprises an antigen binding moiety, and
wherein at least one CAR molecule of the group additionally comprises an endodomain, which comprises at least a signalling region which can transduce a signal via at least one immunoreceptor tyrosine-based activation motif (ITAM) or at least one immunoreceptor tyrosine-based inhibitory motif (ITIM), and
wherein the endodomain of each CAR molecule of the group, in case the respective CAR molecule comprises an endodomain, is located on the intracellular side of a cell membrane, if expressed in a cell, wherein the ectodomain of each CAR molecule of the group translocates to the extracellular side of a cell membrane, if expressed in a cell, and wherein the transmembrane domain of each CAR molecule of the group is located in a cell membrane, if expressed in a cell;
wherein each CAR molecule of the group comprises at least one dimerization domain, which can mediate homo- or heterodimerization with other CAR molecules of the group, wherein this dimerization of a pair of dimerization domains is either induced by a regulating molecule and optionally reduced by another regulating molecule, or occurs in the absence of a regulating molecule and is reduced by a regulating molecule, wherein a regulating molecule is able to bind under physiological conditions to at least one member of a pair of dimerization domains and by inducing or reducing dimerization either induces or reduces the formation of a non-covalently complexed group of CARs consisting of two, three or four CAR molecules, and
wherein the ectodomain of each CAR molecule of the group in its prevalent conformation is free of cysteine amino acid moieties which are able to form intermolecular disulphide bonds with other CAR molecules of the group, respectively, and wherein the antigen binding moieties of the CAR molecules of the group and of the other polypeptides being able to bind to the CAR molecules of the group are either specific for one target antigen or for a non-covalent or a covalent complex of different target antigens, and
wherein the affinity of each individual antigen binding moiety of a CAR molecule of the group to its target antigen is between 1 mM and 100 nM, and
wherein the affinity of each individual antigen binding moiety of another polypeptide to its target antigen or alternatively the affinity of this other polypeptide to the binding site of its respective CAR molecule is between 1 mM and 100 nM.

## DETAILED DESCRIPTION OF PREFERRED EMBODIMENTS

To date, several strategies for conditionally active CARs have been developed. Examples of such CARs have been disclosed in EP 2 956 175 B1, US20170081411A1 and WO2017032777 A1. However, the strategy according to the present invention represents a fundamentally different principle for controlling CAR function. The underlying principle for controlling CAR function according to the present invention is a group of CARs, in which the individual antigen binding moieties of the individual CAR molecules of the group have only a low affinity to their respective target antigen, so that a monovalent interaction only triggers weak intracellular signalling in the CAR-expressing cell, or no signalling at all. In the case of usage of other polypeptides, each of which containing one antigen binding moiety and additionally being able to bind to a CAR molecule of the group, either the interaction between the antigen binding moiety of the other polypeptide and its respective target antigen, or the interaction between the other polypeptide and its binding site on the respective CAR molecule of the group, must be of low affinity, so that a monovalent interaction only triggers weak intracellular signalling in the CAR-expressing cell, or no signalling at all. However, non-covalent assembly of two, three or four CAR molecules of the group results in the formation of multivalent CAR complexes which are able to interact with their respective target antigen (or with a non-covalent or a covalent complex of different target antigens) in a bivalent, trivalent or tetravalent manner, either directly or indirectly via other polypeptides. This multivalent interaction results in synergistic amplification of the low affinities, i.e. avidity. Importantly, such a multivalent interaction is dependent on the non-covalent complexation of the CAR molecules of the group. Since this non-covalent complexation can be controlled, this ultimately facilitates the regulation of CAR function.

Thus, in order to ensure that only multivalent, but not monovalent interactions efficiently trigger the complexed group of CARs, the affinity of each individual antigen binding moiety of a CAR molecule of the group to its target antigen is between 1 mM and 100 nM, and the affinity of each individual antigen binding moiety of another polypeptide to its target antigen or alternatively the affinity of this other polypeptide to the binding site of its respective CAR molecule is between 1 mM and 100 nM. In preferred embodiments, the affinity of each individual antigen binding moiety of a CAR molecule of the group to its target antigen is between 1 mM and 150 nM, preferably between 1 mM and 200 nM, more preferably between 1 mM and 300 nM, especially between 1 mM and 400 nM, and the affinity of each individual antigen binding moiety of another polypeptide to its target antigen or alternatively the affinity of this other polypeptide to the binding site of its respective CAR molecule is between 1 mM and 150 nM, preferably between 1 mM and 200 nM, more preferably between 1 mM and 300 nM, especially between 1 mM and 400 nM. In other preferred embodiments, the affinity of each individual antigen binding moiety of a CAR molecule of the group to its target antigen is between 500 μM and 100 nM, preferably between 250 μM and 100 nM, more preferably between 125 μM and 100 nM, especially between 50 μM and 100 nM, and the affinity of each individual antigen binding moiety of another polypeptide to its target antigen or alternatively the affinity of this other polypeptide to the binding site of its respective CAR molecule is between 500 μM and 100 nM, preferably between 250 μM and 100 nM, more preferably between 125 μM and 100 nM, especially between 50 μM and 100 nM. In other preferred embodiments, the affinity of each individual antigen binding moiety of a CAR molecule of the group to its target antigen is between 500 μM and 150 nM, preferably between 250 μM and 200 nM, more preferably between 125 μM and 300 nM, especially between 50 μM and 400 nM, and the affinity of each individual antigen binding moiety of another polypeptide to its target antigen or alternatively the affinity of this other polypeptide to the binding site of its respective CAR molecule is between 500 μM and 150 nM, preferably between 250 μM and 200 nM, more preferably between 125 μM and 300 nM, especially between 50 μM and 400 nM.

To facilitate defined dimerization, trimerization or tetramerization of a group of CARs according to the present invention, each CAR molecule of the group of CARs comprises at least one dimerization domain. According to the present invention, the dimerization of a pair of those dimerization domains is regulated by a regulating molecule, which is able to bind under physiological conditions to at least one member of this pair of dimerization domains, thereby inducing or preventing the formation of a defined non-covalent complex of two, three or four CAR molecules of the group. Depending on whether the group of CARs contains activating ITAMs- or inhibitory ITIMs, the non-covalently complexed group of CARs can either efficiently activate or inhibit cells modified to express said group of CARs in response to target cells expressing the respective target antigen or the respective non-covalent or covalent complex of different target antigens.

Regulation of avidity, which is facilitated by the design of the group of CARs according to the present invention, would not be possible with currently used formats of CAR molecules. First, this is due to the fact that at least a fraction of currently used CAR molecules, when expressed in cells, forms dimers (or oligomers) resulting from disulphide bonds between extracellular cysteine residues. However, such uncontrolled dimerization or oligomerization of CAR molecules precludes the efficient regulation of CAR complexation and thus of the resulting avidity effect upon regognition of the target antigen or of the covalently or non-covalently complexed target antigens. For this reason according to the present invention the ectodomain of each CAR molecule of the group in its prevalent conformation is free of cysteine amino acid moieties which are able to form intermolecular disulphide bonds with other CAR molecules of the group, respectively. Second, antigen binding moieties of current CARs are usually based on single-chain variable fragments (scFv) which tend to oligomerize due to intermolecular heterodimerization of variable light (VL) and variable heavy (VH) domains

between individual molecules (Hudson et al., J Immunol Methods. 1999; 231(1-2):177-89; Long et al., Nat Med. 2015;21(6):581-90). Therefore, the individual molecules of a group of CARs according to the present invention preferably do not contain scFv-based antigen binding moieties or other molecular components potentially leading to unwanted and uncontrolled covalent or non-covalent complex formation of CAR molecules.

The basic architecture of the molecular design of the group of CARs according to the present invention can be varied at specific sites without disrupting the possibility for conditional regulation of the non-covalent complexation of the group of CARs and, thereby, of the avidity of the group of CARs upon target antigen recognition.

For example, the group of CARs can consist of two CAR molecules or, alternatively, also of three or four CAR molecules in order to further enhance the avidity effect upon target antigen recognition and thereby the capacity for recognizing also low densities of target antigen molecules on a target cell.

According to the present invention, the group of CARs is designed to be functionally dependent on the presence or absence of regulating molecules, which can be any molecule binding to at least one dimerization domain and capable of inducing or reducing interaction of the members of a pair of dimerization domains. Those molecules are typically small molecules, however, can also be, for example, soluble proteins accumulating in the stroma of tumours, which are frequently proteins that itself natively heterodimerize (e.g., the subunits of heterodimeric cytokines as, e.g., IL-12) or natively homodimerize (e.g. VEGF (as used in example 7), TGF-beta 1, etc.). Regulating molecules are able to bind under physiological conditions to at least one member of a pair of dimerization domains located in the endodomain, the transmembrane domain and/or ectodomain of the CAR molecules. In order to exclude the possibility of fratricide by crosslinking of CAR molecules between different cells, the dimerization domains, however, are preferably integrated in the endodomains and/or in the transmembrane domains of the CAR molecules of the group of CARs, more preferably in the endodomains.

An individual CAR molecule of the group of CARs can either directly bind to a target antigen or a non-covalent or covalent complex of different target antigens via an integrated antigen binding moiety or indirectly via another polypeptide being able to bind to the CAR molecule and comprising an antigen binding moiety. Such another polypeptide thereby is defined as a soluble protein that does not belong to the group of CARs and can non-covalently bind to a binding site in a CAR molecule of the group either directly or indirectly via a covalent modification on the other polypeptide such as, for example, a covalently bound fluorescein isothiocyanate (FITC) molecule. This principle of indirect CAR binding to an antigen, which is well known in the CAR field (Cho et al., Cell. 2018;173(6):1426-1438; Ma et al., Proc Natl Acad Sci U S A. 2016;113(4):E450-458; Urbanska et al., Cancer Res. 2012;72(7):1844-1852) and now being tested in the clinic (Labanieh et al., Nat Biomed Eng. 2018; 2:377-391), can also be incorporated into the group of CARs according to the present invention. In this case, in principle, the low affinity-binding does not necessarily need to take place via the antigen binding moiety of the other polypeptide but can also take place at the CAR molecule via the binding site to which the other polypeptide, which comprises an antigen binding moiety, is able to bind.

In order to avoid the necessity to administer another polypeptide comprising an antigen binding moiety and being able to bind to the CAR molecule, however, it is preferred that the ectodomain of each CAR molecule of the group of CARs itself comprises an antigen binding moiety.

As mentioned above, the basic architecture of the CAR molecules of the group, according to the present invention, can be adapted to the needs of different applications. The order of the domains in the CAR molecules of the group from the extracellular to the intracellular side preferably conforms on the surface of a cell to the following basic architecture: an antigen binding moiety or a binding site to which another polypeptide comprising an antigen binding moiety is able to bind, preferably a hinge region for spatial optimization, and a transmembrane domain. In the preferred embodiment of an ITAM-containing group of CARs the transmembrane domain is preferably followed in at least one CAR molecule by a signalling region comprising a co-stimulatory domain, wherein preferably this co-stimulatory signalling region, or optionally the transmembrane domain, is followed by at least one dimerization domain, and further, in at least one CAR molecule, by a signalling region comprising at least one ITAM, wherein the order of the co-stimulatory and the ITAM-containing signalling region can be inverted. CAR molecules that do not comprise an ITAM either lack a co-stimulatory signalling region, or comprise one co-stimulatory signalling region, or two co-stimulatory signalling regions, or even more co-stimulatory signalling regions, but preferably not more than two co-stimulatory signalling regions, or even more preferably only one co-stimulatory signalling region. In the case of an ITIM comprising group of CARs the transmembrane domain is preferentially followed in at least one CAR molecule by an ITIM-comprising inhibitory signalling region. This inhibitory signalling region, or optionally the transmembrane domain, is followed by at least one dimerization domain and optionally a second inhibitory signalling region. Generally, in an ITAM- and ITIM-comprising group of CARs the dimerization domains, of which at least one is mandatory for each CAR molecule of the group, can be located alternatively or additionally in the ectodomain or the transmembrane domain, however, preferably between the transmembrane domain and a signalling region, and/or especially between two signalling regions and/or especially at the intracellular end of the CAR molecules. Finally, any two adjacent components (such as antigen binding moieties, binding sites to which another polypeptide comprising an antigen binding moiety is able to bind, hinge regions, transmembrane domains, signalling regions, dimerization domains) of a CAR molecule of the group can optionally be separated by a linker.

Different groups of CARs directed against different target antigens or different non-covalent or covalent complexes of different target antigens can also be co-expressed in a cell, for example, to inhibit immune escape of tumours triggered by the loss of target antigens. A group of CARs can also be co-expressed with any other protein in a given cell.

1. Antigen binding moiety:

The antigen binding moieties of the individual CAR molecules of a group of CARs according to the present invention can be directed towards a single selected epitope of a target antigen molecule. In this case, efficient signalling by the complexed group of CARs requires either a high density of the target antigen or the capacity of the target antigen to dimerize/oligomerize. For enabling efficient and highly sensitive targeting also of monomeric target antigen molecules, the individual CAR molecules of a group of CARs according to the present invention can be directed also towards different (i.e. non-overlapping) epitopes of a selected target antigen or towards different epitopes located on different molecules which natively form covalent complexes (e.g. TCR alpha/TCR beta chains) or non-covalent complexes (e.g. MHC II alpha/beta chains or ErbB-1/ErbB-2). In those cases, i.e. when targeting non-overlapping epitopes on the same target antigen or epitopes on different, but complexed target antigens, the avidity effect is strongly increased, because the target epitopes of the group of CARs are located on a single molecule or on complexed molecules which corresponds to a very high local concentration of the epitopes.

An antigen binding moiety suitable for use in a group of CARs according to the present invention can be any antigen binding polypeptide (Labanieh et al., Nat Biomed Eng. 2018; 2:377-391), a wide variety of which are known in the art (Simeon et al., Protein Cell. 2017; Gilbreth et al., Curr Opin Struct Biol. 2012;22(4):413-420; Koide et al., ACS Chem Biol. 2009;4(5):325-334; Traxlmayr et al., J Biol Chem. 2016;291(43):22496-22508). Meanwhile, many more non-antibody binding proteins have been reported (Plückthun, Alternative Scaffolds: Expanding the options of antibodies. In: Little M, ed. New York: Cambridge University Press; 2009:244-271; Chapman et al., Cell Chem Biol. 2016;23(5):543-553; Binz et al., Nat Biotechnol. 2005;23(10):1257-1268; Vazquez-Lombardi et al., Drug Discov Today. 2015;20(10):1271-1283), and, in fact, synthetic library design and selection can be applied to any protein which then can potentially serve as antigen binding moiety, too (Plückthun, Alternative Scaffolds: Expanding the options of antibodies. In: Little M, ed. New York: Cambridge University Press; 2009:244-271).

In some instances, the antigen binding moiety can be a single chain Fv (scFv), other antibody based recognition domains like cAb VHH (camelid antibody variable domains) and its humanized versions, IgNAR VH (shark antibody variable domains) and its humanized versions, sdAb VH (single domain antibody variable domains) or "camelized" antibody variable domains. In some instances, T-cell receptor (TCR) based recognition domains such as single chain TCRs (scTv, single chain two-domain TCR containing VaV) can also be suitable for use. Preferably, the antigen binding moiety of each molecule of the group of CARs comprises only one protein domain, preferably a human or non-human VH or VL single domain antibody (nanobody) or an engineered antigen binding moiety based on the Z-domain of staphylococcal Protein A, lipocalins, SH3 domains, fibronectin type III (FN3) domains, knottins, Sso7d, rcSso7d, Sac7d, Gp2, DARPins or ubiquitin; or a ligand, a receptor or a co-receptor which was chosen for or engineered for low affinity binding and lack of homotypic interaction. Ligands include, for example, cytokines (e.g., IL-13, etc.); growth factors (e.g., heregulin; etc.); and the like. The ligand can be a receptor binding fragment of a ligand (e.g., a peptide of HGF (Thayaparan et al., Oncoimmunology. 2014;6(12):e1363137); an integrin-binding peptide (e.g., a peptide comprising the sequence Arg-Gly-Asp); etc.). Similarly, the receptor can be a ligand binding fragment of a receptor. Suitable receptors include, for example, a cytokine receptor (e.g., an IL-13 receptor; an IL-2 receptor; etc.); a cellular adhesion molecule (e.g., CD11a (Park et al., Sci Rep. 2017;7(1):14366); etc); PD-1; and the like. The antigen binding moiety of each molecule of the group of CARs preferably does not cause undesired aggregation of the CAR molecules. As discussed above, such undesired dimerization or oligomerization of CAR molecules of the group precludes efficient control of the dimerization, trimerization or tetramerization status of the CAR molecules of the group. For this reason, the antigen binding moiety is preferably not a single-chain variable fragment (scFv) derived from a monoclonal antibody. With the aim of clinical applicability of the groups of CARs according to the present invention, antigen binding moieties are preferably derived from human single protein domains (e.g., fibronectin type III domain (FN3) based Monobodies).

2. Hinge region:

In some embodiments the ectodomains of the CAR molecules of the group comprise a hinge region interposed between an antigen binding moiety (or a binding site to which another polypeptide comprising at least an antigen binding moiety is able to bind) and the transmembrane domain, preferably a hinge region derived from CD8 alpha (amino acid sequence position 138 - 182 according to UniProtKB/Swiss-Prot P01732-1), or CD28 (amino acid sequence position 114 - 152 according to UniProtKB/Swiss-Prot P10747), or PD-1 (amino acid sequence position 146 - 170 according to UniProtKB/Swiss-Prot Q15116), wherein the sequences derived from CD8 alpha, CD28 or PD-1 can be N-terminally and/or C-terminally truncated and can have any length within the borders of the said sequence region, and wherein the

cysteine residues in the said hinges derived from CD8 alpha and CD28 are deleted or replaced by other amino acid residues. In principle, the flexible membrane anchors and also other parts of many more receptors are suited for use in the hinge regions and/or transmembrane domains of CAR molecules of the group (Labanieh et al., Nat Biomed Eng. 2018; 2:377-391), provided that they are modified, if necessary, for preventing dimerization according to the present invention.

Depending on the individual structural requirements for optimal binding of a selected target antigen, the hinge region of a CAR molecule can have a length of from about 2 amino acids to about 50 amino acids, e.g., from about 4 amino acids (aa) to about 10 aa, from about 10 aa to about 15 aa, from about 15 aa to about 20 aa, from about 20 aa to about 25 aa, from about 25 aa to about 30 aa, from about 30 aa to about 40 aa, or from about 40 aa to about 50 aa. Optionally, hinge regions can comprise more than 50 amino acids, for example, when structured domains are integrated (e.g. from CD34 UniProt P28906-1 aa 42-140 for facilitating enrichment of CAR modified cells, as disclosed in US2018/0094044 A1).

Preferably, also other polypeptides, preferably glycine and glycine-serine polymers can be used for the hinges since both Gly and Ser are relatively unstructured, and therefore can serve as a neutral tether between the CAR components. Glycine accesses significantly more phi-psi space than even alanine, and is much less restricted than residues with longer side chains (Scheraga, Rev. Computational Chem. 1992; 11173-11142). Therefore, for adjusting the CAR molecules for optimal binding to their target antigens, a hinge region interposed between an antigen binding moiety (or a binding site to which another polypeptide comprising at least an antigen binding moiety is able to bind) and the transmembrane domaincan comprise a glycine polymer (G)n and/or glycine-serine polymers (GS)n, (GSGGS)n, (GGS)n (GGGS)n, (GGGGS)n where n is an integer of at least one.

3. Transmembrane Domain:

Each molecule of the group of CARs comprises a transmembrane domain for insertion into a eukaryotic cell membrane. Any transmembrane (TM) domain that provides for insertion of a polypeptide into the cell membrane of a eukaryotic (e.g., mammalian) cell is suitable for use. For example, the TM sequence IYIWAPLAGTCGVLLLSLVITLYC of human CD8 alpha (Uniprot P01732, amino acids (aa) 183-206) can be used. Futher examples of suitable TM sequences include: human CD8 beta derived: LGLLVAGVLVLLVSLGVAIHLCC (Uniprot P10966, aa 173-195); human CD4 derived: ALIVLGGVAGLLLFIGLGIFFCVRC (Uniprot P01730, aa 398-422); human CD3 zeta derived: LCYLLDGILFIYGVILTALFLRV (Uniprot P20963, aa 31-53); human CD28 derived: FWVLVVVGGVLACYSLLVTVAFIIFWV (Uniprot P10747, aa 154-179); human CD134 (OX40) derived: VAAILGLGLVLGLLGPLAILLALYLL (Uniprot P43489, aa 215-240); human CD27 derived: ILVIFSGMFLVFTLAGALFLH (Uniprot P26842, aa 192-212); human CD278 (ICOS) derived: FWLPIGCAAFVVVCILGCILI (Uniprot Q9Y6W8, aa 141-161); human CD279 (PD-1) derived: VGVVGGLLGSLVLLVWVLAVI (Uniprot Q15116, aa 171-191); human DAP12 derived: GVLAGIVMGDLVLTVLIALAV (Uniprot O43914, aa 41-61); and human CD7 derived: ALPAALAVISFLLGLGLGVACVLA (Uniprot P09564, aa 178-201).

4. Immunoreceptor tyrosine-based activation motif (ITAM):

According to the present invention at least one molecule of the group of CARs contains an endodomain that can transduce a signal via at least one immunoreceptor tyrosine-based activation motif (ITAM). An ITAM motif is $YX_1X_2L/I$, where $X_1$ and $X_2$ are independently any amino acid. An ITAM-containing endodomain can comprise 1, 2, 3, 4, 5, 6, 7, 8, 9, 10, 11, 12 or more than 12 ITAM motifs. ITAM-containing portions of signal-transducing endodomains are preferably derived from any ITAM-containing protein and do not need to contain the entire sequence of the entire protein from which they are derived. Examples of suitable ITAM-containing polypeptides include: DAP12; FCERIG (Fc epsilon receptor I gamma chain); CD3D (CD3 delta); CD3E (CD3 epsilon); CD3G (CD3 gamma); CD3Z (CD3 zeta); and CD79A (antigen receptor complex-associated protein alpha chain).

In preferred embodiments, at least one signalling domain in at least one CAR molecule of the group of CARs is derived from the cytoplasmic domain of the T-cell surface glycoprotein CD3 zeta chain (also known as CD3Z, T-cell receptor T3 zeta chain, CD247, CD3-ZETA, CD3H, CD3Q, T3Z, TCRZ, etc.). For example, a suitable ITAM-containing domain can comprise an amino acid sequence having at least about 75%, at least about 80%, at least about 85%, at least about 90%, at least about 95%, at least about 98%, or 100%, amino acid sequence identity to a contiguous stretch of from about 50 amino acids to about 60 amino acids (aa), from about 60 aa to about 70 aa, from about 70 aa to about 80 aa, from about 80 aa to about 90 aa, from about 90 aa to about 100 aa, from about 100 aa to about 110 aa, from about 110 aa to about 115 aa, from about 115 aa to about 120 aa, from about 120 aa to about 130 aa, from about 130 aa to about 140 aa, from about 140 aa to about 150 aa, or from about 150 aa to about 160 aa, of either of the amino acid sequences (2 isoforms) MKWKALFTAAILQAQLPITEAQSFGLLDPKLCYLLDGILFIYGVILTALFLRVKFSRSADAPAY QQGQNQL**YNEL**NLGRREE**YDVL**DKRRGRDPEMGGKPRRKNPQEGL**YNEL**QKDKMAEA**YSEI**GMK GERRRGKGHDGL**YQGL**STATKDT**YDAL**HMQALPPR (Uniprot P20963-3) or

MKWKALFTAAILQAQLPITEAQSFGLLDPKLCYLLDGILFIYGVILTALFLRVKFSRSADAPAY
QQGQNQL**YNEL**NLGRREE**YDVL**DKRRGRDPEMGGKPQRRKNPQEGL**YNEL**QKDKMAEA**YSEI**GM KGERRRGKGHDGL**YQGL**STATKDT**YDAL**HMQALPPR (Uniprot P20963-1), where the ITAM motifs are in bold and are underlined.

Likewise, a suitable ITAM-containing domain can comprise an ITAM-containing portion of the full length CD3 zeta amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to any of the amino acid sequences
RVKFSRSADAPAYQQGQNQL**YNEL**NLGRREE**YDVL**DKRRGRDPEMGGKPRRKNPQEGL**YNEL**QK
DKMAEA**YSEI**GMKGERRRGKGHDGL**YQGL**STATKDT**YDAL**HMQALPPR (Uniprot P20963-3 aa 52-163), NQL**YNEL**NLGRREE**YDVL**DKR (Uniprot P20963-3 aa 69-89), EGL**YNEL**QKDKMAEA**YSEI**GMK (Uniprot P20963-3 aa 107-128), DGL**YQGL**STATKDT**YDAL**HMQ (Uniprot P20963-3 aa 138-158) where the ITAMs are in bold and are underlined.

An ITAM-containing domain can also be derived from T-cell surface glycoprotein CD3 delta chain (also known as CD3D; CD3-DELTA; T3D; CD3 antigen, delta subunit; CD3 delta; CD3d antigen, delta polypeptide (TiT3 complex); OKT3, delta chain; T-cell receptor T3 delta chain; T-cell surface glycoprotein CD3 delta chain; etc.). For example, a suitable ITAM-containing domain can comprise an amino acid sequence having at least about 75%, at least about 80%, at least about 85%, at least about 90%, at least about 95%, at least about 98%, or 100%, amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 170 aa, of either of the following amino acid sequences (2 isoforms): Uniprot P04234-1; Uniprot P04234-2.

Likewise, a suitable ITAM-containing domain can comprise an ITAM-containing portion of the full length CD3 delta amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to the amino acid sequence DQV**YQPL**RDRDDAQ**YSHL**GGN (Uniprot P04234-1 aa 146-166), where the ITAMs are in bold and are underlined.

An ITAM-containing domain can also be derived from T-cell surface glycoprotein CD3 epsilon chain (also known as CD3e, T-cell surface antigen T3/Leu-4 epsilon chain, T-cell surface glycoprotein CD3 epsilon chain, AI504783, CD3, CD3epsilon, T3e, etc.). For example, a suitable ITAM-containing domain can comprise an amino acid sequence having at least about 75%, at least about 80%, at least about 85%, at least about 90%, at least about 95%, at least about 98%, or 100%, amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 205 aa, of the amino acid sequence Uniprot P07766-1.

Likewise, a suitable ITAM-conatining domain can comprise an ITAM-containing portion of the full length CD3 epsilon amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to the amino acid sequence NPD**YEPI**RKGQRDL**YSGL**NQR (Uniprot P07766-1 aa 185-205), where the ITAMs are in bold and are underlined.

An ITAM-containing domain can also be derived from T-cell surface glycoprotein CD3 gamma chain (also known as CD3G, T-cell receptor T3 gamma chain, CD3-GAMMA, T3G, gamma polypeptide (TiT3 complex), etc.). For example, a suitable ITAM-containing domain can comprise an amino acid sequence having at least about 75%, at least about 80%, at least about 85%, at least about 90%, at least about 95%, at least about 98%, or 100%, amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, or from about 150 aa to about 180 aa, of the amino acid sequence
MEQGKGLAVLILAIILLQGTLAQSIKGNHLVKVYDYQEDGSVLLTCDAEAKNITWFKDGKMIGF
LTEDKKKWNLGSNAKDPRGMYQCKGSQNKSKPLQVYYRMCQNCIELNAATISGFLFAEIVSIFV
LAVGVYFIAGQDGVRQSRASDKQTLLPNDQL**YQPL**KDREDDQ**YSHL**QGNQLRRN (Uniprot P09693-1), where the ITAMs are in bold and are underlined.

Likewise, a suitable ITAM-containing domain can comprise an ITAM-containing portion of the full length CD3 gamma amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to the amino acid sequence DQL**YQPL**KDREDDQ**YSHL**QGN (Uniprot P09693-1 aa 157-177), where the ITAMs are in bold and are underlined.

An ITAM-containing domain can also be derived from DAP12 (also known as TYROBP; TYRO protein tyrosine kinase binding protein; KARAP; PLOSL; DNAX-activation protein 12; KAR-associated protein; TYRO protein tyrosine kinase-binding protein; killer activating receptor associated protein; killer-activating receptor-associated protein; etc.).

For example, a suitable ITAM-containing domain can comprise an amino acid sequence having at least about 75%, at least about 80%, at least about 85%, at least about 90%, at least about 95%, at least about 98%, or 100%, amino acid sequence identity to any of the following amino acid sequences (4 isoforms): Uniprot O43914-1; Uniprot O43914-2; Uniprot O43914-3; Uniprot X6RGC9-1.

Likewise, a suitable ITAM-containing domain can comprise an ITAM-containing portion of the full length DAP12 amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to ESP**YQEL**QGQRSDV**YSDL**NTQ (Uniprot O43914-1 aa 88-108), where the ITAMs are in bold and are underlined.

An ITAM-containing domain can also be derived from FCER1G (also known as FCRG; Fc epsilon receptor I gamma chain; Fc receptor gamma-chain; fc-epsilon RI-gamma; fcRgamma; fceRI gamma; high affinity immunoglobulin epsilon receptor subunit gamma; immunoglobulin E receptor, high affinity, gamma chain; etc.). For example, a suitable ITAM-containing domain can comprise an amino acid sequence having at least about 75%, at least about 80%, at least about 85%, at least about 90%, at least about 95%, at least about 98%, or 100% amino acid sequence identity to the amino acid sequence MIPAVVLLLLLLVEQAAALGEPQLCYILDAILFLYGIVLTLLYCRLKIQVRKAAITSYEKSDGV **YTGL**STRNQET**YETL**KHEKPPQ (Uniprot P30273), where the ITAMs are in bold and are underlined.

Likewise, a suitable ITAM-containing domain can comprise an ITAM motif-containing portion of the full length FCER1G amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to the amino acid sequence DGV**YTGL**STRNQET**YETL**KHE (Uniprot P30273 aa 62-82), where the ITAMs are in bold and are underlined.

An ITAM-containing domain can also be derived from CD79A (also known as B-cell antigen receptor complex-associated protein alpha chain; CD79a antigen (immunoglobulin-associated alpha); MB-1 membrane glycoprotein; ig-alpha; membrane- bound immunoglobulin-associated protein; surface IgM-associated protein; etc.). For example, a suitable ITAM-containing domain can comprise an amino acid sequence having at least about 75%, at least about 80%, at least about 85%, at least about 90%, at least about 95%, at least about 98%, or 100%, amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 150 aa, from about 150 aa to about 200 aa, or from about 200 aa to about 220 aa, of either of the amino acid sequences (2 isoforms) MPGGPGVLQALPATIFLLFLLSAVYLGPGCQALWMHKVPASLMVSLGEDAHFQCPHNSSNNANV TWWRVLHGNYTWPPEFLGPGEDPNGTLIIQNVNKSHGGIYVCRVQEGNESYQQSCGTYLRVRQP PPRPFLDMGEGTKNRIITAEGIILLFCAVVPGTLLLFRKRWQNEKLGLDAGDEYEDENL**YEGL**N LDDCSM**YEDI**SRGLQGTYQDVGSLNIGDVQLEKP (Uniprot P11912-1) or MPGGPGVLQALPATIFLLFLLSAVYLGPGCQALWMHKV-PASLMVSLGEDAHFQCPHNSSNNANV TWWRVLHGNYTWPPEFLGPGEDPNEPPPRPFLDMGEGTKNRIITAEGI-ILLFCAVVPGTLLLFR KRWQNEKLGLDAGDEYEDENL**YEGL**NLDDCSM**YEDI**SRGLQGTYQDVGSLNIGDVQLEKP (Uniprot P11912-2), where the ITAMs are in bold and are underlined.

Likewise, a suitable ITAM-containing domain can comprise an ITAM-containing portion of the full length CD79A amino acid sequence. Thus, a suitable ITAM-containing domain can comprise an amino acid sequence having at least about 75%, at least about 80%, at least about 85%, at least about 90%, at least about 95%, at least about 98%, or 100%, amino acid sequence identity to the amino acid sequence ENL**YEGL**NLDDCSM**YEDI**SRG (Uniprot P11912-1 aa 185-205), where the ITAMs are in bold and are underlined.

Therefore, according to the present invention, the endodomain of at least one CAR molecule of the group of CARs preferentially comprises at least one ITAM, said ITAM is preferably selected from CD3 zeta, DAP12, Fc-epsilon receptor 1 gamma chain, CD3 delta, CD3 epsilon, CD3 gamma, and CD79A (antigen receptor complex-associated protein alpha chain).

Since the number of ITAMs correlates with the signalling efficiency of CARs (James, Sci Signal. 2018;11(531)), the group of CARs preferably comprises altogether at last three ITAMs, wherein the ITAMs can be confined to only a single CAR molecule of the group. Alternatively, several or all CAR molecules of the group can comprise at least one ITAM. In some embodiments the ITAM containing portions in the different endodomains of the CAR molecules of the group are derived from the same receptor, whereas in other embodiments the ITAM containing portions in the different endodomains of the CAR molecules of the group are derived from different receptors. In some embodiments the group of CARs comprises only one molecule comprising an ITAM-containing portion, preferably derived from CD3 zeta. In other embodiments the group of CARs consists of two molecules, wherein both comprise parts of the cytoplasmic domain derived from CD3 zeta. With respect to vector payload, the total number of ITAMs in a group of CARs is preferably between three and six. Furthermore, the ITAM containing sequences are preferably chosen and/or engineered for minimal nucleotide sequence homology, in order minimize the risk of homologous recombination.

5. Co-stimulatory domains:

In preferred embodiments, the endodomain of at least one CAR molecule of the group comprises a signalling region containing a co-stimulatory domain derived from 4-1BB (CD137), CD28, ICOS, BTLA, OX-40, CD2, CD6, CD27, CD30, CD40, GITR, and HVEM, whereby the co-stimulatory domains comprised by a group of CARs can optionally be derived from different co-stimulatory receptors.

A co-stimulatory domain suitable for inclusion in a co-stimulatory signalling region of a CAR molecule of the group of CARs can have a length of from about 30 aa to about 70 aa, e.g., a co-stimulatory domain can have a length of from about 30 aa to about 35 aa, from about 35 aa to about 40 aa, from about 40 aa to about 45 aa, from about 45 aa to about 50 aa, from about 50 aa to about 55 aa, from about 55 aa to about 60 aa, from about 60 aa to about 65 aa, or from about 65 aa to about 70 aa. Optionally, the co-stimulatory domain can have a length of from about 70 aa to about 100 aa, from about 100 aa to about 200 aa, or greater than 200 aa.

In preferred embodiments, the co-stimulatory domain in at least one molecule of the group of CARs is derived from an intracellular portion of the transmembrane protein 4-1BB (also known as TNFRSF9; CD137; 4-1BB; CDw137; ILA; etc.). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot Q07011 aa 214-255.

In preferred embodiments, the co-stimulatory domain in at least one molecule of the group of CARs is derived from an intracellular portion of the transmembrane protein CD28 (also known as Tp44). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot P10747 aa 177-220.

In preferred embodiments, the co-stimulatory domain in at least one molecule of the group of CARs is derived from an intracellular portion of the transmembrane protein ICOS (also known as AILIM, CD278, and CVID1). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot Q9Y6W8 aa 165-199.

In preferred embodiments, the co-stimulatory domain in at least one molecule of the group of CARs is derived from an intracellular portion of the transmembrane protein CD27 (also known as S 152, T14, TNFRSF7, and Tp55). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot P26842 aa 212-260.

In other embodiments, the co-stimulatory domain in at least one molecule of the group of CARs can be derived from an intracellular portion of the transmembrane protein OX-40 (also known as TNFRSF4, RP5-902P8.3, ACT35, CD134, OX40, TXGP1L). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot P43489 aa 241-277.

In other embodiments, the co-stimulatory domain in at least one molecule of the group of CARs can be derived from an intracellular portion of the transmembrane protein BTLA (also known as BTLA1 and CD272). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot Q7Z6A9 aa 176-289.

In other embodiments, the co-stimulatory domain in at least one molecule of the group of CARs can be derived from an intracellular portion of the transmembrane protein GITR (also known as TNFRSF18, RP5-902P8.2, AITR, CD357, and GITR-D). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot Q9Y5U5 aa 188-241.

In other embodiments, the co-stimulatory domain in at least one molecule of the group of CARs can be derived from an intracellular portion of the transmembrane protein HVEM (also known as TNFRSF14, RP3-395M20.6, ATAR, CD270, HVEA, HVEM, LIGHTR, and TR2). For example, a suitable co-stimulatory domain can comprise an amino acid sequence having at least about 75%, at least about 80%, at least about 85%, at least about 90%, at least about 95%, at least about 98%, or 100% amino acid sequence identity to the amino acid sequence Uniprot Q92956 aa 224-283.

In other embodiments, the co-stimulatory domain in at least one molecule of the group of CARs can be derived from an intracellular portion of the transmembrane protein CD30 (also known as TNFRSF8, D1S166E, and Ki-1). For example, a suitable co-stimulatory domain can comprise an amino acid sequence having at least about 75%, at least about 80%, at least about 85%, at least about 90%, at least about 95%, at least about 98%, or 100% amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa,

from about 140 aa to about 150 aa, from about 150 aa to about 160 aa, or from about 160 aa to about 185 aa of the amino acid sequence Uniprot P28908 aa 409-595.

6. Inhibitory domains:

In cases, where the group of CARs is intended to trigger an inhibitory signal, the endodomain of at least one CAR molecule of the group contains a signalling region that comprises the (or a part of the) ITIM-containing cytoplasmic portion of an inhibitory receptor preferentially selected from PD-1, CD85A, CD85C, CD85D, CD85J, CD85K, LAIR1, TIGIT, CEACAM1, CD96, KIR2DL, KIR3DL, SLAM family members, CD300/LMIR family members, CD22 and other Siglec family members, whereby the inhibitory signalling regions comprised by a group of CARs can optionally be derived from different inhibitory receptors. Inhibitory ITIMs are well known in the art (Ravetch and Lanier, Science. 2000;290 (5489) :84; Barrow and Trowsdale, Eur J Immunol. 2006; 36 (7) :1646) and sequences of respective inhibitory domains have been disclosed, for example, in US 2018/0044399 A1 and US 2017/0260268 A1. In principle, the cytoplasmic domains of other inhibitory receptors that mediate their inhibitory function independent of ITIMs such as, for example, CTLA-4, LAG3, TIM3, or CD5 are also suited for inclusion in an inhibitory signalling region of a CAR molecule.

An inhibitory domain suitable for inclusion in a inhibitory signalling region of a CAR molecule of the group of CARs can have a length of from about 30 aa to about 100 aa, e.g., an inhibitory domain can have a length of from about 30 aa to about 50 aa, from about 50 aa to about 70 aa, or from about 70 aa to about 100 aa. In other cases, the inhibitory domain can have a length of from about 100 aa to about 200 aa, or greater than 200 aa.

For example, a suitable inhibitory domain can comprise an amino acid sequence having at least about 50%, at least 70%, at least about 80%, at least about 85%, at least about 90%, at least about 95%, at least about 98%, or 100% amino acid sequence identity to any of the following amino acid sequences: PD-1 (Uniprot Q15116 aa 192-288), CD85A (Uniprot O75022 aa 465-631), CD85C (Uniprot O75023 aa 480-590), CD85D (Uniprot Q8N423 aa 483-598), CD85K (Uniprot Q8NHJ6 aa 281-448), KIR3DL3 (Uniprot Q8N743 aa 344-410), KIR3DL1 (Uniprot P43629 aa 361-444), KIR3DL2 (Uniprot P43630 aa 361-455), CTLA4 (Uniprot P16410 aa 183-223), LAG3 (Uniprot P18627 aa 472-525), TIM3 (Uniprot Q8TDQ0 aa 224-301), LAIR1 (Uniprot Q6GTX8 aa 187-287), KIR2DL2 (Uniprot P43627 aa 265-348), CD85J (Uniprot Q8NHL6 aa 483-650), TIGIT (Uniprot Q495A1 aa 163-244), CEACAM1 (Uniprot P13688 aa 453-526), CD5 (Uniprot P06127 aa 403-495), CD96 (Uniprot P40200 aa 541-585), CD22 (Uniprot P20273 aa 707-847), CSF1R (Uniprot P07333 aa 539-972).

7. Linkers:

The molecules of the group of CARs can include a linker between any two adjacent domains (i.e. components of the CAR molecules). For example, a linker can be disposed between the transmembrane domain and a signalling region. As another example, a linker can be disposed between a signalling region and a dimerization domain. As another example, a linker can be disposed between two dimerization domains. As another example, a linker can be disposed between two signalling regions. As another example, a linker can be disposed between a transmembrane domain and a dimerization domain. As another example, a linker can be disposed in the ectodomain of a CAR molecule between an antigen binding moiety and the transmembrane domain. As another example, a linker can be disposed in the ectodomain of a CAR molecule between a binding site to which another polypeptide is able to bind and the transmembrane domain. As another example, a linker can be disposed in the ectodomain of a CAR molecule between a signal sequence and an antigen binding moiety. As another example, a linker can be disposed in the ectodomain of a CAR molecule between a signal sequence and a binding site to which another polypeptide is able to bind. As another example, a linker can be disposed in the ectodomain of a CAR molecule between a signal sequence and a dimerization domain. As another example, a linker can be disposed in the ectodomain of a CAR molecule between a dimerization domain and an antigen binding moiety. As another example, a linker can be disposed in the ectodomain of a CAR molecule between a dimerization domain and a binding site to which another polypeptide is able to bind.

A linker can be a peptide containing about 1 to about 40 amino acids in length. The linking peptides may have virtually any amino acid sequence, bearing in mind that suitable linkers preferably have a sequence that results in a generally flexible peptide. Small amino acids, such as glycine, serine and alanine, are preferably used in creating a flexible peptide. The creation of such sequences is routine to those of skill in the art. Suitable linkers can be readily selected and can be of different lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids. Exemplary flexible linkers include glycine polymers $(G)_n$, glycine-serine polymers (including, for example, $(GS)_n$, $(GSGGS)_n$, $(GGS)_n$ and $(GGGS)_n$, where n is an integer of at least one, or also glycine-alanine polymers, alanine-serine polymers, and other flexible linkers known in the art. Exemplary flexible linkers include GGSG (SEQ ID NO: 1), GGSGG (SEQ ID NO: 2), GSGSG (SEQ ID NO: 3), GSGGG (SEQ ID NO: 4), GGGSG (SEQ ID NO: 5), GSSSG (SEQ ID NO:

6), and the like. The ordinarily skilled artisan will recognize that design of a peptide conjugated to any elements described above can include linkers that are all or partially flexible, such that the linker can include a flexible linker as well as one or more portions that confer less flexible structure.

8. Additional domains:

The molecules of a subject group of CARs can further include one or more additional polypeptide domains, where such domains include, for example, a signal sequence; an epitope tag; and/or a polypeptide that produces a detectable signal. Signal sequences that are suitable for use in a subject group of CARs include any eukaryotic signal sequence, including a naturally occurring signal sequence, a synthetic (e.g., man-made) signal sequence, etc. Suitable epitope tags include, e.g., hemagglutinin (HA; e.g., amino acid sequence YPYDVPDYA (SEQ ID NO: 7)), FLAG (e.g., amino acid sequence DYKDDDDK (SEQ ID NO: 8)) c-myc (e.g., amino acid sequence EQKLISEEDL (SEQ ID NO: 9)), Strep II (e.g., amino acid sequence NWSHPQFEK (SEQ ID NO: 76)), Hexahistidine tag (6xHIS; e.g., amino acid sequence HHHHHH (SEQ ID NO: 77)), and the like. Suitable detectable signal-producing proteins include, e.g., fluorescent proteins and the like. Suitable fluorescent proteins include, e.g., green fluorescent protein (GFP) or variants thereof, blue fluorescent variant of GFP (BFP), cyan fluorescent variant of GFP (CFP), yellow fluorescent variant of GFP (YFP), enhanced GFP (EGFP), enhanced CFP (ECFP), enhanced YFP (EYFP), GFPS65T, Emerald, Topaz (TYFP), Venus, Citrine, mCitrine, GFPuv, destabilised EGFP (dEGFP), destabilised ECFP (dECFP), destabilised EYFP (dEYFP), mCFPm, Cerulean, T-Sapphire, CyPet, YPet, mKO, HcRed, t-HcRed, DsRed, DsRed2, DsRed-monomer, J-Red, dimer2, t-dimer2(12), mRFPl, pocilloporin, Renilla GFP, Monster GFP, paGFP, Kaede protein and kindling protein, Phycobiliproteins and Phycobiliprotein conjugates including B-Phycoerythrin, R-Phycoerythrin and Allophycocyanin. Other examples of fluorescent proteins include mHoneydew, mBanana, mOrange, dTomato, tdTomato, mTangerine, mStrawberry, mCherry, mGrapel, mRaspberry, mGrape2, mPlum (Shaner et al. (2005) Nat. Methods 2:905-909), and the like. Any of a variety of fluorescent and coloured proteins from Anthozoan species, as described in, e.g., Matz et al. (1999) Nature Biotechnol. 17:969-973, is suitable for use.

9. Dimerization domains:

Complexation of groups of CARs comprising two CAR molecules can be mediated by a single dimerization domain per CAR molecule, whereby this domain can be a domain for homodimerization or heterodimerization. In embodiments, where the group of CARs comprises three or four CAR molecules, at least one CAR molecule of the group preferably contains more than one dimerization domain, in order to facilitate the formation of trimers or tetramers through dimerization.

9.1. Dimerization domains for conditional homodimerization:

In preferred embodiments, examples of suitable dimerization domains for homodimerization include: FK506 binding protein (FKBP), FKPB mutant F36V (dmrB), gyrase B (GyrB) and dihydrofolate reductase (DHFR). The sequences of these homodimerization domains as well as the regulating molecules suited for dimerization of these homodimerization domains are well known in the art (Rutkowska et al., Angew Chem Int Ed Engl. 2012;51(33):8166) and are disclosed, for example, in WO2014127261.

For example, a dimerization domain can be derived from FKBP and can comprise an amino acid sequence having at least about 75%, at least about 80%, at least about 85%, at least about 90%, at least about 95%, at least about 98%, or 100% amino acid sequence identity to the amino acid sequence Uniprot P62942-1.

As another example, a dimerization domain can be derived from GyrB (also known as DNA gyrase subunit B) and can comprise an amino acid sequence having at least about 75%, at least about 80%, at least about 85%, at least about 90%, at least about 95%, at least about 98%, or 100% amino acid sequence identity to a contiguous stretch of from about 100 amino acids to about 200 amino acids (aa), from about 200 aa to about 300 aa, from about 300 aa to about 400 aa, from about 400 aa to about 500 aa, from about 500 aa to about 600 aa, from about 600 aa to about 700 aa, or from about 700 aa to about 800 aa, of the GyrB amino acid sequence from Escherichia coli Uniprot P0AES6-1 (or to the DNA gyrase subunit B sequence from any organism). In some cases, a dimerization domain comprises an amino acid sequence having at least about 75%, at least about 80%, at least about 85%, at least about 90%, at least about 95%, at least about 98%, or 100% amino acid sequence identity to amino acids 1-220 of the GyrB amino acid sequence from Escherichia coli.

As another example, a dimerization domain can be derived from DHFR (also known as dihydrofolate reductase, DHFRP1, and DYR) and can comprise an amino acid sequence having at least about 75%, at least about 80%, at least about 85%, at least about 90%, at least about 95%, at least about 98%, or 100% amino acid sequence identity to the amino acid sequence Uniprot P00374-1.

As another example, a dimerization domain can be derived from the DmrB binding domain (i.e., DmrB homodimerization domain) and can comprise an amino acid sequence having at least about 75%, at least about 80%, at least about 85%, at least about 90%, at least about 95%, at least about 98%, or 100% amino acid sequence identity to SEQ ID NO. 10.

Homodimerization can be achieved by different regulating molecules, such as, for example, FK1012 and AP1510 for homodimerization of FKBP (Amara et al. PNAS 1997;94(20):10618); Coumermycin (PubChem CID 54675768) or analogs of coumermycin for homodimerization of GyrB (Farrar et al. Nature. 1996;383:178; and U.S. Pat. No. 6,916,846); a homo-bifunctional dimer of methotrexate (PubChem CID 126941) for homodimerization of DHFR (as disclosed, for example, in U.S. Patent No. 8,236,925); and in preferred embodiments by AP20187 (PubChem CID 78357784) and AP1903 (PubChem CID 16135625) for homodimerization of DmrB;

9.2. Dimerization domains for conditional heterodimerization:

9.2.1. Conditional heterodimerization of CAR molecules based on ligand binding domains from nuclear receptors:

In preferred embodiments at least two CAR molecules of a group of CARs according to the present invention can be heterodimerized by a pair of heterodimerization domains comprising one member which is a ligand binding domain (LBD) from a nuclear receptor and a second member which is a co-regulator peptide. LBDs derived from nuclear receptors upon binding of appropriate small molecules (i.e., regulating molecules according the present invention) can heterodimerize with respective co-regulator peptides. This system can be used for heterodimerization of proteins of interest. Suitable sequences of LBDs and co-regulator peptides together with suitable regulating molecules have been disclosed for example in US 2017/0306303 A1. Suitable LBDs can be selected from any of a variety of nuclear receptors, including ER-alpha, ER-beta, PR, AR, GR, MR, RAR-alpha, RAR-beta, RAR-gamma, TR-alpha, TR-beta, VDR, EcR, RXR-alpha, RXR-beta, RXR-gamma, PPAR-alpha, PPAR-beta, PPAR-gamma, LXR-alpha, LXR-beta, FXR, PXR, SXR, Constitutive Adrostrane Receptor, SF-1, LRH-1, DAX-1, SHP, TLX, PNR, NGF1-B-alpha, NGF1-B-beta, NGF1-B-gamma, ROR-alpha, ROR-beta, ROR-gamma, ERR-alpha, ERR-beta, ERR-gamma, GCNF, TR2/4, HNF-4, COUP-TF-alpha, COUP-TF-beta and COUP-TF-gamma.

Abbreviations for nuclear receptors (synonymus with nuclear hormone receptors) are as follows. ER: Estrogen Receptor; PR: Progesterone Receptor; AR: Androgen Receptor; GR: Glucocorticoid Receptor; MR: Mineralocorticoid Receptor; RAR: Retinoic Acid Receptor; TR-alpha/beta: Thyroid Receptor; VDR: Vitamin D3 Receptor; EcR: Ecdysone Receptor; RXR: Retinoic Acid X Receptor; PPAR: Peroxisome Proliferator Activated Receptor; LXR: Liver X Receptor; FXR: Farnesoid X Receptor; PXR/SXR: Pregnane X Receptor/Steroid and Xenobiotic Receptor; SF-1: Steroidogenic Factor 1; DAX-1: Dosage sensitive sex reversal-adrenal hypoplasia congenital critical region on the X chromosome, gene 1; LRH-1: Liver Receptor Homolog 1; SHP: Small Heterodimer Partner; TLX: Tail-less Gene; PNR: Photoreceptor-Specific Nuclear Receptor; NGF1-B: Nerve Growth Factor; ROR: RAR related orphan receptor; ERR: Estrogen Related Receptor; GCNF: Germ Cell Nuclear Factor; TR2/4: Testicular Receptor; HNF-4: Hepatocyte Nuclear Factor; COUP-TF: Chicken Ovalbumin Upstream Promoter, Transcription Factor.

9.2.1.1. LBDs:

*Mineralocorticoid Receptor:*

In some cases, an LBD suitable for inclusion as a member of a pair of heterodimerization domains can be an LBD of a mineralocorticoid receptor (MR). For example, in some cases, the LBD can comprise an amino acid sequence having at least 75%, at least 80%, at least 85%, at least 90%, at least 95%, at least 98%, at least 99%, or 100%, amino acid sequence identity to the LBD of an MR (Uniprot P08235).

For example, the LBD of a MR can comprise an amino acid sequence having at least 75%, at least 80%, at least 85%, at least 90%, at least 95%, at least 98%, at least 99%, or 100%, amino acid sequence identity to any of the following amino acid sequences: Uniprot Q9IAC6.1 aa 112-359; Uniprot Q91573.1 aa 365-612; Uniprot Q157N1 aa 734-981; GenBank CAG11072.1 aa 173-501; PDB 2AA6_A aa 28-275; PDB 2AA2_A aa 28-275; PDB 2A3I_A aa 6-253; PDB 2OAX_A aa 9-256; PDB 1Y9R_A aa 8-255; PDB 2ABI_A aa 9-256; and has a length of from about 200 amino acids to 250 amino acids (e.g., has a length of from 200 amino acids to 225 amino acids, or from 225 amino acids to 250 amino acids; e.g., has a length of 248 amino acids).

For example, the LBD of a MR can comprise an amino acid sequence having at least 75%, at least 80%, at least 85%, at least 90%, at least 95%, at least 98%, at least 99%, or 100%, amino acid sequence identity to the amino acid sequence Uniprot P08235 aa 686-984 and has a length of from about 250 amino acids to 299 amino acids (e.g., has a length of from 250 amino acids to 275 amino acids, or from 275 amino acids to 299 amino acids).

For example, the LBD of a MR can comprise an amino acid sequence having at least 75%, at least 80%, at

least 85%, at least 90%, at least 95%, at least 98%, at least 99%, or 100%, amino acid sequence identity to the amino acid sequence Uniprot P08235 aa 737-984 and has a length of from about 200 amino acids to 250 amino acids (e.g., has a length of from 200 amino acids to 225 amino acids, or from 225 amino acids to 250 amino acids; e.g., has a length of 248 amino acids).

For example, the LBD of a MR can comprise an amino acid sequence having at least 75%, at least 80%, at least 85%, at least 90%, at least 95%, at least 98%, at least 99%, or 100%, amino acid sequence identity to the amino acid sequence Uniprot P08235 aa 686-984 with S810L substitution, and has a length of from about 250 amino acids to 299 amino acids (e.g., has a length of from 250 amino acids to 275 amino acids, or from 275 amino acids to 299 amino acids).

For example, the LBD of a MR can comprise an amino acid sequence having at least 75%, at least 80%, at least 85%, at least 90%, at least 95%, at least 98%, at least 99%, or 100%, amino acid sequence identity to the amino acid sequence Uniprot P08235 aa 737-984 with S810L substitution, and has a length of from about 200 amino acids to 250 amino acids (e.g., has a length of from 200 amino acids to 225 amino acids, or from 225 amino acids to 250 amino acids; e.g., has a length of 248 amino acids).

Where one member of the pair of heterodimerization domains is an LBD of an MR, the second member of the pair can be a co-regulator peptide comprising the amino acid sequence SLTARHKILHRLLQEGSPSDI (Uniprot Q15788 aa 681-701), where the co-regulator peptide has a length of from about 21 amino acids to about 50 amino acids (e.g., the co-regulator peptide has a length of from 21 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

Where one member of the pair of heterodimerization domains is an LBD of an MR, the second member of the pair can be a co-regulator peptide comprising the amino acid sequence QEAEEPSLLKKLLLAPANTQL (Uniprot Q9UBK2 aa 136-156), where the co-regulator peptide has a length of from about 21 amino acids to about 50 amino acids (e.g., the co-regulator peptide has a length of from 21 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

Where one member of the pair of heterodimerization domains is an LBD of an MR, the second member of the pair can be a co-regulator peptide comprising the amino acid sequence SKVSQNPILTSLLQITGNGGS (Uniprot Q15648 aa 596-616), where the co-regulator peptide has a length of from about 21 amino acids to about 50 amino acids (e.g., the co-regulator peptide has a length of from 21 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

*Androgen Receptor:*

In some cases, an LBD suitable for inclusion as a member of a pair of heterodimerization domains can be an LBD of an androgen receptor (AR). For example, in some cases, the LBD can comprise an amino acid sequence having at least 75%, at least 80%, at least 85%, at least 90%, at least 95%, at least 98%, at least 99%, or 100%, amino acid sequence identity to the LBD of an AR (Uniprot P10275).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 619-919 and has a length of from about 250 amino acids to 301 amino acids (e.g., has a length of from 250 amino acids to 275 amino acids, or from 275 amino acids to 301 amino acids).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 690-919 and has a length of from about 190 amino acids to 230 amino acids (e.g., has a length of from 190 amino acids to 210 amino acids, or from 210 amino acids to 230 amino acids).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 619-919 with T877A substitution, and has a length of from about 250 amino acids to 301 amino acids (e.g., has a length of from 250 amino acids to 275 amino acids, or from 275 amino acids to 301 amino acids).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 690-919 with T877A substitution, and has a length of from about 190 amino acids to 230 amino acids (e.g., has a length of from 190 amino acids to 210 amino acids, or from 210 amino acids to 230 amino acids).

For example, the LBD of an AR can comprise an amino acid sequence having at least 75%, at least 80%, at

least 85%, at least 90%, at least 95%, at least 98%, at least 99%, or 100%, amino acid sequence identity to the amino acid sequence Uniprot P10275 aa 619-919 with F876L substitution, and has a length of from about 250 amino acids to 301 amino acids (e.g., has a length of from 250 amino acids to 275 amino acids, or from 275 amino acids to 301 amino acids).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 690-919 with F876L substitution, and has a length of from about 190 amino acids to 230 amino acids (e.g., has a length of from 190 amino acids to 210 amino acids, or from 210 amino acids to 230 amino acids).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 619-919 with F876L and T877A substitution, and has a length of from about 250 amino acids to 301 amino acids (e.g., has a length of from 250 amino acids to 275 amino acids, or from 275 amino acids to 301 amino acids).

For example, the LBD of an AR can comprise an amino acid sequence having at least 75%, at least 80%, at least 85%, at least 90%, at least 95%, at least 98%, at least 99%, or 100%, amino acid sequence identity to the amino acid sequence Uniprot P10275 aa 690-919 with F876L T877A substitution, and has a length of from about 190 amino acids to 230 amino acids (e.g., has a length of from 190 amino acids to 210 amino acids, or from 210 amino acids to 230 amino acids).

Where one member of the pair of heterodimerization domains is an LBD of an AR, the second member of the pair can be a co-regulator peptide comprising the amino acid sequence ESKGHKKLLQLLTCSSDDR (Uniprot Q9Y6Q9 aa 614-632) where the co-regulator peptide has a length of from about 19 amino acids to about 50 amino acids (e.g., the co-regulator peptide has a length of from 19 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

*Progesterone Receptor:*

In some cases, an LBD suitable for inclusion as a member of a pair of heterodimerization domains can be an LBD of a progesterone receptor (PR). For example, in some cases, the LBD can comprise an amino acid sequence having at least 75%, at least 80%, at least 85%, at least 90%, at least 95%, at least 98%, at least 99%, or 100%, amino acid sequence identity to the LBD of a PR (Uniprot P06401).

For example, the LBD of a PR can comprise an amino acid sequence having at least 75%, at least 80%, at least 85%, at least 90%, at least 95%, at least 98%, at least 99%, or 100%, amino acid sequence identity to any of the following amino acid sequences: Uniprot Q8UVY3 aa 456-703; Uniprot P07812.1 aa 539-786; GenBank CAQ14518.1 aa 306-553; PDB 1SR7_A aa 12-259; PDB 1SQN_A aa 14-261; PDB 1E3K aa 11-258; PDB 1A28_A aa 9-256; and has a length of from about 200 amino acids to 250 amino acids (e.g., has a length of from 200 amino acids to 225 amino acids, or from 225 amino acids to 250 amino acids; e.g., has a length of 248 amino acids).

For example, the LBD of a PR can comprise an amino acid sequence having at least 75%, at least 80%, at least 85%, at least 90%, at least 95%, at least 98%, at least 99%, or 100%, amino acid sequence identity to the amino acid sequence Uniprot P06401 aa 678-933 and has a length of from about 200 amino acids to 256 amino acids (e.g., has a length of from 200 amino acids to 225 amino acids, or from 225 amino acids to 256 amino acids; e.g., has a length of 256 amino acids).

For example, the LBD of a PR can comprise an amino acid sequence having at least 75%, at least 80%, at least 85%, at least 90%, at least 95%, at least 98%, at least 99%, or 100%, amino acid sequence identity to the amino acid sequence Uniprot P06401 aa 686-933 and has a length of from about 200 amino acids to 250 amino acids (e.g., has a length of from 200 amino acids to 225 amino acids, or from 225 amino acids to 250 amino acids; e.g., has a length of 248 amino acids).

Where one member of the pair of heterodimerization domains is an LBD of a PR, the second member of the dimerization pair can be a co-regulator peptide comprising the amino acid sequence GHSFADPASNLGLEDIIRKALMGSF (Uniprot O75376 aa 2251-2275) where the co-regulator peptide has a length of from about 25 amino acids to about 50 amino acids (e.g., the co-regulator peptide has a length of from 25 amino acids to 30 amino acids, from 30 amino acids to 35 amino acids, from 35 amino acids to 40 amino acids, from 40 amino acids to 45 amino acids, or from 45 amino acids to 50 amino acids).

*Thyroid Hormone Receptor-beta:*

In some cases, an LBD suitable for inclusion as a member of a pair of heterodimerization domains can be an

LBD of thyroid hormone receptor-beta (TR-beta). For example, in some cases, the LBD can comprise an amino acid sequence having at least 75%, at least 80%, at least 85%, at least 90%, at least 95%, at least 98%, at least 99%, or 100%, amino acid sequence identity to the LBD of a TR-beta (Uniprot P10828).

For example, the LBD of a TR-beta can comprise an amino acid sequence having at least 75%, at least 80%, at least 85%, at least 90%, at least 95%, at least 98%, at least 99%, or 100%, amino acid sequence identity to one of the following amino acid sequences: Uniprot Q4T8V6 aa 223-502; Uniprot Q90382.1 aa 159-401; Uniprot P18115.2 aa 170-412; Uniprot Q9PVE4.2 aa 141-392; Uniprot P10828.2 aa 216-458; GenBank ABS11249.1 aa 179-419; NCBI REF SEQ XP_001185977.1 aa 186-416; PDB 1NAV_A aa 17-259; PDB 2PIN_A aa 8-250; PDB 3D57_A aa 22-264; PDB 1N46_A aa 13-255; PDB 1BSX_A aa 15-257; and has a length of from about 200 amino acids to 250 amino acids (e.g., has a length of from 200 amino acids to 225 amino acids, from 225 amino acids to 230 amino acids, from 230 amino acids to 240 amino acids, or from 240 amino acids to 250 amino acids).

For example, the LBD of a TR-beta can comprise an amino acid sequence having at least 75%, at least 80%, at least 85%, at least 90%, at least 95%, at least 98%, at least 99%, or 100%, amino acid sequence identity to the amino acid sequence Uniprot P10828 aa 202-461 and has a length of from about 200 amino acids to 260 amino acids (e.g., has a length of from 200 amino acids to 225 amino acids, or from 225 amino acids to 260 amino acids; e.g., has a length of 260 amino acids).

For example, the LBD of a TR-beta can comprise an amino acid sequence having at least 75%, at least 80%, at least 85%, at least 90%, at least 95%, at least 98%, at least 99%, or 100%, amino acid sequence identity to the amino acid sequence Uniprot P10828 aa 216-461 and has a length of from about 200 amino acids to 246 amino acids (e.g., has a length of from 200 amino acids to 225 amino acids, or from 225 amino acids to 246 amino acids; e.g., has a length of 246 amino acids).

Where one member of the pair of heterodimerization domains is an LBD of a TR-beta, the second member of the pair can be an NCOA3/SRC3 polypeptide, for example comprising the amino acid sequence Uniprot Q9Y6Q9 aa 627-829 or Uniprot Q9Y6Q9 aa 673-750 or Uniprot Q15596 aa 721-1021.

*Estrogen Receptor-Alpha:*

In preferred embodiments, an LBD suitable for inclusion as a member of a pair of heterodimerization domains can be an LBD of estrogen receptor-alpha (ER-alpha). For example, in some cases, the LBD can comprise an amino acid sequence having at least 75%, at least 80%, at least 85%, at least 90%, at least 95%, at least 98%, at least 99%, or 100%, amino acid sequence identity to the LBD of an ER-alpha (Uniprot P03372).

For example, the LBD of an ER-alpha can comprise an amino acid sequence having at least 75%, at least 80%, at least 85%, at least 90%, at least 95%, at least 98%, at least 99%, or 100%, amino acid sequence identity to any of the following amino acid sequences: Uniprot P06212.1 aa 304-541; Uniprot P81559.1 aa 302-539; Uniprot Q7ZU32 aa 280-517; GenBank ACB10649.1 aa 303-529; GenBank ABQ42696.1 aa 226-468; GenBank ACC85903.1 aa 141-375; PDB 1XP9_A aa 4-241; PDB 1YY4_A aa 1-236; and has a length of from about 200 amino acids to 240 amino acids (e.g., has a length of from 200 amino acids to 225 amino acids, from 225 amino acids to 230 amino acids, from 230 amino acids to 235 amino acids, or from 235 amino acids to 240 amino acids).

For example, the LBD of an ER-alpha can comprise an amino acid sequence having at least 75%, at least 80%, at least 85%, at least 90%, at least 95%, at least 98%, at least 99%, or 100%, amino acid sequence identity to the amino acid sequence Uniprot P03372 aa 305-533 and has a length of from about 180 amino acids to 229 amino acids (e.g., has a length of from 180 amino acids to 200 amino acids, or from 200 amino acids to 229 amino acids; e.g., has a length of 229 amino acids).

For example, the LBD of an ER-alpha can comprise an amino acid sequence having at least 75%, at least 80%, at least 85%, at least 90%, at least 95%, at least 98%, at least 99%, or 100%, amino acid sequence identity to the amino acid sequence Uniprot P03372 aa 282-595 and has a length of from about 250 amino acids to 314 amino acids (e.g., has a length of from 250 amino acids to 275 amino acids, from 275 amino acids to 300 amino acids, or from 300 amino acids to 314 amino acids; e.g., has a length of 314 amino acids).

For example, the LBD of an ER-alpha can comprise an amino acid sequence having at least 75%, at least 80%, at least 85%, at least 90%, at least 95%, at least 98%, at least 99%, or 100%, amino acid sequence identity to the amino acid sequence Uniprot P03372 aa 310-547 and has a length of from about 190 amino acids to 238 amino acids (e.g., has a length of from 190 amino acids to 220 amino acids, or from 220 amino acids to 238 amino acids; e.g., has a length of 238 amino acids).

For example, the LBD of an ER-alpha can comprise an amino acid sequence having at least 75%, at least 80%, at least 85%, at least 90%, at least 95%, at least 98%, at least 99%, or 100%, amino acid sequence identity to the amino acid sequence Uniprot P03372 aa 305-533 with substitution D351Y; and has a length of from about 180 amino acids to 229 amino acids (e.g., has a length of from 180 amino acids to 200 amino acids, or from 200 amino acids to 229 amino acids; e.g., has a length of 229 amino acids).

For example, the LBD of an ER-alpha can comprise an amino acid sequence having at least 75%, at least 80%, at least 85%, at least 90%, at least 95%, at least 98%, at least 99%, or 100%, amino acid sequence identity to the amino acid sequence Uniprot P03372 aa 282-595 with substitution D351Y; and has a length of from about 250 amino acids to 314 amino acids (e.g., has a length of from 250 amino acids to 275 amino acids, from 275 amino acids to 300 amino acids, or from 300 amino acids to 314 amino acids; e.g., has a length of 314 amino acids).

For example, the LBD of an ER-alpha can comprise an amino acid sequence having at least 75%, at least 80%, at least 85%, at least 90%, at least 95%, at least 98%, at least 99%, or 100%, amino acid sequence identity to the amino acid sequence Uniprot P03372 aa 310-547 with substitution D351Y; and has a length of from about 190 amino acids to 238 amino acids (e.g., has a length of from 190 amino acids to 220 amino acids, or from 220 amino acids to 238 amino acids; e.g., has a length of 238 amino acids).

Where one member of the pair of heterodimerization domains is an LBD of an ER-alpha, the second member of the pair can be a co-regulator peptide comprising the amino acid sequence DAFQLRQLILRGLQDD (SEQ ID NO: 11), where the co-regulator peptide has a length of from about 16 amino acids to about 50 amino acids (e.g., the co-regulator peptide has a length of from 16 amino acids to 20 amino acids, from 20 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

Where one member of the pair of heterodimerization domains is an LBD of an ER-alpha, the second member of the pair can be a co-regulator peptide comprising the amino acid sequence SPGSREWFKDMLS (SEQ ID NO: 12), where the co-regulator peptide has a length of from about 13 amino acids to about 50 amino acids (e.g., the co-regulator peptide has a length of from 13 amino acids to 15 amino acids, from 15 amino acids to 20 amino acids, from 20 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

*Estrogen Receptor-Beta (ER-beta):*

In some cases, an LBD suitable for inclusion as a member of a pair of heterodimerization domains can be an LBD of estrogen receptor-beta (ER-beta). For example, in some cases, the LBD can comprise an amino acid sequence having at least 75%, at least 80%, at least 85%, at least 90%, at least 95%, at least 98%, at least 99%, or 100%, amino acid sequence identity to the LBD of an ER-beta (Uniprot Q92731).

For example, the LBD of an ER-beta can comprise an amino acid sequence having at least 75%, at least 80%, at least 85%, at least 90%, at least 95%, at least 98%, at least 99%, or 100%, amino acid sequence identity to any of the following amino acid sequences: Uniprot P06212.1 aa 304-541; Uniprot P81559.1 aa 302-539; Uniprot Q7ZU32 aa 280-517; GenBank ACB10649.1 aa 303-529; GenBank ABQ42696.1 aa 226-468; GenBank ACC85903.1 aa 141-375; PDB 1XP9_A aa 4-241; PDB 1YY4_A aa 1-236; and has a length of from about 200 amino acids to 243 amino acids (e.g., has a length of from 200 amino acids to 225 amino acids, from 225 amino acids to 230 amino acids, from 230 amino acids to 235 amino acids, or from 235 amino acids to 243 amino acids).

For example, the LBD of an ER-beta can comprise an amino acid sequence having at least 75%, at least 80%, at least 85%, at least 90%, at least 95%, at least 98%, at least 99%, or 100%, amino acid sequence identity to the amino acid sequence Uniprot Q92731 aa 260-502; and has a length of from about 200 amino acids to 243 amino acids (e.g., has a length of from 200 amino acids to 225 amino acids, from 225 amino acids to 230 amino acids, from 230 amino acids to 235 amino acids, or from 235 amino acids to 243 amino acids).

Where one member of the pair of heterodimerization domains is an LBD of an ER-beta, the second member of the dimerization pair can be a co-regulator peptide comprising the amino acid sequence PRQGSILYSMLTSAKQT (SEQ ID NO: 13), where the co-regulator peptide has a length of from about 17 amino acids to about 50 amino acids (e.g., the co-regulator peptide has a length of from 17 amino acids to 20 amino acids, from 20 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

*Peroxisome Proliferator-Activated Receptor-Gamma:*

In some cases, an LBD suitable for inclusion as a member of a pair of heterodimerization domains can be an LBD of peroxisome proliferator-activated receptor-gamma (PPAR-gamma). For example, in some cases, the LBD can comprise an amino acid sequence having at least 75%, at least 80%, at least 85%, at least 90%, at least 95%, at least 98%, at least 99%, or 100%, amino acid sequence identity to the LBD of a PPAR-gamma (Uniprot P37231).

For example, the LBD of a PPAR-gamma can comprise an amino acid sequence having at least 75%, at least 80%, at least 85%, at least 90%, at least 95%, at least 98%, at least 99%, or 100%, amino acid sequence identity to one of the following amino acid sequences: Uniprot Q4H2X4 aa 176-417; Uniprot P37233.1 aa 129-395; Uniprot Q7T029 aa 95-435; GenBank AAL26245.1 aa 95-435; NCBI REF SEQ XP_781750.1 aa 137-378; NCBI REF SEQ XP 784429.2

aa 219-478; NCBI REF SEQ NP_001001460.1 aa 207-474; PDB 2J14_A aa 17-284; PDB 1FM6_D aa 4-271; and has a length of from about 200 amino acids to 269 amino acids (e.g., has a length of from 200 amino acids to 225 amino acids, from 225 amino acids to 250 amino acids, or from 250 amino acids to 269 amino acids).

For example, the LBD of a PPAR-gamma can comprise an amino acid sequence having at least 75%, at least 80%, at least 85%, at least 90%, at least 95%, at least 98%, at least 99%, or 100%, amino acid sequence identity to the amino acid sequence Uniprot P37231 aa 174-475 and has a length of from about 150 amino acids to 202 amino acids (e.g., has a length of from 150 amino acids to 160 amino acids, from 160 amino acids to 170 amino acids, from 170 amino acids to 190 amino acids, or from 190 amino acids to 202 amino acids).

For example, the LBD of a PPAR-gamma can comprise an amino acid sequence having at least 75%, at least 80%, at least 85%, at least 90%, at least 95%, at least 98%, at least 99%, or 100%, amino acid sequence identity to the amino acid sequence Uniprot P37231 aa 181-475 and has a length of from about 200 amino acids to 269 amino acids (e.g., has a length of from 200 amino acids to 225 amino acids, from 225 amino acids to 250 amino acids, or from 250 amino acids to 269 amino acids).

For example, the LBD of a PPAR-gamma can comprise an amino acid sequence having at least 75%, at least 80%, at least 85%, at least 90%, at least 95%, at least 98%, at least 99%, or 100%, amino acid sequence identity to the amino acid sequence Uniprot P37231 aa 205-475 and has a length of from about 200 amino acids to 269 amino acids (e.g., has a length of from 200 amino acids to 225 amino acids, from 225 amino acids to 250 amino acids, or from 250 amino acids to 271 amino acids).

Where one member of the pair of heterodimerization domains is an LBD of a PPAR-gamma, the second member of the pair can be a co-regulator peptide comprising the amino acid sequence CPSSHSSLTERHKILHRLLQEGSPS (Uniprot Q15788-1 aa 676-700), where the co-regulator peptide has a length of from about 25 amino acids to about 50 amino acids (e.g., the co-regulator peptide has a length of from 25 amino acids to 28 amino acids, from 28 amino acids to 29 amino acids, from 29 amino acids to 30 amino acids, from 30 amino acids to 35 amino acids, from 35 amino acids to 40 amino acids, from 40 amino acids to 45 amino acids, or from 45 amino acids to 50 amino acids).

Where one member of the pair of heterodimerization domains is an LBD of a PPAR-gamma, the second member of the pair can be a co-regulator peptide comprising the amino acid sequence PKKENNALLRYLLDRDDPSDV (SEQ ID NO: 14) or PKKKENALLRYLLDKDDTKDI (Uniprot Q15596-1 aa 737-757), where the co-regulator peptide has a length of from about 21 amino acids to about 50 amino acids (e.g., the co-regulator peptide has a length of from 21 amino acids to 23 amino acids, from 23 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

*Glucocorticoid Receptor:*

In some cases, an LBD suitable for inclusion as a member of a pair of heterodimerization domains can be an LBD of glucocorticoid receptor (GR). For example, in some cases, the LBD can comprise an amino acid sequence having at least 75%, at least 80%, at least 85%, at least 90%, at least 95%, at least 98%, at least 99%, or 100%, amino acid sequence identity to the LBD of a GR having the amino acid sequence Uniprot P04150-3.

For example, the LBD of a GR can comprise an amino acid sequence having at least 75%, at least 80%, at least 85%, at least 90%, at least 95%, at least 98%, at least 99%, or 100%, amino acid sequence identity to one of the following amino acid sequences: Uniprot Q4RIR9 aa 110-356; Uniprot P49844.1 aa 530-776; NCBI REF SEQ NP_001032915.1 aa 526-772; PDB 1NHZ_A 34-280; PDB 1M2Z_A aa 11-257; PDB 3BQD_A aa 9-255; PDB 3CLD_A aa 13-259; and has a length of from about 200 amino acids to 247 amino acids (e.g., has a length of from 200 amino acids to 225 amino acids, from 225 amino acids to 230 amino acids, from 230 amino acids to 240 amino acids, or from 240 amino acids to 247 amino acids).

For example, the LBD of a GR can comprise an amino acid sequence having at least 75%, at least 80%, at least 85%, at least 90%, at least 95%, at least 98%, at least 99%, or 100%, amino acid sequence identity to the amino acid sequence Uniprot P04150-3 aa 532-778 and has a length of from about 200 amino acids to 247 amino acids (e.g., has a length of from 200 amino acids to 225 amino acids, or from 225 amino acids to 247 amino acids; e.g., has a length of 247 amino acids).

Where one member of the pair of heterodimerization domains is an LBD of a GR, the second member of the pair can be an NCOA2/SRC2 polypeptide, for example, comprising the amino acid sequence Uniprot Q15788 aa 1172-1441 or a fragment thereof, or Uniprot Q15596 aa 320-1021 or a fragment thereof.

*Vitamin D Receptor:*

In some cases, an LBD suitable for inclusion as a member of a pair of heterodimerization domains can be an LBD of vitamin D receptor (VDR). For example, in some cases, the LBD can comprise an amino acid sequence having

at least 75%, at least 80%, at least 85%, at least 90%, at least 95%, at least 98%, at least 99%, or 100%, amino acid sequence identity to the LBD of a VDR (Uniprot P11473).

For example, the LBD of a VDR can comprise an amino acid sequence having at least 75%, at least 80%, at least 85%, at least 90%, at least 95%, at least 98%, at least 99%, or 100%, amino acid sequence identity to one of the following amino acid sequences: Uniprot O42392.1 aa 147-450; NCBI REF SEQ NP_001079288.1 aa 125-421; PDB 2HBH_A aa 5-301; PDB 1S0Z_A aa 11-262; and has a length of from about 250 amino acids to 310 amino acids (e.g., has a length of from 250 amino acids to 275 amino acids, from 275 amino acids to 300 amino acids, or from 300 amino acids to 310 amino acids).

For example, the LBD of a VDR can comprise an amino acid sequence having at least 75%, at least 80%, at least 85%, at least 90%, at least 95%, at least 98%, at least 99%, or 100%, amino acid sequence identity to the amino acid sequence Uniprot P11473 aa 124-426 and has a length of from about 250 amino acids to 303 amino acids (e.g., has a length of from 250 amino acids to 275 amino acids, from 275 amino acids to 300 amino acids, or from 300 amino acids to 303 amino acids).

Where one member of the pair of heterodimerization domains is an LBD of a VDR, the second member of the pair can be an NCOA1/SRC1 polypeptide, for example, comprising the amino acid sequence Uniprot Q15788 aa 1172-1441 or a fragment thereof, or Uniprot Q15596 aa 320-1021 or a fragment thereof.

For example, in some cases, where one member of the pair of heterodimerization domains is an LBD of a VDR, the other member of the pair can be an NCOA2/SRC2 polypeptide comprising the amino acid sequence Uniprot Q15596 aa 744-751, where the co-regulator peptide has a length of from about from about 8 amino acids to about 50 amino acids (e.g., the co-regulator peptide has a length of from about 8 amino acids to 10 amino acids, from 10 amino acids to 15 amino acids, from 15 amino acids to 20 amino acids, from 20 amino acids to 23 amino acids, from 23 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids).

*Thyroid Hormone Receptor-Alpha:*

In some cases, an LBD suitable for inclusion as a member of a pair of heterodimerization domains can be an LBD of thyroid hormone receptor-alpha (TR-alpha). For example, in some cases, the LBD can comprise an amino acid sequence having at least 75%, at least 80%, at least 85%, at least 90%, at least 95%, at least 98%, at least 99%, or 100%, amino acid sequence identity to the LBD of a TR-alpha (Uniprot P10827-2).

For example, the LBD of a TR-alpha can comprise an amino acid sequence having at least 75%, at least 80%, at least 85%, at least 90%, at least 95%, at least 98%, at least 99%, or 100%, amino acid sequence identity to one of the following amino acid sequences: Uniprot Q4T8V6 aa 223-502; Uniprot Q90382.1 aa 159-401; Uniprot P18115.2 aa 170-412; Uniprot Q9PVE4.2 aa 141-392; Uniprot P10828.2 aa 216-458; GenBank ABS11249.1 aa 179-419; NCBI REF SEQ XP_001185977.1 aa 186-416; PDB 1NAV_A aa 17-259; PDB 2PIN_A aa 8-250; PDB 3D57_A aa 22-264; PDB 1N46_A aa 13-255; PDB 1BSX_A aa 15-257; and has a length of from about 190 amino acids to about 245 amino acids (e.g., has a length of from 190 amino acids to 210 amino acids, from 210 amino acids to 230 amino acids, or from 230 amino acids to 245 amino acids).

For example, the LBD of a TR-alpha can comprise an amino acid sequence having at least 75%, at least 80%, at least 85%, at least 90%, at least 95%, at least 98%, at least 99%, or 100%, amino acid sequence identity to the amino acid sequence Uniprot P10827-2 aa 162-404 and has a length of from about 190 amino acids to about 243 amino acids (e.g., has a length of from 190 amino acids to 210 amino acids, from 210 amino acids to 230 amino acids, or from 230 amino acids to 243 amino acids).

A suitable co-regulator peptide for TR-alpha can be an SRC1 polypeptide or a fragment thereof (e.g., a peptide of from 8 amino acids to 50 amino acids in length, derived from an SRC1 polypeptide).

*Retinoic Acid Receptor-Beta:*

In some cases, an LBD suitable for inclusion as a member of a pair of heterodimerization domains can be an LBD of retinoic acid receptor-beta (RAR-beta). For example, in some cases, the LBD can comprise an amino acid sequence having at least 75%, at least 80%, at least 85%, at least 90%, at least 95%, at least 98%, at least 99%, or 100%, amino acid sequence identity to the LBD of a RAR-beta (Uniprot P10826-2).

For example, the LBD of a RAR-beta can comprise an amino acid sequence having at least 75%, at least 80%, at least 85%, at least 90%, at least 95%, at least 98%, at least 99%, or 100%, amino acid sequence identity to one of the following amino acid sequences: Uniprot Q4H2W2 aa 400-634; Uniprot P22448.2 aa 186-416; Uniprot P28699.2 aa 209-439; Uniprot Q91392.2 aa 176-406; NCBI REF SEQ XP_779976.2 aa 134-362; NCBI REF SEQ XP_002204386.1 aa 179-409; PDB 1XAP_A aa 32-262; PDB 1XDK_B aa 34-264; PDB 1DKF_B aa 5-235; and has a length of from about 180 amino acids to about 235 amino acids (e.g., has a length of from 180 amino acids to 200 amino acids, from 200

amino acids to 220 amino acids, or from 220 amino acids to 235 amino acids).

For example, the LBD of a RAR-beta can comprise an amino acid sequence having at least 75%, at least 80%, at least 85%, at least 90%, at least 95%, at least 98%, at least 99%, or 100%, amino acid sequence identity to the amino acid sequence Uniprot P10826-2 aa 179-409 and has a length of from about 180 amino acids to about 231 amino acids (e.g., has a length of from 180 amino acids to 200 amino acids, from 200 amino acids to 220 amino acids, or from 220 amino acids to 231 amino acids).

A suitable co-regulator peptide for RAR-beta can be an SRC1 polypeptide or a fragment thereof (e.g., a peptide of from 8 amino acids to 50 amino acids in length, derived from an SRC1 polypeptide).

Where one member of a pair of heterodimerization domains is an LBD of a RAR-beta, the other member of the dimerization pair can be an NCOA1/SRC1 polypeptide, for example comprising the amino acid sequence Uniprot Q15788 aa 1172-1441 or a fragment thereof.

Where one member of a pair of heterodimerization domains is an LBD of a RAR-beta, the other member of the dimerization pair can be an NCOA2/SRC2 polypeptide, for example comprising the amino acid sequence Uniprot Q15596 aa 320-1021 or a fragment thereof.

*Farnesoid X Receptor:*

In some cases, an LBD suitable for inclusion as a member of a pair of heterodimerization domains can be an LBD of farnesoid X receptor (FXR). For example, in some cases, the LBD can comprise an amino acid sequence having at least 75%, at least 80%, at least 85%, at least 90%, at least 95%, at least 98%, at least 99%, or 100%, amino acid sequence identity to the LBD of an FXR having the amino acid sequence Uniprot Q96RI1-2.

For example, the LBD of an FXR can comprise an amino acid sequence having at least 75%, at least 80%, at least 85%, at least 90%, at least 95%, at least 98%, at least 99%, or 100%, amino acid sequence identity to the amino acid sequence Uniprot Q96RI1-2 aa 237-472; and has a length of from about 100 amino acids to about 136 amino acids (e.g., has a length of from 100 amino acids to 110 amino acids, from 110 amino acids to 120 amino acids, or from 120 amino acids to 136 amino acids).

A suitable co-regulator peptide for an FXR can be an SRC1 polypeptide or a fragment thereof (e.g., a peptide of from 8 amino acids to 50 amino acids in length, derived from an SRC1 polypeptide).

*LXR-alpha:*

In some cases, an LBD suitable for inclusion as a member of a pair of heterodimerization domains can be an LBD of liver X receptor-alpha (LXR-alpha). For example, in some cases, the LBD can comprise an amino acid sequence having at least 75%, at least 80%, at least 85%, at least 90%, at least 95%, at least 98%, at least 99%, or 100%, amino acid sequence identity to the LBD of an LXR-alpha having the amino acid sequence Uniprot Q13133-1.

For example, the LBD of an LXR-alpha can comprise an amino acid sequence having at least 75%, at least 80%, at least 85%, at least 90%, at least 95%, at least 98%, at least 99%, or 100%, amino acid sequence identity to the amino acid sequence Uniprot Q13133-1 aa 182-447; and has a length of from about 200 amino acids to about 266 amino acids (e.g., has a length of from 200 amino acids to 220 amino acids, from 220 amino acids to 240 amino acids, or from 240 amino acids to 266 amino acids).

A suitable co-regulator peptide for an LXR-alpha can be an SRC1 polypeptide or a fragment thereof (e.g., a peptide of from 8 amino acids to 50 amino acids in length, derived from an SRC1 polypeptide).

*ROR-gamma:*

In some cases, an LBD suitable for inclusion as a member of a pair of heterodimerization domains can be an LBD of a retinoid-related orphan receptor gamma (ROR-gamma). For example, in some cases, the LBD can comprise an amino acid sequence having at least 75%, at least 80%, at least 85%, at least 90%, at least 95%, at least 98%, at least 99%, or 100%, amino acid sequence identity to the LBD of an ROR-gamma having the amino acid sequence Uniprot P51449-2.

For example, the LBD of an ROR-gamma can comprise an amino acid sequence having at least 75%, at least 80%, at least 85%, at least 90%, at least 95%, at least 98%, at least 99%, or 100%, amino acid sequence identity to the amino acid sequence Uniprot P51449-2 aa 237-497; and has a length of from about 200 amino acids to about 261 amino acids (e.g., has a length of from 200 amino acids to 220 amino acids, from 220 amino acids to 240 amino acids, or from 240 amino acids to 261 amino acids).

A suitable co-regulator peptide for an ROR-gamma can be an NCORNR peptide (CDPASNLGLEDIIRKALMGSFDDK, Uniprot Q7Z516-1 aa 2160-2182).

A suitable co-regulator peptide for an ROR-gamma can be an SRC1 polypeptide or a fragment thereof (e.g.,

a peptide of from 8 amino acids to 50 amino acids in length, derived from an SRC1 polypeptide).

*RXR-alpha:*

In some cases, an LBD suitable for inclusion as a member of a pair of heterodimerization domains can be an LBD of a retinoid-X receptor-alpha (RXR-alpha). For example, in some cases, the LBD can comprise an amino acid sequence having at least 75%, at least 80%, at least 85%, at least 90%, at least 95%, at least 98%, at least 99%, or 100%, amino acid sequence identity to the LBD of an RXR-alpha having the amino acid sequence Uniprot P19793-1.

For example, the LBD of an RXR-alpha can comprise an amino acid sequence having at least 75%, at least 80%, at least 85%, at least 90%, at least 95%, at least 98%, at least 99%, or 100%, amino acid sequence identity to the amino acid sequence Uniprot P19793-1 aa 225-462; and has a length of from about 190 amino acids to about 238 amino acids (e.g., has a length of from 190 amino acids to 200 amino acids, from 200 amino acids to 210 amino acids, or from 210 amino acids to 238 amino acids).

A suitable co-regulator peptide for an RXR-alpha can be an SRC1 polypeptide or a fragment thereof (e.g., a peptide of from 8 amino acids to 50 amino acids in length, derived from an SRC1 polypeptide).

*PXR:*

In some cases, an LBD suitable for inclusion as a member of a pair of heterodimerization domains can be an LBD of a Pregnane X Receptor (PXR). For example, in some cases, the LBD can comprise an amino acid sequence having at least 75%, at least 80%, at least 85%, at least 90%, at least 95%, at least 98%, at least 99%, or 100%, amino acid sequence identity to the LBD of a PXR having the amino acid sequence Uniprot O75469-1. In some cases, the LBD comprises an amino acid sequence having at least 75%, at least 80%, at least 85%, at least 90%, at least 95%, at least 98%, at least 99%, or 100%, amino acid sequence identity to amino acids 143-428 of the amino acid sequence Uniprot O75469-1. In some cases, the LBD comprises an amino acid sequence having at least 75%, at least 80%, at least 85%, at least 90%, at least 95%, at least 98%, at least 99%, or 100%, amino acid sequence identity to amino acids 205-434 of the amino acid sequence depicted in Uniprot O75469-1.

For example, the LBD of a PXR can comprise an amino acid sequence having at least 75%, at least 80%, at least 85%, at least 90%, at least 95%, at least 98%, at least 99%, or 100%, amino acid sequence identity to the amino acid sequence Uniprot O75469-1 aa 130-434; and has a length of from about 250 amino acids to about 302 amino acids (e.g., has a length of from 250 amino acids to 275 amino acids, from 275 amino acids to 290 amino acids, or from 290 amino acids to 302 amino acids).

A suitable co-regulator peptide for a PXR can be an SRC1 polypeptide or a fragment thereof (e.g., a peptide of from 8 amino acids to 50 amino acids in length, derived from an SRC1 polypeptide).

9.2.1.2. Co-Regulator Polypeptides:

Suitable co-regulator polypeptides include full-length naturally-occurring nuclear hormone co-regulator polypeptides. Suitable co-regulator polypeptides include fragments of naturally-occurring nuclear hormone co-regulator polypeptides. Suitable co-regulator polypeptides include synthetic or recombinant nuclear hormone co-regulator polypeptides. Suitable co-regulator polypeptides can have a length of from 8 amino acids to 2000 amino acids. Suitable co-regulator polypeptides can have a length of from 8 amino acids to 50 amino acids, e.g., from 8 amino acids to 10 amino acids, from 10 amino acids to 15 amino acids, from 15 amino acids to 20 amino acids, from 20 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids. Suitable co-regulator polypeptides can have a length of from 50 amino acids to 100 amino acids, e.g., from 50 amino acids to 60 amino acids, from 60 amino acids to 70 amino acids, from 70 amino acids to 80 amino acids, from 80 amino acids to 90 amino acids, or from 90 amino acids to 100 amino acids. Suitable co-regulator polypeptides can have a length of from 100 amino acids to 200 amino acids, from 200 amino acids to 300 amino acids, from 300 amino acids to 400 amino acids, from 400 amino acids to 500 amino acids, from 500 amino acids to 600 amino acids, from 600 amino acids to 700 amino acids, from 700 amino acids to 800 amino acids, from 800 amino acids to 900 amino acids, or from 900 amino acids to 1000 amino acids. Suitable co-regulator polypeptides can have a length of from 1000 amino acids to 2000 amino acids.

Suitable co-regulator peptides include Steroid Receptor Coactivator (SRC)-1, SRC-2, SRC-3, TRAP220-1, TRAP220-2, NR0B1, NRIP1, CoRNR box, alpha-betaV, TIF1, TIF2, EA2, TA1, EAB1, SRC1-1, SRC1-2, SRC1-3, SRC1-4a, SRC1-4b, GRIP1-1, GRIP1-2, GRIP1-3, AIB1-1, AIB1-2, AIB1-3, PGC1a, PGC1b, PRC, ASC2-1, ASC2-2, CBP-1, CBP-2, P300, CIA, ARA70-1, ARA70-2, NSD1, SMAP, Tip60, ERAP140, Nix1, LCoR, CoRNR1 (N-CoR), CoRNR2, SMRT, RIP140-C, RIP140-1, RIP140-2, RIP140-3, RIP140-4, RIP140-5, RIP140-6, RIP140-7, RIP140-8, RIP140-9, PRIC285-1, PRIC285-2, PRIC285-3, PRIC285-4, and PRIC285-5.

A co-regulator polypeptide suited for heterodimerization with a respective LBD dimerization partner preferably has a length of from 8 amino acids to 10 amino acids, from 10 amino acids to 15 amino acids, from 15 amino acids to 20 amino acids, from 20 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids; and preferably has at least 75%, at least 80%, at least 85%, at least 90%, at least 95%, at least 98%, at least 99%, or 100%, amino acid sequence identity to a stretch of from 8 to 50 contiguous amino acids of the amino acid sequences: SRC1 (Uniprot Q15788-1), SRC2 (Uniprot Q15596-1), SRC3 (Uniprot Q9Y6Q9-5), PGC1a (Uniprot Q9UBK2-1), PGC1b (Uniprot Q86YN6-1), PPRC-1 (Uniprot Q5VV67-1), TRAP220 (Uniprot Q15648-1), NCOA6 (Uniprot Q14686-1), CREBBP (Uniprot Q92793-1), EP300 (Uniprot Q09472-1), NCOA5 (Uniprot Q9HCD5-1), NCOA4 (Uniprot Q13772-1), TRIM24 (Uniprot O15164-2), NSD1 (Uniprot Q96L73-1), BRD8 (Uniprot Q9H0E9-2), KAT5 (Uniprot Q92993-1), NCOA7 (Uniprot Q8NI08-1), Nix1 (Uniprot Q9BQI9-1), LCoR (Uniprot Q96JN0-1), N-CoR (Uniprot O75376-1), NCOR2 (Uniprot Q9Y618-1), RIP140 (Uniprot P48552-1), PRIC285 (Uniprot Q9BYK8-2);

In preferred embodiments, a suitable co-regulator peptide comprises an LXXLL motif, where X is any amino acid; where the co-regulator peptide has a length of from 8 amino acids to 50 amino acids, e.g., from 8 amino acids to 10 amino acids, from 10 amino acids to 12 amino acids, from 12 amino acids to 15 amino acids, from 15 amino acids to 20 amino acids, from 20 amino acids to 25 amino acids, from 25 amino acids to 30 amino acids, from 30 amino acids to 35 amino acids, from 35 amino acids to 40 amino acids, from 40 amino acids to 45 amino acids, or from 45 amino acids to 50 amino acids.

Examples of suitable co-regulator peptides are as follows: SRC1: (Uniprot Q15788-1 aa 676 - 700) CPSSHSSLTERHKILHRLLQEGSPS; SRC1-2: (Uniprot Q15788-1 aa 682 - 702; SNP rs1049021 E685A) SLTARHKILHRLLQEGSPSDI; SRC3-1: (Uniprot Q9Y6Q9-5 aa 614 - 632) ESKGHKKLLQLLTCSSDDR; SRC3: (SEQ ID NO: 14) PKKENNALLRYLLDRDDPSDV; PGC-1: (Uniprot Q9UBK2-1 aa 138 - 154) AEEPSLLKKLLLAPANT; PGC1a: (Uniprot Q9UBK2-1 aa 136 - 156) QEAEEPSLLKKLLLAPANTQL; TRAP220-1: (Uniprot Q15648-1 aa 596 - 616) SKVSQNPILTSLLQITGNGGS; NCoR: (Uniprot O75376-1 aa 2251 - 2275) GHSFADPASNLGLEDIIRKALMGSF; NR0B1: (Uniprot P51843-1 aa 74 - 90) PRQGSILYSMLTSAKQT; NRIP1: (Uniprot P48552-1 aa 374 - 390) AANNSLLLHLLKSQTIP; TIF2: (Uniprot Q15596-1 aa 737 - 757) PKKKENALLRYLLDKDDTKDI; CoRNR Box: (SEQ ID NO: 11) DAFQLRQLILRGLQDD; abV: (SEQ ID NO: 12) SPGSREWFKDMLS; TRAP220-2: (Uniprot Q15648-1 aa 637 - 657) GNTKNHPMLMNLLKDNPAQDF; EA2: (SEQ ID NO: 15) SSKGVLWRMLAEPVSR; TA1: (SEQ ID NO: 16) SRTLQLDWGTLYWSR; EAB1: (SEQ ID NO: 17) SSNHQSSRLIELLSR; SRC2: (Uniprot Q15596-1 aa 683 - 701) LKEKHKILHRLLQDSSSPV; SRC1-3: (Uniprot Q15788-1 aa 1428 - 1441) QAQQKSLLQQLLTE; SRC1-1: (Uniprot Q15788-1 aa 625 - 645) KYSQTSHKLVQLLTTTAEQQL; SRC1-2: (Uniprot Q15788-1 aa 682 - 702; SNP rs1049021 E685A) SLTARHKILHRLLQEGSPSDI; SRC1-3: (Uniprot Q15788-1 aa 741 - 761) KESKDHQLLRYLLDKDEKDLR; SRC1-4a: (Uniprot Q15788-1 aa 1427 - 1441) PQAQQKSLLQQLLTE; SRC1-4b: (Uniprot Q15788-1 aa 1427 - 1441 L1435R) PQAQQKSLRQQLLTE; GRIP1-1: (Uniprot Q15596-1 aa 633 - 653) HDSKGQTKLLQLLTTKSDQME; GRIP1-2: (Uniprot Q15596-1 aa 682 - 702) SLKEKHKILHRLLQDSSSPVD; GRIP1-3: (Uniprot Q15596-1 aa 737 - 757) PKKKENALLRYLLDKDDTKDI; AIB1-1: (Uniprot Q9Y6Q9-5 aa 613 - 633) LESKGHKKLLQLLTCSSDDRG; AIB1-2: (Uniprot Q9Y6Q9-5 aa 677 - 697) LLQEKHRILHKLLQNGNSPAE; AIB1-3: (Uniprot Q9Y6Q9-5 aa 730 - 750) KKKENNALLRYLLDRDDPSDA; PGC1a: (Uniprot Q9UBK2-1 aa 136 - 156) QEAEEPSLLKKLLLAPANTQL; PGC1b: (Uniprot Q86YN6-1 aa 148 - 168) PEVDELSLLQKLLLATSYPTS; PRC: (Uniprot Q5VV67-1 aa 156 - 176) VSPREGSSLHKLLTLSRTPPE; TRAP220-1: (Uniprot Q15648-1 aa 596 - 616) SKVSQNPILTSLLQITGNGGS; TRAP220-2: (Uniprot Q15648-1 aa 637 - 657) GNTKNHPMLMNLLKDNPAQDF; ASC2-1: (Uniprot Q14686-1 aa 879 - 899) DVTLTSPLLVNLLQSDISAGH; ASC2-2: (Uniprot Q14686-1 aa 1483 - 1503) AMREAPTSLSQLLDNSGAPNV; CBP-1: (Uniprot Q92793-1 aa 62 - 82) DAASKHKQLSELLRGGSGSSI; CBP-2: (Uniprot Q92793-1 aa 350 - 370) KRKLIQQQLVLLLHAHKCQRR; P300: (Uniprot Q09472-1 aa 73 - 93) DAASKHKQLSELLRSGSSPNL; CIA: (Uniprot Q9HCD5-1 aa 337 - 357) GHPPAIQSLINLLADNRYLTA; ARA70-1: (Uniprot Q13772-1 aa 84 - 104) TLQQQAQQLYSLLGQFNCLTH; ARA70-2: (Uniprot Q13772-1 aa 320 - 340) GSRETSEKFKLLFQSYNVNDW; TIF1: (Uniprot O15164-2 aa 718 - 738) NANYPRSILTSLLLNSSQSST; NSD1: (Uniprot Q96L73-1 aa 899 - 919) IPIEPDYKFSTLLMMLKDMHD; SMAP: (Uniprot Q9H0E9-2 aa 263 - 283) ATPPPSPLLSELLKKGSLLPT; Tip60: (Uniprot Q92993-1 aa 481 - 501) VDGHERAMLKRLLRIDSKCLH; ERAP140: (Uniprot Q8NI08-1 aa 514 - 534) HEDLDKVKLIEYYLTKNKEGP; Nix1: (Uniprot Q9BQI9-1 aa 236 - 256) ESPEFCLGLQTLLSLKCCIDL; LCoR: (Uniprot Q96JN0-1 aa 45 - 65) AATTQNPVLSKLLMADQDSPL; CoRNR1 (N-CoR): (Uniprot O75376-1 aa 239 - 268) MGQVPRTHRLITLADHICQIITQDFARNQV; CoRNR2 (N-CoR): (Uniprot O75376-1 aa 2260 - 2273) NLGLEDIIRKALMG; CoRNR1 (SMRT): (Uniprot Q9Y618-1 aa 2131 - 2170) APGVKGHQRVVTLAQHISEVITQDTYRHHPQQLSAPLPAP; CoRNR2 (SMRT): (Uniprot Q9Y618-1 aa 2347 - 2360) NMGLEAIIRKALMG; RIP140-C: (SEQ ID NO: 18) RLTKTNPILYYMLQKGGNSVA; RIP140-1: (Uniprot P48552-1 aa 13 - 33) QDSIVLTYLEGLLMHQAAGGS; RIP140-2: (Uniprot P48552-1 aa 125 - 145) KGKQDSTLLASLLQSFSSRLQ; RIP140-3: (Uniprot P48552-1 aa 177 - 197) CYGVASSHLKTLLKKSKVKDQ; RIP140-4: (Uniprot P48552-1 aa 258 - 278) KPSVACSQLALLLSSEAHLQQ; RIP140-5: (Uniprot P48552-1 aa 372 - 392) KQAANNSLLLHLLKSQTIPKP; RIP140-6: (Uniprot P48552-1 aa 493 - 513) NSHQKVTLLQLLLGHKNEENV; RIP140-7: (SEQ ID NO: 19) NLLERRTVLQLLLGNPTKGRV; RIP140-8: (Uniprot P48552-1 aa 811 -

831) FSFSKNGLLSRLLRQNQDSYL; RIP140-9: (Uniprot P48552-1 aa 928 - 948) RESKSFNVLKQLLLSENCVRD; PRIC285-1: (Uniprot Q9BYK8-2 aa 458 - 518) ELNADDAILRELLDESQKVMV; PRIC285-2: (Uniprot Q9BYK8-2 aa 541 - 561) YENLPPAALRKLLRAEPERYR; PRIC285-3: (Uniprot Q9BYK8-2 aa 596 - 616) MAFAGDEVLVQLLSGDKAPEG; PRIC285-4: (Uniprot Q9BYK8-2 aa 1435 - 1455) SCCYLCIRLEGLLAPTASPRP; and PRIC285-5: (Uniprot Q9BYK8-2 aa 1652 - 1672) PSNKSVDVLAGLLLRRMELKP.

In some cases, a given LBD can be paired with two or more different co-regulator polypeptides. For example, PPAR-gamma (Uniprot P37231) can be paired with SRC1 (Uniprot Q15788-1 aa 625-645; Uniprot Q15788-1 aa 676-700; Uniprot Q15788-1 aa 682-702, SNP rs1049021 E685A; Uniprot Q15788-1 aa 741-761; Uniprot Q15788-1 aa 1428-1441; Uniprot Q15788-1 aa 1427-1441; Uniprot Q15788-1 aa 1427-1441 L1435R), SRC2 (Uniprot Q15596-1 aa 683-701), SRC3 (SEQ ID NO: 14; Uniprot Q9Y6Q9-5 aa 614-632), or TRAP220 (Uniprot Q15648-1 aa 596-616; Uniprot Q15648-1 aa 637-657). As another example, ER-alpha (Uniprot P03372) can be paired with CoRNR (Uniprot O75376-1 aa 239-268; Uniprot O75376-1 aa 2260-2273; Uniprot Q9Y618-1 aa 2131-2170; Uniprot Q9Y618-1 aa 2347-2360), alpha-betaV (SEQ ID NO: 12), or TA1 (SEQ ID NO: 16). As another example, ER-beta (Uniprot Q92731) can be paired with CoRNR (Uniprot O75376-1 aa 239-268; Uniprot O75376-1 aa 2260-2273; Uniprot Q9Y618-1 aa 2131-2170; Uniprot Q9Y618-1 aa 2347-2360), alpha-betaV (SEQ ID NO: 12), or TA1 (SEQ ID NO: 16). As another example, AR (Uniprot P10275) can be paired with SRC1 (Uniprot Q15788-1 aa 625-645; Uniprot Q15788-1 aa 676-700; Uniprot Q15788-1 aa 682-702, SNP rs1049021 E685A; Uniprot Q15788-1 aa 741-761; Uniprot Q15788-1 aa 1428-1441; Uniprot Q15788-1 aa 1427-1441; Uniprot Q15788-1 aa 1427-1441 L1435R), SRC2 (Uniprot Q15596-1 aa 683-701), SRC3 (SEQ ID NO: 14; Uniprot Q9Y6Q9-5 aa 614-632), or TRAP220 (Uniprot Q15648-1 aa 596-616; Uniprot Q15648-1 aa 637-657) . As another example, PR (Uniprot P06401) can be paired with SRC1 (Uniprot Q15788-1 aa 625-645; Uniprot Q15788-1 aa 676-700; Uniprot Q15788-1 aa 682-702, SNP rs1049021 E685A; Uniprot Q15788-1 aa 741-761; Uniprot Q15788-1 aa 1428-1441; Uniprot Q15788-1 aa 1427-1441; Uniprot Q15788-1 aa 1427-1441 L1435R), SRC2 (Uniprot Q15596-1 aa 683-701), SRC3 (SEQ ID NO: 14; Uniprot Q9Y6Q9-5 aa 614-632), TRAP220 (Uniprot Q15648-1 aa 596-616; Uniprot Q15648-1 aa 637-657), NR0B1 (Uniprot P51843-1 aa 74-90), PGC1B (Uniprot Q86YN6-1 aa 148-168), NRIP1 (Uniprot P48552-1 aa 374-390), EA2 (SEQ ID NO: 15), or EAB1 (SEQ ID NO: 17). As another example, TR-beta (Uniprot P10828) can be paired with SRC1 (Uniprot Q15788-1 aa 625-645; Uniprot Q15788-1 aa 676-700; Uniprot Q15788-1 aa 682-702, SNP rs1049021 E685A; Uniprot Q15788-1 aa 741-761; Uniprot Q15788-1 aa 1428-1441; Uniprot Q15788-1 aa 1427-1441; Uniprot Q15788-1 aa 1427-1441 L1435R), SRC2 (Uniprot Q15596-1 aa 683-701), SRC3 (SEQ ID NO: 14; Uniprot Q9Y6Q9-5 aa 614-632), or TRAP220 (Uniprot Q15648-1 aa 596-616; Uniprot Q15648-1 aa 637-657).

9.2.1.3. Regulating molecules for LBD based heterodimerization:

Where one member of a pair of heterodimerization domains is an LBD of a nuclear hormone receptor, at least one type of the used regulating molecules are able to bind the LBD in a first CAR molecule of the group which then can heterodimerize with a co-regulator peptide in a second CAR molecule of the group.

Suitable regulating molecules for LBD-based heterodimerization systems are known in the art. Examples of regulating molecules for LBD based heterodimerization systems include corticosterone ((8S,9S,10R,11S,13S,14S,17S)-11-hydroxy-17-(2-hydroxyacetyl)-10,13-dimethyl-1,2,6,7,8,9,11,12,14,15,16,17-dodecahydrocyclopenta[a]phenanthren-3-one); deoxycorticosterone ((8S,9S,10R,13S,14S,17S)-17-(2-hydroxyacetyl)-10,13-dimethyl-1,2,6,7,8,9,11,12,14,15,16,17-dodecahydrocyclopenta[a]phenanthren-3-one); cortisol ((8S,9S,10R,11S,13S,14S,17R)-11,17-dihydroxy-17-(2-hydroxyacetyl)-10,13-dimethyl-2,6,7,8,9,11,12,14,15,16-decahydro-1H-cyclopenta[a]phenanthren-3-one); 11-deoxycortisol ((8R,9S,10R,13S,14S,17R)-17-hydroxy-17-(2-hydroxyacetyl)-10,13-dimethyl-2,6,7,8,9,11,12,14,15,16-decahydro-1H-cyclopenta[a]phenanthren-3-one); cortisone ((8S,9S,10R,13S,14S,17R)-17-hydroxy-17-(2-hydroxyacetyl)-10,13-dimethyl-1,2,6,7,8,9,12,14,15,16-decahydrocyclopenta[a]phenanthrene-3,11-dione); 18-hydroxycorticosterone ((8S,9S,10R,11S,13R,14S,17S)-11-hydroxy-17-(2-hydroxyacetyl)-13-(hydroxymethyl)-10-methyl-1,2,6,7,8,9,11,12,14,15,16,17-dodecahydrocyclopenta[a]phenanthren-3-one); 1alpha-hydroxycorticosterone ((1S,8S,9S,10R,11S,13S,14S,17S)-1,11-dihydroxy-17-(2-hydroxyacetyl)-10,13-dimethyl-1,2,6,7,8,9,11,12,14,15,16,17-dodecahydrocyclopenta[a]phenanthren-3-one); aldosterone ((8S,9S,10R,11S,13R,14S,17S)-11-hydroxy-17-(2-hydroxyacetyl)-10-methyl-3-oxo-1,2,6,7,8,9,11,12,14,15,16,17-dodecahydrocyclopenta[a]phenanthrene-13-carbaldehyde) ; androstenedione ((8R,9S,10R,13S,14S)-10,13-dimethyl-2,6,7,8,9,11,12,14,15, 16-decahydro-1H-cyclopenta[a]phenanthrene-3,17-dione); 4-hydroxy-androstenedione ((8R,9S,10R,13S,14S)-4-hydroxy-10,13-dimethyl-2,6,7,8,9,11,12,14,15,16-decahydro-1H-cyclopenta[a]phenanthrene-3,17-dione); 11beta-hydroxyandrostenedione ((8S,9S,10R,11S,13S,14S)-11-hydroxy-10,13-dimethyl-2,6,7,8,9,11,12,14,15,16-decahydro-1H-cyclopenta[a]phenanthrene-3,17-dione); androstanediol ((3R,5S,8R,9S,10S,13S,14S)-10,13-dimethyl-2,3,4,5,6,7,8,9,11,12,14,15,16,17-tetradecahydro-1H-cyclopenta[a]phenanthrene-3,17-diol); androsterone ((3R,5S,8R,9S,10S,13S,14S)-3-hydroxy-10,13-dimethyl-1,2,3,4,5,6,7,8,9,11,12,14,15,16-tetradecahydrocyclopenta[a]phenanthren-17-one); epiandrosterone

((3S,5S,8R,9S,10S,13S,14S)-3-hydroxy-10,13-dimethyl-1,2,3,4,5,6,7,8,9,11,12,14,15,16-tetradecahydrocyclopenta[a]phenanthren-17-one); adrenosterone ((8S,9S,10R,13S,14S)-10,13-dimethyl-1,2,6,7,8,9,12,14,15,16-decahydrocyclopenta[a]phenanthrene-3,11,17-trione); dehydroepiandrosterone ((3S,8R,9S,10R,13S,14S)-3-hydroxy-10,13-dimethyl-1,2,3,4,7,8,9,11,12,14,15,16-dodecahydrocyclopenta[a]phenanthren-17-one); dehydroepiandrosterone sulphate ([(3S,8R,9S,10R,13S,14S)-10,13-dimethyl-17-oxo-1,2,3,4,7,8,9,11,12,14,15,16-dodecahydrocyclopenta[a]phenanthren-3-yl] hydrogen sulphate); testosterone ((8R,9S,10R,13S,14S,17S)-17-hydroxy-10,13-dimethyl-1,2,6,7,8,9,11,12,14,15,16,17-dodecahydrocyclopenta[a]phenanthren-3-one); epitestosterone ((8R,9S,10R,13S,14S,17R)-17-hydroxy-10,13-dimethyl-1,2,6,7,8,9,11,12,14,15,16,17-dodecahydrocyclopenta[a]phenanthren-3-one); 5alpha-dihydrotestosterone ((5S,8R,9S,10S,13S,14S,17S)-17-hydroxy-10,13-dimethyl-1,2,4,5,6,7,8,9,11,12,14,15,16,17-tetradecahydrocyclopenta[a]phenanthren-3-one); 5beta-dihydrotestosterone ((5R,8R,9S,10S,13S,14S,17S)-17-hydroxy-10,13-dimethyl-1,2,4,5,6,7,8,9,11,12,14,15,16,17-tetradecahydrocyclopenta[a]phenanthren-3-one); 5beta-dihydrotestosterone ((5R,8R,9S,10S,13S,14S,17S)-17-hydroxy-10,13-dimethyl-1,2,4,5,6,7,8,9,11,12,14,15,16,17-tetradecahydrocyclopenta[a]phenanthren-3-one); 11beta-hydroxytestosterone ((8S,9S,10R,11S,13S,14S,17S)-11,17-dihydroxy-10,13-dimethyl-1,2,6,7,8,9,11,12,14,15,16,17-dodecahydrocyclopenta[a]phenanthren-3-one); 11-ketotestosterone ((8S,9S,10R,13S,14S,17S)-17-hydroxy-10,13-dimethyl-2,6,7,8,9,12,14,15,16,17-decahydro-1H-cyclopenta[a]phenanthrene-3,11-dione); estrone ((8R,9S,13S,14S)-3-hydroxy-13-methyl-7,8,9,11,12,14,15,16-octahydro-6H-cyclopenta[a]phenanthren-17-one); estradiol ((8R,9S,13S,14S,17S)-13-methyl-6,7,8,9,11,12,14,15,16,17-decahydrocyclopenta[a]phenanthrene-3,17-diol); estriol ((8R,9S,13S,14S,16R,17R)-13-methyl-6,7,8,9,11,12,14,15,16,17-decahydrocyclopenta[a]phenanthrene-3,16,17-triol); pregnenolone (1-[(3S,8S,9S,10R,13S,14S,17S)-3-hydroxy-10,13-dimethyl-2,3,4,7,8,9,11,12,14,15,16,17-dodecahydro-1H-cyclopenta[a]phenanthren-17-yl]ethanone); 17-hydroxypregnenolone (1-[(3S,8R,9S,10R,13S,14S,17R)-3,17-dihydroxy-10,13-dimethyl-1,2,3,4,7,8,9,11,12,14,15,16-dodecahydrocyclopenta[a]phenanthren-17-yl]ethanone); progesterone ((8S,9S,10R,13S,14S,17S)-17-acetyl-10,13-dimethyl-1,2,6,7,8,9,11,12,14,15,16,17-dodecahydrocyclopenta[a]phenanthren-3-one); 17-hydroxyprogesterone ((8R,9S,10R,13S,14S,17R)-17-acetyl-17-hydroxy-10,13-dimethyl-2,6,7,8,9,11,12,14,15,16-decahydro-1H-cyclopenta[a]phenanthren-3-one); T3 ((2S)-2-amino-3-[4-(4-hydroxy-3-iodophenoxy)-3,5-diiodophenyl]propanoic acid); T4 ((2S)-2-amino-3-[4-(4-hydroxy-3,5-diiodophenoxy)-3,5-diiodophenyl]propanoic acid); spironolactone (S-[(7R,8R,9S,10R,13S,14S,17R)-10,13-dimethyl-3,5'-dioxospiro[2,6,7,8,9,11,12,14,15,16-decahydro-1H-cyclopenta[a]phenanthrene-17,2'-oxolane]-7-yl] ethanethioate); eplerenone (PubChem CID 443872);

cyproterone acetate (PubChem CID 9880); hydroxyflutamide (2-hydroxy-2-methyl-N-[4-nitro-3-(trifluoromethyl)phenyl]propanamide); enzalutamide (4-[3-[4-cyano-3-(trifluoromethyl)phenyl]-5,5-dimethyl-4-oxo-2-sulfanylideneimidazolidin-1-yl]-2-fluoro-N-methylbenzamide); ARN-509 (4-[7-[6-cyano-5-(trifluoromethyl)pyridin-3-yl]-8-oxo-6-sulfanylidene-5,7-diazaspiro[3.4]octan-5-yl]-2-fluoro-N-methylbenzamide); 3,3'-diindolylmethane (DIM) (3-(1H-indol-3-ylmethyl)-1H-indole); bexlosteride ((4aR,10bR)-8-chloro-4-methyl-1,2,4a,5,6,10b-hexahydrobenzo[f]quinolin-3-one); bicalutamide (N-[4-cyano-3-(trifluoromethyl)phenyl]-3-(4-fluorophenyl)sulfonyl-2-hydroxy-2-methylpropanamide); N-butylbenzenesulfonamide (NBBS) (N-butylbenzenesulfonamide); dutasteride ((1S,3aS,3bS,5aR,9aR,9bS,11aS)-N-[2,5-bis(trifluoromethyl)phenyl]-9a,11a-dimethyl-7-oxo-1,2,3,3a,3b,4,5,5a,6,9b,10,11-dodecahydroindeno[5,4-f]quinoline-1-carboxamide); epristeride ((8S,9S,10R,13S,14S,17S)-17-(tert-butylcarbamoyl)-10,13-dimethyl-2,7,8,9,11,12,14,15,16,17-decahydro-1H-cyclopenta[a]phenanthrene-3-carboxylic acid); finasteride ((1S,3aS,3bS,5aR,9aR,9bS,11aS)-N-tert-butyl-9a,11a-dimethyl-7-oxo-1,2,3,3a,3b,4,5,5a,6,9b,10,11-dodecahydroindeno[5,4-f]quinoline-1-carboxamide); flutamide (2-methyl-N-[4-nitro-3-(trifluoromethyl)phenyl]propanamide); izonsteride ((4aR,10bR)-8-[(4-ethyl-1,3-benzothiazol-2-yl)sulfanyl]-4,10b-dimethyl-2,4a,5,6-tetrahydro-1H-benzo[f]quinolin-3-one); ketoconazole (1-[4-[4-[[(2R,4S)-2-(2,4-dichlorophenyl)-2-(imidazol-1-ylmethyl)-1,3-dioxolan-4-yl]methoxy]phenyl]piperazin-1-yl]ethanone); N-butylbenzenesulfonamide (N-butylbenzenesulfonamide); nilutamide (5,5-dimethyl-3-[4-nitro-3-(trifluoromethyl)phenyl]imidazolidine-2,4-dione); megestrol ((8R,9S,10R,13S,14S,17R)-17-acetyl-17-hydroxy-6,10,13-trimethyl-2,8,9,11,12,14,15,16-octahydro-1H-cyclopenta[a]phenanthren-3-one); turosteride ((1S,3aS,3bS,5aR,9aR,9bS,11aS)-6,9a,11a-trimethyl-7-oxo-N-propan-2-yl-N-(propan-2-ylcarbamoyl)-2,3,3a,3b,4,5,5a,8,9,9b,10,11-dodecahydro-1H-indeno[5,4-f]quinoline-1-carboxamide);

mifepristone ((8S,11R,13S,14S,17S)-11-[4-(dimethylamino)phenyl]-17-hydroxy-13-methyl-17-prop-1-ynyl-1,2,6,7,8,11,12,14,15,16-decahydrocyclopenta[a]phenanthren-3-one); Lilopristone ((8S,11R,13S,14S,17R)-11-[4-(dimethylamino)phenyl]-17-hydroxy-17-[(Z)-3-hydroxyprop-1-enyl]-13-methyl-1,2,6,7,8,11,12,14,15,16-decahydrocyclopenta[a]phenanthren-3-one); onapristone ((8S,11R,13R,14S,17S)-11-[4-(dimethylamino)phenyl]-17-hydroxy-17-(3-hydroxypropyl)-13-methyl-1,2,6,7,8,11,12,14,15,16-decahydrocyclopenta[a]phenanthren-3-one); asoprisnil ((8S,11R,13S,14S,17S)-11-[4-[(E)-hydroxyiminomethyl]phenyl]-17-methoxy-17-(methoxymethyl)-13-methyl-1,2,6,7,8,11,12,14,15,16-decahydrocyclopenta[a]phenanthren-3-one); J912 ((8S,11R,13S,14S,17S)-17-hydroxy-11-[4-[(Z)-hydroxyiminomethyl]phenyl]-17-(methoxymethyl)-13-methyl-1,2,6,7,8,11,12,14,15,16-decahydrocyclopenta[a]phenanthren-3-one); CDB-2914 ((8S,13S,14S,17R)-17-acetyl-11-[4-(dimethylamino)phenyl]-17-hydroxy-13-methyl-1,2,6,7,8,11,12,14,15,16-decahydrocyclopenta[a]phenanthren-3-one); JNJ-1250132 ([(8S,11R,13S,14S,17R)-17-acetyl-13-methyl-3-oxo-11-(4-piperidin-1-ylphenyl)-1,2,6,7,8,11,12,14,15,16-decahydrocyclopenta[a]phenanthren-17-yl] acetate); ORG-31710 ((6R,8S,11R,13S,14S,17R)-11-[4-(dimethylamino)phenyl]-6,13-dimethylspiro[1,2,6,7,8,11,12,14,15,16-decahydrocyclopenta[a]phenanthrene-17,2'-oxolane]-3-one); ORG-33628 ((8S,11R,13S,14S,17R)-11-(4-acetylphenyl)-13-methyl-3'-methylidenespiro[1,2,6,7,8,11,12,14,15,16-decahydrocyclopenta[a]phenanthrene-17,2'-oxolane]-3-one); ORG-31806 ((7S,8S,11R,13S,14S,17R)-11-[4-(dimethylamino)phenyl]-7,13-dimethylspiro[1,2,6,7,8,11,12,14,15,16-decahydrocyclopenta[a]phenanthrene-17,2'-oxolane]-3-one); ZK-112993 ((8S,11R,13S,14S,17S)-11-(4-acetylphenyl)-17-hydroxy-13-methyl-17-prop-1-ynyl-1,2,6,7,8,11,12,14,15,16-decahydrocyclopenta[a]phenanthren-3-one); ORG-31376 ((8S,11R,13S,14R,17S)-11-[4-(dimethylamino)phenyl]-13-methylspiro[1,2,6,7,8,11,12,14,15,16-decahydrocyclopenta[a]phenanthrene-17,2'-oxolane]-3-one); ORG-33245 ((8S,13S,14S,17R)-11-[4-(dimethylamino)phenyl]-13-methyl-3'-methylidenespiro[1,2,6,7,8,11,12,14,15,16-decahydrocyclopenta[a]phenanthrene-17,2'-oxolane]-3-one); ORG-31167; ORG-31343; RU-2992; RU-1479; RU-25056; RU-49295; RU-46556; RU-26819; LG1127; LG120753 (3-(2,2,4-trimethyl-1H-quinolin-6-yl)benzonitrile); LG120830 (3-fluoro-5-(2,2,4-trimethyl-1H-quinolin-6-yl)benzonitrile); LG1447; LG121046; CGP-19984A (sodium;methyl [(2Z)-3-methyl-2-[(Z)-[5-methyl-3-(2-methylprop-2-enyl)-4-oxo-1,3-thiazolidin-2-ylidene]hydrazinylidene]-4-oxo-1,3-thiazolidin-5-yl] phosphate); RTI-3021-012 (8S,11R,13S,14S,17R)-17-acetyl-11-[4-(dimethylamino)phenyl]-17-hydroxy-13-methyl-1,2,6,7,8,11,12,14,15,16-decahydrocyclopenta[a]phenanthren-3-one); RTI-3021-022 ((8S,11R,13S,14S,17R)-17-acetyl-11-[4-(dimethylamino)phenyl]-17-hydroxy-13-methyl-1,2,6,7,8,11,12,14,15,16-decahydrocyclopenta[a]phenanthren-3-one); RTI-3021-020; RWJ-25333 ((3,4-dichlorophenyl)-(6-phenyl-4,5-dihydro-3H-pyridazin-2-yl)methanone); ZK-136796; ZK-114043 ((8S,11R,13S,14S,17S)-11-(4-acetylphenyl)-17-hydroxy-17-[(E)-3-hydroxyprop-1-enyl]-13-methyl-1,2,6,7,8,11,12,14,15,16-decahydrocyclopenta[a]phenanthren-3-one); ZK-230211 ((8S,11R,13S,14S,17S)-11-(4-acetylphenyl)-17-hydroxy-13-methyl-17-(1,1,2,2,2-pentafluoroethyl)-1,2,6,7,8,11,12,14,15,16-decahydrocyclopenta[a]phenanthren-3-one); ZK-136798; ZK-98229; ZK-98734 ((8S,11R,13S,14S,17R)-11-[4-(dimethylamino)phenyl]-17-hydroxy-17-[(Z)-3-hydroxyprop-1-enyl]-13-methyl-1,2,6,7,8,11,12,14,15,16-decahydrocyclopenta[a]phenanthren-3-one); ZK-137316; Asoprisnil ((8S,11R,13S,14S,17S)-11-[4-[(E)-hydroxyiminomethyl]phenyl]-17-methoxy-17-(methoxymethyl)-13-methyl-1,2,6,7,8,11,12,14,15,16-decahydrocyclopenta[a]phenanthren-3-one); 4-[17β-Methoxy-17α-(methoxymethyl)-3-oxoestra-4,9-dien-11β-yl]benzaldehyde-1-(E)-[0-(ethylamino)carbonyl]oxime; (Z)-6'-(4-cyanophenyl)-9,11α-dihydro-17β-hydroxy-17α-[4-(1-oxo-3-methylbutoxy)-1-butenyl]4'H-naphtho[3',2',1'; 10,9,11]estr-4-en-3-one; 11β-(4-acetylphenyl)-17β-hydroxy-17α-(1,1,2,2,2-pentafluoroethyl)estra-4,9-dien-3-one; 11β-(4-Acetylphenyl)-19,24-dinor-17,23-epoxy-17alpha-chola-4,9,20-trie-n-3-one; (Z)-11beta,19-[4-(3-Pyridinyl)-o-phenylene]-17beta-hydroxy-17α-[3-hydroxy-1-propenyl]-4-androsten-3-one; 11beta-[4-(1-methylethenyl)phenyl]-17α-hydroxy-17beta-β-hydroxypropyl)-13α-estra-4,9-dien-3-one; 4',5'-Dihydro-11beta-[4-(dimethylamino)phenyl]-6beta-methylspiro[estra-4,-9-dien-17beta,2'(3'H)-furan]-3-one; drospirenone (PubChem CID 68873); T3 ((2S)-2-amino-3-[4-(4-hydroxy-3-iodophenoxy)-3,5-diiodophenyl]propanoic acid); KB-141 (2-[3,5-dichloro-4-(4-hydroxy-3-propan-2-ylphenoxy)phenyl]acetic acid); sobetirome (2-[4-[(4-hydroxy-3-propan-2-ylphenyl)methyl]-3,5-dimethylphenoxy]acetic acid); GC-24 (2-[4-[(3-benzyl-4-hydroxyphenyl)methyl]-3,5-dimethylphenoxy]acetic acid); 4-OH-PCB106 (2-chloro-4-(2,3,4,5-tetrachlorophenyl)phenol); eprotirome (3-[3,5-dibromo-4-(4-hydroxy-3-propan-2-ylphenoxy)anilino]-3-oxopropanoic acid); MB07811 (PubChem CID 15942005); QH2 (2-[(2E)-3,7-dimethylocta-2,6-dienyl]-5,6-dimethoxy-3-methylbenzene-1,4-diol); MB07344 ([4-[(4-hydroxy-3-propan-2-ylphenyl)methyl]-3,5-dimethylphenoxy]methylphosphonic acid); Tamoxifen (2-[4-[(Z)-1,2-diphenylbut-1-enyl]phenoxy]-N,N-dimethylethanamine); 4-OH-tamoxifen (4-[(Z)-1-[4-[2-(dimethylamino)ethoxy]phenyl]-2-phenylbut-1-enyl]phenol); raloxifene ([6-hydroxy-2-(4-hydroxyphenyl)-1-benzothiophen-3-yl]-[4-(2-piperidin-1-ylethoxy)phenyl]methanone); lasofoxifene ((5R,6S)-6-phenyl-5-[4-(2-pyrrolidin-1-ylethoxy)phenyl]-5,6,7,8-tetrahydronaphthalen-2-ol); bazedoxifene (1-[[4-[2-(azepan-1-yl)ethoxy]phenyl]methyl]-2-(4-hydroxyphenyl)-3-methylindol-5-ol); falsodex ((7R,8R,9S,13S,14S,17S)-13-methyl-7-[9-(4,4,5,5,5-pentafluoropentylsulfinyl)nonyl]-6,7,8,9,11,12,14,15,16,17-decahydrocyclopenta[a]phenanthrene-3,17-diol); clomifene (2-[4-[(E)-2-chloro-1,2-diphenylethenyl]phenoxy]-N,N-diethylethanamine); femarelle (); ormeloxifene (1-[2-[4-[(3R,4R)-7-methoxy-2,2-dimethyl-3-phenyl-3,4-dihydrochromen-4-yl]phenoxy]ethyl]pyrrolidine); toremifiene (2-[4-[(Z)-4-chloro-1,2-diphenylbut-1-enyl]phenoxy]-N,N-dimethylethanamine); ospemifene (2-[4-[(Z)-4-chloro-1,2-diphenylbut-1-enyl]phenoxy]ethanol); and ethinyl estradiol ((8R,9S,13S,14S,17R)-17-ethynyl-13-methyl-7,8,9,11,12,14,15,16-octahydro-6H-cyclopenta[a]phenanthrene-3,17-diol); estradiol ((8R,9S,13S,14S,17S)-13-methyl-6,7,8,9,11,12,14,15,16,17-decahydrocyclopenta[a]phenanthrene-3,17-diol); ethinyl estradiol ((8R,9S,13S,14S,17R)-17-ethynyl-13-methyl-7,8,9,11,12,14,15,16-octahydro-6H-cyclopenta[a]phenanthrene-3,17-diol); Thiazolidinedione: (eg. Rosiglitazone (5-[[4-[2-[methyl(pyridin-2-yl)amino]ethoxy]phenyl]methyl]-1,3-thiazolidine-2,4-dione); pioglitazone (5-[[4-[2-(5-ethylpyridin-2-yl)ethoxy]phenyl]methyl]-1,3-thiazolidine-2,4-dione); lobeglitazone (5-[[4-[2-[[6-(4-methoxyphenoxy)pyrimidin-4-yl]-methylamino]ethoxy]phenyl]methyl]-1,3-thiazolidine-2,4-dione); troglitazone (5-[[4-[(6-hydroxy-2,5,7,8-tetramethyl-3,4-dihydrochromen-2-yl)methoxy]phenyl]methyl]-1,3-thiazolidine-2,4-dione)), farglitazar ((2S)-2-(2-benzoylanilino)-3-[4-[2-(5-methyl-2-phenyl-1,3-oxazol-4-yl)ethoxy]phenyl]propanoic acid); aleglitazar ((2S)-2-methoxy-3-[4-[2-(5-methyl-2-phenyl-1,3-oxazol-4-yl)ethoxy]-1-benzothiophen-7-yl]propanoic acid); and fenofibric acid (2-[4-(4-chlorobenzoyl)phenoxy]-2-methylpropanoic acid); benzopyranoquinoline A 276575, Mapracorat ((2R)-1,1,1-trifluoro-4-(5-fluoro-2,3-dihydro-1-benzofuran-7-yl)-4-methyl-2-[[(2-methylquinolin-5-yl)amino]methyl]pentan-2-ol); ZK 216348 (4-(2,3-dihydro-1-benzofuran-7-yl)-2-hydroxy-4-methyl-N-(4-methyl-1-oxo-2,3-benzoxazin-6-yl)-2-(trifluoromethyl)pentanamide); 55D1E1; dexamethasone ((8S,9R,10S,11S,13S,14S,16R,17R)-9-fluoro-11,17-dihydroxy-17-(2-hydroxyacetyl)-10,13,16-trimethyl-6,7,8,11,12,14,15,16-octahydrocyclopenta[a]phenanthren-3-one); prednisolone ((8S,9S,10R,11S,13S,14S,17R)-11,17-dihydroxy-17-(2-hydroxyacetyl)-10,13-dimethyl-7,8,9,11,12,14,15,16-octahydro-6H-cyclopenta[a]phenanthren-3-one); prednisone ((8S,9S,10R,13S,14S,17R)-17-hydroxy-17-(2-hydroxyacetyl)-10,13-dimethyl-6,7,8,9,12,14,15,16-octahydrocyclopenta[a]phenanthrene-3,11-dione); methylprednisolone ((6S,8S,9S,10R,11S,13S,14S,17R)-11,17-dihydroxy-17-(2-hydroxyacetyl)-6,10,13-trimethyl-7,8,9,11,12,14,15,16-octahydro-6H-cyclopenta[a]phenanthren-3-one); fluticasone propionate ([(6S,8S,9R,10S,11S,13S,14S,16R,17R)-6,9-difluoro-17-(fluoromethylsulfanylcarbonyl)-11-hydroxy-10,13,16-trimethyl-3-oxo-6,7,8,11,12,14,15,16-octahydrocyclopenta[a]phenanthren-17-yl] propanoate); beclomethasone-17-monopropionate ([(8S,9R,10S,11S,13S,14S,16S,17R)-9-chloro-11-hydroxy-17-(2-hydroxyacetyl)-10,13,16-trimethyl-3-oxo-6,7,8,11,12,14,15,16-octahydrocyclopenta[a]phenanthren-17-yl] propanoate); betamethasone ((8S,9R,10S,11S,13S,14S,16S,17R)-9-fluoro-11,17-dihydroxy-17-(2-hydroxyacetyl)-10,13,16-trimethyl-6,7,8,11,12,14,15,16-octahydrocyclopenta[a]phenanthren-3-one); rimexolone ((8S,9S,10R,11S,13S,14S,16R,17S)-11-hydroxy-10,13,16,17-tetramethyl-17-propanoyl-7,8,9,11,12,14,15,16-octahydro-6H-cyclopenta[a]phenanthren-3-one); paramethasone ((6S,8S,9S,10R,11S,13S,14S,16R,17R)-6-fluoro-11,17-dihydroxy-17-(2-hydroxyacetyl)-10,13,16-trimethyl-7,8,9,11,12,14,15,16-octahydro-6H-cyclopenta[a]phenanthren-3-one); and hydrocortisone ((8S,9S,10R,11S,13S,14S,17R)-11,17-dihydroxy-17-(2-hydroxyacetyl)-10,13-dimethyl-2,6,7,8,9,11,12,14,15,16-decahydro-1H-cyclopenta[a]phenanthren-3-one); 1,25-dihydroxyvitamin D3 (calcitriol) ((1R,3S,5Z)-5-[(2E)-2-[(1R,3aS,7aR)-1-[(2R)-6-hydroxy-6-methylheptan-2-yl]-7a-methyl-2,3,3a,5,6,7-hexahydro-1H-inden-4-ylidene]ethylidene]-4-methylidenecyclohexane-1,3-diol), paricalitol ((1R,3R)-5-[(2E)-2-[(1R,3aS,7aR)-1-[(E,2R,5S)-6-hydroxy-5,6-dimethylhept-3-en-2-yl]-7a-methyl-2,3,3a,5,6,7-hexahydro-1H-inden-4-ylidene]ethylidene]cyclohexane-1,3-diol), doxercalciferol ((1R,3S,5Z)-5-[(2E)-2-[(1R,3aS,7aR)-1-[(E,2R,5R)-5,6-dimethylhept-3-en-2-yl]-7a-methyl-2,3,3a,5,6,7-hexahydro-1H-inden-4-ylidene]ethylidene]-4-methylidenecyclohexane-1,3-diol), 25-hydroxyvitamin D3 (calcifediol) ((1S,3Z)-3-[(2E)-2-[(1R,3aS,7aR)-1-[(2R)-6-hydroxy-6-methylheptan-2-yl]-7a-methyl-2,3,3a,5,6,7-hexahydro-1H-inden-4-ylidene]ethylidene]-4-methylidenecyclohexan-1-ol), cholecalciferol ((1S,3Z)-3-[(2E)-2-[(1R,3aS,7aR)-7a-methyl-1-[(2R)-6-methylheptan-2-yl]-2,3,3a,5,6,7-hexahydro-1H-inden-4-ylidene]ethylidene]-4-methylidenecyclohexan-1-ol), ergocalciferol ((1S,3Z)-3-[(2E)-2-[(1R,3aS,7aR)-1-[(E,2R,5R)-5,6-dimethylhept-3-en-2-yl]-7a-methyl-2,3,3a,5,6,7-hexahydro-1H-inden-4-ylidene]ethylidene]-4-methylidenecyclohexan-1-ol), tacalcitol ((1R,3S,5Z)-5-[(2E)-2-[(1R,3aS,7aR)-1-[(2R,5R)-5-hydroxy-6-methylheptan-2-yl]-7a-methyl-2,3,3a,5,6,7-hexahydro-1H-inden-4-ylidene]ethylidene]-4-methylidenecyclohexane-1,3-diol), 22-dihydroergocalciferol ((1S,3Z)-3-[(2E)-2-[(1R,3aS,7aR)-1-[(2R,5S)-5,6-dimethylheptan-2-yl]-7a-methyl-2,3,3a,5,6,7-hexahydro-1H-inden-4-ylidene]ethylidene]-4-methylidenecyclohexan-1-ol), (6Z)-Tacalciol ((1S)-3-[(Z)-2-[(1R,7aR)-7a-methyl-1-[(2R)-6-methylheptan-2-yl]-1,2,3,3a,6,7-hexahydroinden-4-yl]ethenyl]-4-methylcyclohex-3-en-1-ol), 2-methylene-19-nor-20(S)-1α-hydroxy-bishomopregnacalciferol ((1R,3R)-5-[(2E)-2-[(1R,3aS, 7aR)-1-[(2S)-butan-2-yl]-7a-methyl-2,3,3a,5,6,7-hexahydro-1H-inden-4-ylidene]ethylidene]-2-methylidenecyclohexane-1,3-diol), 19-nor-26,27-dimethylene-20(S)-2-methylene-1α,25-dihydroxyvitamin D3, 2-methylene-1α,25-dihydroxy-(17E)-17(20)-dehydro-19-nor-vitamin D3, 2-methylene-19-nor-(24R)-1α,25-dihydroxyvitamin D2, 2-methylene-(20R,25S)-19,26-dinor-1α,25-dihydroxyvitamin D3, 2-methylene-19-nor-1α-hydroxy-pregnacalciferol, 1α-hydroxy-2-methylene-19-nor-homopregnacalciferol, (20R)-1α-hydroxy-2-methylene-19-nor-bishomopregnacalciferol, 2-methylene-19-nor-(20S)-1α-hydroxy-trishomopregnacalciferol, 2-methylene-23,23-difluoro-1α-hydroxy-19-nor-bishomopregnacalcifero-1, 2-methylene-(20S)-23,23-difluoro-1α-hydroxy-19-nor-bishomopregnan-calciferol, (2-(3' hydroxypropyl-1',2'-idene)-19,23,24-trinor-(20S)-1α-hydroxyvitamin D3, 2-methylene-18,19-dinor-(20S)-1α,25-dihydroxyvitamin D3, and the like.

Retinoic acid ((2E,4E,6E,8E)-3,7-dimethyl-9-(2,6,6-trimethylcyclohexen-1-yl)nona-2,4,6,8-tetraenoic acid), all-trans-retinoic acid ((2E,4E,6E,8E)-3,7-dimethyl-9-(2,6,6-trimethylcyclohexen-1-yl)nona-2,4,6,8-tetraenoic acid), 9-cis-retinoic acid ((2E,4E,6Z,8E)-3,7-dimethyl-9-(2,6,6-trimethylcyclohexen-1-yl)nona-2,4,6,8-tetraenoic acid), tamibarotene (4-[(5,5,8,8-tetramethyl-6,7-dihydronaphthalen-2-yl)carbamoyl]benzoic acid), 13-cis-retinoic acid ((2Z,4E,6E,8E)-3,7-dimethyl-9-(2,6,6-trimethylcyclohexen-1-yl)nona-2,4,6,8-tetraenoic acid), (2E,4E,6Z,8E)-3,7-dimethyl-9-(2,6,6-trimethyl-1-cyclohexeneyl)nona-2,4,6,-8-tetraenoic acid, 9-(4-methoxy-2,3,6-trimethyl-phenyl)-3,7-dimethyl-nona-2,4,6,8-tetraenoic acid, 6-[3-(1-adamantyl)-4-methoxyphenyl]-2-napthoic acid, 4-[1-(3,5,5,8,8-pentamethyl-tetralin-2-yl)ethenyl]benzoic acid, retinobenzoic acid (4-[(5,5,8,8-tetramethyl-6,7-dihydronaphthalen-2-yl)carbamoyl]benzoic acid), ethyl 6-[2-(4,4-dimethylthiochroman-6-yl)ethynyl]pyridine-3-carboxylate, retinoyl t-butyrate, retinoyl pinacol and retinoyl cholesterol. Obeticholic acid ((4R)-4-[(3R,5S,6R,7R,8S,9S,10S,13R,14S,17R)-6-ethyl-3,7-dihydroxy-10,13-dimethyl-2,3,4,5,6,7,8,9,11,12,14,15,16,17-tetradecahydro-1H-cyclopenta[a]phenanthren-17-yl]pentanoic acid), LY2562175 (6-(4-((5-cyclopropyl-3-(2,6-dichlorophenyl)isoxazol-4-yl)methoxy)piperidin-1-yl)-1-methyl-1H-indole-3-carboxylic acid), and GW4064 (3-[2-[2-Chloro-4-[[3-(2,6-dichlorophenyl)-5-(1-methylethyl)-4-isoxazolyl-]methoxy]phenyl]ethenyl]benzoic acid); T0901317 (N-(2,2,2-Trifluoroethyl)-N-[4-[2,2,2-trifluoro-1-hydroxy-1-(trifluoromethyl)ethyl]phenyl]benzenesulfonamide), GW3965 (3-[3-[[[2-Chloro-3-(trifluoromethyl)phenyl]methyl](2,2-diphenylethyl)amino]propoxy]benzeneacetic acid hydrochloride), and LXR-623 (2-[(2-chloro-4-fluorophenyl)methyl]-3-(4-fluorophenyl)-7-(trifluoromethyl)indazole); GNE-3500 (27, 1-{4-[3-fluoro-4-((3S,6R)-3-methyl-1,1-dioxo-6-phenyl-[1,2]thiazinan-2-yl-methyl)-phenyl]-piperazin-1-yl}-ethanone); 7beta, 27-dihydroxycholesterol ((3S,7R,8S,9S,10R,13R,14S,17R)-17-[(2R)-7-hydroxy-6-methylheptan-2-yl]-10, 13-dimethyl-2,3,4,7,8,9,11,12,14,15,16,17-dodecahydro-1H-cyclopenta[a]phenanthrene-3,7-diol), and 7alpha,27-dihydroxycholesterol((3S,7S,8S,9S,10R,13R,14S,17R)-17-[(2R)-7-hydroxy-6-methylheptan-2-yl]-10,13-dimethyl-2,3,4,7,8,9,11,12,14,15,16,17-dodecahydro-1H-cyclopenta[a]phenanthrene-3,7-diol); 9-cis retinoic acid ((2E,4E,6Z,8E)-3,7-dimethyl-9-(2,6,6-trimethylcyclohexen-1-yl)nona-2,4,6,8-tetraenoic acid), LGD100268 (6-[1-(3,5,5,8,8-pentamethyl-6,7-dihydronaphthalen-2-yl)cyclopropyl]pyridine-3-carboxylic acid), CD3254 (3-[4-Hydroxy-3-(5,6,7,8-tetrahydro-3,5,5,8,8-pentamethyl-2-naphthalenyl)-phenyl]-2-propenoic acid), and CD2915 (Sorensen et al. (1997) Skin Pharmacol. 10:144).

Where a pair of heterodimerization domains comprises an LBD of a mineralocorticoid receptor (MR) and a corresponding co-regulator peptide, a suitable regulating molecule includes spironolactone, and eplerenone. Spironolactone can be administered at a dose ranging from 10 to 35 mg per day, e.g., 25 mg per day.

Where a pair of heterodimerization domains comprises an LBD of an androgen receptor (AR) and a corresponding co-regulator peptide, a suitable regulating molecule includes cyproterone acetate, hydroxyflutamide, enzalutamide, ARN-509, 3,3'-diindolylmethane (DIM), bexlosteride, bicalutamide, N-butylbenzene-sulfonamide (NBBS), dutasteride, epristeride, finasteride, flutamide, izonsteride, ketoconazole, N-butylbenzene-sulfonamide, nilutamide, megestrol, a steroidal antiandrogen, and turosteride.

Where a pair of heterodimerization domains comprises an LBD of a progesterone receptor (PR) and a corresponding co-regulator peptide, a suitable regulating molecule includes mifepristone (RU-486; 11beta-[4N,N-dimethylaminophenyl]-17beta-hydroxy-17-(1-propynyl)-estra-4,9-dien-3-one); Lilopristone (11beta-(4N,N-dimethylaminophenyl)-17beta-hydroxy-17-((Z)-3-hydroxypropenyl)estra-4,9-dien-3-one); onapristone (11beta-(4 N,N-dimethylaminophenyl)-17alpha-hydroxy-17-(3-hydroxypropyl)-13alpha-estra-4,9-dien-3-one); asoprisnil (benzaldehyde, 4-[(11beta,17beta)-17-methoxy-17-(methoxymethyl)-3-oxoestra-4,9-dien-11-yl]-1-(E)-oxim; J867); J912 (4-[17beta-Hydroxy-17alpha-(methoxymethyl)-3-oxoestra-4,9-dien-11beta-yl]benzaldehyd-(1E)-oxim); and CDB-2914 (17alpha-acetoxy-11beta-(4-N,N-dimethylaminophenyl)-19-norpregna-4,9-dien-3,20-dione). Other suitable dimerization agents include, e.g., JNJ-1250132, (6alpha,11beta,17beta)-11-(4-dimethylaminophenyl)-6-methyl-4',5'-di-hydrospiro[estra-4,9-diene-17,2' (3'H)-furan]-3-one (ORG-31710); (11beta,17alpha)-11-(4-acetylphenyl)-17,23-epoxy-19,24-dinorchola-4,9-,20-trien-3-one (ORG-33628); (7beta,11beta,17beta)-11-(4-dimethylaminophenyl-7-methyl]-4',5'-dihydrospiro[estra-4,9-diene-17,2'(3'H)-furan]-3-one (ORG-31806); ZK-112993; ORG-31376; ORG-33245; ORG-31167; ORG-31343; RU-2992; RU-1479; RU-25056; RU-49295; RU-46556; RU-26819; LG1127; LG120753; LG120830; LG1447; LG121046; CGP-19984A; RTI-3021-012; RTI-3021-022; RTI-3021-020; RWJ-25333; ZK-136796; ZK-114043; ZK-230211; ZK-136798; ZK-98229; ZK-98734; ZK-137316; 4-[17beta-Methoxy-17alpha-(methoxymethyl)-3-oxoestra-4,9-dien-11beta-yl]benzaldehyde-1-(E)-oxime; 4-[17beta-Methoxy-17alpha-(methoxymethyl)-3-oxoestra-4,9-dien-11beta-yl]benzaldehyde-1-(E)-[O-(ethylamino)carbonyl]oxime; 4-[17beta-Methoxy-17alpha-(methoxymethyl)-3-oxoestra-4,9-dien-11beta-yl]benzaldehyde-1-(E)-[O-(ethylthio)carbonyl]oxime; (Z)-6'-(4-cyanophenyl)-9,11alpha-dihydro-17beta-hydroxy-17alpha-[4-(1-oxo-3-methylbutoxy)-1-butenyl]4'H-naphtho[3',2',1'; 10,9,11]estr-4-en-3-one; 11beta-(4-acetylphenyl)-17beta-hydroxy-17alpha-(1,1,2,2,2-penta-fluoroethyl)estra-4,9-dien-3-one; 11beta-(4-Acetylphenyl)-19,24-dinor-17,23-epoxy-17alpha-chola-4,9,20-trien-3-one; (Z)-11beta,19-[4-(3-Pyridinyl)-o-phenylene]-17beta-hydroxy-17alpha-[3-hydroxy-1-propenyl]-4-androsten-3-one; 11beta-[4-(1-methylethenyl)phenyl]-17alpha-hydroxy-17beta-beta-hydroxypropyl)-13alpha-estra-4,9-dien-3-one; 4',5'-Dihydro-11beta-[4-(dimethylamino)phenyl]-6beta-methylspiro[estra-4,-9-dien-17beta,2'(3'H)-furan]-3-one, and drospirenone.

Where a pair of heterodimerization domains comprises an LBD of thyroid receptor-beta (TR-beta) and a corresponding co-regulator peptide, a suitable regulating molecule includes T3 (3,5,3'-triiodo-L-thyronine); KB-141 (3,5-dichloro-4-(4-hydroxy-3-isopropylphenoxy)phenylacetic acid); sobetirome (also known as GC-1) (3,5-dimethyl-4-(4'-hydroxy-3'-isopropylbenzyl)-phenoxy acetic acid); GC-24 (3,5-dimethyl-4-(4'-hydroxy-3'-benzyl)benzylphenoxyacetic acid); 4-OH-PCB106 (4-OH-2',3,3',4',5'-pentachlorobiphenyl); eprotirome; MB07811 ((2R,4S)-4-(3-chlorophenyl)-2-[(3,5-dimethyl-4-(4'-hydroxy-3'-isopropylbenzyl)phenoxy)methyl]-2-oxido-[1,3,2]-dioxaphosphonane); QH2; and (3,5-dimethyl-4-(4'-hydroxy-3'-isopropylbenzyl)phenoxy)methylphosphonic acid (MB07344).

Where a pair of heterodimerization domains comprises an LBD of estrogen receptor-alpha (ER-alpha) and a corresponding co-regulator peptide, a suitable regulating molecule includes tamoxifen, 4-OH-tamoxifen, raloxifene, lasofoxifene, bazedoxifene, falsodex, clomifene, femarelle, ormeloxifene, toremifiene, ospemifene, and ethinyl estradiol.

Where a pair of heterodimerization domains comprises an LBD of estrogen receptor-beta (ER-beta) and a corresponding co-regulator peptide, a suitable regulating molecule includes estradiol (E2; or 17-beta-estradiol), and ethinyl estradiol.

Where a pair of heterodimerization domains comprises an LBD of PPAR-gamma and a corresponding co-regulator peptide, a suitable regulating molecule includes a thiazolidinedione (e.g., rosiglitazone, pioglitazone, lobeglitazone, troglitazone), farglitazar, aleglitazar, and fenofibric acid.

Where a pair of heterodimerization domains comprises an LBD of a GR and a corresponding co-regulator peptide, a suitable regulating molecule can be a selective GR agonist (SEGRA) or a selective GR modulator (SEGRM).

Where a pair of heterodimerization domains comprises an LBD of a GR and a corresponding co-regulator peptide, a suitable regulating molecule includes benzopyranoquinoline A 276575, Mapracorat, ZK 216348, 55D1E1, dexamethasone, prednisolone, prednisone, methylprednisolone, fluticasone propionate, beclomethasone-17-monopropionate, betamethasone, rimexolone, paramethasone, and hydrocortisone.

Where a pair of heterodimerization domains comprises an LBD of a VDR and a corresponding co-regulator peptide, a suitable regulating molecule can be 1,25-dihydroxyvitamin D3 (calcitriol), paricalitol, doxercalciferol, 25-hydroxyvitamin D3 (calcifediol), cholecalciferol, ergocalciferol, tacalciol, 22-dihydroergocalciferol, (6Z)-Tacalciol, 2-methylene-19-nor-20(S)-1alpha-hydroxy-bishomopregnacalciferol, 19-nor-26,27-dimethylene-20(S)-2-methylene-1alpha,25-dihydroxyvitamin D3, 2-methylene-1alpha,25-dihydroxy-(17E)-17(20)-dehydro-19-nor-vitamin D3, 2-methylene-19-nor-(24R)-1alpha,25-dihydroxyvitamin D2, 2-methylene-(20R,25S)-19,26-dinor-1alpha,25-dihydroxyvitamin D3, 2-methylene-19-nor-1alpha-hydroxy-pregnacalciferol, 1alpha-hydroxy-2-methylene-19-nor-homopregnacalciferol, (20R)-1alpha-hydroxy-2-methylene-19-nor-bishomopregnacalciferol, 2-methylene-19-nor-(20S)-1alpha-hydroxy-trishomopregnacalciferol, 2-methylene-23,23-difluoro-1alpha-hydroxy-19-nor-bishomopregnacalcifero-1, 2-methylene-(20S)-23,23-difluoro-1alpha-hydroxy-19-nor-bishomopregna-n-calciferol, (2-(3' hydroxypropyl-1',2'-idene)-19,23,24-trinor-(20S)-1alpha-hydroxyvitamin D3, 2-methylene-18,19-dinor-(20S)-1alpha,25-dihydroxyvitamin D3, and the like.

Where a pair of heterodimerization domains comprises an LBD of a RAR-beta and a corresponding co-regulator peptide, a suitable regulating molecule can be retinoic acid, all-trans-retinoic acid, 9-cis-retinoic acid, tamibarotene, 13-cis-retinoic acid, (2E,4E,6Z,8E)-3,7-dimethyl-9-(2,6,6-trimethyl-1-cyclohexeneyl)nona-2,4,6,-8-tetraenoic acid, 9-(4-methoxy-2,3,6-trimethyl-phenyl)-3,7-dimethyl-nona-2,4,6,8-tetraenoic acid, 6-[3-(1-adamantyl)-4-methoxyphenyl]-2-napthoic acid, 4-[1-(3,5,5,8,8-pentamethyl-tetralin-2-yl)ethenyl]benzoic acid, retinobenzoic acid, ethyl 6-[2-(4,4-dimethylthiochroman-6-yl)ethynyl]pyridine-3-carboxylate, retinoyl t-butyrate, retinoyl pinacol, and retinoyl cholesterol.

Where a pair of heterodimerization domains comprises an FXR and a corresponding co-regulator peptide, a suitable regulating molecule includes obeticholic acid, LY2562175 (6-(4-((5-cyclopropyl-3-(2,6-dichlorophenyl)isoxazol-4-yl)methoxy)piperidin-1-yl)-1-methyl-1H-indole-3-carboxylic acid), and GW4064 (3-[2-[2-Chloro-4-[[3-(2,6-dichlorophenyl)-5-(1-methylethyl)-4-isoxazolyl-]methoxy]phenyl]ethenyl]benzoic acid).

Where a pair of heterodimerization domains comprises an LBD of LXR-alpha and a corresponding co-regulator peptide, a suitable regulating molecule includes T0901317 (N-(2,2,2-Trifluoroethyl)-N-[4-[2,2,2-trifluoro-1-hydroxy-1-(trifluoromethyl)ethyl]phenyl]benzenesulfonamide), GW3965 (3-[3-[[[2-Chloro-3-(trifluoromethyl)phenyl]methyl](2,2-diphenylethyl)amino]propoxy]benzeneacetic acid hydrochloride), and LXR-623 (2-[(2-chloro-4-fluorophenyl)methyl]-3-(4-fluorophenyl)-7-(trifluoromethyl)indazole).

Where a pair of heterodimerization domains comprises an LBD of an ROR-gamma and a corresponding co-regulator peptide, a suitable regulating molecule includes GNE-3500 (27, 1-{4-[3-fluoro-4-((3S,6R)-3-methyl-1,1-dioxo-6-phenyl-[1,2]thiazinan-2-yl-methyl)-phenyl]-piperazin-1-yl}-ethanone).

Where a pair of heterodimerization domains comprises an LBD of an ROR-gamma and a corresponding co-regulator peptide, a suitable regulating molecule includes 7beta, 27-dihydroxycholesterol, and 7alpha, 27-dihydroxycholesterol.

Where a pair of heterodimerization domains comprises an LBD of an RXR-alpha and a corresponding co-regulator peptide, a suitable regulating molecule includes 9-cis retinoic acid, LGD100268, CD3254 (3-[4-Hydroxy-3-(5,6,7,8-tetrahydro-3,5,5,8,8-pentamethyl-2-naphthalenyl)-phenyl]-2-propenoic acid), and CD2915 (Sorensen et al. (1997) Skin Pharmacol. 10:144).

Where a pair of heterodimerization domains comprises an LBD of a PXR and a corresponding co-regulator peptide, a suitable regulating molecule can be rifampicin, chlotrimazole, and lovastatin.

9.2.2. Conditional heterodimerization of CAR molecules based on lipocalin-fold molecules:

In other preferred embodiments at least two CAR molecules of a group of CARs according to the present invention can be heterodimerized by a pair of heterodimerization domains comprising one member which is a lipocalin-fold molecule and a second member which is a lipocalin-fold binding interaction partner as disclosed in EP17208924.5 filed on 20 December 2017. According to a preferred embodiment, a lipocalin-fold based heterodimerization system comprises:

(a) a lipocalin-fold molecule
(b) a lipocalin-fold ligand with a low molecular weight of 1500 Da or below, and
(c) a lipocalin-fold binding interaction partner,

wherein the lipocalin-fold molecule can bind to the lipocalin-fold ligand; and
wherein the lipocalin-fold molecule bound to the lipocalin-fold ligand binds to the lipocalin-fold binding interaction partner with an affinity which is at least 10-fold higher than the affinity of the lipocalin-fold molecule not bound to the lipocalin-fold ligand,
and wherein the lipocalin-fold binding interaction partner is not a naturally occurring protein which has an affinity of <10 μM to any naturally occurring lipocalin-fold molecule in the presence of any lipocalin-fold ligand.

According to a further preferred embodiment, a lipocalin-fold based heterodimerization system comprises:

(a) a lipocalin-fold molecule
(b) a lipocalin-fold ligand with a low molecular weight of 1500 Da or below, and
(c) a lipocalin-fold binding interaction partner,

wherein the lipocalin-fold molecule has at least a first conformation when the lipocalin-fold ligand is not bound to the lipocalin-fold molecule and at least a second conformation when the lipocalin-fold ligand is bound to the lipocalin-fold molecule; and
wherein the lipocalin-fold molecule bound to the lipocalin-fold ligand in the second conformation binds to the lipocalin-fold binding interaction partner with an affinity which is at least 10-fold higher than the affinity of the lipocalin-fold molecule not bound to the lipocalin-fold ligand in the first conformation,
and wherein the lipocalin-fold binding interaction partner is not a naturally occurring protein which has an affinity of <10 μM to any naturally occurring lipocalin-fold molecule in the presence of any lipocalin-fold ligand.

This lipocalin-fold molecule based system for conditional heterodimerization generally relies on a substantial difference in the affinities of the lipocalin-fold molecule to the lipocalin-fold binding interaction partner depending on whether the lipocalin-fold ligand is bound or not. It is preferred that the affinity window (i.e. the affinities of the lipocalin-fold binding interaction partner to the lipocalin-fold molecule bound or not bound to the lipocalin-fold ligand, respectively) is present in a reasonable affinity range which allows for regulation of heterodimerization under physiological conditions. Therefore, it is preferred that the affinity of the lipocalin-fold binding interaction partner to the lipocalin-fold molecule in the ligand-bound state is below 10 μM, preferably below 2 μM, especially below 400 nM.

Depending on whether a lipocalin-fold binding interaction partner is engineered for binding to a lipocalin-fold molecule charged with a lipocalin-fold ligand or not charged with a lipocalin-fold ligand a lipocalin-fold molecule based system can be used for conditional heterodimerization (i.e., for on-switching) or for constitutive heterodimerization, respectively. Since a lipocalin-fold binding interaction partner can also be engineered for binding to a lipocalin-fold molecule in the absence but not in the presence of a lipocalin-fold ligand, the system can also be used for conditionally preventing heterodimerization (i.e., for off-switching). In principle, a lipocalin-fold molecule based system can optionally also be engineered for binding at least two different lipocalin-fold ligands, wherein an accordingly selected lipocalin-fold binding interaction partner can distinguish between the two differentially induced conformational states which then allows for conditional on- and off-switching by sequentially adding the two different lipocalin-fold ligands.

A lipocalin-fold molecule, which can be used as a heterodimerization domain according to the present invention, may be any protein that contains the structural motif of a lipocalin-fold to which (or in which) the lipocalin-fold ligand binds and which enables binding of the lipocalin-fold molecule to the lipocalin-fold binding interaction partner.

A lipocalin-fold molecule is defined as any naturally occurring molecule classified into the lipocalin superfamily in the SCOP database (version 1.75), or a mutant thereof. However, it is preferred to exchange only a limited number of amino acids.

According to a preferred embodiment, the lipocalin-fold molecule is a molecule identical with a naturally occurring iLBP (intracellular lipid binding protein), a naturally occurring lipocalin or an anticalin, and derivatives of any of these molecules with 1-30 amino acid exchanges and fragments thereof. In another preferred embodiment, the lipocalin-fold molecule is a derivative of a naturally occurring lipocalin or iLBP with at least one, two, three, four, five, six, seven, eight, nine, ten, 25 or 30 amino acid exchanges.

According to a preferred embodiment of the present invention, the lipocalin-fold molecule is engineered by one or more amino acid exchanges, insertions and/or deletions to optimize lipocalin-fold ligand binding. According to a preferred embodiment, the lipocalin-fold molecule is a derivative of a naturally occurring or otherwise disclosed (by its amino acid sequence) lipocalin-fold molecule with at least 70%, preferably at least 80%, especially at least 90% sequence identity in the β-barrel structure, whereby this β-barrel structure is defined as the regions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;
- amino acid residues 14-23, 37-43, 48-54, 62-69, 76-79, 84-91, 96-102 and 111-117 in human tear lipocalin (TLC;

as defined by Schiefner et al., Acc Chem Res. 2015;48(4):976-985), which define the structurally conserved β-strands in human TLC;

- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;
- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;
- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1;

According to a preferred embodiment, the lipocalin-fold molecule is a fragment of a naturally occurring lipocalin or a derivative thereof with a length of at least 80, preferably at least 100, especially at least 120, amino acids covering at least the structurally conserved β-barrel structure of the lipocalin-fold, or wherein the lipocalin-fold molecule is a fragment of a naturally occurring iLBP or a derivative thereof with a length of at least 80, preferably at least 85, especially at least 90, amino acids covering at least the structurally conserved β-barrel structure of the lipocalin-fold, wherein the structurally conserved β-barrel structure comprises or consists of amino acid positions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;
- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;
- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;
- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;
- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1; according to the amino acid residue numbering scheme in PDB entry 2F73).

According to a further preferred embodiment, the lipocalin-fold molecule is a derivative of a naturally occurring lipocalin or iLBP with up to 15, up to 30, or up to 50 amino acid deletions and/or up to 15, up to 30, or up to 50 amino acid insertions outside of the structurally conserved β-barrel structure, preferably corresponding structurally to the regions of amino acid residues selected from

- amino acid residues 1-20, 31-40, 48-51, 59-70, 79-84, 89-101, 110-113, 121-131 and 139-183 in human RBP4, which define the regions adjoining the structurally conserved β-strands in human RBP4 according to the amino acid residue numbering scheme in the PDB entry 1RBP;
- amino acid residues 1-13, 24-36, 44-47, 55-61, 70-75, 80-83, 92-95, 103-110 and 118-158 in human TLC (according to the amino acid residue numbering scheme in Schiefner et al., Acc Chem Res. 2015;48(4):976-985), which define the regions adjoining the structurally conserved β-strands in human TLC;
- amino acid residues 1-43, 54-68, 76-80, 88-95, 104-109, 114-118, 127-130, 138-141 and 149-188 in human ApoM (according to the amino acid residue numbering scheme in Schiefner et al., Acc Chem Res. 2015;48(4):976-985), which define the regions adjoining the structurally conserved β-strands in human ApoM;
- amino acid residues 1-4, 13-40, 46-49, 55-60, 66-70, 74-80, 88-92, 97-107, 113-118, 125-128 and 136-137 in human CRABPII (according to the amino acid residue numbering scheme in PDB entry 2FS6), which define the regions adjoining the structurally conserved β-strands in human CRABPII;
- amino acid residues 1-4, 13-38, 44-47, 53-58, 64-68, 72-78, 86-90, 95-98, 104-108, 115-118 and 126-127 in human FABP1 (according to the amino acid residue numbering scheme in PDB entry 2F73), which define the regions adjoining the structurally conserved β-strands in human FABP1.

In another preferred embodiment, the lipocalin-fold molecule is a derivative of a naturally occurring member of the lipocalin superfamily with at least one, two, three, four, five, six, seven, eight, nine, ten, eleven, twelve, 13, 14, 15, 16, 17, 18, 19, 20, 21, 22, 23, 24, 25, 26, 27, 28, 29, or 30 amino acid exchanges.

According to a further preferred embodiment, the lipocalin-fold molecule used as heterodimerization domain according to the present invention is a lipocalin, i.e., a protein containing an eight-stranded up-and-down β-barrel arranged in a +1 topology, followed by an α-helix after the C-terminal end of the eighth β-strand.

The lipocalin-fold ligand, which can be used as a regulating molecule according to the present invention, is a "small molecule", e.g. "small" compared to polypeptides and proteins, such as the lipocalin-fold molecule. Accordingly, the lipocalin-fold ligand has a molecular weight of 1500 Da or less, preferably 1000 Da or less, especially 750 Da or less. Preferred Mw ranges of the lipocalin-fold ligand are 50 to 1500 Da, preferably 75 to 1500 Da, especially 150 to 750 Da. Preferably, the lipocalin-fold ligand can bind in the calyx of the lipocalin-fold molecule formed by the barrel and the loop regions of the lipocalin-fold structure.

It is preferred that the lipocalin-fold ligand has an affinity to the lipocalin-fold molecule of below 1 mM, preferably of below 100 μM, especially of below 10 μM. This affinity between the lipocalin-fold ligand and the lipocalin-fold molecule is defined as a $K_d$ (dissociation constant) value and preferably determined by isothermal titration calorimetry (ITC) using an automated MicroCal PEAQ-ITC instrument (Malvern Instruments) .

Examples from which lipocalin-fold ligands can be selected are:

| Nr | database HMDB |
|---|---|
| 1 | Nafcillin |
| 2 | Gerberinol |
| 3 | Montelukast |
| 4 | Flurazepam |
| 5 | Quinidine barbiturate |
| 6 | Glyceollin I |
| 7 | Glyceollin II |
| 8 | (-)-Shinpterocarpin |
| 9 | Kanzonol W |
| 10 | 6alpha-Hydroxyphaseollin |
| 11 | (1a,5b,6a)-7-Protoilludene-1,5,6,14-tetrol 14-(2,4-dihydroxy-6-methylbenzoic acid) |
| 12 | 4'-O-Methylkanzonol W |
| 13 | Cyclokievitone |
| 14 | Licofuranone |
| 15 | Armillatin |
| 16 | 2-(4-Methyl-3-pentenyl)anthraquinone |
| 17 | Sorafenib beta-D-Glucuronide |
| 18 | Heterophyllin |
| 19 | 2'-O-Methylphaseollinisoflavan |
| 20 | Tiapride |
| 21 | Gluten exorphin C |
| 22 | Mulberrofuran M |
| 23 | Dulxanthone G |
| 24 | Sclareol |
| 25 | Colupdox a |
| 26 | Kanzonol F |
| 27 | Mangostinone |
| 28 | Gancaonin X |
| 29 | Rubraflavone D |
| 30 | Cyclokievitone hydrate |
| 31 | Glyceollidin II |
| 32 | Cyclandelate |
| 33 | Dulxanthone E |
| 34 | Morusin |
| 35 | (E)-2',4,4'-Trihydroxy-3-prenylchalcone |
| 36 | Dulxanthone H |
| 37 | Judeol |
| 38 | Artonin E |
| 39 | Kanzonol T |
| 40 | Fragransol A |
| 41 | Dulxanthone F |
| 42 | Mulberrofuran T |
| 43 | Garcimangosone A |
| 44 | Artonin B |
| 45 | Asteltoxin |
| | **database KEGG** |
| 46 | Oxyfedrine |
| 47 | Profluthrin |
| 48 | Momfluorothrin |
| 49 | Xyloylsulfamine |
| 50 | Cetotiamine hydrochloride hydrate |
| 51 | Dicethiamine hydrochloride hydrate |
| 52 | Dicetamin |
| 53 | Oxolamine |
| 54 | Oksalamin |
| 55 | Crisnatol mesylate |
| 56 | Ioflubenzamide I |
| 57 | Ceritinib |
| 58 | Zykadia |
| 59 | Imiprothrin |
| 60 | Triclabendazole |
| 61 | Fasinex |
| 62 | Brivanib alaninate |
| 63 | Transfluthrin |
| 64 | Enolicam sodium |
| 65 | Enolicam sodium monohydrate |
| 66 | Dibucaine |
| 67 | Cinchocaine |
| 68 | Nupercaine |
| 69 | Trametinib |
| | **database WDI** |
| 70 | FLUCLOXACILLIN |
| 71 | S-FARNESYLTHIOSALICYLIC ACID |
| 72 | 2-FLUOROTROPAPRIDE |
| 73 | BUTAMPICILLIN |
| 74 | DICLOXACILLIN SULFATE |
| 75 | FUROXACILLIN |
| 76 | IBUCILLIN |
| 77 | PRAZOCILLIN |
| 78 | SALETAMIDE |
| 79 | TETRACHLORSALICYLANILIDE |
| 80 | CARBENICILLIN |
| 81 | DICLOMETIDE |
| 82 | METHYL-SULFOMETURON |
| 83 | TRICHLOROSALICYLANILIDE |
| 84 | CLOMETOCILLIN |
| 85 | CYSTODYTIN-F |
| 86 | DETANOSAL |

87 DIARBARONE
88 DICLOFOP
89 EPIPHENETHICILLIN
90 ETALIL-MEDEYOL
91 SALETAMIDE HYDROCHLORIDE
92 TIAPRIDE HYDROCHLORIDE
93 TRUNCULIN-A
94 DEOXYPENTALENYLGLUCURON
95 LAFLUNIMUS
96 MERAZOLAM
97 DIBUSADOL
98 PHENETICILLIN POTASSIUM
99 PRANOSAL
100 DALEFORMIS
101 DIPHENICILLIN
102 FENOTEROL HYDROCHLORIDE
103 GIGANTIC-ACID
104 HALOLITORALIN-B
105 ISOPROPICILLIN
106 PROGUANIL HYDROCHLORIDE
107 PYRANOKUNTHONE-B
108 TUBEROSIN
109 ZOPFIELLAMIDE-B
110 CLOXACILLIN SODIUM
111 LETIMIDE HYDROCHLORIDE
112 BAIGENE-B
113 BIDWILLON-B
114 CARBENICILLIN DISODIUM
115 HYDROXYPROCAINE
116 OCHRATOXIN-A
117 THEROX
118 MACARANGAFLAVANONE-B
119 MENOXYMYCIN-B
120 PENICILLIN-S
121 PSORALIDIN
122 RUBIGINONE-C1
123 SECOPSEUDOPTEROSIN-E
124 ASADISULFIDE
125 BARANGCADOIC-ACID-A
126 BEPHEDON
127 MELLEDONAL-B
128 NERAMINOL
129 PHOMOPSOLIDE-A
130 ROBUSTIC-ACID
131 ZOPFIELLAMIDE-A
132 CYSTODYTIN-B
133 DICLOXACILLIN
134 FLUOROPROPRANOLOL
135 ILICICOLIN-B
136 INDICANINE-B
137 JACAREUBIN
138 KOTTAMIDE-C
139 MEXOLAMINE
140 MYCAPEROXIDE-H
141 OTOGIRIN
142 OXOLAMINE HYDROCHLORIDE
143 OXOPROPALINE-A
144 PURVALANOL-A
145 RUBIGINONE-C2
146 TERACRYLSHIKONIN
147 CLODINAFOP-PROPARGYL-ESTER
148 MAZATICOL
149 SETHOXYDIM
150 SULFAGUANOLE
151 BALAPERIDONE
152 FLUCLOXACILLIN SODIUM
153 GEODIAMOLIDE-TA
154 LUCANTHONE-SULFOXIDE
155 MELLEOLIDE-D
156 NADOXOLOL HYDROCHLORIDE
157 BECLOBRIC-ACID-GLUCURONIDE
158 CLOXACILLIN
159 HYPERGUINONE-B
160 OLIGOSPOROL-A
161 PROPOXYCAINE HYDROCHLORIDE
162 RONIFIBRATE
163 SUDAN-BLUE-GN
164 TRICHODERMAMIDE-B
165 BOTRYLLAMIDE-A
166 CARFECILLIN
167 CLETHODIM
168 DUTADRUPINE
169 EPICOCHLIOQUINONE-B-14
170 FLURAZEPAM
171 HYDROXYFLUCLOXACILLIN
172 RHINACANTHIN-C
173 TEFLUBENZURON
174 XENYSALATE
175 ANTIMYCIN-A8A
176 ARTOINDONESIANIN-U
177 BRONCHOCAINE
178 ENOLICAM
179 IPAZILIDE
180 MORDANT-BROWN-1
181 PROPRANOLOL PHENOBARBITAL
182 PUGHIININ-A
183 AMPHIBINE-H
184 ARMILLARIC-ACID
185 CARINDACILLIN
186 CHLOROBIOCATE
187 CHLORPROGUANIL
188 CYLINDROL-B
189 ECLIPTALBINE
190 CARCICERRIN-A
191 O-DEMETHYLCHLOROTHRICIN
192 SANGGENON-C
193 TETRAPTEROL-G
194 CHLORSULFURON
195 DEXAMETHASONE-DIETHYLAMINOACETATE
196 DIETHYXIME
197 PYRIDOVERICIN
198 SUBENDAZOLE
199 THIOCAINE
200 TRAPEZIFOLIXANTHONE
201 TRICLAZAN
202 3',4'-DICHLOROBENZAMIL
203 CHAETOVIRIDIN-C
204 CYCLOGREGATIN
205 FLURAZEPAM MONOHYDROCHLORIDE
206 FUSIDILACTONE-B
207 GRISEOCHELIN-METHYL-ESTER
208 ISOBUTYL-SHIKONIN
209 MICINICATE
210 AJUDAZOL-B
211 CYSTODYTIN-E
212 DEMETHYLPRAECANSONE-A
213 DESTRUXIN-A
214 DIFENIDOL EMBONATE
215 DISCOKIOLIDE-B
216 IRUMANOLIDE-2
217 LACTOQUINOMYCIN
218 NEOBORNYVAL
219 SAROTHRALEN-D
220 ABYSSINONE-V
221 AXITIROME
222 CHLORFLUAZURON
223 CHONDRILLIDIENE-18,20
224 EUGLOBAL-G2
225 IDARUBICIN HYDROCHLORIDE
226 MUTISICOUMARANONE-A
227 3-O-METHYLCALOPOCARPIN
228 ALLOCLAMIDE HYDROCHLORIDE
229 ANOPTERINE
230 DIOXATION
231 EUGLOBAL-IIC
232 ILICICOLIN-C

233 MYCINOLIDE-II
234 SUILLIN
235 TOCOTRIENOL-GAMMA
236 INDOMYCINONE-BETA
237 ISOTHIORBAMINE
238 MEBEVERINE
239 MUTISICOUMARANONE-D
240 NYMPHAEOL-C
241 PRUSOCAIN
242 ZIMET-20-84
243 2,4-D-BUTOXYPROPYL
244 ABYSSINONE-IV
245 BAIGENE-A
246 CHRYSOCHLAMIC-ACID
247 EPOLONE-B
248 ETHOXYCAINE
249 FLUORENAMIL
250 GUAIACOL-MEFENAMATE
251 MAXIMA-ISOFLAVONE-C
252 SARCODICTYIN-B
253 TRICLABENDAZOLE
254 ACHYOFURAN
255 ASTERRIQUINONE
256 DIETHYLGLYCOLATE-TOLYHYDRAZIDE
257 MALLOTOPHILIPPENS-D
258 NAFTOXATE
259 PACHYDICTYOL-A-EPOXIDE
260 RATJADONE
261 SALVERINE HYDROCHLORIDE
262 4-MENAHYDROQUINONE
263 BOTRYLLAMIDE-C
264 ELSAMICIN
265 FENFLUTHRIN
266 MUTISIPHENONE-A
267 SANGGENON-A
268 SCHWEINFURTHIN-A
269 VANYLIDILOL
270 4-O-METHYLMELLECLIDE
271 ACETYLVISMIONE-F
272 ALFENTANIL-HYDROCHLORIDE
273 ASPERTETRONIN-A
274 BETA-HYDROXYISOVALERYLSHIKONIN
275 CARBISOCAINE
276 CHLORPROGUANIL HYDROCHLORIDE
277 CRYPTOPHYCIN-52
278 DIDEMETHYL-TOCOTRIENOL
279 FLUOSOL-DA
280 PYRIDOXYPHEN
281 TIAZESIM HYDROCHLORIDE
282 BUTOCTAMIDE
283 DEOXYSHIKONIN
284 GAMBIERIC-ACID-A
285 LICOFURANONE
286 PREDNYLIDENE-DIETHYLAMINOACETATE
287 PSEUDOPTEROSIN-G
288 TRIKENDIOL
289 ZOAPATANOL
290 ERGOKININ-C
291 PENTAMOXANE HYDROCHLORIDE
292 SCANDENIN
293 ACTINOPYRONE-C
294 AMITON
295 CRATOXYARBORENONE-C
296 CYMOPOL
297 DOXYCYCLINE-HYCLATE
298 FLAVIDULOL-C
299 FRAN-12
300 MYRIAPORONE-3
301 ORINOCINOLIDE
302 TONABERSAT
303 VISMIONE-B
304 AMIKHELLINE
305 BUTAMOXANE HYDROCHLORIDE
306 CHLOROBIOCIN
307 CYCLOCOMMUNIN
308 FENAZAFLOR
309 VISMIONE-D
310 3-TRICHLORMETAPHOS
311 AMINOPROPYLONE PHENYLBUTAZONE
312 DIOCLENOL
313 GRIFOLIN
314 HYPERJOVINOL-A
315 TAMOLARIZINE
316 TOMENTOL
317 TRANS-DELTA-TOCOTRIENOLOIC-ACID
318 DIACETOLOL HYDROCHLORIDE
319 DUTOMYCIN
320 LIGUROBUSTOSIDE-O
321 RAISPAILOL-B
322 ERECTQUIONE-A
323 SAROTHRALEN-A
324 SITAMAQUINE
325 TRICHOPOLYN-II
326 3-HYDROXYTOLUFAZEPAM
327 DIMETPRAMIDE
328 FENOTEROL HYDROBROMIDE
329 PHENKAPTON
330 CRATOXYARBORENONE-B
331 ETHOMOXANE HYDROCHLORIDE
332 ISOCENTRATHERIN
333 KALIMANTACIN-A
334 HISPAGLABRIDIN-A
335 LANKACYCLINOL
336 MACLURAXANTHONE
337 PIVAMPICILLIN HYDROCHLORIDE
338 PLURAFLAVIN-A
339 SIGMOIDIN-I
340 FENALAMIDE
341 HYDROXYACLACINOMYCIN-M,2
342 SOPHORADIN
343 ASCOCHLORIN
344 BETA-HYDROXYSANSHOOL
345 BISTRAMIDE-K
346 CHLORTETRACYCLINE BORATE
347 DEHYDROASCOCHLORIN-8+,9+
348 DIMETHIALIUM CHLORIDE
349 MACARANGIN
350 MARCELLOMYCIN
351 BENZOYLGOMISIN-H
352 CLINDAMYCIN HYDROCHLORIDE
353 HYDROXYASCOCHLORIN-8+
354 NEOCARZILIN-A
355 CHAETOVIRIDIN-B
356 CHLORTETRACYCLINE DISULFATE
357 NAPYRADIOMYCIN-C1
358 SAROASPIDIN-B
359 SARRACINE
360 ERECTONE-B
361 HOMOHARZIANIC-ACID
362 ASTERRIQUINONE-B1
363 DETAMID
364 RUBRAXANTHONE
365 DOLASTATIN-19
366 EDETOL
367 PHASEOLLIDIN
368 GEDOCARNIL
369 MANUMYCIN-B
370 SANTALOL-BETA-

SALICYLATE
371 COWANIN
372 INDIBULIN
373 COWANOL
374 KARATAVICIN
375 PICLOXYDINE
376 PROXAZOLE
377 STROBILURIN-E
378 ERECTQUIONE-B
379 SURICAINIDE
380 DIETHYLAMINOMETHYLRUTIN
381 TROPESIN
382 PICLOXYDINE HYDROCHLORIDE
383 HEX-1
384 DECLOVANILLOBIOCIN

**database MDDR**

385 RUBIGINONE C2
386 FLOBUFEN
387 TETRONOTHIODIN
388 LAFLUNIMUS
389 MYCAPEROXIDE A
390 FLURAZEPAM HYDROCHLORIDE
391 RUBIGINONE C1
392 CRISNATOL MESYLATE
393 DOLASTATIN D
394 EPOLACTAENE
395 LEXIPAFANT

**database CHEMBL**

396 CARBENICILLIN
397 DICLOFOP
398 EPIPHENIIICILLIN
399 FLOBUFEN
400 GIGANTIC ACID
401 PHENETICILLIN POTASSIUM
402 DIPHENICILLIN

**database Pubchem**

403 Acotiamide
404 Acoziborole
405 Acumapimod
406 Apalutamide
407 ASP3026
408 AZD1480
409 BIIB021
410 Branplam
411 Brequinar
412 Chlorproguanil
413 Emricasan
414 Enasidenib
415 Enolicam
416 Flurazepam
417 ILX295501
418 Indibulin
419 Metoclopramide
420 Mevastatin
421 MK0686
422 Navarixin
423 Nefazodone
424 Pantoprazole
425 Pavinetant
426 SCYX-7158
427 Siccanin
428 Sulfoguanole
429 Sunitinib
430 Suvorexant
431 Tiapride
432 Tonabersat
433 Ulimorelin
434 Xipamide
435 MGGBYMDAPCCKCI-UHFFFAOYSA-N
436 VNBRGSXVFBYQNN-UHFFFAOYSA-N
437 YJHNXUAATAMVKD-PZJWPPBQSA-N

9.2.3. Further systems for conditional heterodimerization:

According to the present invention, a pair of dimerization domains for heterodimerization of two CAR molecules of the group of CARs, for example, can also be selected from:

a) FKBP and FKBP-rapamycin associated protein (FRB, mutant T82L)
b) GAI and GID1
c) FKBP and calcineurin catalytic subunit A (CnA)
d) FKBP and cyclophilin
e) PYL and ABI

The sequences of these heterodimerization domains as well as the regulating molecules suited for dimerization of these heterodimerization domains are well known in the art (Rutkowska et al., Angew Chem Int Ed Engl. 2012;51(33):8166) and are disclosed, for example, in WO2014127261.

The members of a pair of heterodimerization domains selected from GAI, GID1, FKBP, CnA, cyclophilin, PYL and ABI can have a length of from about 50 amino acids to about 300 amino acids or more; e.g., the members of a pair of heterodimerization domains can have a length of from about 50 aa to about 100 aa, from about 100 aa to about 150 aa, from about 150 aa to about 200 aa, from about 200 aa to about 250 aa, from about 250 aa to about 300 aa, or more than 300 aa.

For example, a preferred heterodimerization domain can be derived from FKBP and can comprise an amino acid sequence having at least about 75%, at least about 80%, at least about 85%, at least about 90%, at least about 95%, at least about 98%, or 100% amino acid sequence identity to the amino acid sequence Uniprot P62942-1.

As another example, a heterodimerization domain can be derived from calcineurin catalytic subunit A (also known as PPP3CA; CALN; CALNA; CALNA1; CCN1; CNA1; PPP2B; CAM-PRP catalytic subunit; calcineurin A alpha; calmodulin-dependent calcineurin A subunit alpha isoform; protein phosphatase 2B, catalytic subunit, alpha isoform; etc.) and can comprise an amino acid sequence having at least about 75%, at least about 80%, at least about 85%, at least about 90%, at least about 95%, at least about 98%, or 100% amino acid sequence identity to the amino acid sequence Uniprot Q08209-1 amino acids (aa) 56-347 (PP2Ac domain).

As another example, a heterodimerization domain can be derived from cyclophilin (also known cyclophilin A, PPIA, CYPA, CYPH, PPIase A, etc.) and can comprise an amino acid sequence having at least about 75%, at least about 80%, at least about 85%, at least about 90%, at least about 95%, at least about 98%, or 100% amino acid sequence identity to the amino acid sequence Uniprot P62937-1.

As another example, a heterodimerization domain domain can be derived from MTOR (also known as FKBP-rapamycin associated protein; FK506 binding protein 12-rapamycin associated protein 1; FK506 binding protein 12-rapamycin associated protein 2; FK506-binding protein 12-rapamycin complex-associated protein 1; FRAP; FRAP1; FRAP2; RAFT1; and RAPT1) and can comprise an amino acid sequence having at least about 75%, at least about 80%, at least about 85%, at least about 90%, at least about 95%, at least about 98%, or 100% amino acid sequence identity to the amino acid sequence Uniprot P42345-1 aa 2021-2113 (also known as "Frb": Fkbp-Rapamycin Binding Domain).

As another example, a heterodimerization domain can be derived from a PYL protein (also known as abscisic acid receptor and as RCAR) and can comprise an amino acid sequence having at least about 75%, at least about 80%, at least about 85%, at least about 90%, at least about 95%, at least about 98%, or 100% amino acid sequence identity to any of the following amino acid sequences: PYL10 (Uniprot Q8H1R0-1); PYL11 (Uniprot Q9FJ50); PYL12 (Uniprot Q9FJ49-1); PYL13 (Uniprot Q9SN51-1); PYL1 (Uniprot Q8VZS8-1); PYL2 (Uniprot 080992-1); PYL3 (Uniprot Q9SSM7-1); PYL4 (Uniprot 080920-1); PYL5 (Uniprot Q9FLB1-1); PYL6 (Uniprot Q8S8E3-1); PYL7 (Uniprot Q1ECF1-1); PYL8 (Uniprot Q9FGM1-1); PYL9 (Uniprot Q84MC7-1); PYR1 (Uniprot 049686-1).

As another example, a heterodimerization domain can be derived from an ABI protein (also known as Abscisic Acid-Insensitive) and can be derived from proteins such as those of Arabidopsis thaliana: ABI1 (Also known as ABSCISIC ACID-INSENSITIVE 1, Protein phosphatase 2C 56, AtPP2C56, P2C56, and PP2C ABI1) and/or ABI2 (also known as P2C77, Protein phosphatase 2C 77, AtPP2C77, ABSCISIC ACID-INSENSITIVE 2, Protein phosphatase 2C ABI2, and PP2C ABI2). For example, a suitable heterodimerization domain can comprise an amino acid sequence having at least about 75%, at least about 80%, at least about 85%, at least about 90%, at least about 95%, at least about 98%, or 100% amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, from about 150 aa to about 160 aa, from about 160 aa to about 170 aa, from about 170 aa to about 180 aa, from about 180 aa to about 190 aa, or from about 190 aa to about 200 aa of any of the following amino acid sequences: ABI1 (Uniprot P49597-1); ABI2 (Uniprot 004719-1).

As another example, a heterodimerization domain can be derived from the GAI Arabidopsis thaliana protein (also known as Gibberellic Acid Insensitive, and DELLA protein GAI) and can comprise an amino acid sequence having

at least about 75%, at least about 80%, at least about 85%, at least about 90%, at least about 95%, at least about 98%, or 100% amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, from about 150 aa to about 160 aa, from about 160 aa to about 170 aa, from about 170 aa to about 180 aa, from about 180 aa to about 190 aa, or from about 190 aa to about 200 aa of the amino acid sequence Uniprot Q9LQT8-1.

As another example, a heterodimerization domain can be derived from a GID1 Arabidopsis thaliana protein (also known as Gibberellin receptor GID1) and can comprise an amino acid sequence having at least about 75%, at least about 80%, at least about 85%, at least about 90%, at least about 95%, at least about 98%, or 100% amino acid sequence identity to a contiguous stretch of from about 100 amino acids to about 110 amino acids (aa), from about 110 aa to about 115 aa, from about 115 aa to about 120 aa, from about 120 aa to about 130 aa, from about 130 aa to about 140 aa, from about 140 aa to about 150 aa, from about 150 aa to about 160 aa, from about 160 aa to about 170 aa, from about 170 aa to about 180 aa, from about 180 aa to about 190 aa, or from about 190 aa to about 200 aa of any of the following amino acid sequences: GID1A (Uniprot Q9MAA7-1); GID1B (Uniprot Q9LYC1-1); GID1C (Uniprot Q940G6-1).

Heterodimerization of the heterodimerization domains described in 9.2.3 can be achieved by different regulating molecules (shown in the parentheses following the pair of heterodimerization domains):

b) FKBP and CnA (rapamycin);
c) FKBP and cyclophilin (rapamycin);
d) FKBP and FRG (rapamycin);
h) PYL and ABI (abscisic acid);
j) GAI and GID1 (gibberellin or the gibberellin analog GA-3M).

As noted above, rapamycin (PubChem CID 5284616) can serve as a regulating molecule. Alternatively, a rapamycin derivative or analog can be used. See, e.g., WO96/41865; WO 99/36553; WO 01/14387; and Ye et al (1999) Science 283:88-91. For example, analogs, homologs, derivatives and other compounds related structurally to rapamycin ("rapalogs") include, among others, variants of rapamycin having one or more of the following modifications relative to rapamycin: demethylation, elimination or replacement of the methoxy at C7, C42 and/or C29; elimination, derivatization or replacement of the hydroxy at CI 3, C43 and/or C28; reduction, elimination or derivatization of the ketone at C14, C24 and/or C30; replacement of the 6-membered pipecolate ring with a 5-membered prolyl ring; and alternative substitution on the cyclohexyl ring or replacement of the cyclohexyl ring with a substituted cyclopentyl ring. Additional information is presented in, e.g., U.S. Pat. Nos. 5,525,610; 5,310,903 5,362,718; and 5,527,907. Selective epimerization of the C-28 hydroxyl group has been described; see, e.g., WO 01/14387. Additional synthetic regulating molecules suitable for use as an alternative to rapamycin include those described in U.S. Patent Publication No. 2012/0130076, for example, 28-epirapamycin (PubChem CID 131668123).

Also suitable as a rapalog is a compound of the formula:

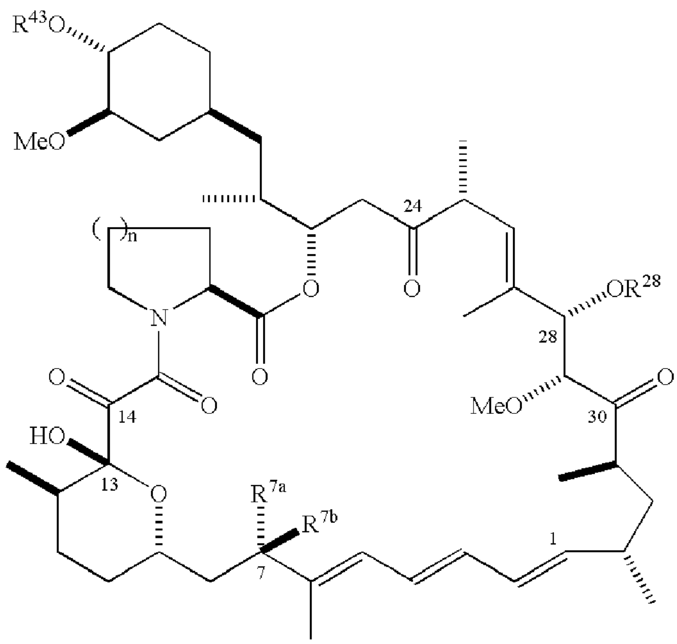

(as disclosed for example in US7067526B1) where n is 1 or 2; $R^{28}$ and $R^{43}$ are independently H, or a substituted or unsubstituted aliphatic or acyl moiety; one of $R^{7a}$ and $R^{7b}$ is H and the other is halo, $R^A$, $OR^A$, $SR^A$, $-OC(O)R^A$, $-OC(O)NR^AR^B$, $-NR^AR^B$, $-NR^BC(OR)R^A$, $NR^BC(O)OR^A$, $-NR^BSO_2R^A$, or $NR^BSO_2NR^AR^{B'}$; or $R^{7a}$ and $R^{7b}$, taken together, are H in the tetraene moiety:

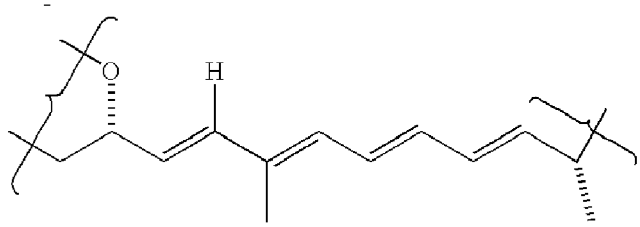

where $R^A$ is H or a substituted or unsubstituted aliphatic, hetero aliphatic, aryl, or heteroaryl moiety and where $R^B$ and $R^{B'}$ are independently H, OH, or a substituted or unsubstituted aliphatic, heteroaliphatic, aryl, or heteroaryl moiety.

9.3. Extracellular dimerization by secreted soluble factors:

The non-covalent complexation of at least two CAR molecules of a group of CARs according to the present invention can also be induced by secreted soluble factors, e.g., proteins accumulating in the tumour stroma, whereby, frequently, these proteins can itself homo- or heterodimerize. In this case, these soluble factors serve as regulating molecules according to the present invention. Dimerization domains then can be, for example, domains of native receptors (or short peptides derived therefrom; e.g., Young et al., J Biol Chem. 2004;279(46) :47633-42) to which the soluble factors are able to bind, or any antigen binding polypeptide (as already described above in chapter 1 "Antigen binding moiety") engineered for binding to a selected soluble factor (for example, a TGF-beta 1 binding peptide (Dotor et al., Cytokine. 2007;39(2):106-15), or a VEGF-binding CH2-CH3-Fc domain (Lobner et al., MAbs. 2017;9(7):1088-1104) as used as dimerization domain in example 7).

10. Target antigens:

In preferred embodiments according to the present invention each antigen binding moiety of a group of CARs and of other polypeptides being able to bind to CAR molecules of the group binds to a target antigen present on a cell, preferably a target antigen of a cell, on a solid surface, or a lipid bilayer.

According to the present invention the specific target antigens specifically recognized by the antigen binding moieties of a group of CARs, or, alternatively, by the antigen binding moieties of the other polypeptides that is able to

bind to the CAR molecules of the group, can be naturally occurring cellular surface antigens or polypeptides, carbohydrates or lipids bound to naturally occurring cellular surface antigens.

In order to maximally exploit the avidity effect, the antigen binding moieties of a group of CARs, or, alternatively, the antigen binding moieties of the other polypeptides that is able to bind to the CAR molecules of the group, are preferably directed against different non-overlapping epitopes on the same target antigen, or against target antigens which natively form covalent or non-covalent complexes. The benefit of targeting such co-localized epitopes or antigens is illustrated in example 6.

Examples of antigens, to which an antigen binding moiety of a group of CARs, and of another polypeptide being able to bind to a CAR molecule of the group, can specifically bind, include, e.g., CD19, CD20, CD22, CD23, CD28, CD30, CD33, CD35, CD38, CD40, CD42c, CD43, CD44, CD44v6, CD47, CD49D, CD52, CD53, CD56, CD70, CD72, CD73, CD74, CD79A, CD79B, CD80, CD82, CD85A, CD85B, CD85D, CD85H, CD85K, CD96, CD107a, CD112, CD115, CD117, CD120b, CD123, CD146, CD148, CD155, CD185, CD200, CD204, CD221, CD271, CD276, CD279, CD280, CD281, CD301, CD312, CD353, CD362, BCMA, CD16V, CLL-1, Ig kappa, TRBC1, TRBC2, CKLF, CLEC2D, EMC10, EphA2, FR-a, FLT3LG, FLT3, Lewis-Y, HLA-G, ICAM5, IGHA1/IgA1, IL-1RAP, IL-17RE, IL-27RA, MILR1, MR1, PSCA, PTCRA, PODXL2, PTPRCAP, ULBP2, AJAP1, ASGR1,CADM1, CADM4, CDH15, CDH23, CDHR5, CELSR3, CSPG4, FAT4, GJA3, GJB2, GPC2, GPC3, IGSF9, LRFN4, LRRN6A/LING01, LRRC15, LRRC8E, LRIG1, LGR4, LYPD1, MARVELD2, MEGF10, MPZLI1, MTDH, PANX3, PCDHB6, PCDHB10, PCDHB12, PCDHB13, PCDHB18, PCDHGA3, PEP, SGCB, vezatin, DAGLB, SYT11, WFDC10A, ACVR2A, ACVR2B, anaplastic lymphoma kinase, cadherin 24, DLK1, GFRA2, GFRA3, EPHB2, EPHB3, EPHB4, EFNB1, EPOR, FGFR2, FGFR4, GALR2, GLG1, GLP1R, HBEGF, IGF2R, UNC5C, VASN, DLL3, FZD10, KREMEN2, TMEM169, TMEM198, NRG1, TMEFF1, ADRA2C, CHRNA1, CHRNB4, CHRNA3, CHRNG, DRD4, GABRB3, GRIN3A, GRIN2C, GRIK4, HTR7, APT8B2, NKAIN1, NKAIN4, CACNA1A, CACNA1B, CACNA1I, CACNG8, CACNG4, CLCN7, KCNA4, KCNG2, KCNN3, KCNQ2, KCNU1, PKD1L2, PKD2L1, SLC5A8, SLC6A2, SLC6A6, SLC6A11, SLC6A15, SLC7A1, SLC7A5P1, SLC7A6, SLC9A1, SLC10A3, SLC10A4, SLC13A5, SLC16A8, SLC18A1, SLC18A3, SLC19A1, SLC26A10, SLC29A4, SLC30A1, SLC30A5, SLC35E2, SLC38A6, SLC38A9, SLC39A7, SLC39A8, SLC43A3, TRPM4, TRPV4, TMEM16J, TMEM142B, ADORA2B, BAI1, EDG6, GPR1, GPR26, GPR34, GPR44, GPR56, GPR68, GPR173, GPR175, LGR4, MMD, NTSR2, OPN3, OR2L2, OSTM1, P2RX3, P2RY8, P2RY11, P2RY13, PTGE3, SSTR5, TBXA2R, ADAM22, ADAMTS7, CST11, MMP14, LPPR1, LPPR3, LPPR5, SEMA4A, SEMA6B, ALS2CR4, LEPROTL1, MS4A4A, ROM1, TM4SF5, VANGL1, VANGL2, C18orf1, GSGL1, ITM2A, KIAA1715, LDLRAD3, OZD3, STEAP1, MCAM, CHRNA1, CHRNA3, CHRNA5, CHRNA7, CHRNB4, KIAA1524, NRM.3, RPRM, GRM8, KCNH4, Melanocortin 1 receptor, PTPRH, SDK1, SCN9A, SORCS1, CLSTN2, Endothelin converting enzyme like-1, Lysophosphatic acid receptor 2, LTB4R, TLR2, Neurotropic tyrosine kinase 1, MUC16, B7-H4, epidermal growth factor receptor (EGFR), ERBB2, HER3, EGFR variant III (EGFRvIII), HGFR, FOLR1, MSLN, CA-125, MUC-1, prostate-specific membrane antigen (PSMA), mesothelin, epithelial cell adhesion molecule (EpCAM), L1-CAM, CEACAM1, CEACAM5, CEACAM6, VEGFR1, VEGFR2, high molecular weight-melanoma associated antigen (HMW-MAA), MAGE-A 1, IL-13R-$\alpha$2, disialogangliosides (GD2 and GD3), tumour-associated carbohydrate antigens (CA-125, CA-242, Tn and sialyl-Tn), 4-1BB, 5T4, BAFF, carbonic anhydrase 9 (CA-IX), C-MET, CCR1, CCR4, FAP, fibronectin extra domain-B (ED-B), GPNMB, IGF-1 receptor, integrin $\alpha5\beta1$, integrin $\alpha v\beta3$, ITB5, ITGAX, embigin, PDGF-R$\alpha$, ROR1, Syndecan 1, TAG-72, tenascin C, TRAIL-R1, TRAIL-R2, NKG2D-Ligands, a major histocompatibility complex (MHC) molecule presenting a tumour-specific peptide epitope, preferably PR1/HLA-A2, a lineage-specific or tissue-specific tissue antigen, preferably CD3, CD4, CD5, CD7, CD8, CD24, CD25, CD34, CD80, CD86, CD133, CD138, CD152, CD319, endoglin, MHC molecules, and the like.

11. Nucleic acids, preparation of cells, therapeutic use:

Another aspect of the present invention relates to nucleic acids comprising nucleotide sequences encoding the CAR molecules of a group of CARs according to the present invention. The nucleic acid according to the present invention will in some embodiments be DNA or RNA, including, e.g., an expression vector. The nucleic acids according to the present invention may also be provided in other form, e.g. in viral vectors. The nucleic acids may be active or conditionally active in cells and be present or presents in some embodiments as RNA, e.g., in vitro synthesized RNA. Introducing RNA or DNA into a host cell can be carried out in vitro or ex vivo or in vivo. For example, a host cell (e.g., an NK cell, a cytotoxic T lymphocyte, etc.) can be electroporated in vitro or ex vivo with RNA comprising a nucleotide sequence encoding the CAR molecules of the group of CARs.

In some cases, the nucleic acid of the present disclosure comprises a nucleotide sequence encoding the CAR molecules of a group of CARs according to the present invention, consisting either of two, or three or four CAR molecules. In some cases, the nucleic acids of the present disclosure comprise one, two, three, or four separate nucleotide sequences each encoding one molecule of the group of CARs, consisting either of two, three or four CAR molecules.

In the case where the CAR molecules of the group of CARs are encoded by different nucleic acid molecules, the present invention provides a kit of at least two nucleic acids encoding one, two, three or four molecules of a group

of CAR, wherein, again, the nucleic acids are preferably selected from DNA, RNA or in vitro transcribed RNA.

The present invention also provides a vector comprising the nucleic acids according to the present invention (i.e. encoding the CAR molecules of the group of CARs) and/or the kit of nucleic acids (encoding the CAR molecules of the group of CARs).

Such a vector can include a selectable marker, an origin of replication, and other features that provide for replication and/or maintenance of the vector. Suitable vectors include, e.g., plasmids, viral vectors, and the like. Large numbers of suitable vectors and promoters are known to those of skill in the art; many are commercially available for generating the recombinant constructs according to the present invention. The following vectors are provided by way of example. Bacterial: pBs, phagescript, PsiX 174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXRl, pSG (Stratagene) pSVK3, pBPV, pMSG and pSVL (Pharmacia). Vectors can have convenient restriction sites located near the promoter sequence to provide for the insertion of nucleic acid sequences encoding heterologous proteins. A selectable marker operative in the expression host may be present. Suitable vectors include viral vectors (e.g. viral vectors based on vaccinia virus, poliovirus, adenovirus, adeno-associated virus, SV40, herpes simplex virus, human immunodeficiency virus, a retroviral vector (e.g., Murine Leukaemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus); and the like). Preferred vectors, due to the ability of efficiently integrating into the genome of the transduced cells, are retroviral vectors, especially gamma-retroviral vectors and lentiviral vectors, i.e. vectors derived from at least a portion of a retrovirus genome. An example of a preferred retroviral vector is a self-inactivating lentiviral vector (as provided in Milone et al., Mol Ther. 2009;17(8):1453-1464). Other examples of lentivirus vectors that may be used in the clinic include, e.g., the LENTIVECTOR® gene delivery technology from Oxford BioMedica, the LENTIMAX™ vector System from Lentigen and the like. Nonclinical types of lentiviral vectors are also available and would be known to one skilled in the art. Other types of preferred vectors that can efficiently integrate into the genome of transfected cells are transposon vectors, preferably PiggyBAC-based vectors and Sleeping beauty-based vectors. Further important non-viral strategies for integrating a gene of interest into the genome of a cell are based on site-specific nuclease technologies (e.g., based on Zinc-finger nucleases (ZFNs) or transcription activator-like effector nucleases (TALENs)) or on CRISPR/Cas-technology (as described, e.g., by Gaj et al., Trends Biotechnol. 2013;31(7):397-405; and Ren et al., Protein Cell 2017;8(9):634-643). These technologies allow for integration of defined nucleotide sequences from any DNA molecule (single stranded DNA or double stranded DNA; in the form of a vector, PCR amplicon etc.) and are attractive because the gene of interest can be integrated into the genome downstream of endogenous promoters (as described, e.g., by Eyquem et al., Nature. 2017;543(7643):113-117).

The present invention also provides a kit of at least two vectors, wherein each vector comprises nucleic acid sequences encoding one, two, three or four CAR molecules of the group of CARs according to the present invention. The vectors may be provided with the same or different regulation sequences in order to achieve expression in the same or different host systems (e.g. suitable cells where the vectors express the CAR molecules after transformation with the vector or propagation).

In the vector or kit of vectors according to the present invention, the nucleic acids encoding the CAR molecules of the group of CARs can be operably linked to a transcriptional control element, yielding an expression vector. Such a transcriptional control element can be a promoter, an enhancer, etc., wherein suitable promoter and enhancer elements are known in the art. For expression in a bacterial cell, suitable promoters include lacl, lacZ, T3, T7, gpt, lambda P and trc. For expression in a eukaryotic cell, suitable promoters include light and/or heavy chain immunoglobulin gene promoter and enhancer elements, cytomegalovirus immediate early promoter, herpes simplex virus thymidine kinase promoter, early and late SV40 promoters, promoter present in long terminal repeats from a retrovirus (e.g. the 5'-LTR of a gamma retrovirus or a promoter sequence comprising subelements R and U3 of the 5'-LTR of the Moloney murine leukaemia virus (MMLV)), promoter present in the murine stem cell virus (MSCV), mouse metallothionein-I promoter, EF1-alpha with or without intron, promoter of phosphoglycerate kinase (PGK), and various art-known tissue specific promoters. Suitable reversible promoters, including reversible inducible promoters are known in the art. Such reversible promoters may be isolated and derived from many organisms, e.g. eukaryotes and prokaryotes. Modification of reversible promoters derived from a first organism for use in a second organism, e.g. a first prokaryote and a second a eukaryote, a first eukaryote and a second a prokaryote, etc., is well known in the art. Such reversible promoters, and systems based on such reversible promoters but also comprising additional control proteins, include alcohol regulated promoters (e.g. alcohol dehydrogenase I (alcA) gene promoter, promoters responsive to alcohol transactivator proteins (AlcR), etc.), tetracycline regulated promoters, (e.g. promoter systems including TetActivators, TetON, TetOFF, etc.), steroid regulated promoters (e.g. rat glucocorticoid receptor promoter systems, human estrogen receptor promoter systems, retinoid promoter systems, thyroid promoter systems, ecdysone promoter systems, mifepristone promoter systems, etc.), metal regulated promoters (e.g. metallothionein promoter systems, etc.), pathogenesis-related regulated promoters (e.g. salicylic acid regulated promoters, ethylene regulated promoters, benzothiadiazole regulated promoters, etc.), temperature

regulated promoters (e.g., heat shock inducible promoters (e.g. HSP-70, HSP-90, soybean heat shock promoter, etc.), light regulated promoters, synthetic inducible promoters, and the like.

In some instances, the locus or construct or transgene containing the suitable promoter can be irreversibly switched through the induction of an inducible system. Suitable systems for induction of an irreversible switch are well known in the art, e.g., induction of an irreversible switch may make use of a Cre-lox-mediated recombination. Any suitable combination of recombinase, endonuclease, ligase, recombination sites, etc. known to the art may be used in generating an irreversibly switchable promoter. Methods, mechanisms, and requirements for performing site-specific recombination, described elsewhere herein, find use in generating irreversibly switched promoters and are well known in the art. In some cases, the promoter is a CD8 cell-specific promoter, a CD4 cell-specific promoter, a neutrophil-specific promoter, or an NK-specific promoter. For example, a CD4 gene promoter can be used. As another example, a CD8 gene promoter can be used. NK cell-specific expression can be achieved by use of a Neri (p46) promoter. In some embodiments, e.g. for expression in a yeast cell, a suitable promoter is a constitutive promoter such as an ADH1 promoter, a PGK 1 promoter, an ENO promoter, a PYK 1 promoter and the like; or a regulatable promoter such as a GAL1 promoter, a GAL1O promoter, an ADH2 promoter, a PH05 promoter, a CUP1 promoter, a GAL7 promoter, a MET25 promoter, a MET3 promoter, a CYC1 promoter, a HIS3 promoter, an ADH1 promoter, a PGK promoter, a GAPDH promoter, an ADC1 promoter, a TRP1 promoter, a URA3 promoter, a LEU2 promoter, an ENO promoter, a TP1 promoter, and AOX 1 (e.g. for use in Pichia). Selection of the appropriate vector and promoter is well within the level of ordinary skill in the art. Suitable promoters for use in prokaryotic host cells include a bacteriophage T7 RNA polymerase promoter; a trp promoter; a lac operon promoter; a hybrid promoter, e.g. a lac/tac hybrid promoter, a tac/trc hybrid promoter, a trp/lac promoter, a T7/lac promoter; a trc promoter; a tac promoter, and the like; an araBAD promoter; in vivo regulated promoters, such as an ssaG promoter or a related promoter, a pagC promoter, a nirB promoter, and the like; a sigma70 promoter, e.g. a consensus sigma70 promoter (see, e.g., GenBank Accession Nos. AX798980, AX798961, and AX798183); a stationary phase promoter, e.g. a dps promoter, an spv promoter, and the like; a promoter derived from the pathogenicity island SPI-2; an actA promoter; an rpsM promoter; a tet promoter; an SP6 promoter; and the like. Suitable strong promoters for use in prokaryotes such as Escherichia coli include Trc, Tac, T5, T7, and PLambda. Examples of operators for use in bacterial host cells include a lactose promoter operator (Laci repressor protein changes conformation when contacted with lactose, thereby preventing the Laci repressor protein from binding to the operator), a tryptophan promoter operator (when complexed with tryptophan, TrpR repressor protein has a conformation that binds the operator; in the absence of tryptophan, the TrpR repressor protein has a conformation that does not bind to the operator), and a tac promoter operator.

According to a preferred embodiment of the present invention, the vector or the kit of at least two vectors comprise a T lymphocyte-specific promoter or an NK cell-specific promoter or an EF1-alpha promoter operably linked to nucleotide sequences encoding CAR molecules of the group of CARs.

According to a further aspect, the present invention also relates to a genetically modified cell which has been modified to produce all CAR molecules of a group of CARs according to the present invention. The cells of the present invention may also be used to produce the vectors of the present invention (e.g. as virus or plasmid supernatant) from where they may then be further purified and provide these vectors in amplified and purified form.

According to a preferred embodiment, the cell is a mammalian cell which is genetically modified to produce the CAR molecules of a group of CARs according to the present invention. Preferred mammalian cells are stem cells, progenitor cells, or cells derived from a stem cell or a progenitor cell, preferably lymphocytes. Further preferred cells to be genetically modified according to the present invention are primary cells and immortalized cell lines. For pharmaceutical uses, human cells, especially lymphocytes, are specifically preferred. However, also non-human primary cells and cell lines may be suitable cell types, especially for addressing scientific questions with the system according to the present invention, e.g. non-human primate cell lines, rodent (e.g., mouse, rat) cell lines, and the like.

Further preferred cells according to the present invention may be HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL1O), PC12 cells (ATCC No. CRL1721), COS cells, COS-7 cells (ATCC No. CRL1651), RAT1 cells, mouse L cells (ATCC No. CCL1.3), human embryonic kidney (HEK) cells (ATCC No. CRL1573), HLHepG2 cells, Hut-78, Jurkat, HL-60, NK cell lines (e.g., NKL, NK92, and YTS), and the like. In some preferred instances, the cell according to the present invention is not an immortalized cell line, but is instead a cell (e.g. a primary cell) obtained from an individual. For example, in some cases, the cell is an immune cell obtained from an individual. As an example, the cell is a T lymphocyte obtained from an individual. As another example, the cell is a cytotoxic cell obtained from an individual. As another example, the cell is a stem cell or progenitor cell obtained from an individual.

According to a specifically preferred embodiment, the mammalian cell according to the present invention, which is transformed with a vector or a kit of at least two vectors encoding the individual CAR molecules of a group of CARs according to the present invention, is a T cell or an NK cell.

According to a further aspect, the present invention relates to a pharmaceutical preparation which comprises

a nucleic acid according to the present invention, a kit of nucleic acids according to the present invention, a vector or a kit of vectors according to the present invention, or a cell or a kit of cells according to the present invention.

The present disclosure provides a method of generating a conditionally activatable cell. The method generally involves genetically modifying a mammalian cell with a vector, or a kit of vectors, or an RNA (e.g., in vitro transcribed RNA), comprising nucleotide sequences encoding a conditionally active group of CARs according to the present disclosure. In a preferred embodiment the genetically modified cell is conditionally activated in the presence of: a) a target antigen or natively complexed target antigens to which the antigen binding moieties of the group of CARs bind and b) at least one kind of regulating molecule. Optionally, the genetically modified cell is activatable in the presence of a target antigen or natively complexed target antigens to which the antigen binding moieties of the group of CARs bind and less activatable in the additional presence of at least one kind of regulating molecule. The genetic modification can be carried out in vivo, in vitro, or ex vivo. The cell can be an immune cell (e.g., a T lymphocyte or NK cell), a stem cell, a progenitor cell, etc.

The genetic modification is preferably carried out ex vivo. For example, a T lymphocyte (i.e., a T cell), a stem cell, or an NK cell can be obtained from an individual and the cell obtained from the individual is genetically modified to express a group of CARs according to the present disclosure. In a preferred embodiment the genetically modified cell is conditionally activatable in the presence of: a) a target antigen or natively complexed target antigens to which the antigen binding moieties of the group of CARs, or, alternatively, the antigen binding moieties of the other polypeptide being able to bind to the group of CARs, bind; and b) at least one kind of regulating molecule. In some cases, the genetically modified cell is activated ex vivo, for example, during the process of expansion of the cells before administration. Where the genetically modified cell is introduced into an individual (e.g., the individual from whom the cell was obtained), the genetically modified cell can be activated in vivo, e.g., by administering to the individual at least one regulating molecule, provided that the respective target antigen(s) of the antigen binding moieties is/are present at physiological expression levels on the surface of a cell in the individual. The genetically modified cell comes into contact with the target antigen(s) present at physiological expression levels on the surface of a cell in the individual; and in preferred embodiments, upon administration to the individual of at least one kind of regulating molecule, the genetically modified cell is activated. Optionally, in embodiments where complex formation of the group of CARs can be inhibited by the presence of a regulating molecule, the genetically modified cell can be less activatable after administration of at least one kind of regulating molecule to the individual.

For example, where the genetically modified cell is a T lymphocyte or an NK cell, the genetically modified cell upon activation can exhibit cytotoxicity toward a cell that presents the respective target antigen(s), to which the group of CARs (or the antigen binding moieties of the other polypeptides) is able to bind, on its surface.

The present disclosure provides various treatment methods using a subject group of CARs.

A non-covalently complexed group of CARs according to the present invention, when present in a T lymphocyte or an NK cell, can mediate cytotoxicity toward a target cell. A non-covalently complexed group of CARs according to the present invention, in some cases dependent on the presence of (an)other polypeptide(s) comprising at least an antigen binding moiety and being able to bind to a CAR molecule, can bind to a selected target antigen or a selected complex of natively complexed target antigens present on a target cell, thereby, in preferred embodiments, mediating killing of a target cell by a T lymphocyte or an NK cell genetically modified to produce the group of CARs.

Target cells include cancer cells. Thus, the present disclosure provides methods of killing, or inhibiting the growth of, a target cancer cell, the method involving contacting a target cancer cell with a cytotoxic immune effector cell (e.g., a cytotoxic T cell, or an NK cell) that is genetically modified to produce a subject group of CARs, such that the T lymphocyte or NK cell recognizes a target antigen or a target antigen complex present on the surface of a target cancer cell, and mediates killing of the target cell.

The present disclosure provides a method of treating cancer in an individual having a cancer. In a preferred embodiment the method comprises: i) genetically modifying NK cells or preferably T lymphocytes obtained from the individual with at least one vector comprising nucleotide sequences encoding the respective CAR molecules of a group of CARs according to the present invention, wherein each antigen binding moiety of the group of CARs is specific for a target antigen on a cancer cell in the individual, and wherein said genetic modification is carried out in vitro or ex vivo; ii) introducing the genetically modified cells into the individual; and iii) administering to the individual an effective amount of at least one regulating molecule for either inducing or reducing dimerization of the respective CAR molecules of the group, preferably inducing dimerization of the respective CAR molecules of the group, thereby either inducing or reducing non-covalent complexation of the group of CARs, preferably inducing non-covalent complexation of the group of CARs, wherein the non-covalently complexed group of CARs upon contact with a cancer cell expressing the respective target antigen or the respective covalent or non-covalent complex of different target antigens mediates activation of the genetically modified cell, which leads to killing of the cancer cell and thereby enables treating the cancer.

Carcinomas that can be amenable to therapy by a method disclosed herein include esophageal carcinoma, hepatocellular carcinoma, basal cell carcinoma (a form of skin cancer), squamous cell carcinoma (various tissues), bladder carcinoma, including transitional cell carcinoma (a malignant neoplasm of the bladder), bronchogenic carcinoma,

colon carcinoma, colorectal carcinoma, gastric carcinoma, lung carcinoma, including small cell carcinoma and non-small cell carcinoma of the lung, adrenocortical carcinoma, thyroid carcinoma, pancreatic carcinoma, breast carcinoma, ovarian carcinoma, prostate carcinoma, adenocarcinoma, sweat gland carcinoma, sebaceous gland carcinoma, papillary carcinoma, papillary adenocarcinoma, cystadenocarcinoma, medullary carcinoma, renal cell carcinoma, ductal carcinoma in situ or bile duct carcinoma, choriocarcinoma, seminoma, embryonal carcinoma, Wilm's tumor, cervical carcinoma, uterine carcinoma, testicular carcinoma, osteogenic carcinoma, epithelial carcinoma, and nasopharyngeal carcinoma. Sarcomas that can be amenable to therapy by a method disclosed herein include fibrosarcoma, myxosarcoma, liposarcoma, chondrosarcoma, chordoma, osteogenic sarcoma, osteosarcoma, angiosarcoma, endotheliosarcoma, lymphangiosarcoma, lymphangioendothelio-sarcoma, synovioma, mesothelioma, Ewing's sarcoma, leiomyosarcoma, rhabdomyosarcoma, and other soft tissue sarcomas. Other solid tumors that can be amenable to therapy by a method disclosed herein include glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma. Leukaemias that can be amenable to therapy by a method disclosed herein include a) chronic myeloproliferative syndromes (neoplastic disorders of multipotential hematopoietic stem cells); b) acute myelogenous leukaemias (neoplastic transformation of a multipotential hematopoietic stem cell or a hematopoietic cell of restricted lineage potential; c) chronic lymphocytic leukaemias (CLL; clonal proliferation of immunologically immature and functionally incompetent small lymphocytes), including B- cell CLL, T-cell CLL prolymphocytic leukaemia, and hairy cell leukaemia; and d) acute lymphoblastic leukaemias (characterized by accumulation of lymphoblasts). Lymphomas that can be treated using a subject method include B-cell lymphomas (e.g., Burkitt's lymphoma); Hodgkin's lymphoma; non-Hodgkin's lymphoma, and the like. Other cancers that can be amenable to treatment according to the methods disclosed herein include atypical meningioma (brain), islet cell carcinoma (pancreas), medullary carcinoma (thyroid), mesenchymoma (intestine), hepatocellular carcinoma (liver), hepatoblastoma (liver), clear cell carcinoma (kidney), and neurofibroma mediastinum.

A subject method can also be used to treat inflammatory conditions and autoimmune disease. A subject group of CARs can be expressed in a T-helper cell or a regulatory T (Treg) cell for use in an immunomodulatory method. Immunomodulatory methods include, e.g., enhancing an immune response in a mammalian subject toward a pathogen; enhancing an immune response in a subject who is immunocompromised; reducing an inflammatory response; reducing an immune response in a mammalian subject to an autoantigen, e.g., to treat an autoimmune disease; and reducing an immune response in a mammalian subject to a transplanted organ or tissue, to reduce organ or tissue rejection. Where the method involves reducing an immune response to an autoantigen, at least one of the target antigens used to activate the group of CARs preferably is an autoantigen. Where the method involves reducing an immune response to a transplanted organ or tissue, at least one of the antigens used to activate the group of CARs preferably is an antigen specific to the transplanted organ.

As discussed above, a treatment method of the present disclosure involves the administration to an individual in need thereof of an effective amount of one or more different regulating molecules and optionally one or more different other polypeptides, wherein each of the other polypeptides comprises antigen binding moiety and being able to bind to an extracellular binding site of a CAR molecule of the group of CARs.

The required effective amount of each regulating molecule, administered to an individual in need thereof having received T lymphocytes or NK cells expressing a group of CARs according to the present invention ("effector cells"), is defined by the different response of those effector cells upon contact with antigen expressing target cells in presence vs. absence of each required regulating molecule. The response of those effector cells thereby is defined by the excretion of interferon-gamma, and/or Macrophage inflammatory protein-1 (MIP-1) alpha, and/or MIP-1 beta, and/or granzyme B, and/or IL-2, and/or TNF, and/or IL-10, and/or IL-4, and/or by effector cell degranulation, wherein cell degranulation is preferably detected by the percentage of effector cells translocating CD107a onto their surface, i.e., the percentage of CD107a-positive effector cells detected by flow cytometric analysis using a degranulation assay (for example, as described in Proff et al., Front Micro-biol. 2016;7:844), after contact with a target cell expressing more than 100,000 target antigen molecules or target antigen complexes per cell, optionally in the presence of an effective concentration of each required other polypeptide comprising at least an antigen binding moiety and being able to bind to binding sites of the CAR molecules of the group of CARs. In preferred embodiments, the response of the effector cells in presence vs. absence of an effective concentration of each required regulating molecule differs by at least 20%, preferably by at least 50%, or even more preferably by at least 100%, wherein the effective concentration of each required regulating molecule is the concentration achieved by administration of an effective amount of each required regulating molecule in one or more doses to an individual in need thereof. The effective concentration of each required other polypeptide comprising at least an antigen binding moiety and being able to bind to the group of CARs is defined by the response of the fully complexed subject group of CARs (i.e., all dimerization domains comprised by the group of CARs are dimerized), after contact with a target cell expressing more than 100,000 target antigen molecules or target antigen complexes per cell, in presence vs. absence of each required other polypeptide comprising at least an antigen binding moiety and being able to bind to the group of CARs, wherein the response preferably differs by at least 20%, preferably by at least 50%, or even more preferably by at least 100%, and wherein the effective concentration of each required other polypeptide

comprising at least an antigen binding moiety and being able to bind to the group of CARs is the concentration achieved by administration of an effective amount of each of those other polypeptides in one or more doses to an individual in need thereof having received T lymphocytes or NK cells expressing the subject group of CARs.

Both the regulating molecules and the antigen-specific other polypeptides being able to bind to a CAR molecule of a group of CARs according to the present invention are hereafter together referred to as "agents specifically binding to the group of CARs".

In the subject methods, an "agent specifically binding to the group of CARs" can be administered to the host using any convenient means capable of resulting in the desired therapeutic effect or diagnostic effect. Thus, the "agent(s) specifically binding to the group of CARs" can be incorporated into a variety of formulations for therapeutic administration. More particularly, an "agent specifically binding to the group of CARs" can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants and aerosols. In pharmaceutical dosage forms, an "agent specifically binding to the group of CARs" can be administered in the form of their pharmaceutically acceptable salts, or they may also be used alone or in appropriate association, as well as in combination, with other pharmaceutically active compounds. The following methods and excipients are merely exemplary:

Suitable excipient vehicles can be, for example, water, saline, dextrose, glycerol, ethanol, or the like, and combinations thereof. In addition, if desired, the vehicle may contain minor amounts of auxiliary substances such as wetting or emulsifying agents or pH buffering agents. Actual methods of preparing such dosage forms are known, or will be apparent, to those skilled in the art. See, e.g., Remington's Pharmaceutical Sciences, Mack Publishing Company, Easton, Pennsylvania, 17th edition, 1985. The composition or formulation to be administered will, in any event, contain a quantity of the required "agent(s) specifically binding to the group of CARs" adequate to achieve the desired state in the subject being treated. The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public. For oral preparations, an "agent specifically binding to the group of CARs" can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatines; with disintegrators, such as corn starch, potato starch or sodium carboxymethylcellulose; with lubricants, such as talc or magnesium stearate; and if desired, with diluents, buffering agents, moistening agents, preservatives and flavouring agents.

An "agent specifically binding to the group of CARs" can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Pharmaceutical compositions comprising an "agent specifically binding to the group of CARs" can be prepared by mixing the "agent(s) specifically binding to the group of CARs" having the desired degree of purity with optional physiologically acceptable carriers, excipients, stabilizers, surfactants, buffers and/or tonicity agents. Acceptable carriers, excipients and/or stabilizers are preferably nontoxic to recipients at the dosages and concentrations employed, and include buffers such as phosphate, citrate, and other organic acids; antioxidants including ascorbic acid, glutathione, cysteine, methionine and citric acid; preservatives (such as ethanol, benzyl alcohol, phenol, m-cresol, p-chlor-m-cresol, methyl or propyl parabens, benzalkonium chloride, or combinations thereof); amino acids such as arginine, glycine, ornithine, lysine, histidine, glutamic acid, aspartic acid, isoleucine, leucine, alanine, phenylalanine, tyrosine, tryptophan, methionine, serine, proline and combinations thereof; monosaccharides, disaccharides and other carbohydrates; low molecular weight (less than about 10 residues) polypeptides; proteins, such as gelatin or serum albumin; chelating agents such as EDTA; sugars such as trehalose, sucrose, lactose, glucose, mannose, maltose, galactose, fructose, sorbose, raffinose, glucosamine, N- methylglucosamine, galactosamine, and neuraminic acid; and/or non-ionic surfactants such as Tween, Brij Pluronics, Triton-X, or polyethylene glycol (PEG).

The pharmaceutical composition may be in a liquid form, a lyophilized form or a liquid form reconstituted from a lyophilized form, wherein the lyophilized preparation is to be reconstituted with a sterile solution prior to administration. The standard procedure for reconstituting a lyophilized composition is usually to add back a volume of pure water (typically equivalent to the volume removed during lyophilization); however solutions comprising antibacterial agents may be used for the production of pharmaceutical compositions for parenteral administration; see also Chen (1992) Drug Dev Ind Pharm 18, 1311-54.

An "agent specifically binding to the group of CARs" can be optionally formulated also in a controlled release formulation. Sustained-release preparations may be prepared using methods well known in the art. Suitable examples of sustained-release preparations include semipermeable matrices of solid hydrophobic polymers containing the "agent(s) specifically binding to the group of CARs" in which the matrices are in the form of shaped articles, e.g. films

or microcapsules. Examples of sustained-release matrices include polyesters, copolymers of L-glutamic acid and ethyl-L-glutamate, non-degradable ethylene-vinyl acetate, hydrogels, polylactides, degradable lactic acid-glycolic acid copolymers and poly-D-(-)-3-hydroxybutyric acid. Possible loss of biological activity may be prevented by using appropriate additives, by controlling moisture content and by developing specific polymer matrix compositions.

A suitable dosage can be determined by an attending physician or other qualified medical personnel, based on various clinical factors. As is well known in the medical arts, dosages for any one patient depend upon many factors, including the patient's size, body surface area, age, the particular "agent(s) specifically binding to the group of CARs" to be administered, sex of the patient, time, and route of administration, general health, and other drugs being administered concurrently. An "agent specifically binding to the group of CARs" may be administered in amounts between 1 ng/kg body weight and 20 mg/kg body weight per dose, e.g. between 0.1 mg/kg body weight to 10 mg/kg body weight, e.g. between 0.5 mg/kg body weight to 5 mg/kg body weight; however, doses below or above this exemplary range are envisioned, especially considering the aforementioned factors. If the regimen is a continuous infusion, it can also be in the range of 1 $\mu$g to 10 mg per kilogram of body weight per minute.

Those of skill will readily appreciate that dose levels can vary as a function of the specific "agent(s) specifically binding to the group of CARs", the severity of the symptoms and the susceptibility of the subject to side effects. Preferred dosages for a given compound are readily determinable by those of skill in the art by a variety of means.

One or more "agents specifically binding to the group of CARs" can be administered to an individual using any available method and route suitable for drug delivery, including in vivo and ex vivo methods, as well as systemic and localized routes of administration. Conventional and pharmaceutically acceptable routes of administration include intratumoral, peritumoral, intramuscular, intratracheal, intracranial, subcutaneous, intradermal, topical application, intravenous, intraarterial, rectal, nasal, oral, and other enteral and parenteral routes of administration. Routes of administration may be combined, if desired, or adjusted depending upon the "agent(s) specifically binding to the group of CARs" and/or the desired effect. An "agent specifically binding to the group of CARs" can be administered in a single dose or in multiple doses. In preferred embodiments an "agent specifically binding to the group of CARs" can be administered orally or alternatively intravenously. In other embodiments, an "agent specifically binding to the group of CARs" can be administered via an inhalational route. In yet other embodiments, an "agent specifically binding to the group of CARs" can be administered intranasally, locally, or also intratumourally. In yet other embodiments, an "agent specifically binding to the group of CARs" can be administered peritumourally. In other embodiments for treatment of brain tumours, an "agent specifically binding to the group of CARs" can be administered intracranially.

The "agent(s) specifically binding to the group of CARs" can be administered to a host using any available conventional methods and routes suitable for delivery of conventional drugs, including systemic or localized routes. In general, routes of administration contemplated by the invention include enteral, parenteral, or inhalational routes. Parenteral routes of administration other than inhalation administration include topical, transdermal, subcutaneous, intramuscular, intraorbital, intracapsular, intraspinal, intrasternal, intratumoral, peritumoral, and intravenous routes. Parenteral administration can be carried to effect systemic or local delivery of an "agent specifically binding to the group of CARs". Where systemic delivery is desired, administration typically involves invasive or systemically absorbed topical or mucosal administration of pharmaceutical preparations. An "agent specifically binding to the group of CARs" can also be delivered to the subject by enteral administration. Enteral routes of administration include oral and rectal (e.g., using a suppository) delivery.

By treatment is meant at least an amelioration of the symptoms associated with the pathological condition afflicting the host, where amelioration is used in a broad sense to refer to at least a reduction in the magnitude of a parameter, e.g. symptom, associated with the pathological condition being treated, such as cancer. As such, treatment also includes situations where the pathological condition, or at least symptoms associated therewith, are completely inhibited, e.g. prevented from happening, or stopped, e.g. terminated, such that the host no longer suffers from the pathological condition, or at least the symptoms that characterize the pathological condition.

An "agent specifically binding to the group of CARs" can be administered by injection and/or delivery, e.g., to a site in a brain artery or directly into brain tissue. An "agent specifically binding to the group of CARs" can also be administered directly to a target site e.g., by direct injection, by implantation of a drug delivery device such as an osmotic pump or slow release particle, by biolistic delivery to the target site, etc. Furthermore, an "agent specifically binding to the group of CARs" can be administered as an adjuvant therapy to a standard cancer therapy. Standard cancer therapies include surgery (e.g., surgical removal of cancerous tissue), radiation therapy, bone marrow transplantation, chemotherapeutic treatment, antibody treatment, biological response modifier treatment, and certain combinations of the foregoing.

A variety of subjects are suitable for treatment with a subject method of treating cancer. Suitable subjects include any individual, e.g., a human or non-human animal who has cancer, who has been diagnosed with cancer, who is at risk for developing cancer, who has had cancer and is at risk for recurrence of the cancer, who has been treated with other therapeutics and failed to respond to such treatment, or who relapsed after initial response to such treatment.

Subjects suitable for treatment with a subject immunomodulatory method include individuals who have an

autoimmune disorder; individuals who are organ or tissue transplant recipients; and the like; individuals who are immunocompromised; and individuals who are infected with a pathogen.

**SHORT DESCRIPTION OF THE FIGURES:**

Fig. 1 shows the schematics of exemplary architectures of the group of CARs.
Fig. 2 shows the $K_d$ values of different rcSso7d-based antigen binding moieties towards human EGFR.
Fig. 3 shows that extracellular disulphide bond forming cysteines can prevent the exploitation of the avidity effect for regulation of CAR function.
Fig. 4 shows that dimerization/oligomerization of scFv-comprising CAR molecules prevents the exploitation of the avidity effect for regulation of CAR function.
Fig. 5 shows the regulation of CAR function by regulating the avidity of a group of CARs by conditional homodimerization.
Fig. 6 shows the regulation of the function of stably transduced T cells expressing a group of CARs *in vivo.*
Fig. 7 shows the functional *in vitro* characterization of the CAR modified T cells used for the *in vivo* experiments.
Fig. 8 shows the regulation of the avidity of a group of CARs by heterodimerization and the sensitivity of CAR T cells depending on whether the target antigen is monomeric or dimeric.
Fig. 9 shows the regulation of the avidity of a group of CARs by VEGF.
Fig. 10 shows the generation and function of an affibody-based group of CARs directed against HER2.
Fig. 11 shows groups of CARs consisting of three or four CAR molecules.
Fig. 12 shows groups of CARs comprising different co-stimulatory domains.
Fig. 13 shows the expression of CAR molecules comprising different rcSso7d and affibody based binding moieties fused to different CAR signalling backbones.
Fig. 14 shows the schematics of the design of different CAR molecules.
Fig. 15 shows the amino acid sequences of different CAR molecules.

**EXAMPLES:**

Fig. 1: Schematics of exemplary architectures of the group of CARs. Fig. 1A schematically shows the basic architecture of a CAR molecule of a group of CARs in the version in which the antigen binding moiety is integrated into the CAR molecule (left) and in the version in which the CAR molecule comprises a binding site which binds to a binding site in another polypeptide comprising an antigen binding moiety (right). Low affinity interaction occurs either between the antigen binding moiety and the target antigen or between the binding site in the CAR molecule and a binding site in the other polypeptide binding to the binding site in the CAR molecule. At least one of the CAR molecules of a group must comprise at least one signalling region comprising either at least one ITAM or at least one ITIM. In the shown example of a CAR molecule, the endodomain exemplarily comprises a single signalling region. The lines between each component of the shown CAR molecules indicate optional linkers. The dimerization domains (of which at least one is mandatory for each CAR molecule of the group) and optional additional domains or components are not indicated.

Fig. 1B schematically illustrates how many CAR molecules in groups of CARs consisting of two, three or four CAR molecules can comprise at least one signalling region (totality of the signalling regions of a given CAR molecule is symbolized by a white box). Of the CAR molecules comprising at least one signalling region either all or only some, but at least one CAR molecule comprise at least one ITAM or alternatively at least one ITIM. For simplicity, the ectodomains and dimerization domains and optional additional domains or components are not shown. The lines between each component of the shown CAR molecules indicate optional linkers.

Fig. 1C schematically illustrates the arrangement of signalling regions with the example of a group of CARs consisting of two CAR molecules. The depicted examples show only some of the possible combinations of the different arrangements. For example, a CAR molecule may comprise two or more ITAM-comprising signalling regions or two or more co-stimulatory signalling regions. In inhibitory groups of CARs (not shown) the ITAM-comprising signalling regions are substituted by ITIM-comprising signalling regions and co-stimulatory signalling regions are either omitted or substituted by other inhibitory domains. For simplicity, the ectodomains, the dimerization domains and optional additional domains or components are not shown. The lines between each component of the shown CAR molecules indicate optional linkers.

Figs. 1D - 1N schematically exemplify how dimerization domains can be used for non-covalent complexation of groups of CARs. The depicted examples show only some of the possible arrangements. In the depicted examples different pairs of homo- and heterodimerization domains are shown at different positions in the CAR molecules each exemplarily comprising one or two signalling regions. In a preferred example (Fig. 1E, left) a group of CARs consisting

of three CAR molecules comprises only the two members of a single pair of heterodimerization domains and correspondingly can be regulated by a single kind of regulating molecule. For simplicity, the illustrations show only the signalling regions, the transmembrane domain and dimerization domains. The lines between some of the components of the shown CAR molecules indicate optional linkers. Similar arrangements of dimerization domains can also be incorporated extracellularly.

Fig. 1O exemplifies non-covalent complexation of a group of CARs by an extracellular soluble factor acting as a regulating molecule. The regulating molecule is schematically exemplified with a monomeric protein and with proteins which natively either homo- or heterodimerize. The shown CAR molecules exemplarily comprise only one signalling region. Optional additional dimerization domains and optional additional domains or components are not indicated. The order of the antigen binding domains (or binding sites) and the dimerization domains may also be inverted. That is, the antigen binding domains (or binding sites) may also be more proximal to the plasma membrane than the dimerization domains. The lines between each component of the shown CAR molecules indicate optional linkers.

Fig. 2 shows the $K_d$ values of different rcSso7d-based antigen binding moieties (fused to superfolder GFP (sfGFP)) towards human EGFR as determined by three complementary methods: (a) Flow cytometric quantification of the amount of the different sfGFP-fusion proteins bound to Jurkat T cells expressing high levels of truncated EGFR (tEGFR), (b) and (c) surface plasmon resonance (SPR) analysis by using a matrix coated with a chimeric EGFR protein comprising the extracellular domain of EGFR fused to the Fc domain of IgG1. Affinities were either determined in a kinetic (b) or a steady-state analysis mode (c).

Fig. 3: Extracellular disulphide bond forming cysteines prevent the exploitation of the avidity effect for regulation of CAR function. (A) Schematic representation of the architecture of the CAR signalling backbones "Cys" for S-8cys-BB-3z ("Cys") and "Ser" for S-8ser-BB-3z ("Ser"), which are capable for disulphide bond formation or not, respectively. These signalling backbones were fused to the different rcSso7d-based antigen binding moieties and expressed for functional testing in primary human T cells. (B) Typical CAR expression (shown with rcSso7d variant "E11.4.1-WT" fused to either the "Cys" or "Ser" CAR backbone) 20 hours after electroporation of 5 $\mu$g of mRNA in primary human T cells. T cells expressing no transgene ("no CAR") served as negative controls. (C) Expression of tEGFR in Jurkat cells, which were used as target cells, 20 hours after electroporation of 3 $\mu$g tEGFR-encoding mRNA. Jurkat T cells without construct ("no construct") and a respective isotype control ("isotype control") served as negative controls. The function of the CARs was tested by determining the capacity of primary human T cells modified with the different CARs for target cell killing (D) and IFN-$\gamma$ production (E). The T cells of four different donors (indicated by different symbols) were electroporated with 5 $\mu$g mRNA of the indicated CAR construct and co-cultured on the next day with Jurkat T cells (electroporated with 3 $\mu$g of tEGFR-mRNA) for 4 hours at 37°C at an effector:target (E:T) ratio of 2:1. T cells without CAR ("no CAR") served as negative controls.

Fig. 4: Dimerization/multimerization of scFv-comprising CAR molecules prevents the exploitation of the avidity effect for regulation of CAR function. (A) Schematic representation of the CARs used in the experiments. (B, C and D) Expression of CAR constructs in human primary T cells 20 hours after electroporation of 5 $\mu$g the respective mRNAs. T cells without a CAR ("no CAR") served as a negative control. (E) Expression of tHER2 in Jurkat cells, which were used as target cells, 20 hours after electroporation of 5 $\mu$g tEGFR-encoding mRNA. Jurkat T cells without construct ("no construct") served as negative controls. CAR T cells were co-cultured with Jurkat-tHER2 cells for 4 hours at 37°C at an E:T ratio of 2:1. Figures 4F and 4G show the capacity of the CARs in which the scFv 4D5-5 was fused to the two different CAR signalling backbones (capable for disulphide bond formation or not, i.e., "Cys" for 4D5-5-8cys-BB-3z (SEQ ID NO: 59) and "Ser" for 4D5-5-8ser-BB-3z (SEQ ID NO: 60)) to trigger cytotoxicity (G) and IFN-$\gamma$ production (F). The function of CARs in which $V_H$ and $V_L$ were fused onto two separate polypeptides ("$V_H$ and $V_L$"; 4D5-5(split)-8ser-BB-FKBP(36V)-3z (SEQ ID NO: 56 and SEQ ID NO: 57)) is shown in (H). Primary T cells expressing the CAR 4D5-5-8ser-BB-3z (SEQ ID NO: 60), in which the monomeric signalling backbone was fused to the 4D5-5-scFv, served as a positive control. CAR T cells of three different donors (indicated by different symbols) were co-cultured with a mixture of tHER2-transfected and non-transfected Jurkat T cells for 4 hours at 37°C at an E:T ratio of 4:1:1 (T cells:tHER2$^{pos}$ Jurkat cells:tHER2$^{neg}$ Jurkat cells) and the ratio of viable tHER2$^{pos}$ and tHER2$^{neg}$ target cells was determined by flow cytometry. To induce dimerization, the T cells were pre-treated with the dimerization agent AP20187 (10 nM, 30 minutes, 37°C). Treatment with the same concentration of DMSO served as a control condition. T cells without CAR ("no CAR") and T cells expressing only the $V_H$ chain ("4D5-5 ($V_H$) -8ser-BB-FKBP (36V) -3z" (SEQ ID NO: 57)) served as negative controls.

Fig. 5: Regulating CAR avidity by homodimerization of CAR molecules. (A) Schematic representation of the group of CARs complexed by the regulating molecule AP20187. In the shown example an rcSso7d-based antigen binding moiety directed against EGFR was used as an antigen binding moiety. (B) Expression of CAR molecules comprising an antigen binding moiety with either high or low affinity to EGFR (i.e., S(WT)-8ser-BB-FKBP(36V)-3z "E11.4.1-WT" (SEQ ID NO: 49) and S(G32A)-8ser-BB-FKBP(36V)-3z "E11.4.1-G32A" (SEQ ID NO: 46), respectively) in primary human T cells 14 days after activation by anti-CD3/CD28-antibody coated beads (i.e., 13 days after stable transduction with the respective CAR constructs). T cells without a CAR ("no CAR") served as negative controls. (C) Expression of EGFR in the target cell line "A431-fLuc". Unstained cells ("unstained") and a respective isotype control ("isotype control") served

as negative controls. (D) Capacity of primary human T cells transduced with different CAR constructs for killing of the luciferase-expressing target cell line A431-fLuc. Primary T cells of three donors (indicated by different symbols) were stably transduced with vectors coding for either the rcSso7d-based CARs S(WT)-8ser-BB-FKBP(36V)-3z ("E11.4.1-WT") and S(32)-8ser-BB-FKBP(36V)-3z ("E11.4.1-G32A")) or the scFv-based CAR directed against CD19 (CD19-8cys-BB-3z "CD19-BBz" (SEQ ID NO: 58)), which served as a negative control. T cells without CAR ("no CAR") served as an additional negative control. AP20187 served as regulating molecule for non-covalent dimerization of "E11.4.1-WT" (S(WT)-8ser-BB-FKBP(36V)-3z) and "E11.4.1-G32A" (S(G32A)-8ser-BB-FKBP(36V)-3z). Dimerization was induced by pretreatment of the T cells with 10 nM AP20187 for 30 minutes at 37°C. Treatment with the same concentration of DMSO served as a control condition. Cytotoxicity of the modified T cells was determined by quantifying viable, luciferase expressing A431-fLuc target cells after co-culture for 4 hours at 37°C at an E:T ratio of 10:1.

Fig. 6: Regulation of the function of stably transduced CAR T cells *in vivo* in an NSG mouse model. (A) Efficacy of dimerization induced activation of CARs in an *in vivo* NSG mouse model. 9-16 weeks old NSG mice were intravenously injected with $0.5x10^6$ Nalm6 cells that were stably transduced with a vector coding for luciferase and tEGFR ("Nalm6-tEGFR-fLuc"). Three days later, the NSG mice were treated by intravenous administration of $10x10^6$ CAR T cells (14 days after activation by anti-CD3/CD28-antibody coated beads, i.e., 13 days after stable transduction). An injection with phosphate buffered saline (PBS) served as a control condition. Five NSG mice were used for each treatment group, except for the group treated with S(WT)-8ser-BB-FKBP(36V)-3z (SEQ ID NO: 49), which consisted of four mice. Dimerization was induced by intraperitoneal administration of 2 mg/kg of the homodimerization agent AP20187 over a period of 11 days (days of injection indicated by arrows). Groups that did not receive a dimerization agent, received the respective vehicle solution intraperitoneally as a control condition. Tumour size (mean of each treatment group) is shown as total photon flux determined within the region of interest that encompassed the entire body of the NSG mice. (B) Shows that postponed administration of the dimerizer in mice, which were treated with the S(32)-8ser-BB-FKBP(36V)-3z (SEQ ID NO: 46) CAR T cells but did not receive the dimerizer AP20187 until day 11, results in efficient CAR activation and control of tumour growth.

Fig. 7: Regulation of the function of stably transduced CAR T cells *in vitro.* (A) Expression of CAR molecules comprising an antigen binding moiety with either high or low affinity to EGFR (i.e., S(WT)-8ser-BB-FKBP(36V)-3z "E11.4.1-WT" (SEQ ID NO: 49) and S(G32A)-8ser-BB-FKBP(36V)-3z "E11.4.1-G32A" (SEQ ID NO: 46), respectively) in primary human T cells 14 days after activation by anti-CD3/CD28-antibody coated beads (i.e., 13 days after stable transduction with the respective CAR constructs). T cells without a CAR ("no CAR") served as negative controls. (B) Expression of the anti-CD19 CAR CD19-8cys-BB-3z "CD19-BBz" (SEQ ID NO: 58) in primary human T cells 14 days after activation by anti-CD3/CD28-antibody coated beads (i.e., 13 days after stable transduction). T cells without a CAR ("no CAR") served as negative controls. (C and D) Expression of tEGFR in Nalm6-fLuc and Nalm6-tEGFR-fLuc cells, respectively. Unstained cells and a respective isotype control served as negative controls. (E and F) Lysis of Nalm6-fLuc and Nalm6-tEGFR-fLuc cells by primary human T cells expressing different CAR molecules. Primary T cells of three donors (indicated by different symbols) were stably transduced with vectors coding for either the rcSso7d-based CARs (S(WT)-8ser-BB-FKBP(36V)-3z "E11.4.1-WT" and S(G32A)-8ser-BB-FKBP(36V)-3z "E11.4.1-G32A") or the scFv-based CAR directed against CD19 (CD19-8cys-BB-3z "CD19-BBz"), which served as a positive control. T cells without CAR ("no CAR") served as a negative control. AP20187 served as regulating molecule for non-covalent dimerization of "E11.4.1-WT" (S(WT)-8ser-BB-FKBP(36V)-3z) and "E11.4.1-G32A" (S(G32A)-8ser-BB-FKBP(36V)-3z). Dimerization was induced by pre-treatment of the T cells with 10 nM AP20187 for 30 minutes at 37°C. Treatment with the same concentration of DMSO served as a control condition. Cytotoxicity of the modified T cells was determined by quantifying viable, luciferase expressing target cells after co-culture for 4 hours at 37°C at an E:T ratio of 10:1.

Fig. 8: Regulating the avidity of a group of CARs by heterodimerization of CAR molecules and influence of the colocalization of target antigens on the sensitivity of the CAR T cells. Depicted in (A) is the expression of a tEGFR fusion protein ("FKBP F36V", for conditional homodimerization) in Jurkat cells, which served as target cells, 20 hours after electroporation of 5 μg of the respective mRNA. Unstained cells ("mock") and a respective isotype control ("isotype") served as negative controls. (B and C) Correlation of the expression levels of tEGFR in Jurkat T cells electroporated with different amounts of the respective mRNAs. (D and E) Expression of the different CARs in primary human T cells 20 hours after electroporation of 5 μg of the respective mRNA. The rcSso7d variants with high and low affinity ("E11.4.1-WT" and "E11.4.1.-G32A, respectively) were fused to both a 8ser-BB-FKBP-3z and a 8ser-BB-FRB-3z backbone for conditional heterodimerization by the regulating molecule AP21967. The expression of the resulting constructs (S(G32A)-8ser-BB-FKBP-3z (SEQ ID NO: 48), S(G32A)-8ser-BB-FRB-3z (SEQ ID NO: 47), S(WT)-8ser-BB-FKBP-3z (SEQ ID NO: 50), S(WT)-8ser-FRB-3z (SEQ ID NO: 51)) was detected via their integrated Tags (Strep II for 8ser-BB-FRB-3z and FLAG for 8ser-BB-FKBP-3z). (F) Molecular architecture of the experimental system used for determining the sensitivity of dimerization-induced activation of CAR T cells in response to target cells expressing either monomeric or dimeric target antigen tEGFR. In this system, the CAR molecules were conditionally heterodimerized by the regulating molecule AP21967 and the target antigen tEGFR was conditionally homodimerized by AP20187. Depicted in (G) and (H) is the capacity of the CARs for triggering cytotoxicity and IFN-γ production, respectively, (mean ± standard deviation,

n=3, three different donors) in primary human T cells in dependence of both the number of tEGFR molecules expressed in the target cells and the presence or absence of AP21967 and AP20187. Primary T cells from three donors were electroporated with 5 μg mRNA of each CAR molecule (i.e., either the low-affinity constructs S(G32A)-8ser-BB-FKBP-3z plus S(G32A)-ser8-BB-FRB-3z or the high-affinity constructs S(WT)-8ser-BB-FKBP-3z plus S(WT)-8ser-BB-FRB-3z, as indicated) and co-cultured with Jurkat T cells expressing varying amounts of tEGFR. Co-culture was done for 4 hours at 37°C at an E:T ratio of 4:1:1 (T cells:tEGFR$^{pos}$ Jurkat cells:tEGFR$^{neg}$ Jurkat cells). The cytotoxicity of the CAR T cells was determined by flow cytometric quantification of the ratio of viable tEGFR$^{pos}$ and tEGFR$^{neg}$ target cells. To induce CAR-dimerization, the primary human T cells were pre-treated with AP21967 (500 nM, 30 minutes, 37°C) and to induce EGFR-dimerization, Jurkat T cells were pre-treated with AP20187 (10 nM, 30 minutes, 37°C). Treatment with the same concentration of ethanol (in the case of AP21967) or DMSO (in the case of AP20187) served as a control condition.

Fig. 9: Generation of a CAR which can be regulated by VEGF as example of an extracellular factor accumulating in the tumour microenvironment. In the shown example the soluble factor VEGF was used as a regulating molecule and the EGFR-specific rcSso7d-based binder E11.4.1-G32A was used as antigen binding moiety. The schematic of the architecture of a respective CAR is shown in (A) and (B). Expression of the target antigen tEGFR in Jurkat T cells 20 hours after electroporation of 5 μg of mRNA is depicted in (C). Expression of the two polypeptides in primary human T cells was detected using an anti-IgGl-antibody (D). The anti-Strep II-tag antibody was additionally used for detection of the CAR molecule containing the VEGF binding site (Janus-CT6-Fc domain without transmembrane domain) and for the control CAR without IgG-Fc domains. Cytotoxicity triggered by the different CARs in primary T cells was determined in a FACS-based cytotoxicity assay (E). CAR T cells from three different donors (indicated by different symbols) were co-cultured with tEGFR-transfected Jurkat cells for 4 hours at 37°C at an E:T ratio of 4:1:1 (T cells: tEGFR$^{pos}$ Jurkat cells:tEGFR$^{neg}$ Jurkat cells). Dimerization of the CARs was induced by pre-treatment of the T cells with VEGF (concentration as indicated, 30 minutes, 37°C). T cells without a CAR ("no CAR") served as a negative control and T cells with the CAR S(WT)-8ser-BB-FKBP(36V)-3z served as a positive control.

Fig. 10: Screening of affibody-based binding moieties suited for use in a group of CARs according to the present invention. The affinity of the affibody zHER2-WT was reduced by replacing all amino acids that are potentially involved in epitope binding by alanine. The different variants of zHER2-WT were fused to the CAR signalling backbone 8ser-BB-FKBP(36V)-3z containing the intracellular homodimerization domain FKBP(F36V) for conditional dimerization. The architecture of these CARs is shown in (A). (B and C) Expression of the target antigen tHER2 in Jurkat T cells and the expression of affibody-based CARs in primary T cells, respectively. Primary T cells and Jurkat T cells were electroporated with 5 μg mRNA coding for the respective construct and expression was determined 20 hours after electroporation. Primary T cells and Jurkat T cells expressing no construct ("no CAR" and "no construct", respectively) were used as negative controls. (D) Expression of 13 different affibody-based CARs in primary T cells ("L9A", "R10A", "Q11A", "Y13A", "W14A", "Q17A", "W24A", "T25A", "S27A", "R28A", "R32A", "Y35A" and "zHER2-WT", respectively) (the sequences of the affibody based antigen binding moieties fused to the CAR signalling backbone are depicted in SEQ ID NO:26 to SEQ ID NO:38). Primary T cells were electroporated with 5 μg mRNA coding for the respective construct and expression was determined 20 hours after electroporation. T cells expressing no construct ("no CAR") were used as a negative control. (E) Dimerization-induced activation of affibody-based CARs. Primary T cells from two donors (indicated by different symbols) were electroporated with 5 μg for each construct. Specific lysis of target cells was determined in a luciferase-based cytotoxicity assay after co-incubation of the different CAR T cells with tHER2-transfected (4 hours at 37°C at an E:T ratio of 2:1). Dimerization of the CARs was induced by pre-treatment of the T cells with AP20187 (10 nM, 30 minutes, 37°C). Treatment with the same concentration of DMSO served as a control condition. T cells without a CAR ("no CAR") served as a negative control. Figure 10F shows the $K_d$ values of respective Affibody-based binding moietiess (fused to superfolder GFP (sfGFP)) towards human HER2 as determined by SPR analysis by using a matrix coated with a chimeric HER2 protein comprising the extracellular domain of HER2 fused to the Fc domain of IgG1. Affinities were determined in a kinetic mode.

Fig. 11: Functional characterization of groups of CARs consisting ofthree and four CAR molecules. (A and B) Schematic representation of groups of CARs consisting of three or four CAR molecules, respectively. (C and D) Expression of trimeric and tetrameric groups of CARs in Jurkat T cells 20 hours after electroporation of 5 μg mRNA of each construct. Jurkat T cells expressing no construct ("no CAR") were used as a negative control.

Fig. 12: Expression and function of groups of CARs comprising different co-stimulatory molecules. (A) Schematic representation of a group of CARs that consists of two CAR molecules each containing either the co-stimulatory domain of CD28 or ICOS or OX40 in its co-stimulatory signalling region. (B and C) Expression of the CAR molecules (red histograms) 20 hours after electroporation of 5 μg mRNA in Jurkat T cells or primary human T cells, respectively. Jurkat T cells or primary human T cells, respectively, expressing no construct ("no CAR") were used as a negative control (filled blue histograms). (D) Induction of NF-κB and NF-AT promoters in Jurkat T cells electroporated with 5 μg of mRNA encoding S(G32A)-8ser-OX40-FKBP(36V)-3z. These cells were either co-cultured or not for furher 20 hours in the presence or absence of AP20187 with Jurkat T cells electroporated with 5 μg of tEGFR encoding mRNA. Induction of

NF-κB and NF-AT promoters in the CAR expressing reporter cells was detected by flow cytometric analysis of the expression of enhanced green fluorescent protein (eGFP) and cyan fluorescent variant of GFP (CFP), respectively. The cytotoxicity of primary human T cells expressing the indicated CAR molecules is shown in (E). 20 hours after electroporation of 5 μg mRNA encoding the respective CAR molecules the T cells were co-cucultured with target cells for further 4 or 20 hours at 37°C at an E:T ratio of 4:1:1 (T cells:tEGFR$^{pos}$ Jurkat cells:tEGFR$^{neg}$ Jurkat cells).
The specific lysis of the respective target cells was calculated by subtracting the lysis of the target cells observed in the co-cultures with T cells that did not express a CAR. Dimerization of the CAR molecules of the group was induced by pre-treatment of the Jurkat cells (D) and primary human T cells (E) with 10 nM AP20187 for 30 minutes at 37°C. Treatment with the same concentration of DMSO served as a control condition.

Fig. 13: Expression of CAR molecules comprising rcSso7d and affibody based binding moieties fused to different CAR signalling backbones. (A) Expression of CAR constructs "Myc-S(18.4.2)-8cys-BB-3z" (SEQ ID NO: 39), "S(18.4.2)-8cys-BB-3z" (SEQ ID NO: 40) and "S(18.4.2)-G4S-8cys-BB-3z" (SEQ ID NO: 41) in primary human T cells 20 hours after electroporation of 5 μg of the respective mRNAs. Primary T cells without a CAR served as negative controls (filled histogram). Expression was detected using a fusion protein consisting of the extracellular domain of human EGFR and the Fc domain of IgG1 and an anti-human-IgGl-antibody. (B) Expression of CAR constructs "S(G32A)-G4S-myc-8cys-BB-3z" (SEQ ID NO: 42), "S(G32A)-G4S-StrepII-8cys-BB-3z" (SEQ ID NO: 43) and "S(G32A)-G4S-his-8cys-BB-3z" (SEQ ID NO: 45) in primary human T cells 20 hours after electroporation of 5 μg of the respective mRNAs. Primary T cells without a CAR served as negative controls (filled histogram). CAR expression was detected using either an anti-c-myc antibody, an anti-Strep II antibody, or an anti-hexahistidine antibody, respectively. (C) Expression of CAR constructs "S(WT)-8cys-BB-3z" (SEQ ID NO: 74), "S(G25A)-8cys-BB-3z" (SEQ ID NO: 72), "S(G32A)-8cys-BB-3z" (SEQ ID NO: 43), "S (WT)-8ser-BB-3z" (SEQ ID NO: 75), "S (G25A)-8ser-BB-3z" (SEQ ID NO: 73), "S(G32A)-8ser-BB-3z" (SEQ ID NO: 44) in human primary T cells 20 hours after electroporation of 5 μg of the respective mRNAs. Primary T cells without a CAR served as negative controls (filled histogram). Expression was detected using an anti-Strep II antibody. (D) Expression of CAR constructs "S (G32A)-8ser-BB-FKBP(36V)-3z" (SEQ ID NO: 46), "S(G32A)-8ser-BB-FRB-3z" (SEQ ID NO: 47), "S(G32A)-8ser-BB-FKBP-3z" (SEQ ID NO: 48), "A(WT)-8ser-BB-FKBP(36V)-3z" (SEQ ID NO: 52) in primary human T cells 20 hours after electroporation of 5 μg of the respective mRNAs. Primary T cells without a CAR served as negative controls (filled histogram). Expression was detected using either an anti-FLAG antibody, an anti-Strep II antibody, or an anti-hexahistidine antibody, as indicated.

Fig. 14 shows the schematics of the design of different CAR molecules. The corresponding amino acid sequences are shown in Fig. 15.

Fig. 15 shows the amino acid sequences of different CAR molecules.

**Example 1: Generation of a low-affinity single domain binding moiety based on rcSso7d for use in a group of CARs according to the present invention**

The first example shows a strategy for generating an antigen binding moiety with low affinity that is suited for use as an antigen binding moiety in a group of CARs, according to the present invention. Reduced charge Sso7d (rcSso7d) is a charge-reduced version of a small (~7 kDa) DNA-binding protein from the archaeon *Sulfolobus solfataricus.* Charge-reduction minimizes unspecific binding due to reduced electrostatic interactions. rcSso7d is a single-domain protein antigen binding moiety with high thermal stability and monomeric behaviour and therefore is an example of a suited binding scaffold. Starting from the well characterized antigen binding moiety rcSso7d E11.4.1, which binds to human EGFR with a $K_d$ of 19 nM (Traxlmayr et al., J Biol Chem. 2016;291(43):22496-22508), we generated low affinity mutants by performing an alanine scan in which we replaced all amino acids potentially involved in epitope binding by alanine, i.e. in each mutant one position involved in antigen binding was mutated to alanine. Mutants of rcSso7d E11.4.1 were fused to sfGFP and expressed as soluble proteins in a bacterial expression system. The schematics of the architecture of the fusion proteins are shown in Figure 14G. Binding affinities were determined (i) by performing titration experiments of the soluble fusion proteins of the binding moieties with sfGFP on Jurkat T cells that were engineered by mRNA electroporation to express high levels of the respective target antigen EGFR, and (ii) by performing SPR experiments on protein A chips loaded with the extracellular domain of EGFR fused to IgG-Fc. The result of the alanine scan and the obtained affinities of the antigen binding moieties are shown in Figure 2.

Alanine-scanning of protein antigen binding moieties:

Site-directed mutagenesis of all amino acids involved in epitope binding was performed using the QuikChange Lightning Site-Directed Mutagenesis Kit (Agilent Genomics), according to the manufacturer's instructions. Primers were designed using the QuikChange Primer Design software (Agilent Genomics) and oligonucleotides were synthesized by Biomers.

Expression and purification of rcSso7d-based antigen binding moieties:

Binding scaffolds were expressed as sfGFP fusion proteins (consisting of an N-terminal hexahistidine tag followed by either rcSso7d or the Affibody and sfGFP) using the pE-SUMO vector (Life Sensors). The nucleotide sequence that encodes the sfGFP reporter protein was obtained from Addgene (plasmid #54737). Briefly, *Escherichia coli* cells (Tuner DE3) were transformed with sequence-verified plasmids using heat shock transformation. After overnight cultivation at 37°C, cultures were diluted 1:100 in terrific broth (TB) medium (12 g/L tryptone, 24 g/L yeast extract, 4% glycerol, 2.31 g/L $KH_2PO_4$ and 16.43 g/L $K_2HPO_4$*$3H_2O$) supplemented with kanamycin (50 μg/mL) and incubated at 37°C while shaking. When cultures reached an $A_{600}$ of roughly 2, expression of the transgene was induced by addition of 1 mM of isopropyl β-D-1-thiogalactopyranoside (IPTG) and cells were further cultured overnight at 20°C. Cells were harvested by centrifugation (5000 g, 20 minutes, 4°C), resuspended in sonication buffer (50 mM sodium phosphate, 300 mM NaCl, 3% glycerol, 1% Triton X-100, pH 8.0), sonicated (2x 90 seconds, duty cycle 50 %, amplitude set to 5) and centrifuged again to remove cell debris. Hexahistidine-tagged fusion proteins were purified from crude cell extracts using TALON metal affinity resin (Clontech Laboratories). After addition of 10 mM imidazole, the sonicated supernatants were applied onto the resin twice, followed by washing step with equilibration buffer (50 mM sodium phosphate, 300 mM NaCl, pH 8.0) with increasing amounts of imidazole (5 - 15 mM). Binding scaffolds were eluted by applying equilibration buffer supplemented with 250 mM imidazole. After buffer exchange to PBS using Amicon Ultra-15 10K centrifugal filters (Merck Millipore), concentrations were determined by measuring the absorbance at 280 nm using the respective molar absorption coefficient and finally proteins were directly frozen at -80°C.

Maintenance of human cell lines:

Jurkat T cells were a gift from Dr. Sabine Strehl at the Children's Cancer Research Institute (CCRI) and were maintained in RPMI-1640 (Thermo Scientific) supplemented with 10% FCS (Sigma Aldrich) and 1% penicillin-streptomycin (Thermo Scientific). Cell lines were regularly tested for mycoplasma contamination and authentication was performed at Multiplexion, Germany. Cell densities were monitored with AccuCheck counting beads (Thermo Scientific), a flow cytometer-based cell counting platform.

*In vitro* transcription and electroporation of mRNA:

*In vitro* transcription was performed using the mMessage mMachine T7 Ultra Kit (Ambion) according to the manufacturer's instructions. 50-200 ng of column purified PCR product was used as a reaction template. The resulting mRNA was purified with an adapted protocol using the RNeasy column purification kit (Qiagen). Briefly, the mRNA solution was diluted with a mixture of RLT buffer (Qiagen), ethanol (Merck) and 2-mercaptoethanol (Merck). The mixture was loaded onto an RNeasy column and purification was performed according to the manufacturer's instructions. Elution was performed with nuclease-free water (Thermo Scientific) and purified mRNAs were frozen at -80°C until electroporation. For transient transgene expression, Jurkat T cells were electroporated with varying amounts of the respective mRNA using the Gene Pulser (Biorad). Following protocols were used for the respective cell types: Jurkat T cells (square wave protocol, 500 V, 3 ms and 4 mm cuvettes).

Antibodies and flow cytometry:

Jurkat T cells were resuspended in FACS buffer (PBS (Thermo Scientific), 0.2 % human albumin (CSL Behring) and 0.02 % sodium azide) and treated for 10 minutes at 4°C with 10% human serum. Cells were stained with the respective primary antibody for 25 minutes at 4°C. Stained cells were washed two times in FACS buffer and then directly processed with a BD LSRFortessa. Expression of the engineered target antigen tEGFR was detected either with a PE- or APC-conjugated anti-EGFR antibody (clone AY13, BioLegend). Analysis was done by the FlowJo software.

Determination of binding affinities on cell membranes:

Jurkat T cells were engineered to express high levels of the respective tumour antigen. Hence, 3 μg of mRNA coding for tEGFR were electroporated into Jurkat T cells one day prior to the co-culture with the effector cells. After washing in PBS, cells were resuspended in PBS containing 0.1% BSA (Sigma Aldrich) and incubated with varying concentrations of the binder proteins fused to sfGFP in order to determine the affinities of the antigen binding moieties towards the respective tumour antigen. After incubation for 1 hour at 4°C while shaking, plates were centrifuged (450g, 7 minutes, 4°C), the supernatant was discarded and cells were acquired with a BD LSRFortessa. Cells were kept on ice to avoid endocytosis. The $K_d$ was obtained by curve fitting using Microsoft Excel (Microsoft Corporation).

Determination of binding affinities using surface plasmon resonance (SPR):

SPR experiments were performed with a Biacore T200 instrument (GE Healthcare). All experiments were conducted in degassed and filtered PBS, pH 7.4, containing 0.1% BSA and 0.05% Tween-20 (Merck Millipore), at 25°C. hEGFR-Fc (R&D) was immobilized on a Protein A sensor chip (GE Healthcare) at a flow rate of 10 μL/min for 60 seconds at a concentration 6.67 μg/mL. To determine the affinity of rcSso7d-based antigen binding moieties, five concentrations (depending on the *expected* $K_d$ of the antigen binding moiety) of the respective protein were injected at a flow rate of 30 μL/min for 15 seconds in the single-cycle kinetic mode, followed by a dissociation step (30 seconds). Regeneration was performed using 10 mM Glycine-HCl, pH 1.7 at a flow rate of 30 μL/min for 30 seconds. The $K_d$ was obtained by curve fitting using the Biacore T200 Evaluation Software (GE Healthcare).

**Example 2: Extracellular disulphide bond-forming cysteines prevent the full exploitation of avidity effects for reversible control of CAR function**

Extracellular disulphide-bond forming cysteines in extracellular hinge regions as e.g. CD8α can prevent the exploitation of the avidity effect according to present invention. This is demonstrated in example 2, in which the low affinity mutant of the binding moiety "E11.4.1 G32A" of example 1 was fused to CAR signalling backbones in which the two extracellular cysteine residues in the stalk region of CD8α (UniProt ID P01732, positions C164 and C181) were substituted by serine residues or not, respectively. Whereas the cysteine-containing CAR-variant ("Cys") efficiently triggered T cell activation in response to target cells, the serine-containing variant ("Ser") did not or only poorly trigger the T cells. This example thus illustrates the importance of preventing disulphide-bond formation for generating CAR molecules that are suited for use in a group of CARs according to the present invention. The schematics in Figure 3A illustrate the design of the tested constructs. Figures 3B and 3C show the expression of the CARs and target antigens. Primary human T cells were electroporated with 5 μg mRNA for each construct and CAR expression was detected 20 hours after electroporation via a Strep II Tag. Jurkat T cells were electroporated with 3 μg mRNA encoding a truncated version of EGFR (tEGFR). Full length EGFR was truncated both N- and C-terminally to create a functionally inert human polypeptide that has diminished dimerization properties due to the inability to bind its natural ligand EGF and the absence of the kinase domain (Wang et al., Blood. 2011; 118 (5) :1255-1263) . The resulting transgene consisted of the leader sequence of the granulocyte-macrophage colony-stimulating factor 2 receptor alpha subunit (GM-CSF-Ra) and amino acids 334 to 675 (Uniprot P00533) of human EGFR, comprising two extracellular membrane-proximal domains and the transmembrane domain. Transgene expression was detected 20 hours after electroporation via antibodies directed against EGFR. 20 hours after electroporation of the T cells, the function of the CARs was determined by using a luciferase-based cytotoxicity assay (Figure 3D) and by quantifying cytokines released from the T cells using ELISA (Figure 3E). Figures 3D and 3E show that the antigen binding moiety with the lowest tested affinity ("E11.4.1-G32A") could trigger the T cells only upon bivalent interaction with the target cells, i.e, when fused to the CAR containing the cysteines in the CD8α hinge ("Cys"), but not or only poorly when fused to the CAR in which those cysteines were replaced by serine ("Ser"). Contrary, antigen binding moieties with increased affinities (E11.4.1-WT and E11.4.1-G25A) triggered potent cytotoxicity also upon monovalent interaction, i.e., when fused to the "Ser" CAR backbone.

Maintenance of human cell lines:

Primary human T cells were obtained from de-identified healthy donor's blood after apheresis (Buffy coats from the Austrian Red Cross, Vienna, Austria). $CD3^{pos}$ T cells were enriched by negative selection using RosetteSep Human T cell Enrichment Cocktail (STEMCELL Technologies). Isolated and purified T cells were cryopreserved in RPMI-1640 medium supplemented with 20% FCS and 10% DMSO (Sigma Aldrich) until use. $CD3^{pos}$ T Cells were activated with anti-CD3/CD28 beads (Thermo Scientific) according to the manufacturer's instructions and were expanded in human T cell medium, consisting of RPMI-1640 supplemented with 10% FCS, 1% penicillin-streptomycin and 200 IU/mL recombinant human IL-2 (Peprotech). Primary T cells were cultivated for at least 14 days before experiments were conducted. Jurkat T cells were a gift from Dr. Sabine Strehl at the CCRI and were maintained in RPMI-1640 supplemented with 10% FCS and 1% penicillin-streptomycin. Cell lines were regularly tested for mycoplasma contamination and authentication was performed at Multiplexion, Germany. Cell densities were monitored with AccuCheck counting beads.

*In vitro* transcription and electroporation of mRNA:

*In vitro* transcription was performed using the mMessage mMachine T7 Ultra Kit according to the manufacturer's instructions. 50-200 ng of column purified PCR product was used as a reaction template. The resulting mRNA was purified with an adapted protocol using the RNeasy column purification kit. Briefly, the mRNA solution was diluted with a mixture of RLT buffer, ethanol and 2-mercaptoethanol. The mixture was loaded onto an RNeasy column and purification

was performed according to the manufacturer's instructions. Elution was performed with nuclease-free water and purified mRNAs were frozen at -80°C until electroporation. For transient transgene expression, primary T cells or Jurkat T cells were electroporated with varying amounts of the respective mRNA using the Gene Pulser (Biorad). Following protocols were used for the respective cell types: primary T cells (square wave protocol, 500 V, 5 ms and 4 mm cuvettes), Jurkat T cells (square wave protocol, 500 V, 3 ms and 4 mm cuvettes).

Antibodies and flow cytometry:

Primary human T cells or tumour cell lines were resuspended in FACS buffer (PBS, 0.2 % human albumin and 0.02 % sodium azide) and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected via the Strep II tag using an anti-Strep II tag antibody (clone 5A9F9, Genscript) as a primary antibody and a PE- or APC-conjugated secondary antibody (eBioscience). Expression of the engineered target antigen tEGFR was detected either with a PE- or APC-conjugated anti-EGFR antibody (clone AY13, BioLegend). Analysis was done by the FlowJo software.

Construction of transgene constructs:

The nucleotide sequences encoding the signal peptide CD33, the human CD8α hinge, human monomeric CD8α hinge (UniProt ID P01732, C164S and C181S) and CD8α transmembrane domain, the 4-1BB co-stimulatory domain and the CD3ζ ITAM signalling domain were synthesized by GenScript. Sequences encoding the extracellular and transmembrane domain of EGFR was obtained from Addgene (plasmid #11011). Insertion of a Strep II tag (NWSHPQFEK) and flexible linkers was performed by PCR. Assembly of nucleotide sequences into functional transgenes was performed by using the Gibson Assembly Master Mix (New England BioLabs), according to the manufacturer's instructions. The schematics and sequences are shown in Fig. 14 and 15, respectively. The resulting constructs were amplified by PCR and subsequently used for *in vitro* transcription.

Luciferase-based cytotoxicity assay:

Luciferase-expressing tumour cells were co-cultured with CAR T cells at an E:T cell ratio of 2:1 with 10,000 target cells/well in white round-bottom 96 well plates (Sigma Aldrich) for 4 hours at 37°C in cytotoxicity assay medium, consisting of phenol-free RPMI (Thermo Scientific), 10% FCS, 1% L-Glutamine (Thermo Scientific) and 1% penicillin-streptomycin. Finally, remaining living cells were quantified by determination of the residual luciferase activity of the co-culture. After equilibration to room temperature for 10 minutes, luciferin was added to the cell suspension (150 μg/mL final concentration; Perkin Elmer) and luciferase activity was measured 20 minutes later using the ENSPIRE Multimode plate reader. The percentage of specific lysis was determined with the following formula:

% specific lysis = 100 - ((RLU from well with effector and target cell co-culture) / (RLU from well with target cells only) x 100)).

Cytokine release by CAR T cells:

Cytokine secretion of primary CAR T cells was assessed by co-cultivation with target cells at E:T ratios of 1:1 or 2:1 in flat-bottom 96 well plates for 4 hours or 24 hours at 37°C. In some experiments, released cytokines were quantified in the the supernatants from the co-culture experiments for determining cytotoxicity. The supernatants were centrifuged (1600 rpm, 7 minutes, 4°C) to remove remaining cells and debris and were subsequently frozen at -80°C. For analysis of secreted IFN-γ, ELISA was performed using the Human IFN gamma ELISA Ready-SET-Go!® kit (eBioscience) according to the manufacturer's instructions. Measurements were conducted using the ENSPIRE Multimode plate reader.

**Example 3: Single-chain variable fragments (scFv) can trigger CAR clustering in cell membranes and thereby prevent the exploitation of the avidity effect for reversible control of CAR function**

The third example demonstrates that the integration of scFv-based binding moieties in CAR molecules can

prevent the exploitation of the avidity effect for specific recognition of antigen combinations. The schematics of the CAR constructs shown in Figure 4A illustrate the design of the tested CAR variants (4D5-5-8cys-BB-3z, 4D5-5-8ser-BB-3z, 4D5-5(split)-8ser-BB-FKBP(36V)-3z). In the shown example the scFv 4D5-5 directed against HER2 was used as an antigen binding moiety and incorporated into either a monomeric ("Ser") or a dimeric ("Cys") CAR signalling backbone. Figure 4B shows the expression of the CARs in primary T cells. The effective binding affinity for the scFv 4D5-5 was reported to be 1.1 μM (Liu et al., Cancer Res. 2015;75(17):3596-3607), which is comparable to the affinity of E11.4.1-G32A. Jurkat T cells expressing a truncated form of HER2 (tHER2) served as a target cell line (Figure 3E). IFN-γ secretion by the CAR T cells (Figure 3F) and lysis of target cells (Figure 3G) triggered by the serine-containing CAR "4D5-5-8ser-BB-3z" is only slightly diminished compared to the cysteine-containing CAR "4D5-5-8cys-BB-3z", despite the low-affinity scFv, which is in stark contrast to the observations with the low-affinity rcSso7d-based antigen binding moiety in the CAR S(G32A)-8ser-BB-3z (SEQ ID NO: 44)) of Example 2 (Figure 3). As observed in diabody formation, $V_H$ and $V_L$ linked together in an scFv at the surface of T cells can not only dimerize within the same single-chain molecule (i.e. within the same CAR molecule), but can also form intermolecular links (i.e. between different CAR molecules). This would mediate dimerization or even oligomerization as reported for purified scFv proteins (Atwell et al., Protein Eng. 1999;12(7):597-604) and would explain the previously observed CAR clustering (Long et al., Nat Med. 2015;21(6):581-590). Ultimately, this dimerization or oligomerization of the scFvs would result in the formation of bivalent or multivalent CARs, even when based on a monomeric CAR backbone. Accordingly, cutting the linker between $V_H$ and $V_L$ would prevent oligomerization and therefore activation of low-affinity CARs based on monomeric CAR backbones. We further assumed that $V_H$ and $V_L$ domains without linker still can at least partially heterodimerize on the surface of T cells to form functional $V_H$/$V_L$-heterodimers (i.e., Fvs). Provided that there is no unspecific stickiness between Fvs, these Fvs due to their low affinity (Kd 1.1 μM in the case of 4D5-5) should be able to trigger T cell activation only upon controlled dimerization of two Fvs (via an FKBP F36V-domain intracellularly fused to the $V_H$ carrying chain). Indeed, Fig. 4H illustrates that this is the case, as could be shown by separating $V_H$ and $V_L$ of the low-affinity scFv 4D5-5 onto two separate membrane-anchored molecules (SEQ ID NO: 56 and SEQ ID NO: 57). As predicted, CAR T cells expressing these constructs (Figs. 4C and 4D) were not activated by tHER2$^{pos}$ target cells. However, the T cells were activated by those target cells when the $V_H$ construct was homodimerized by AP20187 (Figure 4H). This T cell activation in the presence of dimerizer confirmed that the two separate constructs indeed formed functional Fvs on the T cell surface and that the lack of activation in the absence of dimerizer was due to the monovalent nature of the Fvs. This agrees with the low affinity of 4D5-5 and with the observations obtained with the rcSso7d-based antigen binding moiety in Example 2. For comparison we roughly adjusted the expression of the scFv-version (i.e. $V_H$ and $V_L$ connected by the linker "218") to the expression levels obtained with the $V_H$ construct (Figure 4C), which despite the low expression still resulted in strong activation of the CAR T cells (Figure 4H). Together, the data in Figure 4 strongly suggest that at least certain versions of scFvs partly dimerize (or oligomerize) by intermolecular heterodimerization of $V_H$ and $V_L$ between neighbouring molecules on the T cell surface. Similar to dimerization or oligomerization caused by cysteine amino acid residues (discussed above), uncontrolled dimerization or oligomerization of CAR molecules mediated by at least certain scFvs variants can cause homodimerization or homooligomerization independent of a regulating molecule. Therefore, in preferred embodiments, the antigen binding moieties of the CAR molecules of the group of CARs, or the antigen binding moieties of the other polypeptides binding to the CAR molecules of the group, according to the present invention are not scFvs.

Maintenance of human cell lines:

Primary human T cells were obtained from de-identified healthy donor's blood after apheresis (Buffy coats from the Austrian Red Cross, Vienna, Austria). CD3$^{pos}$ T cells were enriched by negative selection using RosetteSep Human T cell Enrichment Cocktail. Isolated and purified T cells were cryopreserved in RPMI-1640 medium supplemented with 20% FCS and 10% DMSO until use. CD3$^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. Jurkat T cells were a gift from Dr. Sabine Strehl at the CCRI and were maintained in RPMI-1640 supplemented with 10% FCS and 1% penicillin-streptomycin. Cell lines were regularly tested for mycoplasma contamination and authentication was performed at Multiplexion, Germany. Cell densities were monitored with AccuCheck counting beads.

*In vitro* transcription and electroporation of mRNA:

*In vitro* transcription was performed using the mMessage mMachine T7 Ultra Kit according to the manufacturer's instructions. 50-200 ng of column purified PCR product was used as a reaction template. The resulting mRNA was purified with an adapted protocol using the RNeasy column purification kit. Briefly, the mRNA solution was diluted with

a mixture of RLT buffer, ethanol and 2-mercaptoethanol. The mixture was loaded onto an RNeasy column and purification was performed according to the manufacturer's instructions. Elution was performed with nuclease-free water and purified mRNAs were frozen at -80°C until electroporation. For transient transgene expression, primary T cells or Jurkat T cells were electroporated with varying amounts of the respective mRNA using the Gene Pulser (Biorad). Following protocols were used for the respective cell types: primary T cells (square wave protocol, 500 V, 5 ms and 4 mm cuvettes), Jurkat T cells (square wave protocol, 500 V, 3 ms and 4 mm cuvettes).

Antibodies and flow cytometry:

Primary human T cells or tumour cell lines were resuspended in FACS buffer (PBS, 0.2 % human albumin and 0.02 % sodium azide) and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected via the Strep II tag using an anti-Strep II tag antibody (clone 5A9F9, Genscript) or via the FLAG tag using an anti-FLAG tag antibody (clone L5, BioLegend) as a primary antibody and a PE- or APC-conjugated secondary antibody in case of the Strep II tag antibody. Expression of the engineered target antigen tHER2 was detected with a PE-conjugated anti-HER2 antibody (clone 24D2, BioLegend). Analysis was done by the FlowJo software.

Construction of transgene constructs:

The nucleotide sequences encoding the GM-CSF-Ra signal peptide, the anti-human CD19 scFv FMC63, the human CD8α hinge, the human monomeric CD8α hinge (UniProt ID P01732, C164S and C181S) and CD8α transmembrane domain, the 4-1BB co-stimulatory domain and the CD3ζ ITAM signalling domain were synthesized by GenScript. The nucleotide sequences encoding the signal peptide IgGk, the anti-human HER2 scFv 4D5-5 and the dimerization domain FKBP F36V were synthesized by GeneArt (Thermo Scientific). Sequences encoding the extracellular and transmembrane domain of HER2 were obtained from Addgene (plasmid #16257). Insertion of a Strep II tag (NWSHPQFEK) or FLAG tag (DYKDDDDK) and flexible linkers was performed by PCR. Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for *in vitro* transcription.

Luciferase-based cytotoxicity assay:

Luciferase-expressing tumour cells were co-cultured with CAR T cells at an E:T cell ratioof of 2:1 with 10,000 target cells/well in white round-bottom 96 well plates for 4 hours at 37°C in cytotoxicity assay medium, consisting of phenol-free RPMI, 10% FCS, 1% L-Glutamine and 1% penicillin-streptomycin. Finally, remaining living cells were quantified by determination of the residual luciferase activity of the co-culture. After equilibration to room temperature for 10 minutes, luciferin was added to the cell suspension (150 µg/mL final concentration) and luciferase activity was measured 20 minutes later using the ENSPIRE Multimode plate reader. The percentage of specific lysis was determined with the following formula:

% specific lysis = 100 - ((RLU from well with effector and target cell co-culture) / (RLU from well with target cells only) x 100)).

FACS-based cytotoxicity assay:

For the FACS-based cytotoxicity assay two populations of target cells were generated: (i) Jurkat cells electroporated with mRNA encoding eGFP and RNA encoding the respective target antigen, and (ii) Jurkat cells only electroporated with mRNA encoding mCherry. These two populations were mixed at a 1:1 ratio and co-cultured with CAR T cells at an E:T cell ratio of 4:1:1 with 20,000 target cells/well in round-bottom 96 well plates for 4 hours at 37°C. Target cells without the addition of CAR T cells served as a control condition ("targets only"). After the incubation period, the co-cultures were centrifuged (5 minutes, 1600 rpm, 4°C), supernatants were collected for subsequent cytokine measurements and the remaining cells were resuspended in 100 µL of FACS buffer, consisting of PBS, 0.2 % human albumin and 0.02 % sodium azide. The viability of target antigen$^{pos}$ and target antigen$^{neg}$ cell populations was determined using a BD LSRFortessa flow cytometer and specific lysis was calculated with the following formula:

% specific lysis = (1-(((% $eGFP^{pos}$ cells of the sample)/(% $mCherry^{pos}$ cells of the sample)/(% $eGFP^{pos}$ cells of the "targets only" control)/(% $mCherry^{pos}$ cells of the "targets only" control))))*100.

Cytokine release by CAR T cells:

Cytokine secretion of primary CAR T cells was assessed by co-cultivation with target cells at E:T ratios of 1:1 or 2:1 in flat-bottom 96 well plates for 4 hours or 24 hours at 37°C. In some experiments, released cytokines were quantified in the the supernatants from the co-culture experiments for determining cytotoxicity. The supernatants were centrifuged (1600 rpm, 7 minutes, 4°C) to remove remaining cells and debris and were subsequently frozen at -80°C. For analysis of secreted IFN-γ, ELISA was performed using the Human IFN gamma ELISA Ready-SET-Go!® kit (eBioscience) according to the manufacturer's instructions. Measurements were conducted using the ENSPIRE Multimode plate reader.

*In vitro* dimerization of transgenes:

Dimerization of transgenes was induced prior to co-cultivation experiments. Primary T cells were diluted to the final cell concentration in the respective cell culture medium. The homodimerization agent AP20187 (MedChemExpress) was diluted in cell culture medium and was added at 10 nM final concentration. Addition of the respective vehicle control DMSO at the same concentration served as control. Cells were incubated at 37°C for 30 minutes to ensure efficient dimerization of transgenes and subsequently used for *in vitro* experiments.

**Example 4: Generation of switchable CARs by regulating avidity via homodimerization of the domain FKBP F36V**

The fourth example illustrates the regulation of CAR function by regulating the avidity of a group of CARs by conditional homodimerization. For this purpose, we integrated into a monomeric CAR backbone a homodimerization domain based on the FKBP F36V variant (shown in Figure 5A) and fused this backbone to a binding moiety with high or with low affinity towards EGFR, i.e., S(WT)-8ser-BB-FKBP(36V)-3z "E11.4.1-WT" (SEQ ID NO: 49) and S(G32A)-8ser-BB-FKBP(36V)-3z "E11.4.1-G32A" (SEQ ID NO: 46), respectively. Primary human T cells were transduced with lentiviral vectors encoding the resulting CAR molecules. Expression of the CARs was detected via the Strep II tag and is shown in Fig. 5B. For functional analysis, CAR T cells were co-cultured for 4 hours at 37°C at an E:T ratio of 10:1 with the target cell line A431-fLuc, which expresses high levels of EGFR (Figure 5C). Cytotoxicity of target cells was determined in a luciferase-based cytotoxicity assay. T cells expressing a CD19-specific reference CAR CD19-8cys-BB-3z "CD19-BBz" (SEQ ID NO: 58) and T cells without any CAR ("no CAR") were used as control conditions. CAR dimerization was induced by addition of 10 nM AP20187 to the T cells prior to co-cultivation with the target cells. Addition of the vehicle control DMSO served as control. Figure 5D shows that the function of a CAR with the low affinity-binding moiety E11.4.1-G32A (S(G32A)-8ser-BB-FKBP(36V)-3z (SEQ ID NO: 46)) but not with the high affinity-binding moiety E11.4.1-WT (S(WT)-8ser-BB-FKBP(36V)-3z (SEQ ID NO: 49)) strongly depended on the presence of the dimerization agent AP20187. The dimerization agent itself had no effects on the lysis of target cells, as can be seen in the control conditions with T cells without CAR expression.

Maintenance of human cell lines

Primary human T cells were obtained from de-identified healthy donor's blood after apheresis (Buffy coats from the Austrian Red Cross, Vienna, Austria). $CD3^{pos}$ T cells were enriched by negative selection using RosetteSep Human T cell Enrichment Cocktail. Isolated and purified T cells were cryopreserved in RPMI-1640 medium supplemented with 20% FCS and 10% DMSO until use. $CD3^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. A431 epidermoid carcinoma cells were maintained in DMEM (Thermo Scientific) supplemented with 10% FCS and 1% penicillin-streptomycin. Cell lines were regularly tested for mycoplasma contamination and authentication was performed at Multiplexion, Germany. Cell densities were monitored with Accu-Check counting beads.

Transduction of T cells and cell lines

Virus production of pantropic VSV-G pseudotyped lentivirus was performed from Lenti-X 293T cells (Clontech Laboratories) with a third-generation Puromycin-selectable pCDH transgene vector (System Biosciences) and second-generation viral packaging plasmids pMD2.G and psPAX2 (both obtained from Addgene, plasmids #12259 and #12260 respectively). Co-transfection was performed using Purefection Transfection Reagent (System Biosciences) according to the manufacturer's instructions. Supernatants were collected one and two days after transfection and were concentrated using the Lenti-X Concentrator (Clontech Laboratories) according to the manufacturer's instructions.

Twenty-four hours prior to the lentiviral transduction, primary T cells were activated using anti-CD3/28 beads, according to the manufacturer's instructions. Cell culture plates were coated with RetroNectin (Clontech Laboratories), according to the manufacturer's instructions, to promote co-localization of lentivirus and primary T cells. Cells were exposed to concentrated lentiviral supernatants for one day, followed by removal of the virus particles. After three days, T cells were treated with 1 μg/mL Puromycin (Sigma Aldrich) to ensure high and uniform expression of the transgene. T cells were expanded in T cell transduction medium, consisting of AIM-V (Life Technologies) supplemented with 2% Octaplas (Octapharma), 1% L-Glutamine, 2.5% HEPES (Thermo Scientific) and 200 IU/mL recombinant human IL-2.

Cell lines were split 24 hours before lentiviral transduction to ensure exponential cell growth at the timepoint of transduction. Cells were exposed to varying concentrations of lentiviral supernatants for one day. Puromycin selection was performed three days after transduction with concentrations varying from 1 to 8 μg/mL to exclude non-transduced cells.

Antibodies and flow cytometry

Primary human T cells or tumour cell lines were resuspended in FACS buffer (PBS, 0.2 % human albumin and 0.02 % sodium azide and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected via the Strep II tag using an anti-Strep II tag antibody (clone 5A9F9, Genscript) or with Protein L in case of the CD19-BBz CAR as primary antibodies and a PE- or APC-conjugated secondary antibody. Expression EGFR was detected with a PE-conjugated anti-EGFR antibody (clone AY13, BioLegend). Analysis was done by the FlowJo software.

Construction of transgene constructs

The nucleotide sequences encoding the CD33 signal peptide, the low affinity rcSso7d variant E11.4.1-G32A, the Strep II tag (NWSHPQFEK), a flexible $G_4S$ linker, the human monomeric CD8α hinge (UniProt ID P01732, C164S and C181S) and CD8α transmembrane domain, the 4-1BB co-stimulatory domain, the dimerization domain FKBP F36V and the CD3ζ ITAM signalling domain were synthesized by GeneArt (Thermo Scientific). Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for *in vitro* transcription.

Luciferase-based cytotoxicity assay

Luciferase-expressing tumour cells were co-cultured with CAR T cells at an E:T cell ratio of 2:1 with 10,000 target cells/well in white round-bottom 96 well plates for 4 hours at 37°C in cytotoxicity assay medium, consisting of phenol-free RPMI, 10% FCS, 1% L-Glutamine and 1% penicillin-streptomycin. Finally, remaining living cells were quantified by determination of the residual luciferase activity of the co-culture. After equilibration to room temperature for 10 minutes, luciferin was added to the cell suspension (150 μg/mL final concentration) and luciferase activity was measured 20 minutes later using the ENSPIRE Multimode plate reader. The percentage of specific lysis was determined with the following formula:

% specific lysis = 100 - ((RLU from well with effector and target cell co-culture) / (RLU from well with target cells only) x 100)).

*In vitro* dimerization of transgenes

Dimerization of transgenes was induced prior to co-cultivation experiments. Primary T cells were diluted to the final cell concentration in the respective cell culture medium. The homodimerization agent AP20187 was diluted in cell culture medium and was added at 10 nM final concentration. Addition of the respective vehicle control DMSO at the same concentration served as control. Cells were incubated at 37°C for 30 minutes to ensure efficient dimerization of transgenes and subsequently used for *in vitro* experiments.

**Example 5: Treatment of tumour bearing mice with stably transduced T cells expressing a group of CARs whose avidity can be controlled by drug administration**

In example 5 we show in a leukaemia model with immunodeficient NOD.Cg-*Prkdc*$^{scid}$ *Il2rg*$^{tm1WJl}$/*Sz*J (NSG) mice that tumour growth can efficiently be inhibited by lentivirally transduced T cells expressing the low-affinity CAR "S(G32A)-8ser-BB-FKBP(36V)-3z" (SEQ ID NO: 46) in the presence but not the absence of a regulating molecule.

For the *in vivo* model we used the B-ALL cell line Nalm6 which was transduced with a vector for high expression of tEGFR (approx. $1x10^6$ tEGFR molecules per cell) and firefly luciferase for *in vivo* quantification of tumour growth by using bioluminescence imaging. Intravenous (i.v.) injection of $0.5x10^6$ Nalm6-tEGFR-fLuc cells into NSG mice resulted in exponential tumour growth in untreated mice. Figure 6A shows that the growth of this cell line in NSG mice, however, was efficiently inhibited when $10x10^6$ T cells expressing either an anti-CD19 CAR CD19-8cys-BB-3z (SEQ ID NO: 58) or the high-affinity anti-EGFR CAR "S(WT)-8ser-BB-FKBP(36V)-3z" (SEQ ID NO: 49) were intravenously injected three days after injection of the tumour cells. Importantly, also T cells with the low-affinity EGFR-CAR "S(G32A)-8ser-BB-FKBP(36V)-3z" (SEQ ID NO: 46) inhibited leukaemia outgrowth, but only if the homodimerizer AP20187, i.e., the regulating molecule, was regularly administered (Figure 6A), demonstrating that the CAR S(G32A)-8ser-BB-FKBP(36V)-3z is only fully active in the dimeric state (i.e., complexed group of CARs = ON-state). In the absence of the dimerizer (i.e., non-complexed CARs = OFF-state) we observed only a moderate growth inhibition (Figure 6A). When the dimerizer, i.e., the regulating molecule, was administered to this latter group of mice 11 days after T cell injection, then the triggered complexation of the CAR molecules resulted in a strong reduction of tumour burden in 2 mice and moderate inhibition in the 3 other mice (Figure 6B). Together, these in vivo experiments confirm our *in vitro* data, demonstrating that cells, which express CAR molecules with low affinity antigen binding moieties, are only efficiently triggered, if the CAR molecules are complexed (i.e. assembled) into a group of CARs, thereby resulting in avidity.

Expression of the CARs was detected with an anti-Strep II tag antibody in case of rcSso7d-based CARs and with Protein L in case of the CD19-specific CAR (Figures 7A and 7B, respectively). For the functional characterization *in vitro,* the CAR T cells were co-cultured with Nalm6 cells that were either expressing no EGFR ("Nalm6-fLuc") or high levels of EGFR ("Nalm6-tEGFR-fLuc") (Figures 7E and 7F) for 4 hours at 37°C at an E:T ratio of 10:1. CAR homodimerization was induced by addition of 10 nM AP20187 to the T cells prior to co-cultivation with the target cells. Addition of the vehicle control DMSO served as control. Figures 7C and 7D show the cytolytic capacity of CAR T cells co-cultured with either Nalm6-fLuc or Nalm6-EGFR-fLuc cells, respectively.

Maintenance of human cell lines:

Primary human T cells were obtained from de-identified healthy donor's blood after apheresis (Buffy coats from the Austrian Red Cross, Vienna, Austria). CD3$^{pos}$ T cells were enriched by negative selection using RosetteSep Human T cell Enrichment Cocktail. Isolated and purified T cells were cryopreserved in RPMI-1640 medium supplemented with 20% FCS and 10% DMSO until use. CD3$^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. Cell lines were regularly tested for mycoplasma contamination and authentication was performed at Multiplexion, Germany. Cell densities were monitored with AccuCheck counting beads.

Transduction of T cells and cell lines:

Virus production of pantropic VSV-G pseudotyped lentivirus was performed from Lenti-X 293T cells with a third-generation Puromycin-selectable pCDH transgene vector and second-generation viral packaging plasmids pMD2.G and psPAX2 (both obtained from Addgene, plasmids #12259 and #12260 respectively). Co-transfection was performed using Purefection Transfection Reagent according to the manufacturer's instructions. Supernatants were collected one and two days after transfection and were concentrated using the Lenti-X Concentrator according to the manufacturer's instructions.

Twenty-four hours prior to the lentiviral transduction, primary T cells were activated using anti-CD3/28 beads,

according to the manufacturer's instructions. Cell culture plates were coated with RetroNectin, according to the manufacturer's instructions, to promote co-localization of lentivirus and primary T cells. Cells were exposed to concentrated lentiviral supernatants for one day, followed by removal of the virus particles. After three days, T cells were treated with 1 μg/mL Puromycin to ensure high and uniform expression of the transgene. T cells were expanded in T cell transduction medium, consisting of AIM-V supplemented with 2% Octaplas, 1% L-Glutamine, 2.5% HEPES and 200 IU/mL recombinant human IL-2.

Cell lines were split 24 hours before lentiviral transduction to ensure exponential cell growth at the time point of transduction. Cells were exposed to varying concentrations of lentiviral supernatants for one day. Puromycin selection was performed three days after transduction with concentrations varying from 1 to 8 μg/mL in order to exclude non-transduced cells.

Construction of transgene constructs:

The nucleotide sequences encoding the CD33 signal peptide, the low affinity rcSso7d variant E11.4.1-G32A, the Strep II tag (NWSHPQFEK), a flexible $G_4S$ linker, the human monomeric CD8α hinge (UniProt ID P01732, C164S and C181S) and CD8α transmembrane domain, the 4-1BB co-stimulatory domain, the dimerization domain FKBP F36V and the CD3ζ ITAM signalling domain were synthesized by GeneArt (Thermo Scientific). The nucleotide sequences encoding the GM-CSF-Ra signal peptide and the anti-human CD19 scFv FMC63 were synthesized by GenScript. The nucleotide sequence encoding the extracellular and transmembrane domain of EGFR was obtained from Addgene (plasmid #11011). Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for *in vitro* transcription.

Antibodies and flow cytometry:

Primary human T cells or tumour cell lines were resuspended in FACS buffer (PBS, 0.2 % human albumin and 0.02 % sodium azide and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected via the Strep II tag using an anti-Strep II tag antibody (clone 5A9F9, Genscript) or Protein L in case of the CD19-BBz CAR as primary antibodies and a PE- or APC-conjugated secondary antibody. Expression EGFR was detected with a PE-conjugated anti-EGFR antibody (clone AY13, BioLegend). Analysis was done by the FlowJo software.

Luciferase-based cytotoxicity assay:

Luciferase-expressing tumour cells were co-cultured with CAR T cells at an E:T cell ratio of 2:1 with 10,000 target cells/well in white round-bottom 96 well plates for 4 hours at 37°C in cytotoxicity assay medium, consisting of phenol-free RPMI, 10% FCS, 1% L-Glutamine and 1% penicillin-streptomycin. Finally, remaining living cells were quantified by determination of the residual luciferase activity of the co-culture. After equilibration to room temperature for 10 minutes, luciferin was added to the cell suspension (150 μg/mL final concentration) and luciferase activity was measured 20 minutes later using the ENSPIRE Multimode plate reader. The percentage of specific lysis was calculated with the following formula:

% specific lysis = 100 - ((RLU from well with effector and target cell co-culture) / (RLU from well with target cells only) x 100)).

*In vitro* dimerization of transgenes:

Dimerization of transgenes was induced prior to co-cultivation experiments. Primary T cells were diluted to the final cell concentration in the respective cell culture medium. The homodimerization agent AP20187 was diluted in cell culture medium and was added at 10 nM final concentration. Addition of the respective vehicle control DMSO at the same concentration served as control. Cells were incubated at 37°C for 30 minutes to ensure efficient dimerization of transgenes and subsequently used for *in vitro* experiments.

*In vivo* target cell killing:

NOD. Cg-*Prkdc*$^{scid}$ *Il2rg*$^{tm1WJl}$/SzJ (NSG) mice were housed in the Anna Spiegel facility for animal breeding. For subsequent experiments, mice were transferred to the preclinical research laboratories (PIL) of the Medical University of Vienna. All procedures were performed as approved (GZ: 813267/2015/24) by the Magistratsabteilung 58, Vienna.

Primary T cells were engineered to express the CD19-specific control CAR (CD19-BBz), the EGFR-specific high and low affinity CARs ("E11.4.1-WT" and "E11.4.1-G32A", respectively) using the protocol depicted in "Transduction of T cells and cell lines". After transduction, the CAR T cells were expanded for over 14 days prior to *in vivo* experiments to generate sufficient cell numbers.

The homodimerization agent AP20187 (Clontech Laboratories) was dissolved in vehicle solution according to manufacturer's instructions. Briefly, AP20187 was initially dissolved to a concentration of 12.5 mg/mL in ethanol with rigorous vortexing. The compound was then diluted to the final working concentration of 0.5 mg/mL using an appropriate mixture of PEG-400 (Sigma Aldrich) and Tween-80 (Sigma Aldrich) in water. The resulting vehicle solution consisted of 4% ethanol, 10% PEG-400 and 1.7% Tween-80 in water for injection. The working stock of AP20187 was prepared immediately prior to injection, sterile-filtered and was used within 30 minutes.

Nalm6 cells engineered to express high levels of tEGFR-FKBP and fLuc ("Nalm6-tEGFR-fLuc") were resuspended in PBS, filtered through a 35 μm cell strainer (Corning Falcon) and set to a final concentration of $5x10^6$/mL. $0.5x10^6$ cells were injected intravenously (i.v.) into the tail-vein of each NSG mouse (male and female mice; Medical University of Vienna, Department of Biomedical Research). Three days later, mice were treated with respective CAR T cells ($10x10^6$ CAR T cells *i.v.* into the tail-vein), followed by injection of the homodimerization agent AP20187 (2 mg/kg dosage) or vehicle control using intraperitoneal (*i.p.*) injections. The dimerization agent AP20187 (2 mg/kg) or the vehicle control was administered on day 0 (right after T cell injection), day 1, day 2, day 4, day 7, day 9 and day 11 where indicated. All control conditions were treated with the respective vehicle control (4% ethanol, 10% PEG-400 and 1.7% Tween-80 in water for injection). Tumour growth and control was monitored by bioluminescence imaging (BLI). Mice were sacrificed by cervical dislocation at the end of the experiment.

Bioluminescence imaging (BLI):

BLI imaging of tumour growth was performed at the preclinical research laboratory (PIL) of the Medical University of Vienna using an IVIS Spectrum In Vivo Imaging System (Perkin Elmer). D-Luciferin substrate (Perkin Elmer) was dissolved in PBS to a final concentration of 15 mg/mL and sterile filtered. Mice were anesthetized with isoflurane and received *i.p.* injections of the luciferin working stock (final dosage 150 mg/kg body weight). After 15-20 minutes, 1-3 mice were transferred to the IVIS Imaging System, bioluminescence was measured in medium binning mode at an acquisition time of 1 seconds to 2 minutes to obtain unsaturated images. Luciferase activity was analysed with the Living Image Software (Caliper) and photon flux was determined within the region of interest that encompassed the entire body of the mouse.

**Example 6: Regulating CAR avidity by heterodimerization of CAR molecules and sensitivity of the CAR T cells depending on whether the target antigen is monomeric or dimeric**

In Example 6 the avidity of CAR molecules was regulated by heterodimerization. Further, the example shows the sensitivity of T cells expressing a group of CARs (S(G32A)-8ser-BB-FKBP-3z plus S(G32A)-8ser-BB-FRB-3z) comprising the low affinity binding moiety "E11.4.1-G32A" in comparison to T cells expressing a group of CARs (S(WT)-8ser-BB-FKBP-3z plus S(WT)-8ser-FRB-3z) comprising the high affinity binding moiety "E11.4.1-WT" plus/minus regulating molecule and the impact on whether the target antigens are monomeric or associated.

The target cells (Jurkat T cells) were electroporated with different amounts of mRNA (0.02 μg - 10 μg) coding for tEGFR which yielded expression levels of tEGFR ranging from few hundred molecules/cell up to ~300,000 molecules/cell. The expression of tEGFR (MFI and molecules/cell) in Jurkat T cells is shown in Figures 8A-C. To minimize cross-talk between dimerization domains, orthogonal dimerization systems were used for target and effector cells. For this purpose, we integrated the low-affinity antigen binding moiety E11.4.1-G32A and the high-affinity antigen binding moiety E11.4.1-WT into the monomeric CAR backbones 8ser-BB-FKBP-3z and 8ser-BB-FRB-3z (S(G32A)-8ser-BB-FKBP-3z + S(G32A)-8ser-BB-FRB-3z for E11.4.1-G32A and S(WT)-8ser-BB-FKBP-3z + S(WT)-8ser-BB-FRB-3z for E11.4.1-WT) comprising the heterodimerization domains FKBP or FRB, respectively, for conditional heterodimerization of the resulting CAR molecules by the rapalogue AP21967 (shown in Fig. 8F). Primary human T cells were electroporated with 5 μg mRNA for each construct and expression was detected 20 hours after electroporation with Strep II tag and FLAG tag (Figures 8D and 8E). Figures 8G and 8H show the capacity of T cells expressing the CARs with low and high affinity binding domains to trigger cytotoxicity and secrete cytokines depending on the number of antigen molecules present on surface of Jurkat T cells in presence or absence of AP21967 (for heterodimerization of the CARs) and

AP20187 (for homodimerization of tEGFR). The group of CARs comprising the high-affinity CAR molecules S(WT)-8ser-BB-FKBP-3z and S(WT)-8ser-BB-FRB-3z triggered efficient effector functions at low antigen doses independent of dimerization of the antigen or the group of CARs. The group of CARs comprising the low-affinity CAR molecules S(G32A)-8ser-BB-FKBP-3z and S(G32A)-8ser-BB-FRB-3z was highly dependent on the presence of the regulating molecule AP21967 and did not or only poorly trigger effector functions in the absence of AP21967 even at high antigen doses. Potent cytolytic functions and secretion of cytokines by the T cells was only observed, when both the CAR molecules and the antigen molecules were dimerized.

Maintenance of human cell lines

Primary human T cells were obtained from de-identified healthy donor's blood after apheresis (Buffy coats from the Austrian Red Cross, Vienna, Austria). $CD3^{pos}$ T cells were enriched by negative selection using RosetteSep Human T cell Enrichment Cocktail. Isolated and purified T cells were cryopreserved in RPMI-1640 medium supplemented with 20% FCS and 10% DMSO until use. $CD3^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. Jurkat T cells were a gift from Dr. Sabine Strehl at the CCRI and were maintained in RPMI-1640 supplemented with 10% FCS and 1% penicillin-streptomycin. Cell lines were regularly tested for mycoplasma contamination and authentication was performed at Multiplexion, Germany. Cell densities were monitored with AccuCheck counting beads.

Antibodies and flow cytometry

Primary human T cells or tumour cell lines were resuspended in FACS buffer (PBS, 0.2 % human albumin and 0.02 % sodium azide and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected either via the Strep II tag using an anti-Strep II tag antibody (clone 5A9F9, Genscript) or via the FLAG tag using an anti-FLAG tag antibody (clone L5, BioLegend) as a primary antibody and a PE- or APC-conjugated secondary antibody, in the case of anti-Strep II tag antibodies. Expression of tEGFR was detected via a PE- or APC-conjugated anti-EGFR antibody (clone AY13, BioLegend). Analysis was done by the FlowJo software.

Determination of transgene surface densities

The number of molecules of tEGFR and the different CARs on the surface of the different cells was quantified using the QuantiBRITE Phycoerythrin Fluorescence Quantitation Kit (Becton Dickinson) according to the manufacturer's instructions. Transgene-expressing cells and non-transfected control cells were stained with saturating concentrations of the respective PE-labelled antibodies using the protocol described in "Antibodies and flow cytometry". The geometric mean of the fluorescence intensity was determined, corrected for unspecific binding using control cells and used to estimate the number of antibodies bound per cell (ABC).

Construction of transgene constructs

The nucleotide sequences encoding the CD33 signal peptide, low affinity rcSso7d variant E11.4.1-G32A, the Strep II tag (NWSHPQFEK), a flexible $G_4S$ linker, the human monomeric CD8$\alpha$ hinge (UniProt ID P01732, C164S and C181S) and CD8$\alpha$ transmembrane domain, the 4-1BB co-stimulatory domain, the dimerization domains FKBP F36V and FRB and the CD3$\zeta$ ITAM signalling domain were synthesized by GeneArt (Thermo Scientific). The nucleotide sequences encoding the dimerization domain FKBP was synthesized by Genscript. The sequence encoding the extracellular and transmembrane domain of EGFR was obtained from Addgene (plasmid #11011). Insertion of flexible linkers was performed by PCR. Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for *in vitro* transcription.

*In vitro* transcription and electroporation of mRNA

*In vitro* transcription was performed using the mMessage mMachine T7 Ultra Kit according to the manufacturer's instructions. 50-200 ng of column purified PCR product was used as a reaction template. The resulting mRNA was

purified with an adapted protocol using the RNeasy column purification kit. Briefly, the mRNA solution was diluted with a mixture of RLT buffer, ethanol (Merck) and 2-mercaptoethanol. The mixture was loaded onto an RNeasy column and purification was performed according to the manufacturer's instructions. Elution was performed with nuclease-free water and purified mRNAs were frozen at -80°C until electroporation. For transient transgene expression, primary T cells or Jurkat T cells were electroporated with varying amounts of the respective mRNA using the Gene Pulser (Biorad). Following protocols were used for the respective cell types: primary T cells (square wave protocol, 500 V, 5 ms and 4 mm cuvettes), Jurkat T cells (square wave protocol, 500 V, 3 ms and 4 mm cuvettes).

FACS-based cytotoxicity assay

For the FACS-based cytotoxicity assay two populations of target cells were generated: (i) Jurkat cells electroporated with mRNA encoding eGFP and RNA encoding the respective target antigen, and (ii) Jurkat cells only electroporated with mRNA encoding mCherry. These two populations were mixed at a 1:1 ratio and co-cultured with CAR T cells at an E:T cell ratio of 4:1:1 with 20,000 target cells/well in round-bottom 96 well plates for 4 hours at 37°C. Target cells without the addition of CAR T cells served as a control condition ("targets only"). After the incubation period, the co-cultures were centrifuged (5 minutes, 1600 rpm, 4°C), supernatants were collected for subsequent cytokine measurements and the remaining cells were resuspended in 100 μL of FACS buffer, consisting of PBS, 0.2 % human albumin and 0.02 % sodium azide. The viability of target antigen$^{pos}$ and target antigen$^{neg}$ cell populations was determined using a BD LSRFortessa flow cytometer and specific lysis was calculated with the following formula:

% specific lysis = (1-(((% eGFP$^{pos}$ cells of the sample)/(% mCherry$^{pos}$ cells of the sample)/(% eGFP$^{pos}$ cells of the "targets only" control)/(% mCherry$^{pos}$ cells of the "targets only" control))))*100.

Cytokine release by CAR T cells

Cytokine secretion of primary CAR T cells was assessed by co-cultivation with target cells at E:T ratios of 1:1 or 2:1 in flat-bottom 96 well plates for 4 hours or 24 hours at 37°C. In some experiments, released cytokines were quantified in the the supernatants from the co-culture experiments for determining cytotoxicity. The supernatants were centrifuged (1600 rpm, 7 minutes, 4°C) to remove remaining cells and debris and were subsequently frozen at -80°C. For analysis of secreted IFN-γ, ELISA was performed using the Human IFN gamma ELISA Ready-SET-Go!® kit (eBioscience) according to the manufacturer's instructions. Measurements were conducted using the ENSPIRE Multimode plate reader.

*In vitro* dimerization of transgenes

Dimerization of transgenes was induced prior to co-cultivation experiments. Primary T cells and Jurkat T cells were diluted to the final cell concentration in the respective cell culture medium. The homodimerization agent AP20187 and the heterodimerization agent AP21967 (Clontech Laboratories) were diluted in cell culture medium and were added at 10 nM and 500 nM final concentration, respectively. Addition of the vehicle control DMSO and ethanol at the same concentration, respectively, served as control. Cells were incubated at 37°C for 30 minutes to ensure efficient dimerization of transgenes and subsequently used for *in vitro* experiments.

**Example 7: Regulation of the avidity of a group of CARs by VEGF**

The seventh example demonstrates a strategy to create a group of CARs that can be complexed by an extracellular soluble factor, which in this case serves as the regulating molecule according to the present invention. In the shown example VEGF was used as a potential dimerization agent (i.e. regulating molecule) for homodimerization of the CAR S(G32A)-J.CT6-8ser-BB-3z (SEQ ID NO: 64 and SEQ ID NO: 65). For this purpose, an engineered CH2-CH3-IgG1-Fc domain was integrated into the ectodomain of a CAR molecule (SEQ ID NO: 65) and co-expressed with a soluble construct comprising the CH2-CH3-Fc domain "Janus CT6" (SEQ ID NO: 64), that was engineered for high affinity binding to VEGF (Lobner et al., MAbs. 2017;9(7):1088-1104) and that covalently heterodimerizes via formation of disulphide bridges with the CH2-CH3-Fc domain in the ectodomain of the CAR molecule. The CH2-CH3 domains of both constructs were engineered for minimizing homodimerization (Lobner et al., MAbs. 2017;9(7):1088-1104). In the given example E11.4.1-G32A was used as antigen binding moiety due to its dependence on cooperative binding. Figure

9A shows a schematic representation of a VEGF-dependent EGFR-specific CAR comprising the two constructs (SEQ ID NO: 64 and SEQ ID NO: 65) and Figure 9B shows the effect of addition of VEGF. Jurkat T cells were electroporated with 5 μg mRNA for tEGFR and expression was detected 20 hours after electroporation (Figure 9C). Primary human T cells were electroporated with 5 μg mRNA for each construct. The T cells then expressed the CAR molecule comprising a monomeric second-generation CAR signalling backbone (SEQ ID NO: 65) that associates with the construct comprising the low-affinity E11.4.1-G32A binding moiety which was fused to the Janus-CT6-Fc domain and did not contain a transmembrane domain (SEQ ID NO: 64). CAR T cells expressing the two constructs (Figure 9D) were treated with varying amounts of VEGF and were co-cultivated with Jurkat T cells expressing high levels of tEGFR (Figure 9C). Figure 9E shows the capacity to trigger cytotoxicity as determined by using a FACS-based cytotoxicity assay. Figure 9E shows that the group of CARs triggered T cell cytotoxicity against target cells in a VEGF (i.e. regulating molecule)-dependent manner. This demonstrates that extracellular soluble factors such as VEGF can serve as regulating molecules, thereby promoting complexation of the group of CARs according to the present invention.

Maintenance of human cell lines:

Primary human T cells were obtained from de-identified healthy donor's blood after apheresis (Buffy coats from the Austrian Red Cross, Vienna, Austria). $CD3^{pos}$ T cells were enriched by negative selection using RosetteSep Human T cell Enrichment Cocktail. Isolated and purified T cells were cryopreserved in RPMI-1640 medium supplemented with 20% FCS (Sigma Aldrich) and 10% DMSO until use. $CD3^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. Jurkat T cells were a gift from Dr. Sabine Strehl at the CCRI and were maintained in RPMI-1640 supplemented with 10% FCS and 1% penicillin-streptomycin. Cell lines were regularly tested for mycoplasma contamination and authentication was performed at Multiplexion, Germany. Cell densities were monitored with AccuCheck counting beads.

Antibodies and flow cytometry:

Primary human T cells or tumour cell lines were resuspended in FACS buffer (PBS, 0.2 % human albumin and 0.02 % sodium azide and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected either via the Strep II tag using an anti-Strep II tag antibody (clone 5A9F9, Genscript) or via the Fc domain by using a biotinylated anti-human-IgGI-antibody (clone JDC-10, Biozol) as a primary antibody and a PE-conjugated streptavidin as secondary staining reagent. Expression of the engineered target antigen tEGFR was detected either with a PE- or APC-conjugated anti-EGFR antibody (clone AY13, BioLegend). Analysis was done by the FlowJo software.

Construction of transgene constructs:

The nucleotide sequences encoding the CD33 signal peptide, low affinity rcSso7d variant E11.4.1-G32A, the Strep II tag (NWSHPQFEK), a flexible $G_4S$ linker, the human monomeric CD8α hinge (UniProt ID P01732, C164S and C181S) and CD8α transmembrane domain, the 4-1BB co-stimulatory domain and the CD3ζ ITAM signalling domain were synthesized by GeneArt (Thermo Scientific). Plasmids comprising the CH2-CH3-Fc domain "Janus CT6" and the mutated "WT" CH2-CH3-Fc domain were a kind gift from Elisabeth Lobner at the University of Natural Resources and Life Sciences in Vienna. Sequences encoding the extracellular and transmembrane domains of EGFR were obtained from Addgene (plasmid #11011). Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for *in vitro* transcription.

*In vitro* transcription and electroporation of mRNA:

*In vitro* transcription was performed using the mMessage mMachine T7 Ultra Kit according to the manufacturer's instructions. 50-200 ng of column purified PCR product was used as a reaction template. The resulting mRNA was purified with an adapted protocol using the RNeasy column purification kit. Briefly, the mRNA solution was diluted with a mixture of RLT buffer, ethanol and 2-mercaptoethanol. The mixture was loaded onto an RNeasy column and purification was performed according to the manufacturer's instructions. Elution was performed with nuclease-free water and purified

mRNAs were frozen at -80°C until electroporation. For transient transgene expression, primary T cells or Jurkat T cells were electroporated with varying amounts of the respective mRNA using the Gene Pulser (Biorad). Following protocols were used for the respective cell types: primary T cells (square wave protocol, 500 V, 5 ms and 4 mm cuvettes), Jurkat T cells (square wave protocol, 500 V, 3 ms and 4 mm cuvettes).

FACS-based cytotoxicity assay:

For the FACS-based cytotoxicity assay two populations of target cells were generated: (i) Jurkat cells electroporated with mRNA encoding eGFP and RNA encoding the respective target antigen, and (ii) Jurkat cells only electroporated with mRNA encoding mCherry. These two populations were mixed at a 1:1 ratio and co-cultured with CAR T cells at an E:T cell ratio of 4:1:1 with 20,000 target cells/well in round-bottom 96 well plates for 4 hours at 37°C. Target cells without the addition of CAR T cells served as a control condition ("targets only"). After the incubation period, the co-cultures were centrifuged (5 minutes, 1600 rpm, 4°C), supernatants were collected for subsequent cytokine measurements and the remaining cells were resuspended in 100 μL of FACS buffer, consisting of PBS, 0.2 % human albumin and 0.02 % sodium azide. The viability of target antigen$^{pos}$ and target antigen$^{neg}$ cell populations was determined using a BD LSRFortessa flow cytometer and specific lysis was calculated with the following formula:

% specific lysis = (1-(((% eGFP$^{pos}$ cells of the sample)/(% mCherry$^{pos}$ cells of the sample)/(% eGFP$^{pos}$ cells of the "targets only" control)/(% mCherry$^{pos}$ cells of the "targets only" control))))*100.

Recombinant expression and purification of VEGF:

Recombinant expression of a truncated form of human VEGF (residues 14-108) was previously described by (Lobner et al., MAbs. 2017;9(7):1088-1104).

**Example 8: Generation of an affibody-based group of CARs directed against HER2**

In the eighth example, we generated and identified an affibody based binding moiety directed against HER2 which is suitable for use in a group of CARs according to the present invention. Again, we started from a well characterized existent antigen binding moiety that was engineered for high affinity binding to human HER2 (Wikman et al., Protein Eng Des Sel. 2004;17(5):455-462). To eliminate a potential N-glycosylation site and to reduce IgG binding, two point mutations (N23A and S33K) were introduced into the framework region of the binding scaffold (Feldwisch et al., J. Mol. Biol. 2010;398(2):232-47), which resulted in the antigen binding moiety "zHER2-WT". Low affinity mutants were generated by performing an alanine-scan of "zHER2-WT" by mutating all amino acids involved in antigen binding consecutively to alanine, resulting in various mutants containing one alanine-mutation each. Instead of expressing the 13 mutants in *E.coli* and determining their affinities for selection of appropriate antigen binding moieties, we performed a functional screening by directly integrating all mutants into a CAR backbone (exemplified with binder zHER2-WT (WT) in SEQ ID NO: 52) that could be conditionally homodimerized (Figure 10A). By this way, T cells expressing the different CARs could be directly screened for activation in presence and absence of the homodimerizer in co-culture with Jurkat T cells that were electroporated with 5 μg mRNA encoding for tHER2. Figure 10B shows the expression of tHER2 in the Jurkat cells. Primary human T cells were electroporated with 5 μg mRNA for each CAR construct and expression was detected 20 hours after electroporation via the hexahistidine tag (Figure 10C). Expression of all 13 different affibody-based CARs was comparable (Figure 10D). Primary T cells expressing no construct were used as a negative control ("no CAR"). For the functional screening, dimerization of CAR molecules was induced by treatment of the CAR T cells with 10 nM of AP20187 for 30 minutes at 37°C prior to co-cultivation with Jurkat T cells. Addition of the vehicle control DMSO served as a control. After co-cultivation for 4 hours at 37°C at an E:T ratio of 2:1, the capacity of the CARs to trigger cytotoxicity was determined by performing a luciferase-based cytotoxicity assay. The capacity of the different CARs to trigger cytotoxicity in T cells in presence or absence of 10 nM AP20187 is shown in Figure 10E. The high-affinity affibody zHER2-WT triggered efficient target cell lysis independent of presence of AP20187. Similarly, CARs comprising the mutants Q11A, Q17A, W24A, T25A, S27A and R28A displayed no significant dependence on the presence of the dimerizer. No cytotoxicity was triggered by CARs comprising the affibody antigen binding moieties with substituions Y13A and W14A, whereas Y35A triggered cytotoxicity at low levels. Dimerization-induced activation was observed with the mutants L9A-, R10A- and R32A, which therefore represent binding moieties suited for integration into CAR molecules according to the present invention.

Maintenance of human cell lines:

Primary human T cells were obtained from de-identified healthy donor's blood after apheresis (Buffy coats from the Austrian Red Cross, Vienna, Austria). $CD3^{pos}$ T cells were enriched by negative selection using RosetteSep Human T cell Enrichment Cocktail (STEMCELL Technologies). Isolated and purified T cells were cryopreserved in RPMI-1640 medium supplemented with 20% FCS and 10% DMSO until use. $CD3^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. Jurkat T cells were a gift from Dr. Sabine Strehl at the CCRI and were maintained in RPMI-1640 supplemented with 10% FCS and 1% penicillin-streptomycin. Cell lines were regularly tested for mycoplasma contamination and authentication was performed at Multiplexion, Germany. Cell densities were monitored with AccuCheck counting beads.

Antibodies and flow cytometry:

Primary human T cells or tumour cell lines were resuspended in FACS buffer (PBS, 0.2 % human albumin and 0.02 % sodium azide and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected via the hexahistidine tag using an AF647-conjugated anti-pentahistidine tag antibody (Qiagen). Expression of the engineered target antigen tHER2 was detected with a PE-conjugated anti-HER2 antibody (clone 24D2, BioLegend). Analysis was done by the FlowJo software.

*In vitro* transcription and electroporation of mRNA:

*In vitro* transcription was performed using the mMessage mMachine T7 Ultra Kit according to the manufacturer's instructions. 50-200 ng of column purified PCR product was used as a reaction template. The resulting mRNA was purified with an adapted protocol using the RNeasy column purification kit. Briefly, the mRNA solution was diluted with a mixture of RLT buffer, ethanol and 2-mercaptoethanol. The mixture was loaded onto an RNeasy column and purification was performed according to the manufacturer's instructions. Elution was performed with nuclease-free water and purified mRNAs were frozen at -80°C until electroporation. For transient transgene expression, primary T cells or Jurkat T cells were electroporated with varying amounts of the respective mRNA using the Gene Pulser (Biorad). Following protocols were used for the respective cell types: primary T cells (square wave protocol, 500 V, 5 ms and 4 mm cuvettes), Jurkat T cells (square wave protocol, 500 V, 3 ms and 4 mm cuvettes).

Construction of transgene constructs:

The nucleotide sequences encoding the CD33 signal peptide, the affibody zHER2-WT, the hexahistidine tag, a flexible $G_4S$ linker, the human monomeric CD8$\alpha$ hinge (UniProt ID P01732, C164S and C181S) and CD8$\alpha$ transmembrane domain, the 4-1BB co-stimulatory domain, the dimerization domain FKBP F36V and the CD3$\zeta$ ITAM signalling domain were synthesized by GeneArt. Sequences encoding the extracellular and transmembrane domain of HER2 were obtained from Addgene (plasmid #16257). Insertion of flexible linkers was performed by PCR. Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for *in vitro* transcription.

Alanine-scanning of protein antigen binding moieties:

Site-directed mutagenesis of all amino acids involved in epitope binding was performed using the QuikChange Lightning Site-Directed Mutagenesis Kit, according to the manufacturer's instructions. Primers were designed using the QuikChange Primer Design software (Agilent Genomics) and oligonucleotides were synthesized by Biomers.

Luciferase-based cytotoxicity assay:

Luciferase-expressing tumour cells were co-cultured with CAR T cells at an E:T cell ratio of 2:1 with 10,000 target cells/well in white round-bottom 96 well plates for 4 hours at 37°C in cytotoxicity assay medium, consisting of phenol-free RPMI, 10% FCS, 1% L-Glutamine and 1% penicillin-streptomycin. Finally, remaining living cells were quantified by determination of the residual luciferase activity of the co-culture. After equilibration to room temperature for 10

minutes, luciferin was added to the cell suspension (150 μg/mL final concentration) and luciferase activity was measured 20 minutes later using the ENSPIRE Multimode plate reader. The percentage of specific lysis was calculated with the following formula:

```
% specific lysis = 100 - ((RLU from well with effector and target cell co-culture) / (RLU from well with target cells only) x 100)).
```

*In vitro* dimerization of transgenes:

Dimerization of transgenes was induced prior to co-cultivation experiments. Primary T cells were diluted to the final cell concentration in the respective cell culture medium. The homodimerization agent AP20187 was diluted in cell culture medium and was added at 10 nM final concentration. Addition of the vehicle control DMSO at the same concentration served as control. Cells were incubated at 37°C for 30 minutes to ensure efficient dimerization of transgenes and subsequently used for *in vitro* experiments.

Expression and purification of Affibody-based antigen binding moieties:

Binding scaffolds were expressed as sfGFP fusion proteins (consisting of an N-terminal hexahistidine tag followed by either rcSso7d or the Affibody and sfGFP) using the pE-SUMO vector. The schematics of the architecture of the fusion proteins are shown in Figure 14G. Different mutants of the Affibody-based binder zHER2 were fused to sfGFP in the same way as indicated in Figure 14G. The nucleotide sequence that encodes the sfGFP reporter protein was obtained from Addgene (plasmid #54737). Briefly, *Escherichia coli* cells (Tuner DE3) were transformed with sequence-verified plasmids using heat shock transformation. After overnight cultivation at 37°C, cultures were diluted 1:100 in TB medium (12 g/L tryptone, 24 g/L yeast extract, 4% glycerol, 2.31 g/L $KH_2PO_4$ and 16.43 g/L $K_2HPO_4*3H_2O$) supplemented with kanamycin (50 μg/mL) and incubated at 37°C while shaking. When cultures reached an $A_{600}$ of roughly 2, expression of the transgene was induced by addition of 1 mM of IPTG and cells were further cultured overnight at 20°C. Cells were harvested by centrifugation (5000 g, 20 minutes, 4°C), resuspended in sonication buffer (50 mM sodium phosphate, 300 mM NaCl, 3% glycerol, 1% Triton X-100, pH 8.0), sonicated (2x 90 seconds, duty cycle 50 %, amplitude set to 5) and centrifuged again to remove cell debris. Hexahistidine-tagged fusion proteins were purified from crude cell extracts using TALON metal affinity resin. After addition of 10 mM imidazole, the sonicated supernatants were applied onto the resin twice, followed by washing step with equilibration buffer (50 mM sodium phosphate, 300 mM NaCl, pH 8.0) with increasing amounts of imidazole (5 - 15 mM). Binding scaffolds were eluted by applying equilibration buffer supplemented with 250 mM imidazole. After buffer exchange to PBS using Amicon Ultra-15 10K centrifugal filters, concentrations were determined by measuring the absorbance at 280 nm using the respective molar absorption coefficient and finally proteins were directly frozen at -80°C.

Determination of binding affinities using surface plasmon resonance (SPR):

SPR experiments were performed with a Biacore T200 instrument. All experiments were conducted in degassed and filtered PBS, pH 7.4, containing 0.1% BSA and 0.05% Tween-20 (Merck Millipore), at 25°C. hHER2-Fc (R&D) was immobilized on a Protein A sensor chip at a flow rate of 10 μL/min for 60 seconds at a concentration 4 μg/mL. To determine the affinity of Affibody-based antigen binding moieties, five concentrations (depending on the *expected* $K_d$ of the antigen binding moiety) of the respective protein were injected at a flow rate of 30 μL/min for 15 seconds (zHER2-R10A and zHER2-R32A) or 60 seconds (zHER2-WT) in the single-cycle kinetic mode, followed by a dissociation step (60 seconds for zHER2-R10A and zHER2-R32A and 180 seconds for zHER2-WT). Regeneration was performed using 10 mM Glycine-HCl, pH 1.5 at a flow rate of 30 μL/min for 30 seconds. The $K_d$ was obtained by curve fitting using the Biacore T200 Evaluation Software (GE Healthcare).

**Example 10: Generation of groups of CARs comprising three or four CAR molecules**

By using two orthogonal dimerization platforms (FKBP/FRB using AP21967; FKBP F36V/FKBP F36V using AP20187) and the low affinity binding moiety E11.4.1-G32A, we exemplify a strategy to create conditionally active groups of CARs comprising three (comprising the two constructs (SEQ ID NO: 48) and (SEQ ID NO: 67)) or four CAR molecules (comprising the two constructs (SEQ ID NO: 48) and (SEQ ID NO: 68)) in the complexed state. Complexing three or four CAR molecules into a group of CARs increases the avidity effect which can increase the sensitivity towards the

respective antigen. Figure 11A and Figure 11B show a schematic representation of a trimeric and a tetrameric CAR, respectively. Jurkat T cells were electroporated with 5 μg of mRNA coding for the two separate chains of either the trimeric or the tetrameric group of CARs and CAR expression was detected 20 hours after electroporation via Strep II tag or FLAG tag, depending on the respective signalling chain. Figure 11C shows the expression of the trimeric and tetrameric group of CARs.

Maintenance of human cell lines

Primary human T cells were obtained from de-identified healthy donor's blood after apheresis (Buffy coats from the Austrian Red Cross, Vienna, Austria). $CD3^{pos}$ T cells were enriched by negative selection using RosetteSep Human T cell Enrichment Cocktail (STEMCELL Technologies). Isolated and purified T cells were cryopreserved in RPMI-1640 medium supplemented with 20% FCS and 10% DMSO until use. $CD3^{pos}$ T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. A Jurkat T reporter cell line engineered with an NFκB-dependent eGFP gene and a NFAT-dependent CFP gene is a kind gift from Dr. Peter Steinberger at the Medical University of Vienna and is maintained in RPMI-1640 supplemented with 10% FCS and 1% penicillin-streptomycin. Cell lines are regularly tested for mycoplasma contamination and authentication is performed at Multiplexion, Germany. Cell densities are monitored with AccuCheck counting beads.

*In vitro* transcription and electroporation of mRNA

*In vitro* transcription is performed using the mMessage mMachine T7 Ultra Kit according to the manufacturer's instructions. 50-200 ng of column purified PCR product are used as a reaction template. The resulting mRNA is purified with an adapted protocol using the RNeasy column purification kit. Briefly, the mRNA solution is diluted with a mixture of RLT buffer, ethanol and 2-mercaptoethanol. The mixture is loaded onto an RNeasy column and purification is performed according to the manufacturer's instructions. Elution is performed with nuclease-free water and purified mRNAs are frozen at -80°C until electroporation. For transient transgene expression, Jurkat T cells are electroporated with varying amounts of the respective mRNA using the Gene Pulser (Biorad). Following protocol is used: primary T cells (square wave protocol, 500 V, 5 ms and 4 mm cuvettes), Jurkat T cells (square wave protocol, 500 V, 3 ms and 4 mm cuvettes).

Antibodies and flow cytometry

Primary T cells and Jurkat T cells are resuspended in FACS buffer (PBS, 0.2 % human albumin and 0.02 % sodium azide and treated for 10 minutes at 4°C with 10% human serum. Cells are stained with the respective primary antibody for 25 minutes at 4°C. Stained cells are washed two times in FACS buffer and then either stained with the secondary antibody for 25 minutes at 4°C or processed directly with a BD LSRFortessa. Expression of CAR constructs is detected either via the Strep II tag using an anti-Strep II tag antibody (clone 5A9F9, Genscript) or via the FLAG tag using an anti-FLAG tag antibody (clone L5, BioLegend) as a primary antibody and a PE- or APC-conjugated secondary antibody, in the case of anti-Strep II tag antibodies. Expression of the engineered target antigen tEGFR was detected either with a PE- or APC-conjugated anti-EGFR antibody (clone AY13, BioLegend). Analysis was done by the FlowJo software.

Construction of transgene constructs

The nucleotide sequences encoding the CD33 signal peptide, the low affinity rcSso7d variant E11.4.1-G32A, the Strep II tag, a flexible $G_4S$ linker, the human monomeric CD8α hinge (UniProt ID P01732, C164S and C181S) and CD8α transmembrane domain, the 4-1BB co-stimulatory domain, the dimerization domains FKBP F36V and FRB and the CD3ζ ITAM signalling domain are synthesized by GeneArt (Thermo Scientific). The nucleotide sequence encoding the dimerization domain FKBP is synthesized by Genscript. Sequences encoding the extracellular and transmembrane domain of EGFR are obtained from Addgene (plasmid #11011). Flexible linkers and the FLAG tag are inserted by using respective PCR primers. Assembly of nucleotide sequences into functional transgenes is performed by using the Gibson Assembly Master Mix, according to the manufacturer's instructions. The schematics and sequences are shown in Fig. 14 and 15, respectively. The resulting constructs are amplified by PCR and subsequently used for *in vitro* transcription.

Measuring transcription factor activity by Jurkat T reporter cell line

The Jurkat T reporter cell line is differentially labelled with distinct fluorescent proteins to enable efficient differentiation of Jurkat T reporter cells and Jurkat T target cells expressing the respective tumour antigen within the same well. Suitable fluorescent protein can be dKeima (Addgene #54618), mAmetrine (Addgene #54505) or similar proteins that have minimal cross-talk to the reporter proteins. Activity of the transcription factors NF-AT and NF-κB in Jurkat T reporter cells expressing the respective CAR is assessed by co-cultivation with target cells at an E:T ratios of 0.25:1, 0.5:1, 1:1 or 2:1 in round-bottom 96 well plates for 4 hours, 8 hours, 16 hours or 24 hours at 37°C. Cells are acquired using a BD LSRFortessa and activation of Jurkat T reporter cells is determined by measuring the geometric mean of the fluorescence intensity of the respective reporter protein or the percentage of reporter protein positive cells.

*In vitro* dimerization of transgenes

Dimerization of transgenes is induced prior to co-cultivation experiments. Primary T cells and Jurkat T cells are diluted to the final cell concentration in the respective cell culture medium. The homodimerization agent AP20187 and the heterodimerization agent AP21967 are diluted in cell culture medium and are added at 10 nM and 500 nM final concentration, respectively. Addition of the respective vehicle control DMSO or ethanol, respectively, at the same concentration served as control. Cells are incubated at 37°C for 30 minutes to ensure efficient dimerization of transgenes and subsequently used for *in vitro* experiments.

FACS-based cytotoxicity assay:

For the FACS-based cytotoxicity assay two populations of target cells are generated: (i) Jurkat cells electroporated with mRNA encoding eGFP and RNA encoding the respective target antigen, and (ii) Jurkat cells only electroporated with mRNA encoding mCherry. These two populations are mixed at a 1:1 ratio and co-cultured with CAR T cells at an E:T cell ratio of 4:1:1 with 20,000 target cells/well in round-bottom 96 well plates for 4 hours or 24 hours at 37°C. Target cells without the addition of CAR T cells serve as a control condition ("targets only"). After the incubation period, the co-cultures are centrifuged (5 minutes, 1600 rpm, 4°C), supernatants are collected for subsequent cytokine measurements and the remaining cells are resuspended in 100 μL of FACS buffer, consisting of PBS, 0.2 % human albumin and 0.02 % sodium azide. The viability of target antigen$^{pos}$ and target antigen$^{neg}$ cell populations is determined using a BD LSRFortessa flow cytometer and specific lysis is calculated with the following formula:

% specific lysis = (1-(((% eGFP$^{pos}$ cells of the sample)/(% mCherry$^{pos}$ cells of the sample)/(% eGFP$^{pos}$ cells of the "targets only" control)/(% mCherry$^{pos}$ cells of the "targets only" control))))*100.

**Example 11: Generation of groups of CARs comprising different co-stimulatory domains in the co-stimulatory signalling region of the CAR molecules**

Over the last two decades it has been demonstrated that CAR molecules of the 2$^{nd}$ generation containing different co-stimulatory domains can efficiently activate T cells. Example 11 shows that CAR molecules comprising different co-stimulatory domains in their signalling region also work in the context of a group of CARs of the present invention for exploitation of the aviditiy effect for controlling T cell function by regulating molecules. Figure 12A shows the architecture of EGFR-specific CAR molecules S(G32A)-8ser-28-FKBP(36V)-3z (SEQ-ID NO: 69), S(G32A)-8ser-ICOS-FKBP(36V)-3z (SEQ-ID NO: 70) and S(G32A)-8ser-OX40-FKBP(36V)-3z (SEQ-ID NO:71) containing either CD28 or ICOS or OX40 in the co-stimulatory signalling region. The expression of those CAR molecules in Jurkat cells and primary human T cells, respectively, is illustrated in Figs. 12B and C. Expression was analyzed by flow cytometry via the integrated Strep II tag 20 hours after electroporation of 5 μg of the respective mRNA. The capacity of those CAR molecules in the non-complexed (i.e. monovalent) and complexed (i.e. bivalent) state to activate the promoters NF-κB and NF-AT in Jurkat cells and to trigger cytotoxic effector functions in primary human T cells is shown in Figs. 12D and E, respectively. Figure 12D illustrates that S(G32A)-8ser-OX40-FKBP(36V)-3z, when expressed in Jurkat cells stably transduced with an NF-κB and NF-AT reporter, could efficiently trigger NF-κB and NF-AT when complexed by the regulating molecule AP20187 into a bivalent group of CARs but not in the uncomplexed monovalent state. Similarly, specific lysis was efficiently triggered in primary human T cells by the CAR molecules S(G32A)-8ser-ICOS-FKBP(36V)-3z and S(G32A)-8ser-OX40-FKBP(36V)-3z only if the CAR molecules were complexed by AP20187 into a group of CARs, respectively (Fig. 12E). Together, this confirms that the type of co-stimulatory domain in the co-stimulatory signalling region of CAR molecules of a group of CARs of the present invention can be varied.

Maintenance of human cell lines

Primary human T cells were obtained from de-identified healthy donor's blood after apheresis (Buffy coats from the Austrian Red Cross, Vienna, Austria). CD3pos T cells were enriched by negative selection using RosetteSep Human T cell Enrichment Cocktail (STEMCELL Technologies). Isolated and purified T cells were cryopreserved in RPMI-1640 medium supplemented with 20% FCS and 10% DMSO until use. CD3pos T Cells were activated with anti-CD3/CD28 beads according to the manufacturer's instructions and were expanded in human T cell medium, consisting of RPMI-1640 supplemented with 10% FCS, 1% penicillin-streptomycin and 200 IU/mL recombinant human IL-2. Primary T cells were cultivated for at least 14 days before experiments were conducted. Jurkat T cells were a gift from Dr. Sabine Strehl at the CCRI and were maintained in RPMI-1640 supplemented with 10% FCS and 1% penicillin-streptomycin. A Jurkat T reporter cell line engineered with an NFκB-dependent eGFP gene and a NFAT-dependent CFP gene is a kind gift from Dr. Peter Steinberger at the Medical University of Vienna and is maintained in RPMI-1640 supplemented with 10% FCS and 1% penicillin-streptomycin. Cell lines were regularly tested for mycoplasma contamination and authentication was performed at Multiplexion, Germany. Cell densities were monitored with AccuCheck counting beads.

In vitro transcription and electroporation of mRNA

In vitro transcription was performed using the mMessage mMachine T7 Ultra Kit according to the manufacturer's instructions. 50-200 ng of column purified PCR product were used as a reaction template. The resulting mRNA was purified with an adapted protocol using the RNeasy column purification kit. Briefly, the mRNA solution was diluted with a mixture of RLT buffer, ethanol and 2-mercaptoethanol. The mixture was loaded onto an RNeasy column and purification was performed according to the manufacturer's instructions. Elution was performed with nuclease-free water and purified mRNAs were frozen at -80°C until electroporation. For transient transgene expression, primary T cells were electroporated with varying amounts of the respective mRNA using the Gene Pulser (Biorad). Following protocol is used: primary T cells (square wave protocol, 500 V, 5 ms and 4 mm cuvettes), Jurkat T cells (square wave protocol, 500 V, 3 ms and 4 mm cuvettes).

Antibodies and flow cytometry

Primary T cells were resuspended in FACS buffer (PBS, 0.2 % human albumin and 0.02 % sodium azide and treated for 10 minutes at 4°C with 10% human serum. Cells were stained with the respective primary antibody for 25 minutes at 4°C. Stained cells were washed two times in FACS buffer and then either stained with the secondary antibody for 25 minutes at 4°C or processed directly with a BD LSRFortessa. Expression of CAR constructs was detected via the Strep II tag using an anti-Strep II tag antibody (clone 5A9F9, Genscript) as a primary antibody and a PE- or APC-conjugated secondary antibody. Expression of the engineered target antigens tEGFR was detected with a PE- or APC-conjugated anti-EGFR antibody (clone AY13, BioLegend). Analysis was done by the FlowJo software.

Construction of transgene constructs

The nucleotide sequences encoding the CD33 signal peptide, the low affinity rcSso7d variant E11.4.1-G32A, the Strep II tag, a flexible G4S linker, the human monomeric CD8α hinge (UniProt ID P01732, C164S and C181S) and CD8α transmembrane domain, the 4-1BB co-stimulatory domain, the dimerization domain FKBP F36V and the CD3ζ ITAM signalling domain were synthesized by GeneArt (Thermo Scientific). The nucleotide sequences encoding the intracellular domains of CD28, ICOS and OX40 were derived from cDNA clones (Sino Biological). Sequences encoding the extracellular and transmembrane domain of EGFR were obtained from Addgene (plasmid #11011). Flexible linkers were inserted by using respective PCR primers. Assembly of nucleotide sequences into functional transgenes was performed by using the Gibson Assembly Master Mix, according to the manufacturer's instructions. The schematics and sequences are shown in Fig. 14 and 15, respectively. The resulting constructs were amplified by PCR and subsequently used for in vitro transcription.

Measuring transcription factor activity by Jurkat T reporter cell line

The Jurkat T reporter cell line was differentially labelled with distinct fluorescent proteins to enable efficient differentiation of Jurkat T reporter cells and Jurkat T target cells expressing the respective tumour antigen within the same well. Suitable fluorescent proteins were dKeima (Addgene #54618), mAmetrine (Addgene #54505) or similar proteins that have minimal cross-talk to the reporter proteins. Activity of the transcription factors NF-AT and NF-κB in Jurkat T reporter cells expressing the respective CAR was assessed by co-cultivation with target cells at an E:T ratios of 0.25:1, 0.5:1, 1:1 or 2:1 in round-bottom 96 well plates for 24 hours at 37°C. Cells were acquired using a BD LSRFortessa

and activation of Jurkat T reporter cells was determined by measuring the geometric mean of the fluorescence intensity of the respective reporter protein or the percentage of reporter protein positive cells.

FACS-based cytotoxicity assay:

For the FACS-based cytotoxicity assay two populations of target cells were generated: (i) Jurkat cells electroporated with mRNA encoding eGFP and RNA encoding the respective target antigen, and (ii) Jurkat cells only electroporated with mRNA encoding mCherry. These two populations were mixed at a 1:1 ratio and co-cultured with CAR T cells at an E:T cell ratio of 4:1:1 with 20,000 target cells/well in round-bottom 96 well plates for 4 hours or 24 hours at 37°C. Target cells without the addition of CAR T cells served as a control condition ("targets only"). After the incubation period, the co-cultures were centrifuged (5 minutes, 1600 rpm, 4°C), supernatants were collected for subsequent cytokine measurements and the remaining cells were resuspended in 100 μL of FACS buffer, consisting of PBS, 0.2 % human albumin and 0.02 % sodium azide. The viability of target antigen$^{pos}$ and target antigen$^{neg}$ cell populations was determined using a BD LSRFortessa flow cytometer and specific lysis was calculated with the following formula:

% specific lysis = (1-(((% eGFP$^{pos}$ cells of the sample)/(% mCherry$^{pos}$ cells of the sample)/(% eGFP$^{pos}$ cells of the "targets only" control)/(% mCherry$^{pos}$ cells of the "targets only" control))))*100.

The present invention therefore discloses the following preferred embodiments:

1. A group of chimeric antigen receptors (CARs) consisting of two, three or four CAR molecules,
wherein the members of the group of CARs can be different or identical in their amino acid sequences to one another, and wherein each of the CAR molecules of the group comprise at least a transmembrane domain and an ectodomain comprising either an antigen binding moiety or a binding site to which another polypeptide is able to bind, wherein the another polypeptide comprises an antigen binding moiety, and
wherein at least one CAR molecule of the group additionally comprises an endodomain, which comprises at least a signalling region which can transduce a signal via at least one immunoreceptor tyrosine-based activation motif (ITAM) or at least one immunoreceptor tyrosine-based inhibitory motif (ITIM), and
wherein the endodomain of each CAR molecule of the group, in case the respective CAR molecule comprises an endodomain, is located on the intracellular side of a cell membrane, if expressed in a cell, wherein the ectodomain of each CAR molecule of the group translocates to the extracellular side of a cell membrane, if expressed in a cell, and wherein the transmembrane domain of each CAR molecule of the group is located in a cell membrane, if expressed in a cell;
wherein each CAR molecule of the group comprises at least one dimerization domain, which can mediate homo- or heterodimerization with other CAR molecules of the group, wherein this dimerization of a pair of dimerization domains is either induced by a regulating molecule and optionally reduced by another regulating molecule, or occurs in the absence of a regulating molecule and is reduced by a regulating molecule, wherein a regulating molecule is able to bind under physiological conditions to at least one member of a pair of dimerization domains and by inducing or reducing dimerization either induces or reduces the formation of a non-covalently complexed group of CARs consisting of two, three or four CAR molecules, and
wherein the ectodomain of each CAR molecule of the group in its prevalent conformation is free of cysteine amino acid moieties which are able to form intermolecular disulphide bonds with other CAR molecules of the group, respectively, and
wherein the antigen binding moieties of the CAR molecules of the group and of the other polypeptides being able to bind to the CAR molecules of the group are either specific for one target antigen or for a non-covalent or a covalent complex of different target antigens, and
wherein the affinity of each individual antigen binding moiety of a CAR molecule of the group to its target antigen is between 1 mM and 100 nM, and
wherein the affinity of each individual antigen binding moiety of another polypeptide to its target antigen or alternatively the affinity of this other polypeptide to the binding site of its respective CAR molecule is between 1 mM and 100 nM.

2. A group of CARs according to embodiment 1, wherein the antigen binding moiety comprises only one protein domain.

3. A group of CARs according to embodiment 1 or 2, wherein the antigen binding moiety comprises only one protein domain and does not cause dimerization or oligomerization of CAR molecules of the group when expressed on the surface of a human cell, and wherein said protein domain preferably is selected from the group consisting of a human or non-human VH or VL single domain antibody (nanobody) or an engineered antigen binding moiety based on the Z-domain of staphylococcal Protein A, lipocalins, SH3 domains, fibronectin type III (FN3) domains, knottins, Sso7d, rcSso7d, Sac7d, Gp2, DARPins, ubiquitin, a receptor, a ligand of a receptor, or a co-receptor.

4. A group of CARs according to any one of embodiments 1 to 3, wherein the affinity of each individual antigen binding moiety of a CAR molecule of the group to its target antigen is between 1 mM and 150 nM, preferably between 1 mM and 200 nM, more preferably between 1 mM and 300 nM, especially between 1 mM and 400 nM, and
wherein the affinity of each individual antigen binding moiety of another polypeptide to its target antigen or alternatively the affinity of this other polypeptide to the binding site of its respective CAR molecule is between 1 mM and 150 nM, preferably between 1 mM and 200 nM, more preferably between 1 mM and 300 nM, especially between 1 mM and 400 nM.

5. A group of CARs according to any one of embodiments 1 to 3, wherein the affinity of each individual antigen binding moiety of a CAR molecule of the group to its target antigen is between 500 μM and 100 nM, preferably between 250 μM and 100 nM, more preferably between 125 μM and 100 nM, especially between 50 μM and 100 nM, and
wherein the affinity of each individual antigen binding moiety of another polypeptide to its target antigen or alternatively the affinity of this other polypeptide to the binding site of its respective CAR molecule is between 500 μM and 100 nM, preferably between 250 μM and 100 nM, more preferably between 125 μM and 100 nM, especially between 50 μM and 100 nM.

6. A group of CARs according to any one of embodiments 1 to 3, wherein the affinity of each individual antigen binding moiety of a CAR molecule of the group to its target antigen is between 500 μM and 150 nM, preferably between 250 μM and 200 nM, more preferably between 125 μM and 300 nM, especially between 50 μM and 400 nM, and
wherein the affinity of each individual antigen binding moiety of another polypeptide to its target antigen or alternatively the affinity of this other polypeptide to the binding site of its respective CAR molecule is between 500 μM and 150 nM, preferably between 250 μM and 200 nM, more preferably between 125 μM and 300 nM, especially between 50 μM and 400 nM.

7. A group of CARs according to any one of embodiments 1 to 6, wherein each target antigen specifically recognized by the antigen binding moieties of the group of CARs or of other polypeptides being able to bind to CAR molecules of the group is a naturally occurring cellular surface antigen or a polypeptide, carbohydrate or lipid bound to a naturally occurring cellular surface antigen.

8. A group of CARs according to any one of embodiments 1 to 7, wherein the antigen binding moieties of the group of CARs and of other polypeptides being able to bind to CAR molecules of the group bind to one or more target antigens present on a cell, preferably one or more target antigens of a cell, on a solid surface, or a lipid bilayer.

9. A group of CARs according to any one of embodiments 1 to 8, wherein at least one target antigen, to which at least one antigen binding moiety of the group of CARs, or of another polypeptide being able to bind to a CAR molecule of the group, specifically binds, comprises a molecule preferably selected from the group consisting of CD19, CD20, CD22, CD23, CD28, CD30, CD33, CD35, CD38, CD40, CD42c, CD43, CD44, CD44v6, CD47, CD49D, CD52, CD53, CD56, CD70, CD72, CD73, CD74, CD79A, CD79B, CD80, CD82, CD85A, CD85B, CD85D, CD85H, CD85K, CD96, CD107a, CD112, CD115, CD117, CD120b, CD123, CD146, CD148, CD155, CD185, CD200, CD204, CD221, CD271, CD276, CD279, CD280, CD281, CD301, CD312, CD353, CD362, BCMA, CD16V, CLL-1, Ig kappa, TRBC1, TRBC2, CKLF, CLEC2D, EMC10, EphA2, FR-a, FLT3LG, FLT3, Lewis-Y, HLA-G, ICAM5, IGHA1/IgA1, IL-1RAP, IL-17RE, IL-27RA, MILR1, MR1, PSCA, PTCRA, PODXL2, PTPRCAP, ULBP2, AJAP1, ASGR1, CADM1, CADM4, CDH15, CDH23, CDHR5, CELSR3, CSPG4, FAT4, GJA3, GJB2, GPC2, GPC3, IGSF9, LRFN4, LRRN6A/LINGO1, LRRC15, LRRC8E, LRIG1, LGR4, LYPD1, MARVELD2, MEGF10, MPZLI1, MTDH, PANX3, PCDHB6, PCDHB10, PCDHB12, PCDHB13, PCDHB18, PCDHGA3, PEP, SGCB, vezatin, DAGLB, SYT11, WFDC10A, ACVR2A, ACVR2B, anaplastic lymphoma kinase, cadherin 24, DLK1, GFRA2, GFRA3, EPHB2, EPHB3, EPHB4, EFNB1, EPOR, FGFR2, FGFR4, GALR2, GLG1, GLP1R, HBEGF, IGF2R, UNC5C, VASN, DLL3, FZD10, KREMEN2, TMEM169, TMEM198, NRG1, TMEFF1, ADRA2C, CHRNA1, CHRNB4, CHRNA3, CHRNG, DRD4, GABRB3, GRIN3A, GRIN2C, GRIK4, HTR7, APT8B2, NKAIN1, NKAIN4, CACNA1A, CACNA1B, CACNA1I,

CACNG8, CACNG4, CLCN7, KCN§A4, KCNG2, KCNN3, KCNQ2, KCNU1, PKD1L2, PKD2L1, SLC5A8, SLC6A2, SLC6A6, SLC6A11, SLC6A15, SLC7A1, SLC7A5P1, SLC7A6, SLC9A1, SLC10A3, SLC10A4, SLC13A5, SLC16A8, SLC18A1, SLC18A3, SLC19A1, SLC26A10, SLC29A4, SLC30A1, SLC30A5, SLC35E2, SLC38A6, SLC38A9, SLC39A7, SLC39A8, SLC43A3, TRPM4, TRPV4, TMEM16J, TMEM142B, ADORA2B, BAI1, EDG6, GPR1, GPR26, GPR34, GPR44, GPR56, GPR68, GPR173, GPR175, LGR4, MMD, NTSR2, OPN3, OR2L2, OSTM1, P2RX3, P2RY8, P2RY11, P2RY13, PTGE3, SSTR5, TBXA2R, ADAM22, ADAMTS7, CST11, MMP14, LPPR1, LPPR3, LPPR5, SEMA4A, SEMA6B, ALS2CR4, LEPROTL1, MS4A4A, ROM1, TM4SF5, VANGL1, VANGL2, C18orf1, GSGL1, ITM2A, KIAA1715, LDLRAD3, OZD3, STEAP1, MCAM, CHRNA1, CHRNA3, CHRNA5, CHRNA7, CHRNB4, KIAA1524, NRM.3, RPRM, GRM8, KCNH4, Melanocortin 1 receptor, PTPRH, SDK1, SCN9A, SORCS1, CLSTN2, Endothelin converting enzyme like-1, Lysophosphatic acid receptor 2, LTB4R, TLR2, Neurotropic tyrosine kinase 1, MUC16, B7-H4, epidermal growth factor receptor (EGFR), ERBB2, HER3, EGFR variant III (EGFRvIII), HGFR, FOLR1, MSLN, CA-125, MUC-1, prostate-specific membrane antigen (PSMA), mesothelin, epithelial cell adhesion molecule (EpCAM), L1-CAM, CEACAM1, CEACAM5, CEACAM6, VEGFR1, VEGFR2, high molecular weight-melanoma associated antigen (HMW-MAA), MAGE-A 1, IL-13R-$\alpha$2, disialogangliosides (GD2 and GD3), tumour-associated carbohydrate antigens (CA-125, CA-242, Tn and sialyl-Tn), 4-1BB, 5T4, BAFF, carbonic anhydrase 9 (CA-IX), C-MET, CCR1, CCR4, FAP, fibronectin extra domain-B (ED-B), GPNMB, IGF-1 receptor, integrin $\alpha5\beta1$, integrin $\alpha v\beta3$, ITB5, ITGAX, embigin, PDGF-R$\alpha$, ROR1, Syndecan 1, TAG-72, tenascin C, TRAIL-R1, TRAIL-R2, NKG2D-Ligands, a major histocompatibility complex (MHC) molecule presenting a tumour-specific peptide epitope, preferably PR1/HLA-A2, a lineage-specific or tissue-specific tissue antigen, preferably CD3, CD4, CD5, CD7, CD8, CD24, CD25, CD34, CD80, CD86, CD133, CD138, CD152, CD319, endoglin, and an MHC molecule.

10. A group of CARs according to any one of embodiments 1 to 9, wherein the ectodomain of the CAR molecules of the group comprises a structurally flexible hinge region interposed between the antigen binding moiety and the transmembrane domain, preferably a hinge region derived from CD8 alpha (amino acid sequence position 138 - 182 according to UniProtKB/Swiss-Prot P01732-1), or CD28 (amino acid sequence position 114 - 152 according to UniProtKB/Swiss-Prot P10747), or PD-1 (amino acid sequence position 146 - 170 according to UniProtKB/Swiss-Prot Q15116), wherein the sequences derived from CD8 alpha, CD28 or PD-1 can be N-terminally and/or C-terminally truncated and can have any length within the borders of the said sequence region, and wherein the cysteine residues in the said hinges derived from CD8 alpha and CD28 are deleted or replaced by other amino acid residues.

11. A group of CARs according to any one of embodiments 1 to 10, wherein the domains of the CAR molecules are derived from different proteins, wherein at least two of these domains are connected via amino acid linker sequences, wherein the linker preferably comprises 1 to 40 amino acids in length.

12. A group of CARs according to any one of embodiments 1 to 11, wherein dimerization of the dimerization domains of at least two CAR molecules of the group, preferably of all CAR molecules of the group, is enhanced by binding of a regulating molecule.

13. A group of CARs according to any one of embodiments 1 to 12, wherein in the case of at least two heterodimerization domains within a CAR molecule the heterodimerization domains of that CAR molecule are separated by the cell membrane, and/or are each the same member of a pair of heterodimerization domains, and/or are members of different pairs of heterodimerization domains and therefore not able to bind to each other in the presence and absence of a regulating molecule in order to prevent the formation of complexes comprising an uncontrolled number of CAR molecules of the group.

14. A group of CARs according to any one of embodiments 1 to 13, wherein the dimerization domains of at least two CAR molecules of the group are selected from: calcineurin catalytic subunit A (CnA), cyclophilin, dihydrofolate reductase (DHFR), gyrase B (GyrB), GAI, GID1, PYL, ABI, preferably from FK506 binding protein 12 (FKBP12, FKBP), FKBP mutant F36V, and FKBP-rapamycin associated protein (FRB) mutant T82L.

15. A group of CARs according to any one of embodiments 1 to 14, wherein dimerization of at least two CAR molecules of the group is mediated by a pair of heterodimerization domains comprising a ligand binding domain from a nuclear receptor and a co-regulator peptide.

16. A group of CARs according to any one of embodiments 1 to 15, wherein dimerization of at least two CAR molecules of the group is mediated by a pair of heterodimerization domains comprising a lipocalin-fold molecule and a lipocalin-fold binding interaction partner.

17. A group of CARs according to any one of embodiments 1 to 16, wherein dimerization of at least two CAR molecules of the group is mediated by a pair of heterodimerization domains comprising a ligand binding domain from a nuclear receptor and a co-regulator peptide, and wherein the ligand binding domain from a nuclear receptor is selected from an estrogen receptor, an ecdysone receptor, a glucocorticoid receptor, an androgen receptor, a thyroid hormone receptor, a mineralocorticoid receptor, a progesterone receptor, a vitamin D receptor, a PPARγ receptor, a PPAPβ receptor, a PPARα receptor, a pregnane X receptor, a liver X receptor, a farnesoid X receptor, a retinoid X receptor, a RAR- related orphan receptor, a retinoic acid receptor, and the respective compatible co-regulators of the nuclear receptors selected from SRC1, GRIP1, AIB1, PGC1a, PGC1b, PRC, TRAP220, ASC2, ASC2-1, ASC2-2, CBP, CBP-1, CBP-2, P300, CIA, ARA70, ARA70-1, ARA70-2, NSD1, SMAP, Tip60, ERAP140, Nix1, LCoR, N-CoR, SMRT, RIP140, RIP140-1, RIP140-2, RIP140-3, RIP140-4, RIP140-5, RIP140-6, RIP140-7, RIP140-8, RIP140-9, PRIC285, PRIC285-1, PRIC285-2, PRIC285-3, PRIC285-4, PRIC285-5, SRC1-1, SRC1-2, SRC1-3, SRC1-4a, SRC1-4b, SRC2, SRC3, SRC3-1, PGC1, TRAP220-1, TRAP220-2, NR0B1, NRIP1, TIF1, TIF2, CoRNR Box, CoRNR1, CoRNR2, αβV, EA2, TA1, EAB1, GRIP1-1, GRIP1-2, GRIP1-3, AIB1-1, AIB1-2, AIB1-3, PGC1a, PGC1b.

18. A group of CARs according to any one of embodiments 1 to 17, wherein dimerization of at least two CAR molecules of the group is mediated by a pair of heterodimerization domains comprising a lipocalin-fold molecule and a lipocalin-fold binding interaction partner, and wherein the lipocalin-fold molecule is a derivative of a naturally occurring lipocalin or iLBP with up to 15, up to 30, or up to 50 amino acid deletions and/or up to 15, up to 30, or up to 50 amino acid insertions outside of the structurally conserved β-barrel structure, preferably corresponding structurally to the regions of amino acid residues selected from

- amino acid residues 1-20, 31-40, 48-51, 59-70, 79-84, 89-101, 110-113, 121-131 and 139-183 in human RBP4, which define the regions adjoining the structurally conserved β-strands in human RBP4 according to the amino acid residue numbering scheme in the PDB entry 1RBP;
- amino acid residues 1-13, 24-36, 44-47, 55-61, 70-75, 80-83, 92-95, 103-110 and 118-158 in human TLC (according to the amino acid residue numbering scheme in Schiefner et al., Acc Chem Res. 2015;48(4):976-985), which define the regions adjoining the structurally conserved β-strands in human TLC;
- amino acid residues 1-43, 54-68, 76-80, 88-95, 104-109, 114-118, 127-130, 138-141 and 149-188 in human ApoM (according to the amino acid residue numbering scheme in Schiefner et al., Acc Chem Res. 2015;48(4):976-985), which define the regions adjoining the structurally conserved β-strands in human ApoM;
- amino acid residues 1-4, 13-40, 46-49, 55-60, 66-70, 74-80, 88-92, 97-107, 113-118, 125-128 and 136-137 in human CRABPII (according to the amino acid residue numbering scheme in PDB entry 2FS6), which define the regions adjoining the structurally conserved β-strands in human CRABPII;
- amino acid residues 1-4, 13-38, 44-47, 53-58, 64-68, 72-78, 86-90, 95-98, 104-108, 115-118 and 126-127 in human FABP1 (according to the amino acid residue numbering scheme in PDB entry 2F73), which define the regions adjoining the structurally conserved β-strands in human FABP1.

19. A group of CARs according to any one of embodiments 1 to 18, wherein dimerization of at least two CAR molecules of the group is mediated by a pair of heterodimerization domains comprising a lipocalin-fold molecule and a lipocalin-fold binding interaction partner, and wherein the lipocalin-fold molecule is a derivative of a naturally occurring lipocalin or iLBP with at least 70%, preferably at least 80%, especially at least 90% sequence identity in the β-barrel structure, whereby this β-barrel structure is defined as the regions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;
- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;
- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;
- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;
- amino acid residues 5-12, 39-43, 48-52, 59-63, 69-71, 79-85, 91-94, 99-103, 109-114 and 119-125 in human

fatty acid binding protein 1 (FABP1; according to the amino acid residue numbering scheme in PDB entry 2F73), which define the structurally conserved β-strands in human FABP1;

20. A group of CARs according to any one of embodiments 1 to 19, wherein dimerization of at least two CAR molecules of the group is mediated by a pair of heterodimerization domains comprising a lipocalin-fold molecule and a lipocalin-fold binding interaction partner, and wherein the lipocalin-fold molecule is a fragment of a naturally occurring lipocalin or a derivative thereof with a length of at least 80, preferably at least 100, especially at least 120, amino acids covering at least the structurally conserved β-barrel structure of the lipocalin-fold, or wherein the lipocalin-fold molecule is a fragment of a naturally occurring iLBP or a derivative thereof with a length of at least 80, preferably at least 85, especially at least 90, amino acids covering at least the structurally conserved β-barrel structure of the lipocalin-fold,
wherein the structurally conserved β-barrel structure comprises or consists of amino acid positions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;
- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;
- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;
- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;
- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1;

21. A group of CARs according to any one of embodiments 1 to 20, wherein a regulating molecule is a molecule which is soluble at the concentrations that can be achieved in the physiological environment in the human body, or under physiological conditions within a cell, at the surface of a cell or under standardised physiological conditions, preferably at PBS conditions, wherein PBS conditions are 137 mM NaCl, 2.7 mM KCl, 10 mM $Na_2HPO_4$ and 18 mM $KH_2PO_4$).

22. A group of CARs according to any one of embodiments 1 to 21, wherein at least one regulating molecule is selected from rapamycin, a rapamycin-analog, abscisic acid, gibberellin, or gibberellin-analog GA3-AM, coumermycin, bis-methotrexate, AP20187, or AP1903.

23. A group of CARs according to any one of embodiments 1 to 22, wherein at least one regulating molecule binds to the ligand binding domain from a nuclear receptor and is selected from corticosterone (11beta,21-dihydroxy-4-pregnene-3,20-dione); deoxycorticosterone (21-hydroxy-4-pregnene-3,20-dione); cortisol (11beta,17,21-trihydroxy-4-pregnene-3,20-dione); 11-deoxycortisol (17,21-dihydroxy-4-pregnene-3,20-dione); cortisone (17,21-dihydroxy-4-pregnene-3,11,20-trione); 18-hydroxycorticosterone (11beta,18,21-trihydroxy-4-pregnene-3,20-dione); 1.alpha.-hydroxycorticosterone (1alpha,11beta,21-trihydroxy-4-pregnene-3,20-dione); aldosterone 18,11-hemiacetal of 11beta,21-dihydroxy-3,20-dioxo-4-pregnen-18-a1 ; androstenedione (4-androstene-3,17-dione); 4-hydroxyandrostenedione; 11β-hydroxyandrostenedione (11 beta-4-androstene-3,17-dione); androstanediol (3-beta,17-beta-Androstanediol); androsterone (3alpha-hydroxy-5alpha-androstan-17-one); epiandrosterone (3beta-hydroxy-5alpha-androstan-17-one); adrenosterone (4-androstene-3,11,17-trione); dehydroepiandrosterone (3beta-hydroxy-5-androsten-17-one); dehydroepiandrosterone sulphate (3beta-sulfoxy-5-androsten-17-one); testosterone (17beta-hydroxy-4-androsten-3-one); epitestosterone (17alpha-hydroxy-4-androsten-3-one); 5α-dihydrotestosterone (17beta-hydroxy-5alpha-androstan-3-one 5β-dihydrotestosterone; 5-beta-dihydroxy testosterone (17beta-hydroxy-5beta-androstan-3-one); 11β-hydroxytestosterone (11beta,17beta-dihydroxy-4-androsten-3-one); 11-ketotestosterone (17beta-hydroxy-4-androsten-3,17-dione); estrone (3-hydroxy-1,3,5(10)-estratrien-17-one); estradiol (1,3,5(10)-estratriene-3,17beta-diol); estriol 1,3,5(10)-estratriene-3,16alpha,17beta-triol; pregnenolone (3-beta-hydroxy-5-pregnen-20-one); 17-hydroxypregnenolone (3-beta,17-dihydroxy-5-pregnen-20-one); progesterone (4-pregnene-3,20-dione); 17-hydroxyprogesterone (17-hydroxy-4-pregnene-3,20-dione); progesterone (pregn-4-ene-3,20-dione); T3; T4; spironolactone; eplerenone; cyproterone acetate, hydroxyflutamide; enzalutamide; ARN-509; 3,3'-diindolylmethane (DIM); bexlosteride; bicalutamide; N-butylbenzene-sulfonamide (NBBS); dutasteride; epristeride; finasteride; flutamide; izonsteride; ketoconazole; N-butylbenzene-sulfonamide; nilutamide; megestrol; turosteride; mifepristone (RU-486; 11β-[4N,N-dimethylaminophenyl]-17β-hydroxy-17-(1-propynyl)-estra-4,9-dien-3-one); Lilopristone (11β-(4N,N-dimethylaminophenyl)-17β-hydroxy-17-((Z)-3-hydroxypropenyl)estra-4,9-dien-3-one); onapristone (11β-(4 N,N-dimethylaminophenyl)-17α-hydroxy-17-(3-hydroxypropyl)-13α-estra-4,9-dien-3-one); asoprisnil (benzaldehyde, 4-[(11β,17β)-17-methoxy-17-(methoxymethyl)-3-oxoestra-4,9-dien-11-yl]-1-(E)-oxim; J867); J912 (4-[17β-Hydroxy-17α-(methoxymethyl)-3-oxoestra-4,9-dien-11β-yl]benzaldehyd-(1E)-oxim); CDB-2914 (17α-acetoxy-11β-(4-N,N-dimethylaminophenyl)-19-norpregna-4,9-dien-3,20-dione); JNJ-1250132 (6α,11β,17β)-11-(4-dimethylaminophenyl)-6-methyl-4',5'-dihydrospiro[estra-4,9-diene-17,2'(3'H)-furan]-3-one (ORG-31710); (11β,17α)-11-(4-acetylphenyl)-17,23-epoxy-19,24-dinorchola-4,9-,20-trien-3-one (ORG-33628); (7β,11β,17β)-11-(4-dimethylaminophenyl-7-methyl]-4',5'-dihydrospiro[estra-4,9-diene-17,2'(3'H)-furan]-3-one (ORG-31806); ZK-112993; ORG-31376; ORG-33245; ORG-31167; ORG-31343; RU-2992; RU-1479; RU-25056; RU-49295; RU-46556; RU-26819; LG1127; LG120753; LG120830; LG1447; LG121046; CGP-19984A; RTI-3021-012; RTI-3021-022; RTI-3021-020; RWJ-25333; ZK-136796; ZK-114043; ZK-230211; ZK-136798; ZK-98229; ZK-98734; ZK-137316; 4-[17β-Methoxy-17α-(methoxymethyl)-3-oxoestra-4,9-dien-11β-yl]benzaldehyde-1-(E)-oxime; 4-[17β-Methoxy-17α-(methoxymethyl)-3-oxoestra-4,9-dien-11β-yl]benzaldehyde-1-(E)-[O-(ethylamino)carbonyl]oxime; 4-[17β-Methoxy-17α-(methoxymethyl)-3-oxoestra-4,9-dien-11β-yl]benzaldehyde-1-(E)-[O-(ethylthio)carbonyl]oxime; (Z)-6'-(4-cyanophenyl)-9,11a-dihydro-17β-hydroxy-17α-[4-(1-oxo-3-methylbutoxy)-1-butenyl]4'H-naphtho[3',2',1';10,9,11]estr-4-en-3-one; 11β-(4-acetylphenyl)-17β-hydroxy-17α-(1,1,2,2,2-penta-fluoroethyl)estra-4,9-dien-3-one; 11β-(4-Acetylphenyl)-19,24-dinor-17,23-epoxy-17alpha-chola-4,9,20-trie-n-3-one; (Z)-11beta,19-[4-(3-Pyridinyl)-o-phenylene]-17beta-hydroxy-17α-[3-hydroxy-1-propenyl]-4-androsten-3-one; 11beta-[4-(1-methylethenyl)phenyl]-17α-hydroxy-17beta-β-hydroxypropyl)-13α-estra-4,9-dien-3-one; 4',5'-Dihydro-11beta-[4-(dimethylamino)phenyl]-6beta-methylspiro[estra-4,-9-dien-17beta,2'(3'H)-furan]-3-one; drospirenone; T3 (3,5,3'-triiodo-L-thyronine); KB-141 (3,5-dichloro-4-(4-hydroxy-3-isopropylphenoxy)phenylacetic acid); sobetirome (3,5-dimethyl-4-(4'-hydroxy-3'-isopropylbenzyl)-phenoxy acetic acid); GC-24 (3,5-dimethyl-4-(4'-hydroxy-3'-benzyl)benzylphenoxyacetic acid); 4-OH-PCB106 (4-OH-2',3,3',4',5'-pentachlorobiphenyl); eprotirome; MB07811 ((2R,4S)-4-(3-chlorophenyl)-2-[(3,5-dimethyl-4-(4'-hydroxy-3'-isopropylbenzyl)phenoxy)methyl]-2-oxido-[1,3,2]-dioxaphosphonane); QH2; (3,5-dimethyl-4-(4'-hydroxy-3'-isopropylbenzyl)phenoxy)methylphosphonic acid (MB07344); tamoxifen; 4-OH-tamoxifen; raloxifene; lasofoxifene, bazedoxifene; falsodex; clomifene; femarelle; ormeloxifene; toremifiene; ospemifene; estradiol (17-beta-estradiol); ethinyl estradiol; a thiazolidinedione (preferably rosiglitazone, pioglitazone, lobeglitazone, troglitazone); farglitazar; aleglitazar; fenofibric acid; benzopyranoquinoline A 276575; Mapracorat; ZK 216348; 55D1E1; dexamethasone; prednisolone; prednisone; methylprednisolone; fluticasone propionate; beclomethasone-17-monopropionate; betamethasone; rimexolone; paramethasone; hydrocortisone; 1,25-dihydroxyvitamin D3 (calcitriol); paricalitol; doxercalciferol; 25-hydroxyvitamin D3 (calcifediol); cholecalciferol; ergocalciferol; tacalciol; 22-dihydroergocalciferol; (6Z)-Tacalciol; 2-methylene-19-nor-20(S)-1a-hydroxy-bishomopregnacalciferol; 19-nor-26,27-dimethylene-20(S)-2-methylene-1a,25-dihydroxyvitamin D3; 2-methylene-1α,25-dihydroxy-(17E)-17(20)-dehydro-19-nor-vi-tamin D3; 2-methylene-19-nor-(24R)-1α,25-dihydroxyvitamin D2; 2-methylene-(20R,25S)-19,26-dinor-1α,25-dihydroxyvitamin D3; 2-methylene-19-nor-1α-hydroxy-pregnacalciferol; 1α-hydroxy-2-methylene-19-nor-homopregnacalciferol; (20R)-1α-hydroxy-2-methylene-19-nor-bishomopregnacalciferol; 2-methylene-19-nor-(20S)-1α-hydroxy-trishomopregnacalciferol; 2-methylene-23,23-difluoro-1α-hydroxy-19-nor-bishomopregnacalcifero-1; 2-methylene-(20S)-23,23-difluoro-1α-hydroxy-19-nor-bishomopregnan-calciferol; (2-(3' hydroxypropyl-1',2'-idene)-19,23,24-trinor-(20S)-1α-hydroxyvitamin D3; 2-methylene-18,19-dinor-(20S)-1α,25-dihydroxyvitamin D3; retinoic acid; all-trans-retinoic acid; 9-cis-retinoic acid; tamibarotene; 13-cis-retinoic acid; (2E,4E,6Z,8E)-3,7-dimethyl-9-(2,6,6-trimethyl-1-cyclohexeneyl)nona-2,4,6,-8-tetraenoic acid; 9-(4-methoxy-2,3,6-trimethyl-phenyl)-3,7-dimethyl-nona-2,4,6,8-tetraenoic acid; 6-[3-(1-adamantyl)-4-methoxyphenyl]-2-napthoic acid; 4-[1-(3,5,5,8,8-pentamethyl-tetralin-2-yl)ethenyl]benzoic acid; retinobenzoic acid; ethyl 6-[2-(4,4-dimethylthiochroman-6-yl)ethynyl]pyridine-3-carboxylate; retinoyl t-butyrate; retinoyl pinacol; retinoyl cholesterol; obeticholic acid; LY2562175 (6-(4-((5-cyclopropyl-3-(2,6-dichlorophenyl)isoxazol-4-yl)methoxy)piperidin-1-yl)-1-methyl-1H-indole-3-carboxylic acid); GW4064 (3-[2-[2-Chloro-4-[[3-(2,6-dichlorophenyl)-5-(1-methylethyl)-4-isoxazolyl]methoxy]phenyl]ethenyl]benzoic acid); T0901317 (N-(2,2,2-Trifluoroethyl)-N-[4-[2,2,2-trifluoro-1-hydroxy-1-(trifluoromethyl)ethyl]phenyl]benzenesulfonamide); GW3965 (3-[3-[[[2-Chloro-3-(trifluoromethyl)phenyl]methyl](2,2-diphenylethyl)amino]propoxy]benzeneacetic acid hydrochloride); LXR-623; GNE-3500 (27, 1-{4-[3-fluoro-4-((3S,6R)-3-methyl-1,1-dioxo-6-phenyl-[1,2]thiazinan-2-ylmethyl)-phenyl]-piperazin-1-yl}-ethanone); 7β, 27-dihydroxycholesterol; 7α, 27-dihydroxycholesterol; 9-cis retinoic acid; LGD100268; CD3254 (3-[4-Hydroxy-3-(5,6,7,8-tetrahydro-3,5,5,8,8-pentamethyl-2-naphthalenyl)phenyl]-2-propenoic acid); CD2915 (Sorensen et al. (1997) Skin Pharmacol. 10:144); rifampicin; chlotrimazole; and lovastatin.

24. A group of CARs according to any one of embodiments 1 to 23, wherein at least one regulating molecule is Tamoxifen and binds to the ligand binding domain from a nuclear receptor, preferably from estrogen receptor alpha or from estrogen receptor beta.

25. A group of CARs according to any one of embodiments 1 to 24, wherein at least one regulating molecule binds to a lipocalin-fold molecule and is selected from fenretinide (Pubchem CID 5288209), N-Ethylretinamide (Pubchem CID 5288173), all-trans retinoic acid (Pubchem CID 444795), axerophthene (Pubchem CID 5287722), A1120 (Pubchem CID 25138295) and derivatives thereof (Cioffi et al., J Med Chem. 2014;57(18):7731-7757; Cioffi et al., J Med Chem. 2015; 58(15) : 5863-5888), 1,4-butanediol (Pubchem CID 8064), sphingosine-1-phosphate (Pubchem CID 5283560), tetradecanoic acid (Pubchem CID 11005), indicaxanthin (Pubchem CID 6096870 and 12310796), vulgaxanthin I (Pubchem CID 5281217), Montelukast (Pubchem CID 5281040), Cyclandelate (Pubchem CID 2893), Oxolamine (Pubchem CID 13738), Mazaticol (Pubchem CID 4019), Butoctamid (Pubchem CID 65780), Tonabersat (Pubchem CID 6918324), Novazin (Pubchem CID 65734), Diphenidol (Pubchem CID 3055), Alloclamide (Pubchem CID 71837), Diacetolol (Pubchem CID 50894), Acotiamide (Pubchem CID 5282338), Acoziborole (Pubchem CID 44178354), Acumapimod (Pubchem CID 11338127), Apalutamide (Pubchem CID 24872560), ASP3026 (Pubchem CID 25134326), AZD1480 (Pubchem CID 16659841), BIIB021 (Pubchem CID 16736529), Branaplam (Pubchem CID 89971189), Brequinar (Pubchem CID 57030), Chlorproguanil (Pubchem CID 9571037), Clindamycin (Pubchem CID 446598), Emricasan (Pubchem CID 12000240), Enasidenib (Pubchem CID 89683805), Enolicam (Pubchem CID 54679203), Flurazepam (Pubchem CID 3393), ILX-295501 (Pubchem CID 127737), Indibulin (Pubchem CID 2929), Metoclopramide (Pubchem CID 4168), Mevastatin (Pubchem CID 64715), MGGBYMDAPCCKCT-UHFFFAOYSA-N (Pubchem CID 25134326), MK0686 (Pubchem CID 16102897), Navarixin (Pubchem CID 11281445), Nefazodone (Pubchem CID 4449), Pantoprazole (Pubchem CID 4679), Pavinetant (Pubchem CID 23649245), Proxazole (Pubchem CID 8590), SCYX-7158 (Pubchem CID 44178354), Siccanin (Pubchem CID 71902), Sulfaguanole (Pubchem CID 9571041), Sunitinib (Pubchem CID 5329102), Suvorexant (Pubchem CID 24965990), Tiapride (Pubchem CID 5467), Tonabersat (Pubchem CID 6918324), VNBRGSXVFBYQNN-UHFFFAOYSA-N (Pubchem CID 24794418), YUHNXUAATAMVKD-PZJWPPBQSA-N (Pubchem CID 44548240), Ulimorelin (Pubchem CID 11526696), Xipamide (Pubchem CID 26618), Tropesin (Pubchem CID 47530), Triclabendazole (Pubchem CID 50248), Triclabendazole sulfoxide (Pubchem CID 127657), Triclabendazole sulfone (Pubchem CID 10340439) and Trametinib (Pubchem CID 11707110).

26. A group of CARs according to any one of embodiments 1 to 25, wherein at least one regulating molecule is selected from rapamycin, a rapamycin-analog, AP20187, AP1903, Tamoxifen, Emricasan and A1120.

27. A group of CARs according to any one of embodiments 1 to 26, wherein the order of the domains in the CAR molecules of the group from the extracellular to the intracellular side on the surface of a cell is: an antigen binding moiety or a binding site to which another polypeptide comprising an antigen binding moiety is able to bind, preferably a hinge region for spatial optimization, and a transmembrane domain,
wherein in the preferred case of an ITAM-containing group of CARs the transmembrane domain is preferably followed in at least one CAR molecule by a signalling region comprising a co-stimulatory domain, wherein preferably this co-stimulatory signalling region, or optionally the transmembrane domain, is followed by at least one dimerization domain, and further, in at least one CAR molecule, by a signalling region comprising at least one ITAM, wherein the order of the co-stimulatory and the ITAM-containing signalling region can be inverted, and wherein CAR molecules not comprising an ITAM either lack a co-stimulatory signalling region, or comprise one co-stimulatory signalling region, or two co-stimulatory signalling regions, or even more co-stimulatory signalling regions, but preferably not more than two co-stimulatory signalling regions, or even more preferably only one co-stimulatory signalling region, and wherein in the case of an ITIM comprising group of CARs the transmembrane domain is preferably followed in at least one CAR molecule by an ITIM-comprising inhibitory signalling region, wherein preferably this inhibitory signalling region, or optionally the transmembrane domain, is followed by at least one dimerization domain, and optionally a second inhibitory signalling region, and
wherein in an ITAM- and ITIM-comprising group of CARs any two adjacent components of a CAR molecule can optionally be separated by a linker, and
wherein in an ITAM- and ITIM-comprising group of CARs the dimerization domains, of which at least one is mandatory for each CAR molecule of the group, can be located alternatively or additionally in the ectodomain or the transmembrane domain, however, preferably between the transmembrane domain and a signalling region, and/or especially between two signalling regions and/or especially at the intracellular end of the CAR molecules.

28. A group of CARs according to any one of embodiments 1 to 27, wherein each CAR molecule of the group comprises at least a signalling region which can transduce a signal via at least one immunoreceptor tyrosine-based

activation motif (ITAM) or at least one immunoreceptor tyrosine-based inhibitory motif (ITIM).

29. A group of CARs according to any one of embodiments 1 to 28, wherein the dimerization domains are located in the endodomains and/or the transmembrane domains, preferably in the endodomains, of the CAR molecules of the group.

30. A group of CARs according to any one of embodiments 1 to 28, wherein the ectodomains of at least two of the CAR molecules of the group comprise a dimerization domain which preferably comprises only one protein domain, and wherein the regulating molecule is a molecule secreted from a cell and induces dimerization of said dimerization domains.

31. A group of CARs according to any one of embodiments 1 to 30, wherein at least one CAR molecule of the group comprises an endodomain containing a signalling region which can transduce a signal via at least one ITAM, and wherein the group of CARs preferably comprises at least three ITAMs.

32. A group of CARs according to any one of embodiments 1 to 31, wherein the endodomain of at least one CAR molecule of the group comprises at least one ITAM, said ITAM is selected from CD3 zeta, DAP12, Fc-epsilon receptor 1 gamma chain, CD3 delta, CD3 epsilon, CD3-gamma, and CD79A (antigen receptor complex-associated protein alpha chain).

33. A group of CARs according to any one of embodiments 1 to 32, wherein the co-stimulatory domain of a co-stimulatory signalling region in the endodomain of a CAR molecule of the group is derived from 4-1BB (CD137), CD28, ICOS, BTLA, OX-40, CD2, CD6, CD27, CD30, CD40, GITR, and HVEM.

34. A group of CARs according to any one of embodiments 1 to 30, wherein at least one CAR molecule of the group comprises an endodomain containing a cytoplasmic inhibitory domain derived from PD-1, CD85A, CD85C, CD85D, CD85J, CD85K, LAIR1, TIGIT, CEACAM1, CD96, KIR2DL, KIR3DL, SLAM family members, CD300/LMIR family members, CD22 and other Siglec family members.

35. A group of CARs according to any one of embodiments 1 to 30 and 34, wherein at least one CAR molecule of the group comprises an endodomain containing a signalling region which can transduce a signal via at least one ITIM which is derived from PD-1, CD85A, CD85C, CD85D, CD85J, CD85K, LAIR1, TIGIT, CEACAM1, CD96, KIR2DL, KIR3DL, SLAM family members, ITIM comprising CD300/LMIR family members, CD22 and other ITIM comprising Siglec family members.

36. A group of CARs according to any one of embodiments 1 to 35, wherein the ectodomain of each CAR molecule comprises an antigen binding moiety.

37. A group of CARs according to any one of embodiments 1 to 36, wherein the group consists of two or three CAR molecules, preferably two CAR molecules.

38. A group of CARs according to any one of embodiments 1 to 14, 21, 22, 26 to 29 or 31 to 37, wherein the group consists of three CAR molecules, and wherein the endodomain of one of the three CAR molecules comprises two copies of the same member of a pair of dimerization domains selected from either FKBP12 or alternatively FRB (mutant T82L), and wherein the endodomains of each of the other two CAR molecules comprise one copy of the other member of the pair of dimerization domains, respectively, and wherein the regulating molecule is a rapalog.

39. A group of CARs according to any one of embodiments 1 to 13, 15, 17, 23, 24, 26 to 29 or 31 to 37, wherein the group consists of three CAR molecules, and wherein the endodomain of one of the three CAR molecules comprises two copies of the same member of a pair of dimerization domains selected from either the ligand binding domain of a nuclear receptor or alternatively a compatible co-regulator of the same nuclear receptor, and wherein the endodomains of each of the other two CAR molecules comprise one copy of the other member of the pair of dimerization domains, respectively, and wherein the regulating molecule is Tamoxifen.

40. A group of CARs according to any one of embodiments 1 to 13, 16, 18 to 21, 25 to 29 or 31 to 37, wherein the group consists of three CAR molecules, and wherein the endodomain of one of the three CAR molecules comprises two copies of the same member of a pair of dimerization domains selected from either a lipocalin-fold molecule or a lipocalin-fold binding interaction partner, and wherein the endodomains of each of the other two CAR molecules

comprise one copy of the other member of the pair of dimerization domains, respectively, and
wherein the regulating molecule is selected from fenretinide (15-[(4-hydroxyphenyl)amino]retinal), N-Ethylretinamide, all-trans retinoic acid, axerophthene, A1120 (PubChem CID 25138295) and derivatives thereof, 1,4-butanediol, sphingosine-1-phosphate, tetradecanoic acid, indicaxanthin, vulgaxanthin, Montelukast, Cyclandelate, Oxolamine, Mazaticol, Butoctamid, Tonabersat, Novazin, Diphenidol, Neobornyval, Alloclamide, Diacetolol, Acotiamide, Acoziborole, Acumapimod, Apalutamide, ASP3026, AZD1480, BIIB021, Branaplam, Brequinar, Chlorproguanil, Clindamycin, Emricasan, Enasidenib, Enolicam, Flurazepam, ILX-295501, Indibulin, Metoclopramide, Mevastatin, MGGBYMDAPCCKCT-UHFFFAOYSA-N, MK0686, Navarixin, Nefazodone, Pantoprazole, Pavinetant, Proxazole, SCYX-7158, Siccanin, Sul-faguanole, Sunitinib, Suvorexant, Tiapride, Tonabersat, VNBRGSXVFBYQNN-UHFFFAOYSA-N, YUHNXUAATAMVKD-PZJWPPBQSA-N, Ulimorelin, Xipamide, Tropesin, Triclabendazole, Triclabendazole sulfoxide, Triclabendazole sulfone and Trametinib.

41. A nucleic acid molecule comprising nucleotide sequences encoding the individual CAR molecules of a group of CARs according to any one of embodiments 1 to 40, 64 or 65, wherein the nucleic acid is selected from DNA, RNA, or in vitro transcribed RNA.

42. A kit of nucleic acid molecules comprising nucleotide sequences encoding the individual CAR molecules of a group of CARs according to any one of embodiments 1 to 40, 64 or 65, wherein the nucleic acid is selected from DNA, RNA, or in vitro transcribed RNA.

43. A kit of nucleic acid molecules according to embodiment 42, wherein the nucleic acid molecules are present in a vector and preferably packaged as DNA or RNA into an infectious virus particle.

44. A nucleic acid molecule or a kit of nucleic acid molecules according to any one of embodiments 41 to 43, wherein the nucleic acid sequences are linked to a sequence mediating strong and stable transgene expression in lymphocytes, wherein such a sequence preferably comprises the 5'-LTR of a gamma retrovirus or subelements R and U3 of a 5'-LTR of the Moloney murine leukaemia virus (MMLV) or the promoter of the murine stem cell virus (MSCV) or the promoter of phosphoglycerate kinase (PGK) or even more preferably the human elongation factor 1 (EF-1) alpha promoter.

45. A kit of nucleic acid molecules according to any one of embodiments 42 to 44, wherein the first nucleic acid comprises nucleotide sequences encoding a first CAR molecule of the group and wherein the second nucleic acid comprises nucleotide sequences encoding a second CAR molecule of the group and, optionally, wherein the kit further comprises a third nucleic acid, said third nucleic acid comprising nucleotide sequences encoding a third CAR molecule of the group if the group of CARs consists of at least three different CAR molecules and, optionally, wherein the kit further comprises a fourth nucleic acid, said fourth nucleic acid comprising nucleotide sequences encoding a fourth CAR molecule of the group if the group of CARs consists of four different CAR molecules.

46. A vector or a kit of vectors comprising nucleotide sequences encoding the individual CAR molecules of a group of CARs according to any one of embodiments 1 to 40, 64 or 65, wherein the nucleic acid is DNA or RNA.

47. A vector or a kit of vectors according to embodiment 46, wherein the vector is a recombinant adeno-associated virus (rAAV) vector or a transposon vector, preferably a Sleeping Beauty transposon vector or PiggyBac transposon vector, or wherein a vector is a retroviral vector, preferably a gamma-retroviral vector or a lentiviral vector.

48. A vector or a kit of vectors according to embodiments 46 or 47, wherein the vector is an expression vector, preferably an expression vector in which the nucleotide sequences are operably linked to a sequence mediating strong and stable transgene expression in lymphocytes, wherein such a sequence preferably comprises the 5'-LTR of a gamma retrovirus or subelements R and U3 of a 5'-LTR of the Moloney murine leukaemia virus (MMLV) or the promoter of the murine stem cell virus (MSCV) or the promoter of phosphoglycerate kinase (PGK) or even more preferably the human elongation factor 1 (EF-1) alpha promoter.

49. A kit of vectors according to any one of embodiments 46 to 48, wherein the first vector comprises a nucleotide sequence encoding a first CAR molecule of the group and wherein the second vector comprises a nucleotide sequence encoding a second CAR molecule of the group and, optionally, wherein the kit further comprises a third vector, said third vector comprising a nucleotide sequence encoding a third CAR molecule of the group if the group of CARs consists of at least three different CAR molecules and, optionally, wherein the kit further comprises a fourth vector, said fourth vector comprising a nucleotide sequence encoding a fourth CAR molecule of the group if the

group of CARs consists of four different CAR molecules.

50. A cell modified in vitro or ex vivo with a nucleic acid molecule or a kit of nucleic acid molecules according to any one of embodiments 41 to 45 or with a vector or a kit of vectors according to any one of embodiments 46 to 49 to produce the individual CAR molecules of a group of CARs according to any one of embodiments 1 to 40, 64 or 65, or a kit comprising two or more of said modified cells.

51. A cell or kit of cells according to embodiment 50, wherein the cell is a mammalian cell, preferably a hematopoietic stem cell (HSC), or a cell derived from a HSC, more preferably an NK cell or a T cell, especially a T cell.

52. A cell or kit of cells according to embodiments 50 or 51, wherein the cell is transfected or transduced with a vector or with a kit of vectors according to any one of embodiments 46 to 49.

53. A cell or kit of cells according to any one of embodiments 50 to 52, wherein the cell has stably integrated the nucleotide sequences encoding a group of CARs according to any one of embodiments 1 to 40, 64 or 65 into its genome.

54. A cell or kit of cells according to any one of embodiments 50 to 52, wherein the cell has stably integrated the nucleotide sequences encoding a group of CARs according to any one of embodiments 1 to 40, 64 or 65 into its genome by the use of site directed nuclease technology, preferably by the use of zinc finger nucleases or TALENs, or even more preferably CRISPR/Cas technology.

55. A pharmaceutical preparation comprising a nucleic acid or a kit of nucleic acids according to any one of embodiments 41 to 45, a vector or a kit of vectors according to any one of embodiments 46 to 49, or a cell or a kit of cells according to any one of embodiments 50 to 54.

56. A pharmaceutical preparation according to embodiment 55, wherein the viral vectors are preferably contained in infectious virus particles.

57. A method of making a cell according to any one of embodiments 50 to 54, the method comprising introducing into the cell, preferably stably integrating into the genome of the cell, in vitro or ex vivo a nucleic acid molecule or a kit of nucleic acid molecules according to any one of embodiments 41 to 45, or a vector or a kit of vectors according to any one of embodiments 46 to 49.

58. A group of CARs according to any one of embodiments 1 to 40, 64 or 65 for use in a method of treatment of a cancer in an individual, wherein the method comprises:

i) genetically modifying NK cells or preferably T lymphocytes obtained from the individual with at least one nucleic acid molecule comprising sequences encoding the respective CAR molecules of the group of CARs, wherein each antigen binding moiety of the group of CARs is specific for a target antigen on a cancer cell in the individual, and wherein said genetic modification is carried out in vitro or ex vivo;
ii) introducing the genetically modified cells into the individual; and
iii) administering to the individual an effective amount of at least one regulating molecule for either inducing or reducing dimerization of the respective CAR molecules of the group, preferably inducing dimerization of the respective CAR molecules of the group, thereby either inducing or reducing non-covalent complexation of the group of CARs, preferably inducing non-covalent complexation of the group of CARs, wherein the non-covalently complexed group of CARs upon contact with a cancer cell expressing the respective target antigen or the respective covalent or non-covalent complex of different target antigens mediates activation of the genetically modified cell, which leads to killing of the cancer cell and thereby enables treating the cancer.

59. A cell according to any one of embodiments 50 to 54 for use in a method of treatment of a cancer in an individual, wherein each antigen binding moiety of the group of CARs is specific for a target antigen on a cancer cell in the individual, and wherein the method comprises:

i) introducing the cell into the individual; and
ii) administering to the individual an effective amount of at least one regulating molecule for either inducing or alternatively reducing, preferably inducing, the formation of a non-covalent complex comprising two, three or four CAR molecules of the group, preferably three CAR molecules of the group, even more preferably two CAR

molecules of the group, wherein the non-covalently complexed group of CARs upon contact with a cancer cell expressing the respective target antigen or the respective covalent or non-covalent complex of different target antigens mediates activation of the genetically modified cell, which leads to killing of the cancer cell and thereby enables treating the cancer.

60. A kit comprising:

- a group of CARs according to any one of embodiments 1 to 40, 64 or 65, a vector or kit of vectors according to any one of embodiments 46 to 49, or a cell or a kit of cells according to any one of embodiments 50 to 54, and
- one, two or three regulating molecules, preferably two, even more preferably one regulating molecule.

61. A group of CARs according to any one of embodiments 1 to 40, 64 or 65, a vector or kit of vectors according to any one of embodiments 46 to 49, a cell or kit of cells according to any one of embodiments 50 to 54, especially a T lymphocyte or NK cell, or a kit according to any one of embodiments 42 to 49 or 60 for use in the treatment of a disease which is characterised by the need to bind a T lymphocyte or an NK cell to a target antigen on a cell, preferably for use in the treatment of a tumour patient, especially a tumour patient with a tumour selected from Ewing's sarcoma, rhabdomyosarcoma, osteosarcoma, osteogenic sarcoma, mesothelioma, fibrosarcoma, myxosarcoma, liposarcoma, chondrosarcoma, chordoma, angiosarcoma, endotheliosarcoma, lymphangiosarcoma, lymphangioendotheliosarcoma, synovioma, leiomyosarcoma, melanoma, glioma, astrocytoma, medulloblastoma, neuroblastoma, retinoblastoma, oligodendroglioma, menangioma, craniopharyngioma, ependymoma, pinealoma, hemangioblastoma, acoustic neuroma, chronic myeloproliferative syndromes, acute myelogenous leukemias, chronic lymphocytic leukemias (CLL) including B-cell CLL, T-cell CLL, prolymphocytic leukemia and hairy cell leukemia, acute lymphoblastic leukemias, B-cell lymphomas, Hodgkin's lymphoma, non-Hodgkin's lymphoma, esophageal carcinoma, hepatocellular carcinoma, basal cell carcinoma, squamous cell carcinoma, bladder carcinoma, transitional cell carcinoma, bronchogenic carcinoma, colon carcinoma, colorectal carcinoma, gastric carcinoma, lung carcinoma, including small cell carcinoma and non-small cell carcinoma of the lung, adrenocortical carcinoma, thyroid carcinoma, pancreatic carcinoma, breast carcinoma, ovarian carcinoma, prostate carcinoma, adenocarcinoma, sweat gland carcinoma, sebaceous gland carcinoma, papillary carcinoma, papillary adenocarcinoma, cystadenocarcinoma, medullary carcinoma, renal cell carcinoma, ductal carcinoma, bile duct carcinoma, choriocarcinoma, seminoma, embryonal carcinoma, Wilm's tumor, cervical carcinoma, uterine carcinoma, testicular carcinoma, osteogenic carcinoma, epithelial carcinoma, and nasopharyngeal carcinoma, atypical meningioma, islet cell carcinoma, medullary carcinoma, mesenchymoma, hepatocellular carcinoma, hepatoblastoma, clear cell carcinoma, and neurofibroma mediastinum.

62. A method of modulating the activity of a T lymphocyte or an NK cell, the method comprising contacting the T lymphocyte or NK cell in vivo or ex vivo in the presence of at least one regulating molecule with a target antigen, or a non-covalent or a covalent complex of different target antigens, on a cell or solid surface, wherein the T lymphocyte or NK cell is genetically modified to produce the group of CARs according to any one of embodiments 1 to 40, 64 or 65, and wherein the presence of the regulating molecule(s) either induces or alternatively reduces, preferably induces the formation of a non-covalently complexed group of CARs and thereby modulates at least one activity of the genetically modified cell.

63. A method according to embodiment 62, wherein the activity of the genetically modified cell is proliferation, cell survival, apoptosis, gene expression, or immune activation.

64. A group of CARs according to any one of embodiments 1 to 40, wherein the non-covalent complexation of the group of CARs is induced in the presence of an effective concentration of one or more regulating molecules, and wherein the group of CARs, when expressed in an NK cell or preferably a T lymphocyte, elicits in its non-covalently complexed state a response in the host cell upon contact with a target antigen expressing target cell, wherein this response is defined by the excretion of interferon-gamma, and/or Macrophage inflammatory protein-1 (MIP-1) alpha, and/or MIP-1 beta, and/or granzyme B, and/or IL-2, and/or TNF, and/or IL-10, and/or IL-4, and/or by cell degranulation, wherein cell degranulation is preferably detected by the percentage of CD107a positive effector cells, after contact with a target cell expressing at least 100,000 molecules of each target antigen recognized by the group of CARs, wherein the response elicited in the presence of an effective concentration of all regulating molecules required for inducing the non-covalent complexation of the group of CARs is at least 20% higher, preferably at least 50% higher, and even more preferably at least 100% higher than the response elicited in the absence of any regulating molecule, wherein the effective concentration of each required regulating molecule is the concentration achieved by administration of an effective amount of each required regulating molecule in one or more doses to an individual in need

thereof.

65. A group of CARs according to any one of embodiments 1 to 11 or 13 to 40, wherein the non-covalent complexation of the group of CARs is reduced by the presence of an effective concentration of one or more regulating molecules, and wherein the group of CARs, when expressed in an NK cell or preferably a T lymphocyte, elicits in its non-covalently complexed state a response in the host cell upon contact with a target antigen expressing target cell, wherein this response is defined by the excretion of interferon-gamma, and/or Macrophage inflammatory protein-1 (MIP-1) alpha, and/or MIP-1 beta, and/or granzyme B, and/or IL-2, and/or TNF, and/or IL-10, and/or IL-4, and/or by cell degranulation, wherein cell degranulation is preferably detected by the percentage of CD107a positive effector cells, after contact with a target cell expressing at least 100,000 molecules of each target antigen recognized by the group of CARs, wherein the response elicited by the group of CARs in the absence of any regulating molecule is at least 20% higher, preferably at least 50% higher, and even more preferably at least 100% higher than the response elicited by the group of CARs in the presence of an effective concentration of each regulating molecule required for reducing dimerization of the dimerization domains comprised by the group of CARs, wherein the effective concentration of each required regulating molecule is the concentration achieved by administration of an effective amount of each required regulating molecule in one or more doses to an individual in need thereof.

## Claims

1. A group of chimeric antigen receptors (CARs) consisting of two, three or four CAR molecules,
wherein the members of the group of CARs can be different or identical in their amino acid sequences to one another, and wherein each of the CAR molecules of the group comprises at least a transmembrane domain and an ectodomain comprising either an antigen binding moiety or a binding site to which another polypeptide is able to bind, wherein the another polypeptide comprises an antigen binding moiety, and
wherein at least one CAR molecule of the group additionally comprises an endodomain, which comprises at least a signalling region which can transduce a signal via at least one immunoreceptor tyrosine-based activation motif (ITAM) or at least one immunoreceptor tyrosine-based inhibitory motif (ITIM), and
wherein the endodomain of each CAR molecule of the group, in case the respective CAR molecule comprises an endodomain, is located on the intracellular side of a cell membrane, if expressed in a cell, wherein the ectodomain of each CAR molecule of the group translocates to the extracellular side of a cell membrane, if expressed in a cell, and wherein the transmembrane domain of each CAR molecule of the group is located in a cell membrane, if expressed in a cell;
wherein each CAR molecule of the group comprises at least one dimerization domain, which can mediate homo- or heterodimerization with other CAR molecules of the group, wherein this dimerization of a pair of dimerization domains is either induced by a regulating molecule and optionally reduced by another regulating molecule, or occurs in the absence of a regulating molecule and is reduced by a regulating molecule, wherein a regulating molecule is able to bind under physiological conditions to at least one member of a pair of dimerization domains and by inducing or reducing dimerization either induces or reduces the formation of a non-covalently complexed group of CARs consisting of two, three or four CAR molecules, and
wherein the ectodomain of each CAR molecule of the group in its prevalent conformation is free of cysteine amino acid moieties which are able to form intermolecular disulphide bonds with other CAR molecules of the group, respectively, and
wherein the antigen binding moieties of the CAR molecules of the group and of the other polypeptides being able to bind to the CAR molecules of the group are either specific for one target antigen or for a non-covalent or a covalent complex of different target antigens, and
wherein the affinity of each individual antigen binding moiety of a CAR molecule of the group to its target antigen is between 1 mM and 100 nM, and
wherein the affinity of each individual antigen binding moiety of another polypeptide to its target antigen or alternatively the affinity of this other polypeptide to the binding site of its respective CAR molecule is between 1 mM and 100 nM.

2. A group of CARs according to claim 1, wherein the affinity of each individual antigen binding moiety of a CAR molecule of the group to its target antigen is between 1 mM and 150 nM, preferably between 1 mM and 200 nM, more preferably between 1 mM and 300 nM, especially between 1 mM and 400 nM, and
wherein the affinity of each individual antigen binding moiety of another polypeptide to its target antigen or alternatively the affinity of this other polypeptide to the binding site of its respective CAR molecule is between 1 mM and 150 nM, preferably between 1 mM and 200 nM, more preferably between 1 mM and 300 nM, especially between 1 mM and 400 nM.

3. A group of CARs according to claim 1, wherein the affinity of each individual antigen binding moiety of a CAR molecule of the group to its target antigen is between 500 μM and 100 nM, preferably between 250 μM and 100 nM, more preferably between 125 μM and 100 nM, especially between 50 μM and 100 nM, and
wherein the affinity of each individual antigen binding moiety of another polypeptide to its target antigen or alternatively the affinity of this other polypeptide to the binding site of its respective CAR molecule is between 500 μM and 100 nM, preferably between 250 μM and 100 nM, more preferably between 125 μM and 100 nM, especially between 50 μM and 100 nM.

4. A group of CARs according to claim 1, wherein the affinity of each individual antigen binding moiety of a CAR molecule of the group to its target antigen is between 500 μM and 150 nM, preferably between 250 μM and 200 nM, more preferably between 125 μM and 300 nM, especially between 50 μM and 400 nM, and wherein the affinity of each individual antigen binding moiety of another polypeptide to its target antigen or alternatively the affinity of

this other polypeptide to the binding site of its respective CAR molecule is between 500 μM and 150 nM, preferably between 250 μM and 200 nM, more preferably between 125 μM and 300 nM, especially between 50 μM and 400 nM.

**5.** A group of CARs according to any one of claims 1 to 4, wherein each target antigen specifically recognized by the antigen binding moieties of the group of CARs or of other polypeptides being able to bind to CAR molecules of the group is a naturally occurring cellular surface antigen or a polypeptide, carbohydrate or lipid bound to a naturally occurring cellular surface antigen.

**6.** A group of CARs according to any one of claims 1 to 5, wherein the antigen binding moieties of the group of CARs and of other polypeptides being able to bind to CAR molecules of the group bind to one or more target antigens present on a cell, preferably one or more target antigens of a cell, on a solid surface, or a lipid bilayer, especially wherein at least one target antigen comprises a molecule preferably selected from the group consisting of CD19, CD20, CD22, CD23, CD28, CD30, CD33, CD35, CD38, CD40, CD42c, CD43, CD44, CD44v6, CD47, CD49D, CD52, CD53, CD56, CD70, CD72, CD73, CD74, CD79A, CD79B, CD80, CD82, CD85A, CD85B, CD85D, CD85H, CD85K, CD96, CD107a, CD112, CD115, CD117, CD120b, CD123, CD146, CD148, CD155, CD185, CD200, CD204, CD221, CD271, CD276, CD279, CD280, CD281, CD301, CD312, CD353, CD362, BCMA, CD16V, CLL-1, Ig kappa, TRBC1, TRBC2, CKLF, CLEC2D, EMC10, EphA2, FR-a, FLT3LG, FLT3, Lewis-Y, HLA-G, ICAM5, IGHA1/IgA1, IL-1RAP, IL-17RE, IL-27RA, MILR1, MR1, PSCA, PTCRA, PODXL2, PTPRCAP, ULBP2, AJAP1, ASGR1, CADM1, CADM4, CDH15, CDH23, CDHR5, CELSR3, CSPG4, FAT4, GJA3, GJB2, GPC2, GPC3, IGSF9, LRFN4, LRRN6A/LINGO1, LRRC15, LRRC8E, LRIG1, LGR4, LYPD1, MARVELD2, MEGF10, MPZLI1, MTDH, PANX3, PCDHB6, PCDHB10, PCDHB12, PCDHB13, PCDHB18, PCDHGA3, PEP, SGCB, vezatin, DAGLB, SYT11, WFDC10A, ACVR2A, ACVR2B, anaplastic lymphoma kinase, cadherin 24, DLK1, GFRA2, GFRA3, EPHB2, EPHB3, EPHB4, EFNB1, EPOR, FGFR2, FGFR4, GALR2, GLG1, GLP1R, HBEGF, IGF2R, UNC5C, VASN, DLL3, FZD10, KREMEN2, TMEM169, TMEM198, NRG1, TMEFF1, ADRA2C, CHRNA1, CHRNB4, CHRNA3, CHRNG, DRD4, GABRB3, GRIN3A, GRIN2C, GRIK4, HTR7, APT8B2, NKAIN1, NKAIN4, CACNA1A, CACNA1B, CACNA1I, CACNG8, CACNG4, CLCN7, KCN§A4, KCNG2, KCNN3, KCNQ2, KCNU1, PKD1L2, PKD2L1, SLC5A8, SLC6A2, SLC6A6, SLC6A11, SLC6A15, SLC7A1, SLC7A5P1, SLC7A6, SLC9A1, SLC10A3, SLC10A4, SLC13A5, SLC16A8, SLC18A1, SLC18A3, SLC19A1, SLC26A10, SLC29A4, SLC30A1, SLC30A5, SLC35E2, SLC38A6, SLC38A9, SLC39A7, SLC39A8, SLC43A3, TRPM4, TRPV4, TMEM16J, TMEM142B, ADORA2B, BAI1, EDG6, GPR1, GPR26, GPR34, GPR44, GPR56, GPR68, GPR173, GPR175, LGR4, MMD, NTSR2, OPN3, OR2L2, OSTM1, P2RX3, P2RY8, P2RY11, P2RY13, PTGE3, SSTR5, TBXA2R, ADAM22, ADAMTS7, CST11, MMP14, LPPR1, LPPR3, LPPR5, SEMA4A, SEMA6B, ALS2CR4, LEPROTL1, MS4A4A, ROM1, TM4SF5, VANGL1, VANGL2, C18orf1, GSGL1, ITM2A, KIAA1715, LDLRAD3, OZD3, STEAP1, MCAM, CHRNA1, CHRNA3, CHRNA5, CHRNA7, CHRNB4, KIAA1524, NRM.3, RPRM, GRM8, KCNH4, Melanocortin 1 receptor, PTPRH, SDK1, SCN9A, SORCS1, CLSTN2, Endothelin converting enzyme like-1, Lysophosphatic acid receptor 2, LTB4R, TLR2, Neurotropic tyrosine kinase 1, MUC16, B7-H4, epidermal growth factor receptor (EGFR), ERBB2, HER3, EGFR variant III (EGFRvIII), HGFR, FOLR1, MSLN, CA-125, MUC-1, prostate-specific membrane antigen (PSMA), mesothelin, epithelial cell adhesion molecule (EpCAM), L1-CAM, CEACAM1, CEACAM5, CEACAM6, VEGFR1, VEGFR2, high molecular weight-melanoma associated antigen (HMW-MAA), MAGE-A 1, IL-13R-α2, disialogangliosides (GD2 and GD3), tumour-associated carbohydrate antigens (CA-125, CA-242, Tn and sialyl-Tn), 4-1BB, 5T4, BAFF, carbonic anhydrase 9 (CA-IX), C-MET, CCR1, CCR4, FAP, fibronectin extra domain-B (ED-B), GPNMB, IGF-1 receptor, integrin α5β1, integrin αvβ3, ITB5, ITGAX, embigin, PDGF-Rα, ROR1, Syndecan 1, TAG-72, tenascin C, TRAIL-R1, TRAIL-R2, NKG2D-Ligands, a major histocompatibility complex (MHC) molecule presenting a tumour-specific peptide epitope, preferably PR1/HLA-A2, a lineage-specific or tissue-specific tissue antigen, preferably CD3, CD4, CD5, CD7, CD8, CD24, CD25, CD34, CD80, CD86, CD133, CD138, CD152, CD319, endoglin, and an MHC molecule.

**7.** A nucleic acid molecule comprising nucleotide sequences encoding the individual CAR molecules of a group of CARs according to any one of claims 1 to 6, wherein the nucleic acid is selected from DNA, RNA, or in vitro transcribed RNA.

**8.** A kit of nucleic acid molecules comprising nucleotide sequences encoding the individual CAR molecules of a group of CARs according to any one of claims 1 to 6, wherein the nucleic acid is selected from DNA, RNA, or in vitro transcribed RNA.

**9.** A vector or a kit of vectors comprising nucleotide sequences encoding the individual CAR molecules of a group of CARs according to any one of claims 1 to 6, wherein the nucleic acid is DNA or RNA.

**10.** A cell modified in vitro or ex vivo with a nucleic acid molecule or a kit of nucleic acid molecules according to claim

7 or 8 or with a vector or a kit of vectors according to claim 9 to produce the individual CAR molecules of a group of CARs according to any one of claims 1 to 6, or a kit comprising two or more of said modified cells.

**11.** A pharmaceutical preparation comprising a nucleic acid or a kit of nucleic acids according to claim 7 or 8, a vector or a kit of vectors according to claim 9, or a cell or a kit of cells according to claim 10.

**12.** A group of CARs according to any one of claims 1 to 6 for use in a method of treatment of a cancer in an individual, wherein the method comprises:

i) genetically modifying NK cells or preferably T lymphocytes obtained from the individual with at least one nucleic acid molecule comprising sequences encoding the respective CAR molecules of the group of CARs, wherein each antigen binding moiety of the group of CARs is specific for a target antigen on a cancer cell in the individual, and wherein said genetic modification is carried out in vitro or ex vivo;
ii) introducing the genetically modified cells into the individual; and
iii) administering to the individual an effective amount of at least one regulating molecule for either inducing or reducing dimerization of the respective CAR molecules of the group, preferably inducing dimerization of the respective CAR molecules of the group, thereby either inducing or reducing non-covalent complexation of the group of CARs, preferably inducing non-covalent complexation of the group of CARs, wherein the non-covalently complexed group of CARs upon contact with a cancer cell expressing the respective target antigen or the respective covalent or non-covalent complex of different target antigens mediates activation of the genetically modified cell, which leads to killing of the cancer cell and thereby enables treating the cancer.

**13.** A cell according to claim 10 for use in a method of treatment of a cancer in an individual, wherein each antigen binding moiety of the group of CARs is specific for a target antigen on a cancer cell in the individual, and wherein the method comprises:

i) introducing the cell into the individual; and
ii) administering to the individual an effective amount of at least one regulating molecule for either inducing or alternatively reducing, preferably inducing, the formation of a non-covalent complex comprising two, three or four CAR molecules of the group, preferably three CAR molecules of the group, even more preferably two CAR molecules of the group, wherein the non-covalently complexed group of CARs upon contact with a cancer cell expressing the respective target antigen or the respective covalent or non-covalent complex of different target antigens mediates activation of the genetically modified cell, which leads to killing of the cancer cell and thereby enables treating the cancer.

**14.** A kit comprising:

- a group of CARs according to any one of claims 1 to 6, a vector or kit of vectors according to claim 9, or a cell or a kit of cells according to claim 10, and
- one, two or three regulating molecules, preferably two, even more preferably one regulating molecule.

**15.** A group of CARs according to any one of claims 1 to 6, a vector or kit of vectors according to claim 9, a cell or a kit of cells according to claim 10, especially a T lymphocyte or NK cell, or a kit according to any one of claims 8, 9 or 14 for use in the treatment of a disease which is **characterised by** the need to bind a T lymphocyte or an NK cell to a target antigen on a cell, preferably for use in the treatment of a tumour patient, especially a tumour patient with a tumour selected from Ewing's sarcoma, rhabdomyosarcoma, osteosarcoma, osteogenic sarcoma, mesothelioma, fibrosarcoma, myxosarcoma, liposarcoma, chondrosarcoma, chordoma, angiosarcoma, endotheliosarcoma, lymphangiosarcoma, lymphangioendotheliosarcoma, synovioma, leiomyosarcoma, melanoma, glioma, astrocytoma, medulloblastoma, neuroblastoma, retinoblastoma, oligodendroglioma, menangioma, craniopharyngioma, ependymoma, pinealoma, hemangioblastoma, acoustic neuroma, chronic myeloproliferative syndromes, acute myelogenous leukemias, chronic lymphocytic leukemias (CLL) including B-cell CLL, T-cell CLL, prolymphocytic leukemia and hairy cell leukemia, acute lymphoblastic leukemias, B-cell lymphomas, Hodgkin's lymphoma, non-Hodgkin's lymphoma, esophageal carcinoma, hepatocellular carcinoma, basal cell carcinoma, squamous cell carcinoma, bladder carcinoma, transitional cell carcinoma, bronchogenic carcinoma, colon carcinoma, colorectal carcinoma, gastric carcinoma, lung carcinoma, including small cell carcinoma and non-small cell carcinoma of the lung, adrenocortical carcinoma, thyroid carcinoma, pancreatic carcinoma, breast carcinoma, ovarian carcinoma, prostate carcinoma, adenocarcinoma, sweat gland carcinoma, sebaceous gland carcinoma, papillary carcinoma, papillary adenocarcinoma, cystadenocarcinoma, medullary carcinoma, renal cell carcinoma, ductal carcinoma, bile duct carcinoma,

choriocarcinoma, seminoma, embryonal carcinoma, Wilm's tumor, cervical carcinoma, uterine carcinoma, testicular carcinoma, osteogenic carcinoma, epithelial carcinoma, and nasopharyngeal carcinoma, atypical meningioma, islet cell carcinoma, medullary carcinoma, mesenchymoma, hepatocellular carcinoma, hepatoblastoma, clear cell carcinoma, and neurofibroma mediastinum.

Fig. 1

A

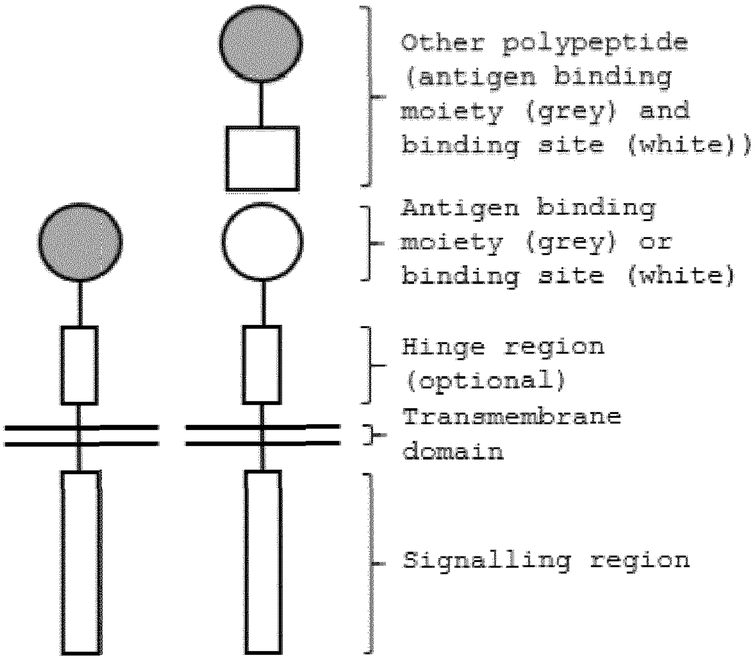
Other polypeptide
(antigen binding
moiety (grey) and
binding site (white))
Antigen binding
moiety (grey) or
binding site (white)
Hinge region
(optional)
Transmembrane
domain
Signalling region

Fig. 1

B

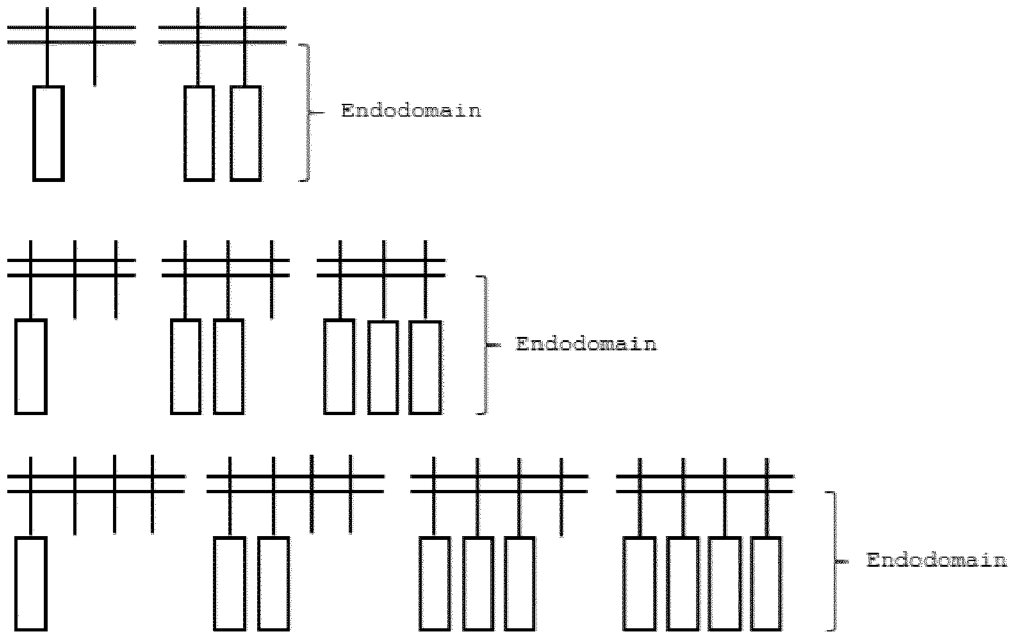
Endodomain
Endodomain
Endodomain

C

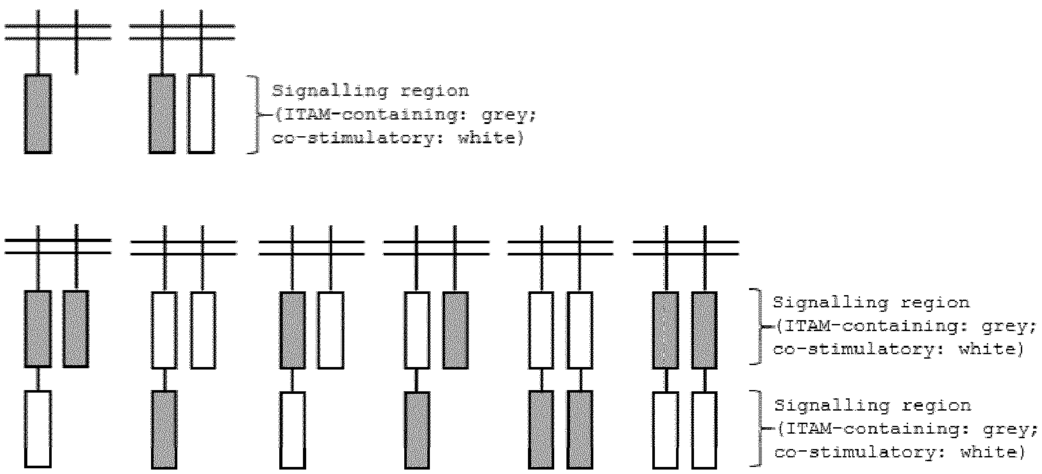
Signalling region
(ITAM-containing: grey;
co-stimulatory: white)
Signalling region
(ITAM-containing: grey;
co-stimulatory: white)
Signalling region
(ITAM-containing: grey;
co-stimulatory: white)

Fig. 1

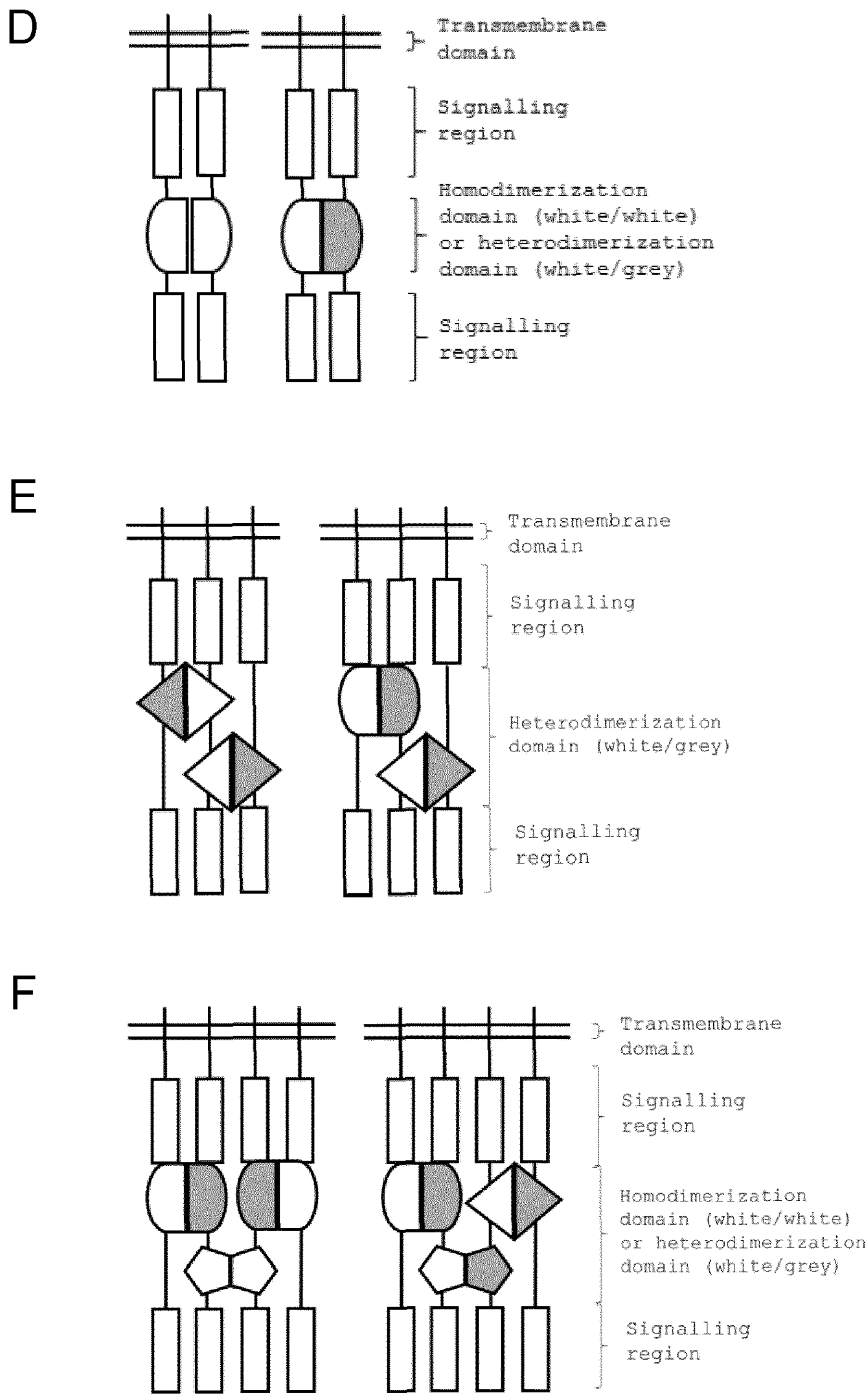
D
Transmembrane domain
Signalling region
Homodimerization domain (white/white) or heterodimerization domain (white/grey)
Signalling region
E
Transmembrane domain
Signalling region
Heterodimerization domain (white/grey)
Signalling region
F
Transmembrane domain
Signalling region
Homodimerization domain (white/white) or heterodimerization domain (white/grey)
Signalling region

Fig. 1

G

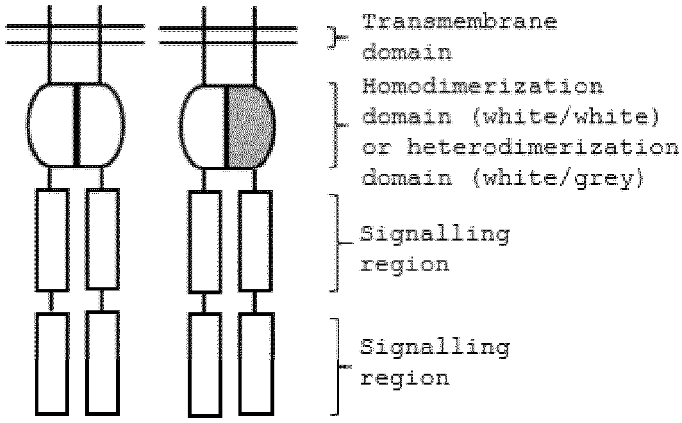
Transmembrane
domain
Homodimerization
domain (white/white)
or heterodimerization
domain (white/grey)
Signalling
region
Signalling
region

H

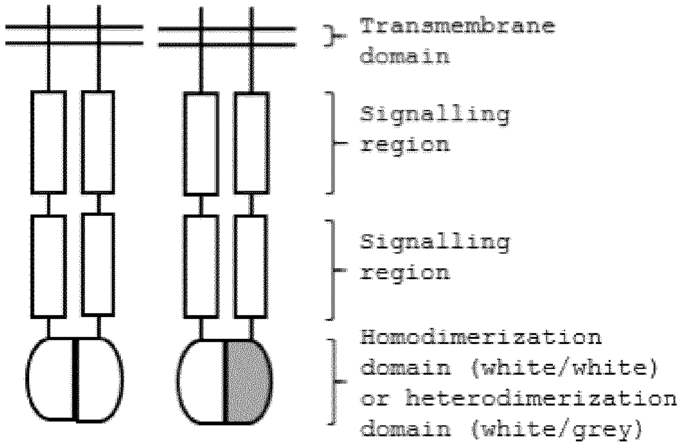
Transmembrane
domain
Signalling
region
Signalling
region
Homodimerization
domain (white/white)
or heterodimerization
domain (white/grey)

Fig. 1

I

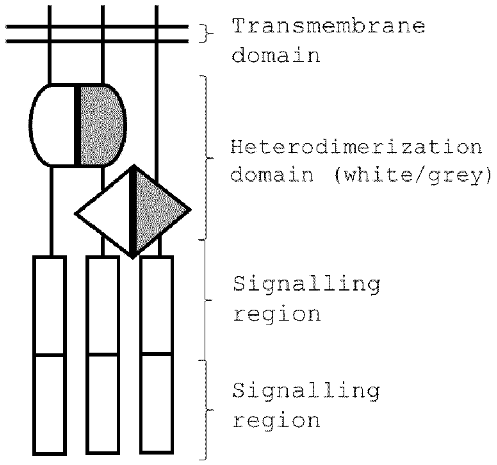
Transmembrane domain
Heterodimerization domain (white/grey)
Signalling region
Signalling region

J

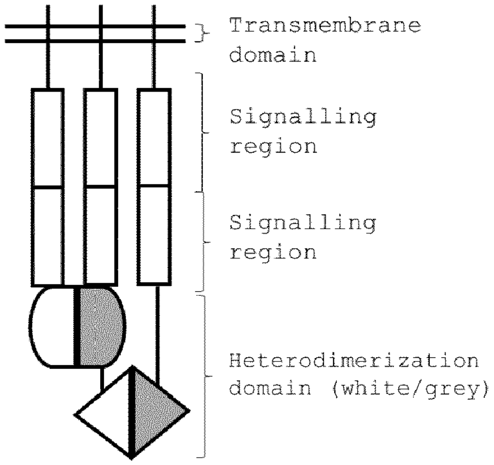
Transmembrane domain
Signalling region
Signalling region
Heterodimerization domain (white/grey)

K

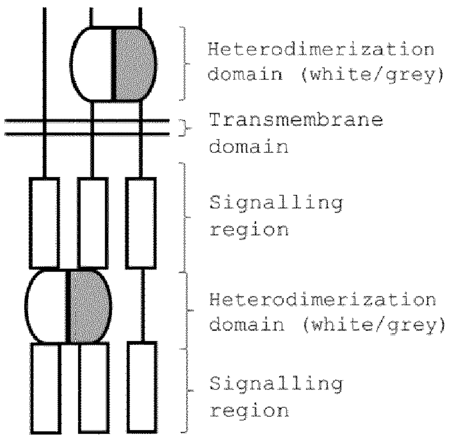
Heterodimerization domain (white/grey)
Transmembrane domain
Signalling region
Heterodimerization domain (white/grey)
Signalling region

Fig. 1

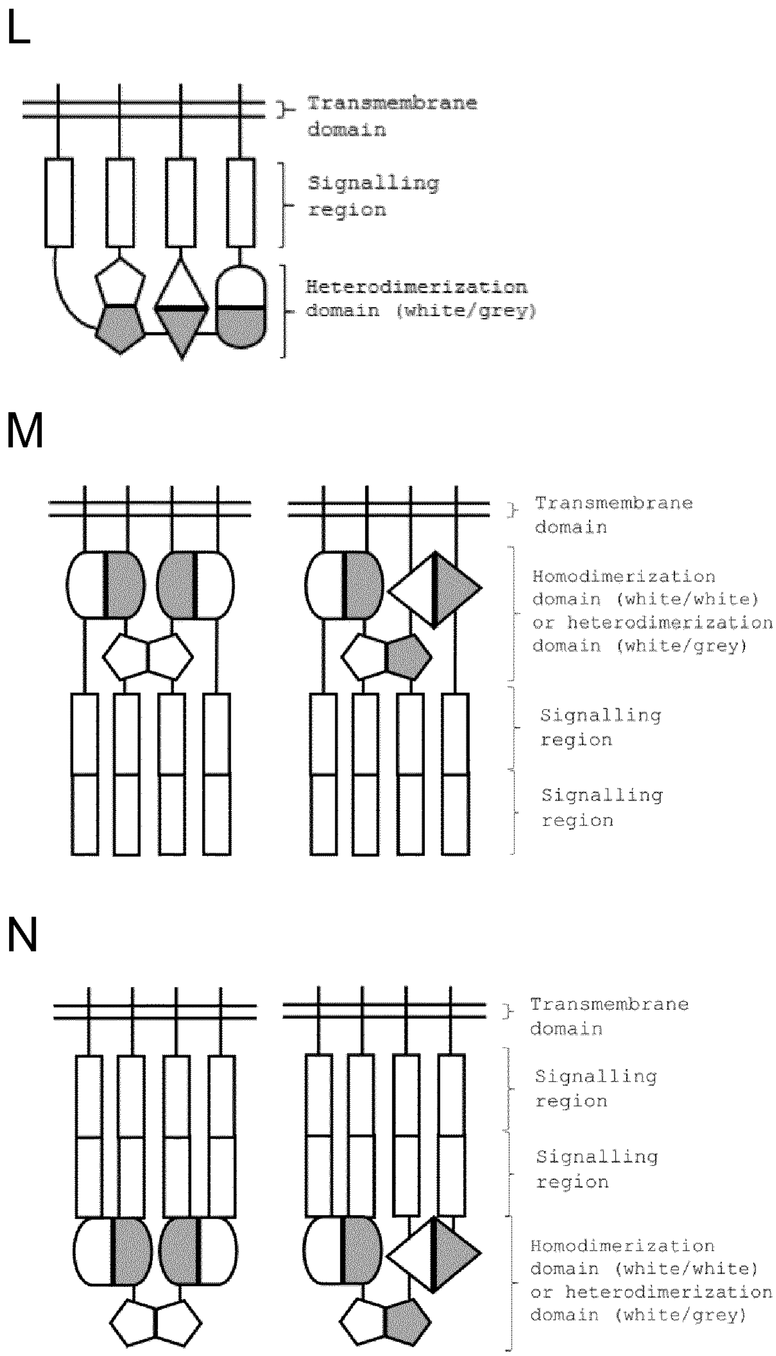
L
Transmembrane domain
Signalling region
Heterodimerization domain (white/grey)
M
Transmembrane domain
Homodimerization domain (white/white) or heterodimerization domain (white/grey)
Signalling region
Signalling region
N
Transmembrane domain
Signalling region
Signalling region
Homodimerization domain (white/white) or heterodimerization domain (white/grey)

Fig. 1

O

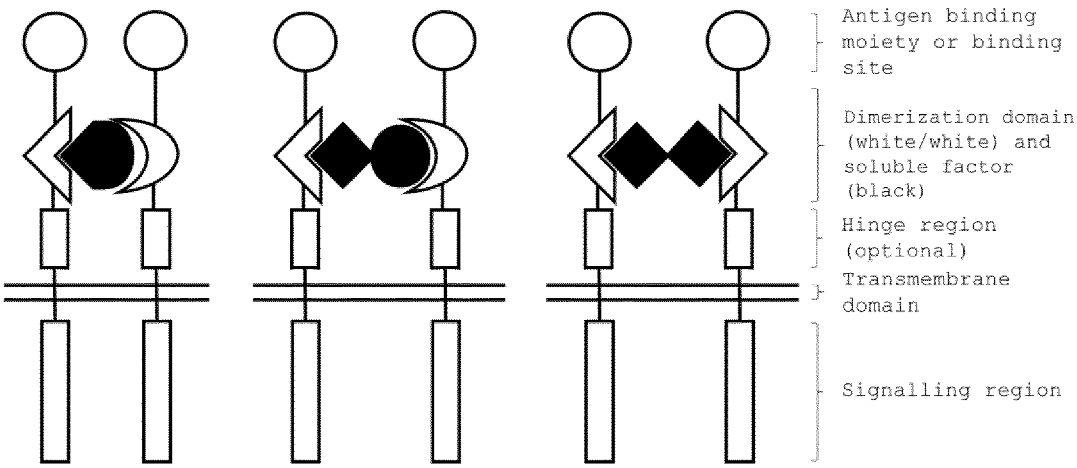
Antigen binding moiety or binding site
Dimerization domain (white/white) and soluble factor (black)
Hinge region (optional)
Transmembrane domain
Signalling region

Fig. 2

| | FACS titration[a)] | Kinetic analysis mode – SPR[b)] | Steady state analysis mode – SPR[c)] |
|---|---|---|---|
| **Ligand** | tEGFR (expressed on Jurkat T cells) | EGFR-Fc (immobilized on Protein A sensor chip) | EGFR-Fc (immobilized on Protein A sensor chip) |
| | | | |
| **Analyte** | **Mean $K_d$ [nM]** | **Mean $K_d$ [nM]** | **Mean $K_d$ [nM]** |
| E11.4.1-WT | 12.9 (n = 3) | 17.7 (n = 3) | n.a. |
| E11.4.1-G25A | 121.3 (n = 3) | 196.0 (n = 2) | 135.0 (n = 2) |
| E11.4.1-G32A | 771.7 (n = 3) | 436.0 (n = 3) | 512.0 (n = 3) |

Fig. 3

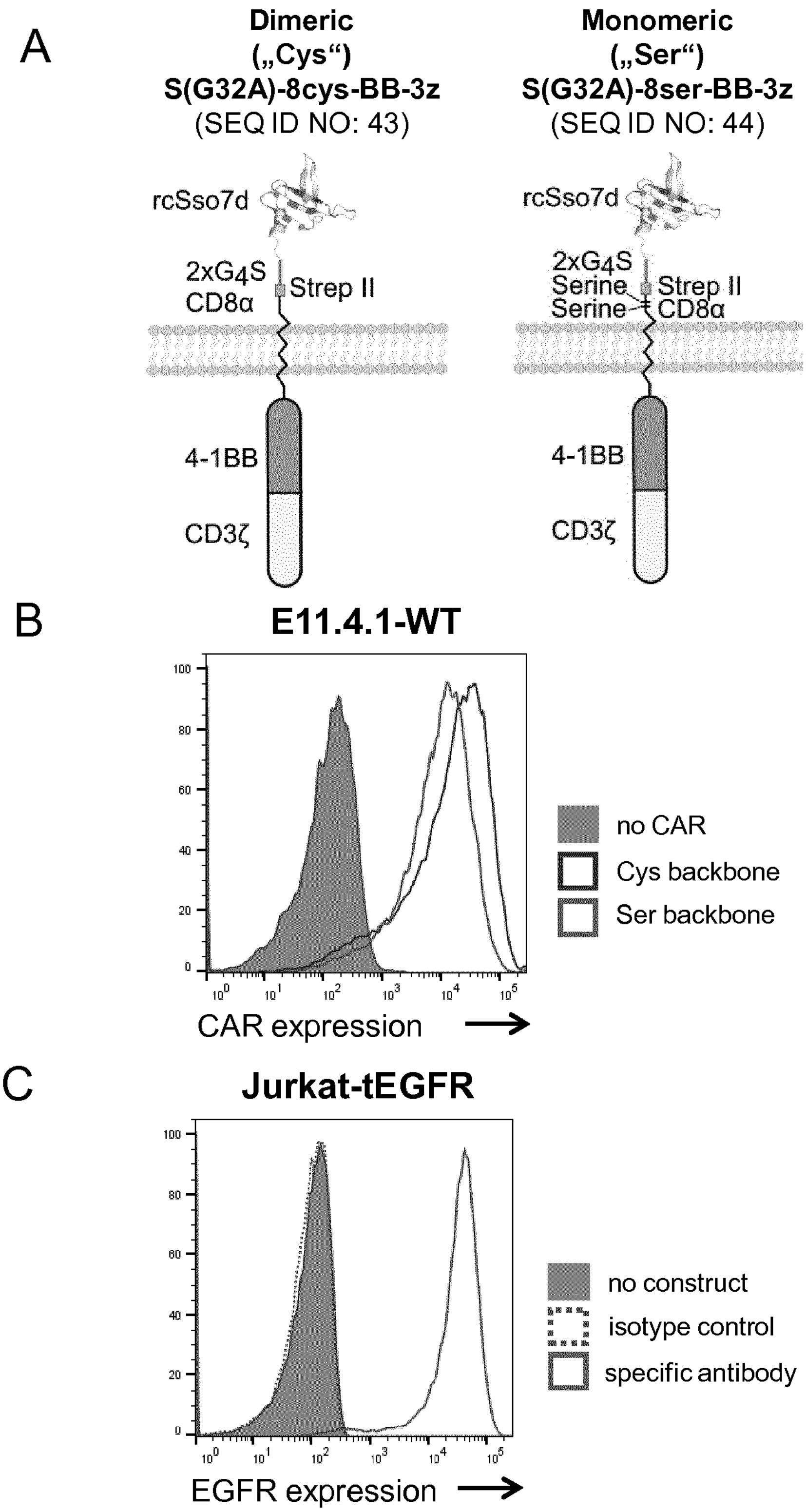
A
Dimeric
(„Cys“)
S(G32A)-8cys-BB-3z
(SEQ ID NO: 43)
Monomeric
(„Ser“)
S(G32A)-8ser-BB-3z
(SEQ ID NO: 44)
rcSso7d
2xG4S
Strep II
CD8α
Serine
Serine
4-1BB
CD3ζ
B
E11.4.1-WT
no CAR
Cys backbone
Ser backbone
CAR expression
C
Jurkat-tEGFR
no construct
isotype control
specific antibody
EGFR expression

Fig. 3

D

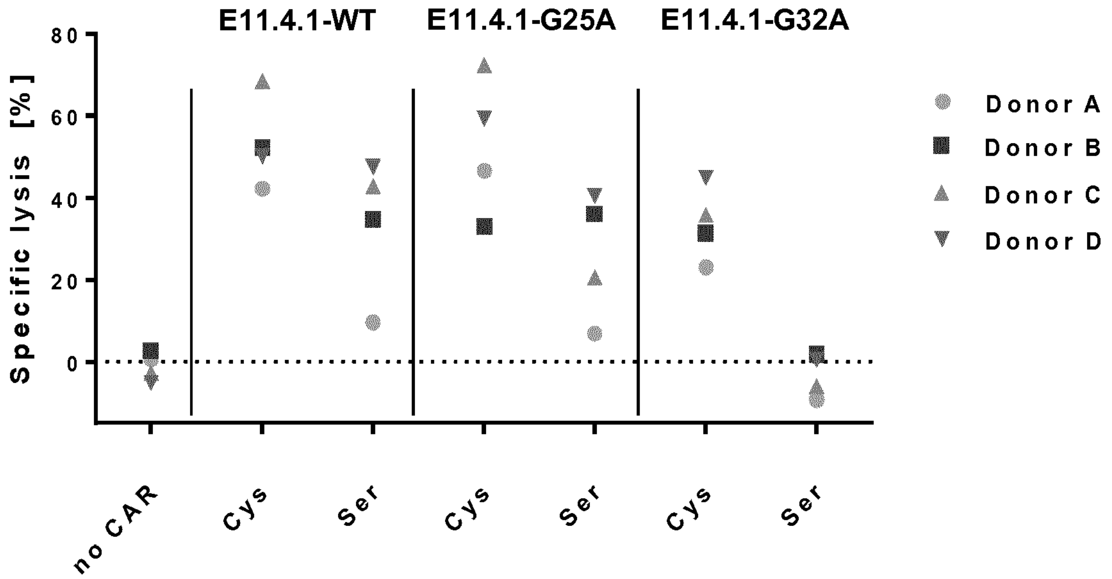
E11.4.1-WT
E11.4.1-G25A
E11.4.1-G32A
Specific lysis [%]
80
60
40
20
0
no CAR
Cys
Ser
Cys
Ser
Cys
Ser
Donor A
Donor B
Donor C
Donor D

E

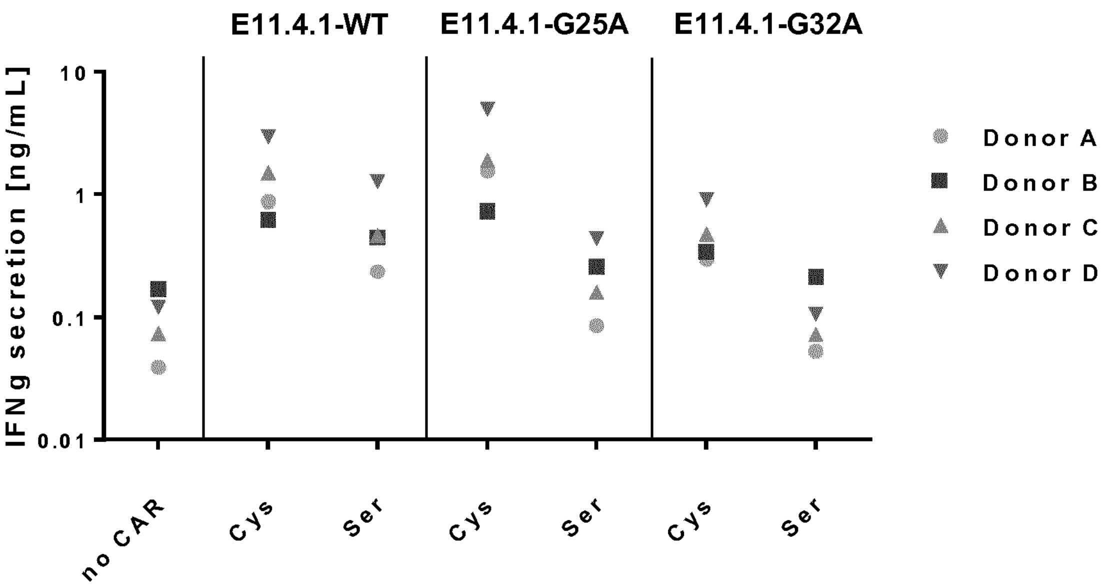
E11.4.1-WT
E11.4.1-G25A
E11.4.1-G32A
IFNg secretion [ng/mL]
10
1
0.1
0.01
no CAR
Cys
Ser
Cys
Ser
Cys
Ser
Donor A
Donor B
Donor C
Donor D

Fig. 4

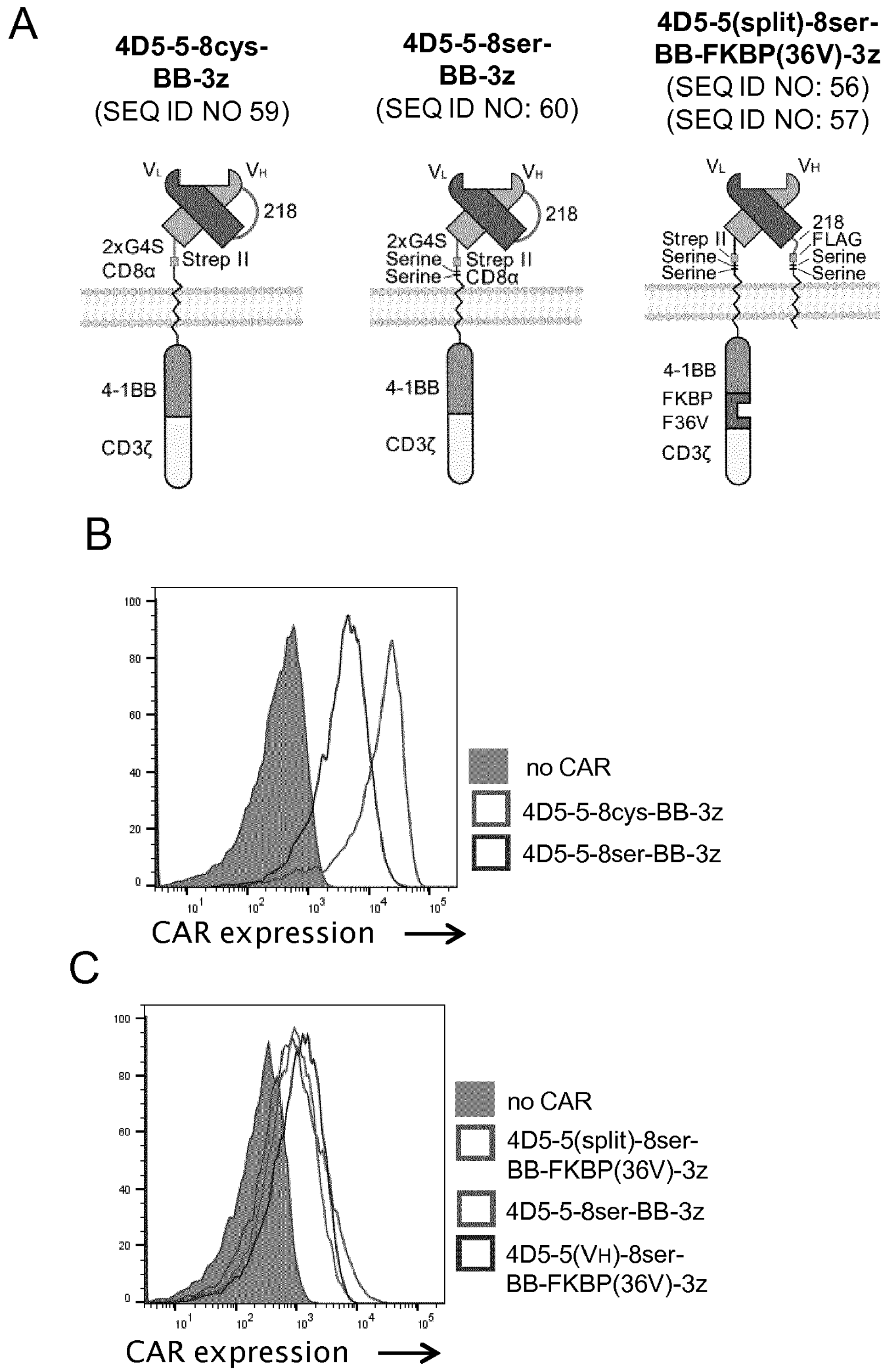
A
4D5-5-8cys-BB-3z
(SEQ ID NO 59)
4D5-5-8ser-BB-3z
(SEQ ID NO: 60)
4D5-5(split)-8ser-BB-FKBP(36V)-3z
(SEQ ID NO: 56)
(SEQ ID NO: 57)
VL
VH
218
2xG4S
CD8α
Strep II
Serine
FLAG
4-1BB
CD3ζ
FKBP
F36V
B
no CAR
4D5-5-8cys-BB-3z
4D5-5-8ser-BB-3z
CAR expression
C
no CAR
4D5-5(split)-8ser-BB-FKBP(36V)-3z
4D5-5-8ser-BB-3z
4D5-5(VH)-8ser-BB-FKBP(36V)-3z
CAR expression

Fig. 4

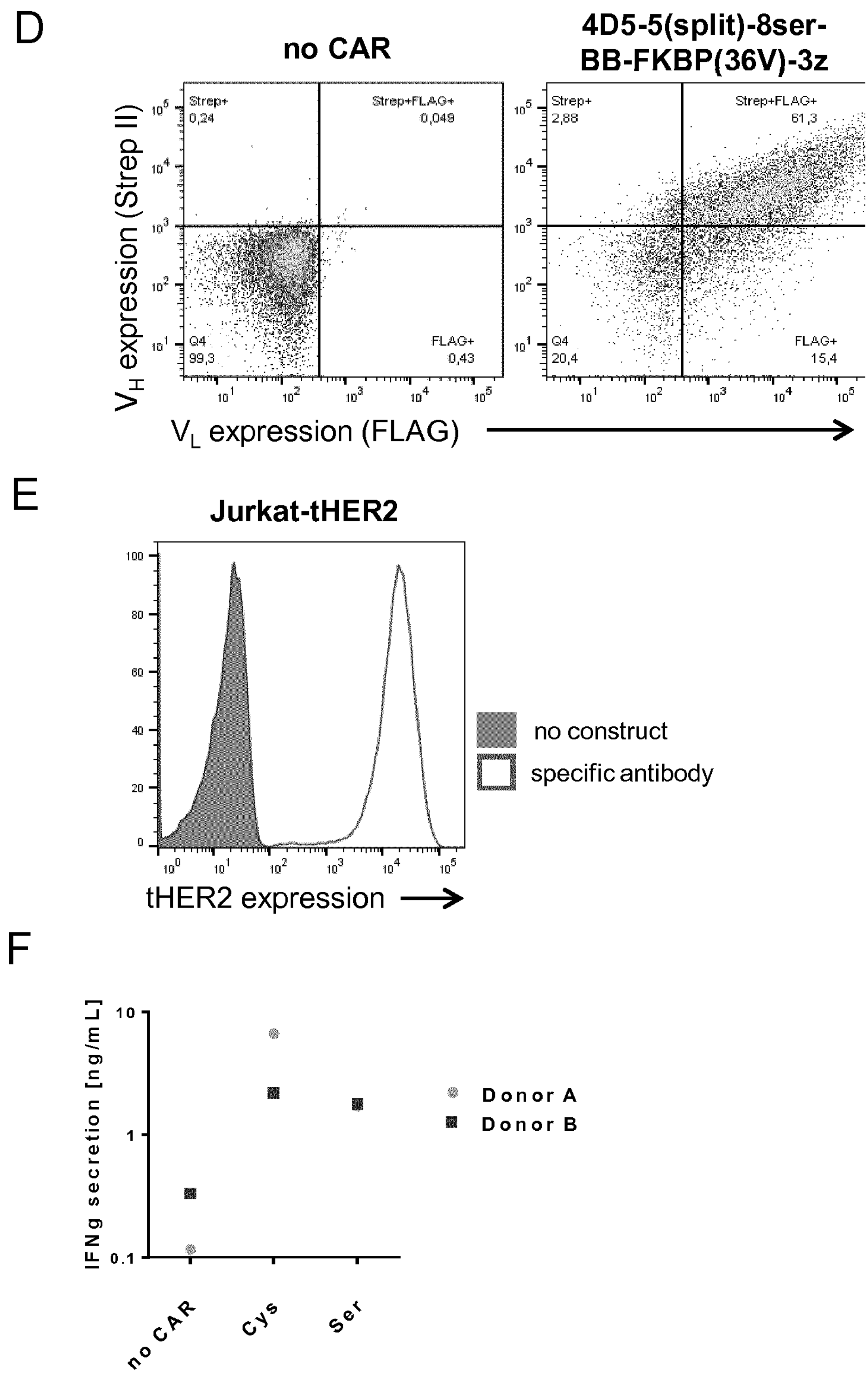
D
no CAR
4D5-5(split)-8ser-BB-FKBP(36V)-3z
Strep+
0,24
Strep+FLAG+
0,049
Q4
99,3
FLAG+
0,43
Strep+
2,88
Strep+FLAG+
61,3
Q4
20,4
FLAG+
15,4
$V_H$ expression (Strep II)
$V_L$ expression (FLAG)
E
Jurkat-tHER2
no construct
specific antibody
tHER2 expression
F
IFNg secretion [ng/mL]
Donor A
Donor B
no CAR
Cys
Ser

Fig. 4

G

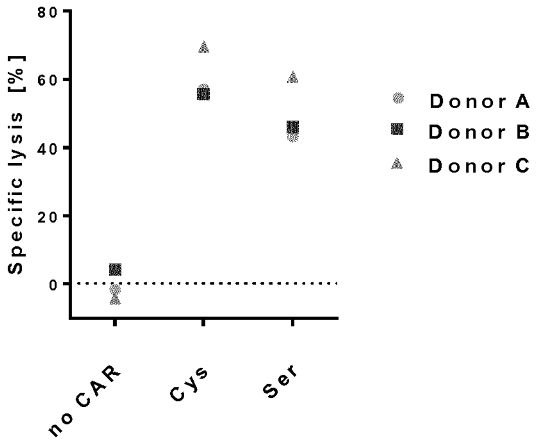
80
60
40
20
0
Specific lysis [%]
no CAR
Cys
Ser
Donor A
Donor B
Donor C

H

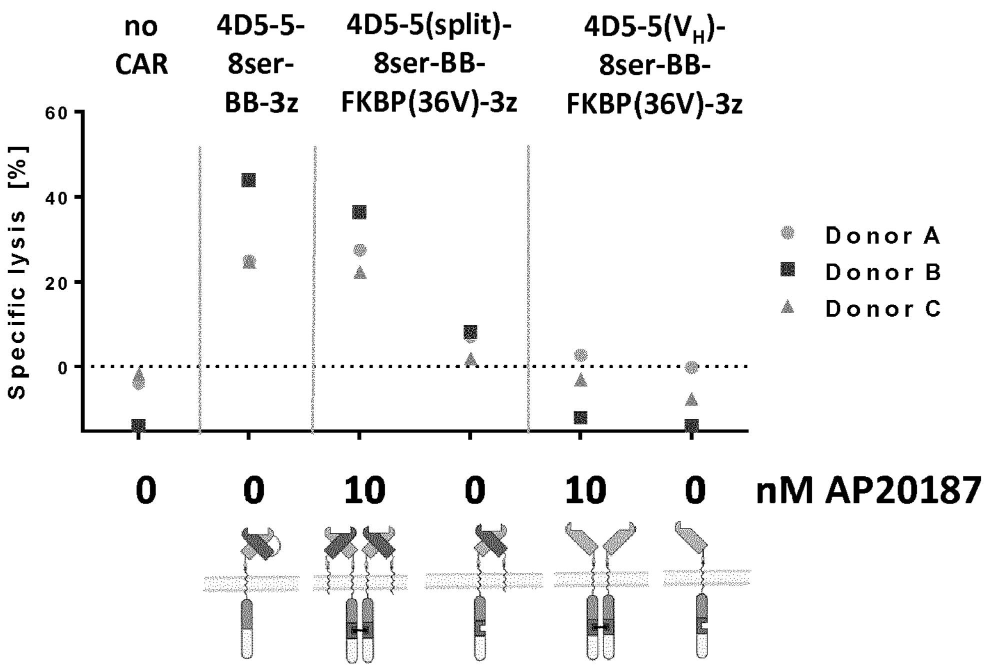
no CAR
4D5-5-8ser-BB-3z
4D5-5(split)-8ser-BB-FKBP(36V)-3z
4D5-5($V_H$)-8ser-BB-FKBP(36V)-3z
60
40
20
0
Specific lysis [%]
Donor A
Donor B
Donor C
0
0
10
0
10
0
nM AP20187

Fig. 5

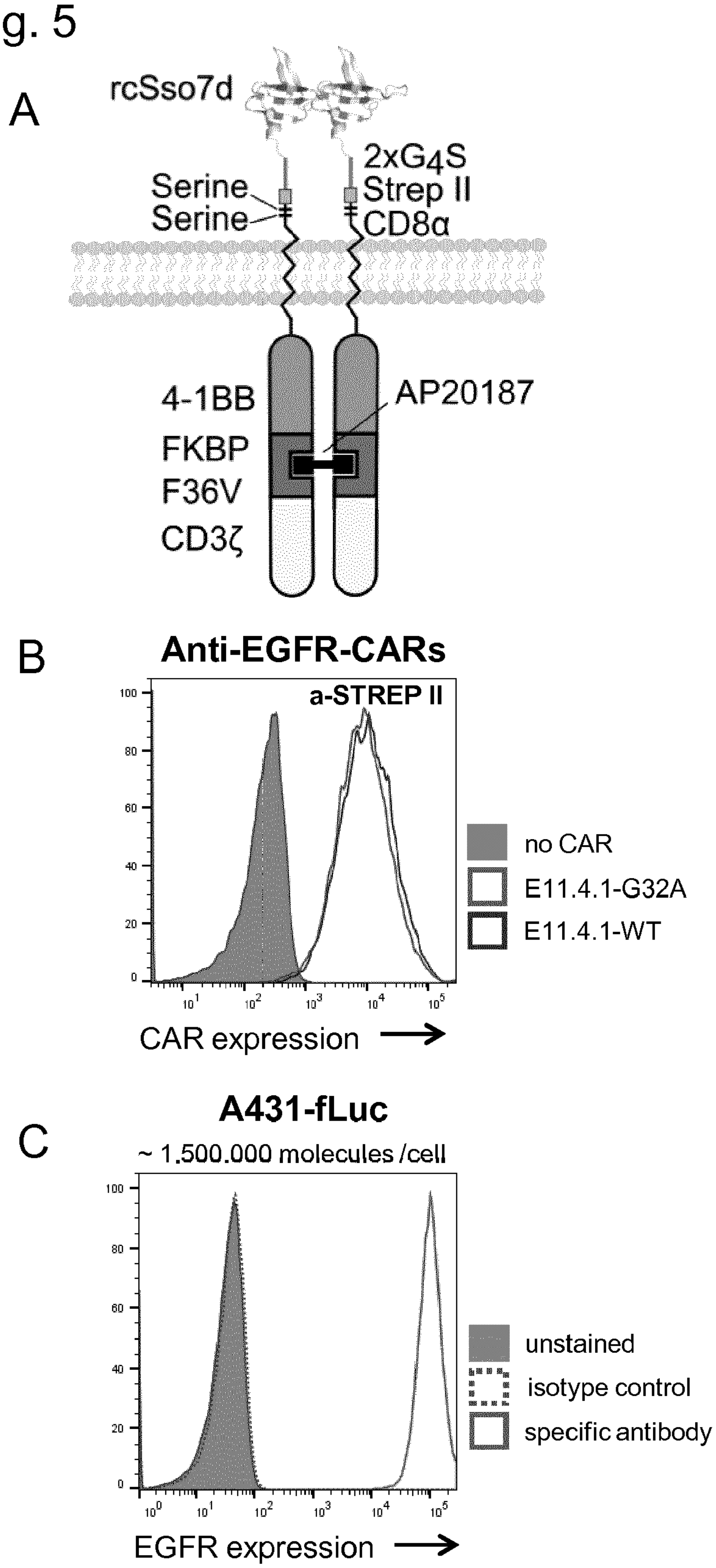
A
rcSso7d
2xG4S
Strep II
Serine
Serine
CD8α
4-1BB
FKBP
F36V
CD3ζ
AP20187
B
Anti-EGFR-CARs
a-STREP II
no CAR
E11.4.1-G32A
E11.4.1-WT
CAR expression
C
A431-fLuc
~ 1.500.000 molecules /cell
unstained
isotype control
specific antibody
EGFR expression

Fig. 5

D

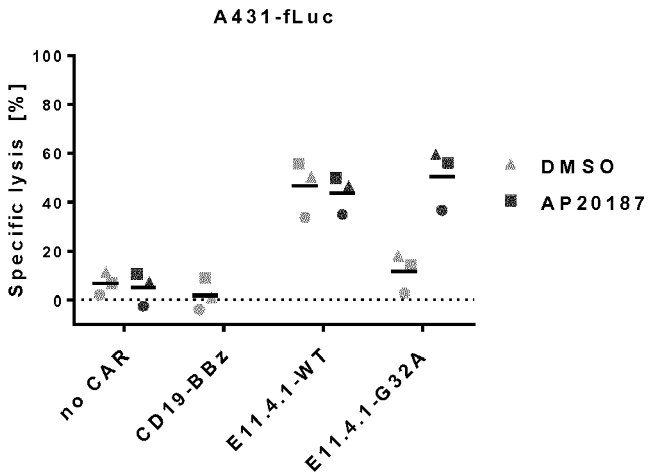
A431-fLuc
Specific lysis [%]
100
80
60
40
20
0
no CAR
CD19-BBz
E11.4.1-WT
E11.4.1-G32A
DMSO
AP20187

Fig. 6

A

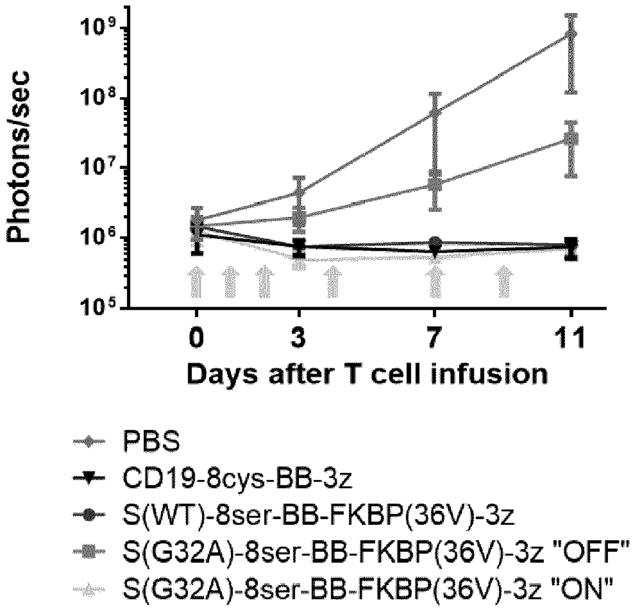
Photons/sec
10⁹
10⁸
10⁷
10⁶
10⁵
0
3
7
11
Days after T cell infusion
PBS
CD19-8cys-BB-3z
S(WT)-8ser-BB-FKBP(36V)-3z
S(G32A)-8ser-BB-FKBP(36V)-3z "OFF"
S(G32A)-8ser-BB-FKBP(36V)-3z "ON"

B

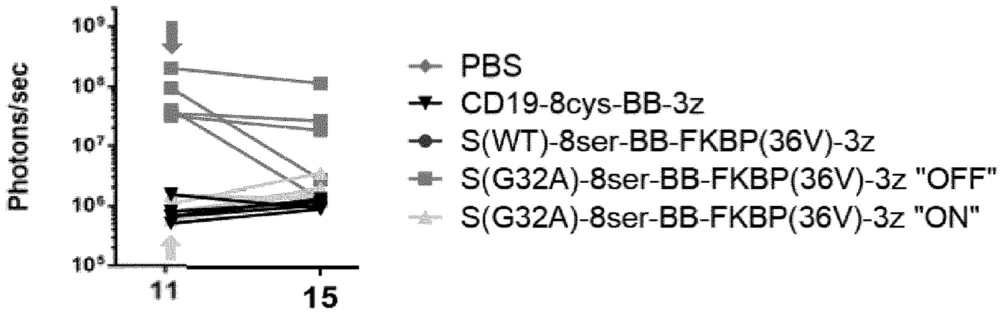
Photons/sec
10⁹
10⁸
10⁷
10⁶
10⁵
11
15
PBS
CD19-8cys-BB-3z
S(WT)-8ser-BB-FKBP(36V)-3z
S(G32A)-8ser-BB-FKBP(36V)-3z "OFF"
S(G32A)-8ser-BB-FKBP(36V)-3z "ON"

Fig. 7

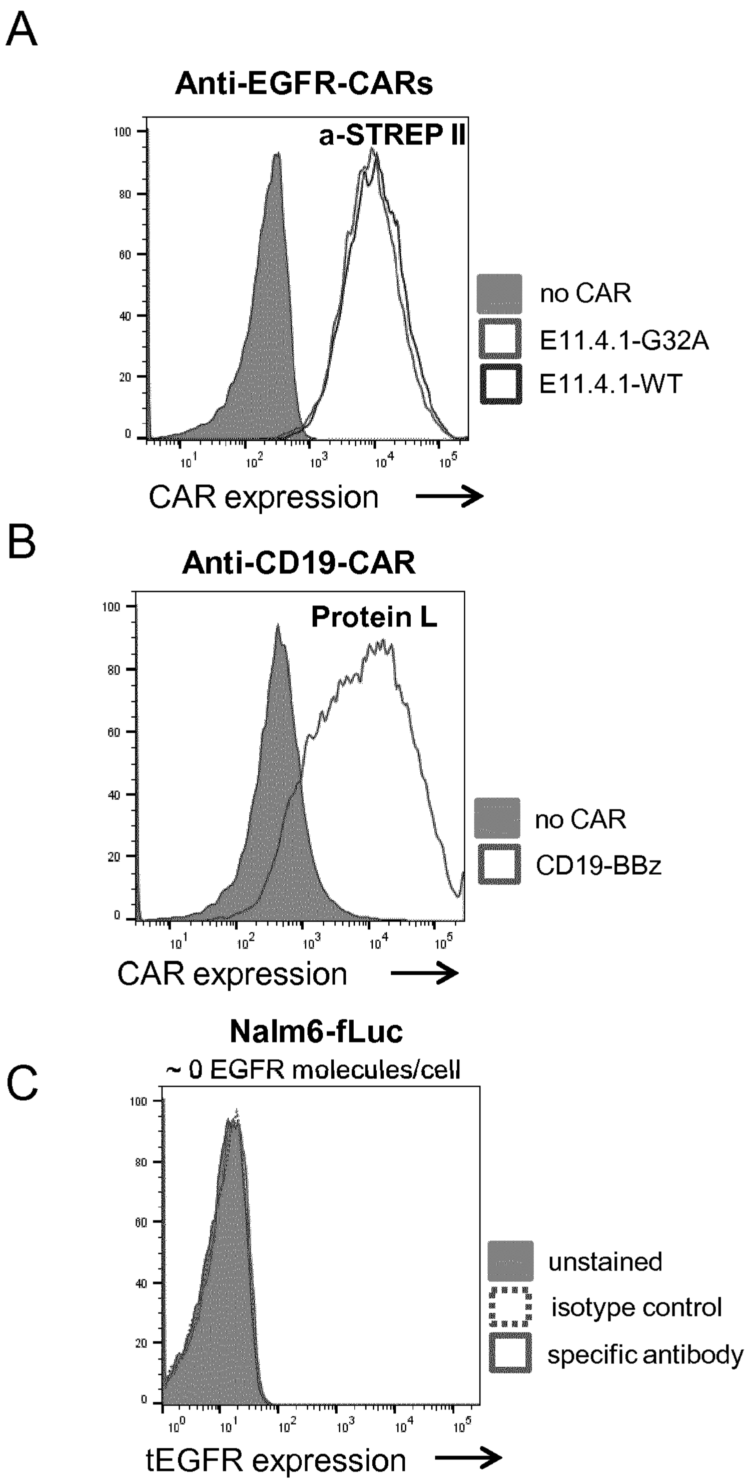
A
Anti-EGFR-CARs
a-STREP II
100
80
60
40
20
0
$10^1$
$10^2$
$10^3$
$10^4$
$10^5$
CAR expression →
no CAR
E11.4.1-G32A
E11.4.1-WT
B
Anti-CD19-CAR
Protein L
100
80
60
40
20
0
$10^1$
$10^2$
$10^3$
$10^4$
$10^5$
CAR expression →
no CAR
CD19-BBz
C
Nalm6-fLuc
~ 0 EGFR molecules/cell
100
80
60
40
20
0
$10^0$
$10^1$
$10^2$
$10^3$
$10^4$
$10^5$
tEGFR expression →
unstained
isotype control
specific antibody

Fig. 7

D

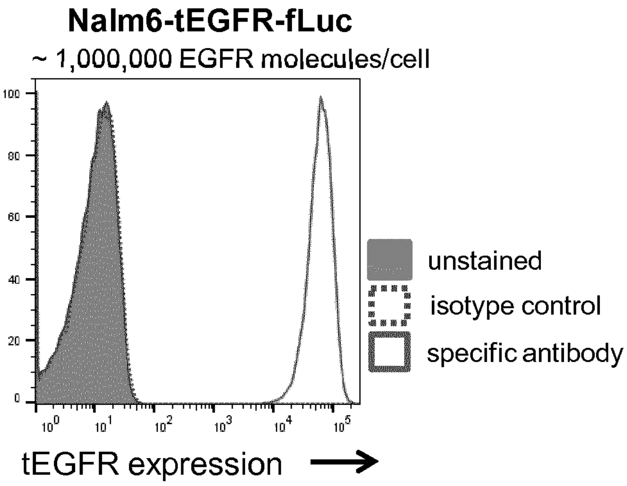
Nalm6-tEGFR-fLuc
~ 1,000,000 EGFR molecules/cell
100
80
60
40
20
0
$10^0$
$10^1$
$10^2$
$10^3$
$10^4$
$10^5$
unstained
isotype control
specific antibody
tEGFR expression

E

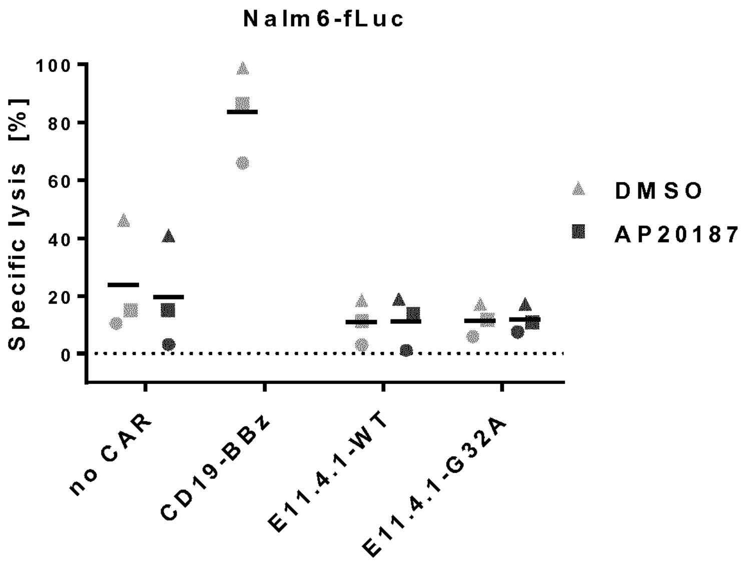
Nalm6-fLuc
Specific lysis [%]
100
80
60
40
20
0
DMSO
AP20187
no CAR
CD19-BBz
E11.4.1-WT
E11.4.1-G32A

Fig. 7

F

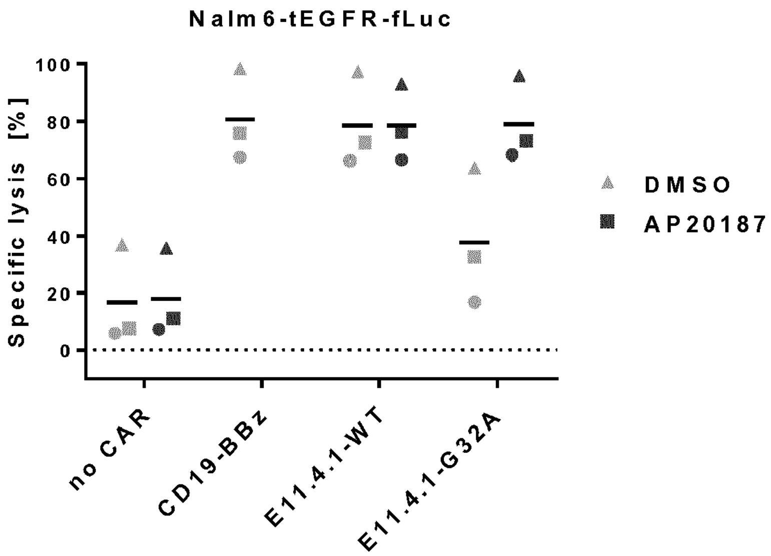
Nalm6-tEGFR-fLuc
Specific lysis [%]
100
80
60
40
20
0
no CAR
CD19-BBz
E11.4.1-WT
E11.4.1-G32A
DMSO
AP20187

Fig. 8

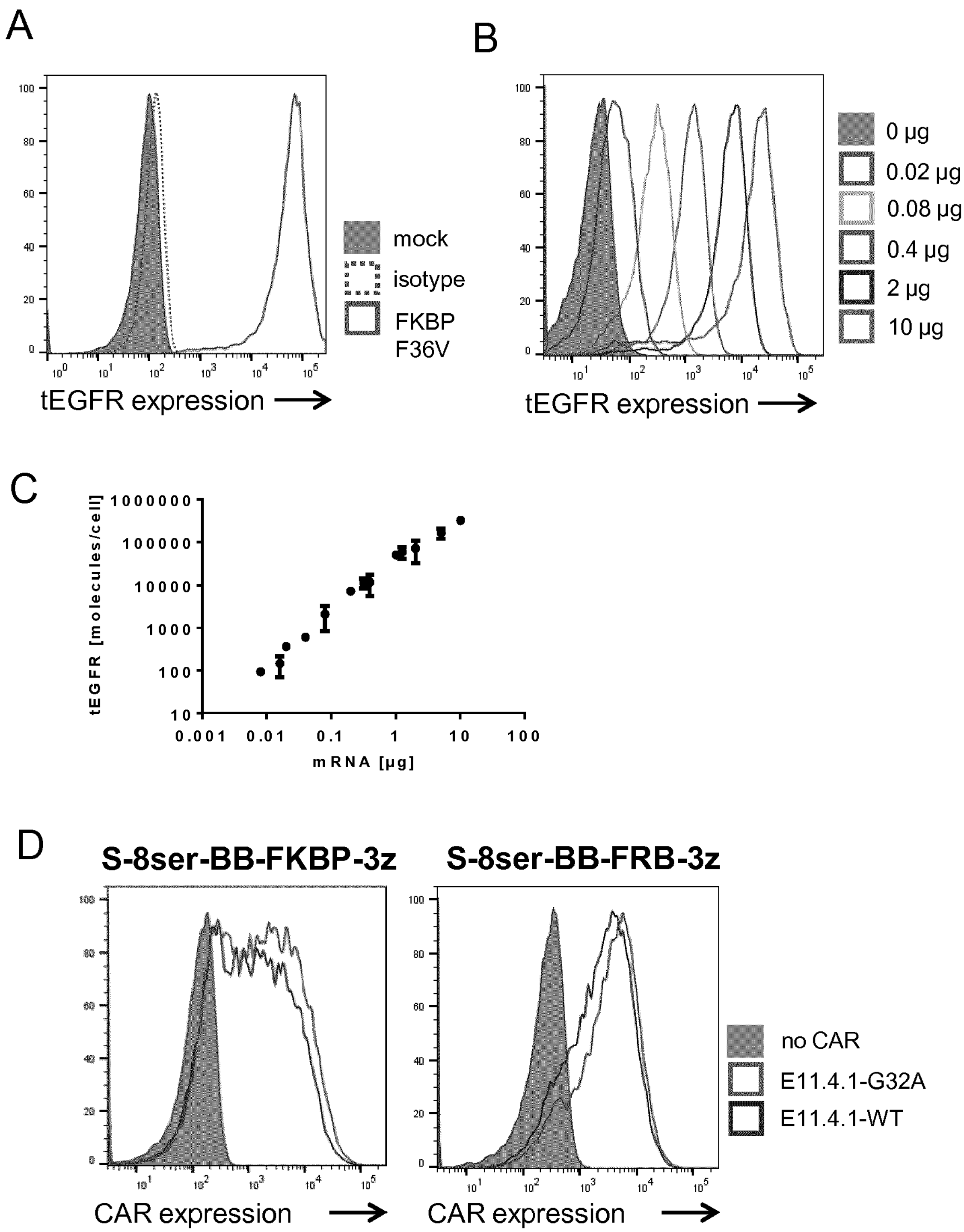
A
mock
isotype
FKBP F36V
tEGFR expression
B
0 µg
0.02 µg
0.08 µg
0.4 µg
2 µg
10 µg
tEGFR expression
C
tEGFR [molecules/cell]
mRNA [µg]
D
S-8ser-BB-FKBP-3z
S-8ser-BB-FRB-3z
no CAR
E11.4.1-G32A
E11.4.1-WT
CAR expression
CAR expression

Fig. 8

E

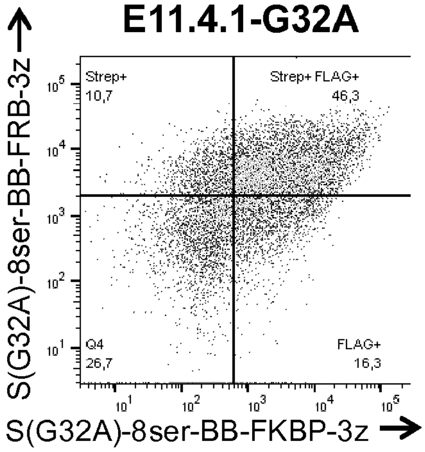
E11.4.1-G32A
Strep+
10,7
Strep+ FLAG+
46,3
Q4
26,7
FLAG+
16,3
S(G32A)-8ser-BB-FRB-3z
S(G32A)-8ser-BB-FKBP-3z

F

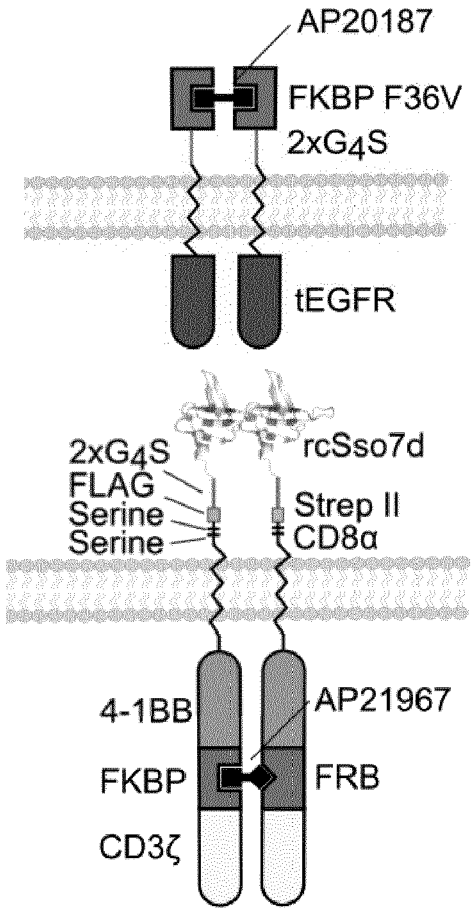
AP20187
FKBP F36V
2xG4S
tEGFR
2xG4S
FLAG
Serine
Serine
rcSso7d
Strep II
CD8α
4-1BB
AP21967
FKBP
FRB
CD3ζ

Fig. 8

G

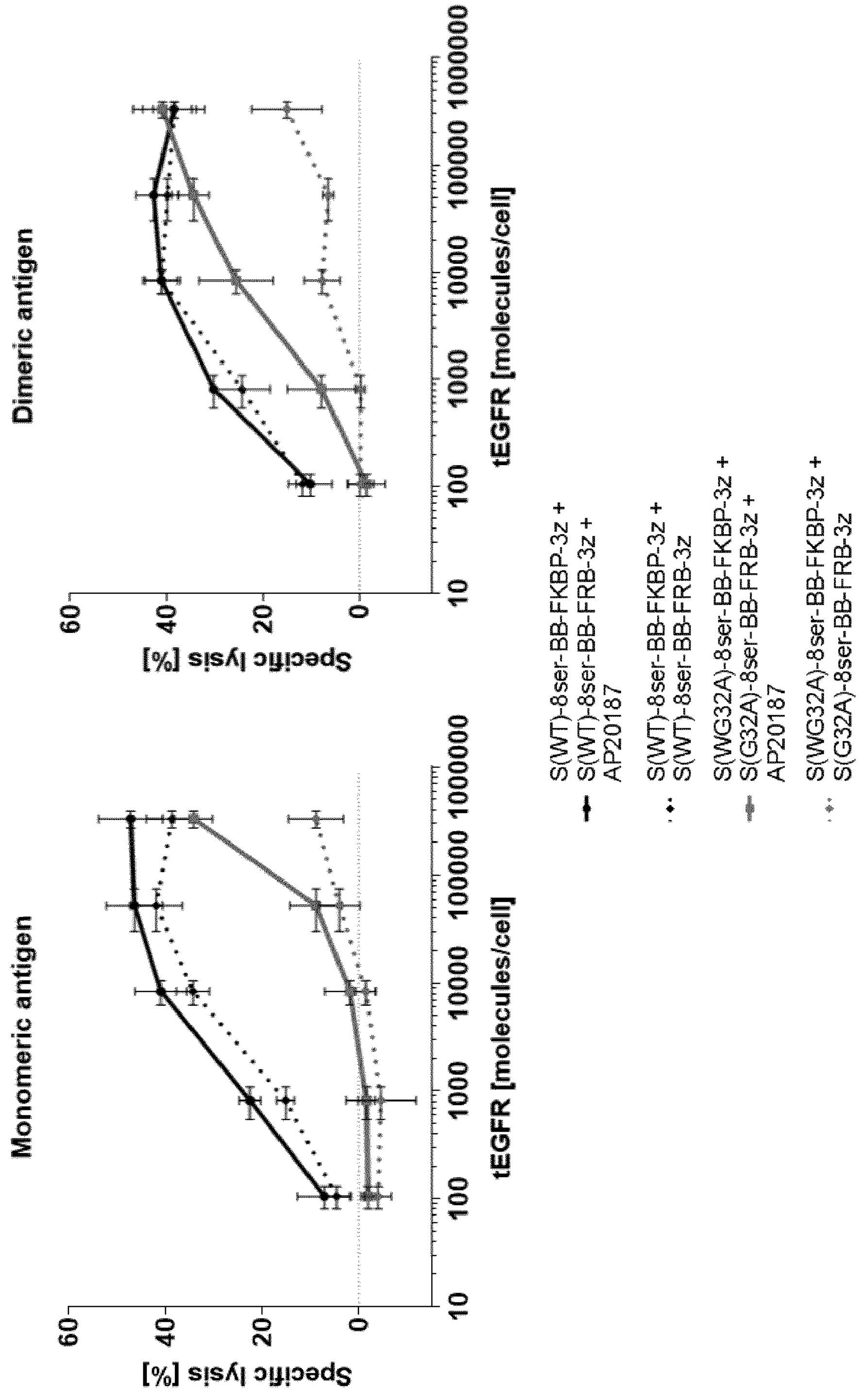
Monomeric antigen
Dimeric antigen
Specific lysis [%]
tEGFR [molecules/cell]
S(WT)-8ser-BB-FKBP-3z + S(WT)-8ser-BB-FRB-3z + AP20187
S(WT)-8ser-BB-FKBP-3z + S(WT)-8ser-BB-FRB-3z
S(WG32A)-8ser-BB-FKBP-3z + S(G32A)-8ser-BB-FRB-3z + AP20187
S(WG32A)-8ser-BB-FKBP-3z + S(G32A)-8ser-BB-FRB-3z

Fig. 8

H

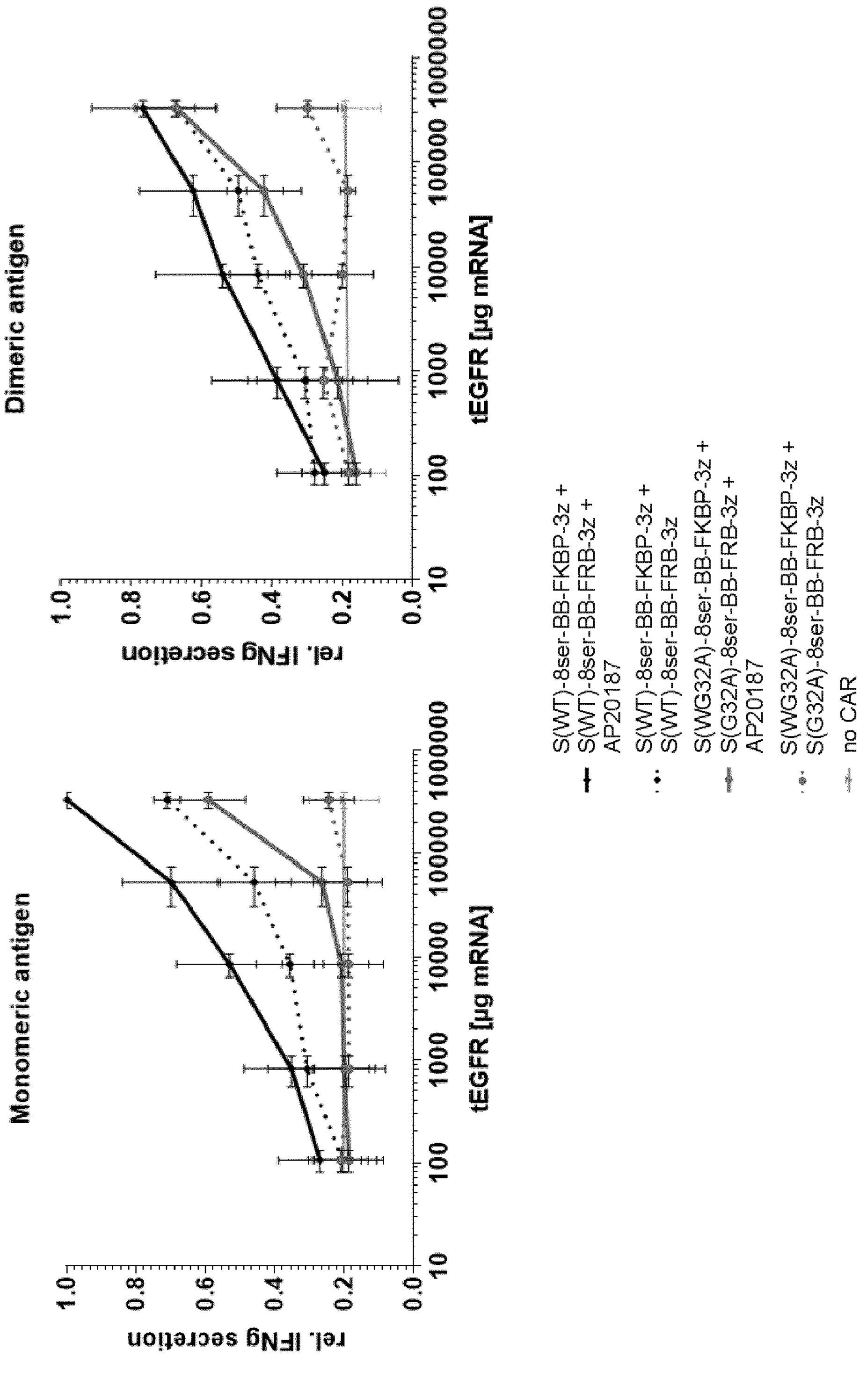

Monomeric antigen
Dimeric antigen
rel. IFNg secretion
tEGFR [µg mRNA]
1.0
0.8
0.6
0.4
0.2
0.0
10
100
1000
10000
100000
1000000
S(WT)-8ser-BB-FKBP-3z + S(WT)-8ser-BB-FRB-3z + AP20187
S(WT)-8ser-BB-FKBP-3z + S(WT)-8ser-BB-FRB-3z
S(WG32A)-8ser-BB-FKBP-3z + S(G32A)-8ser-BB-FRB-3z + AP20187
S(WG32A)-8ser-BB-FKBP-3z + S(G32A)-8ser-BB-FRB-3z
no CAR

Fig. 9

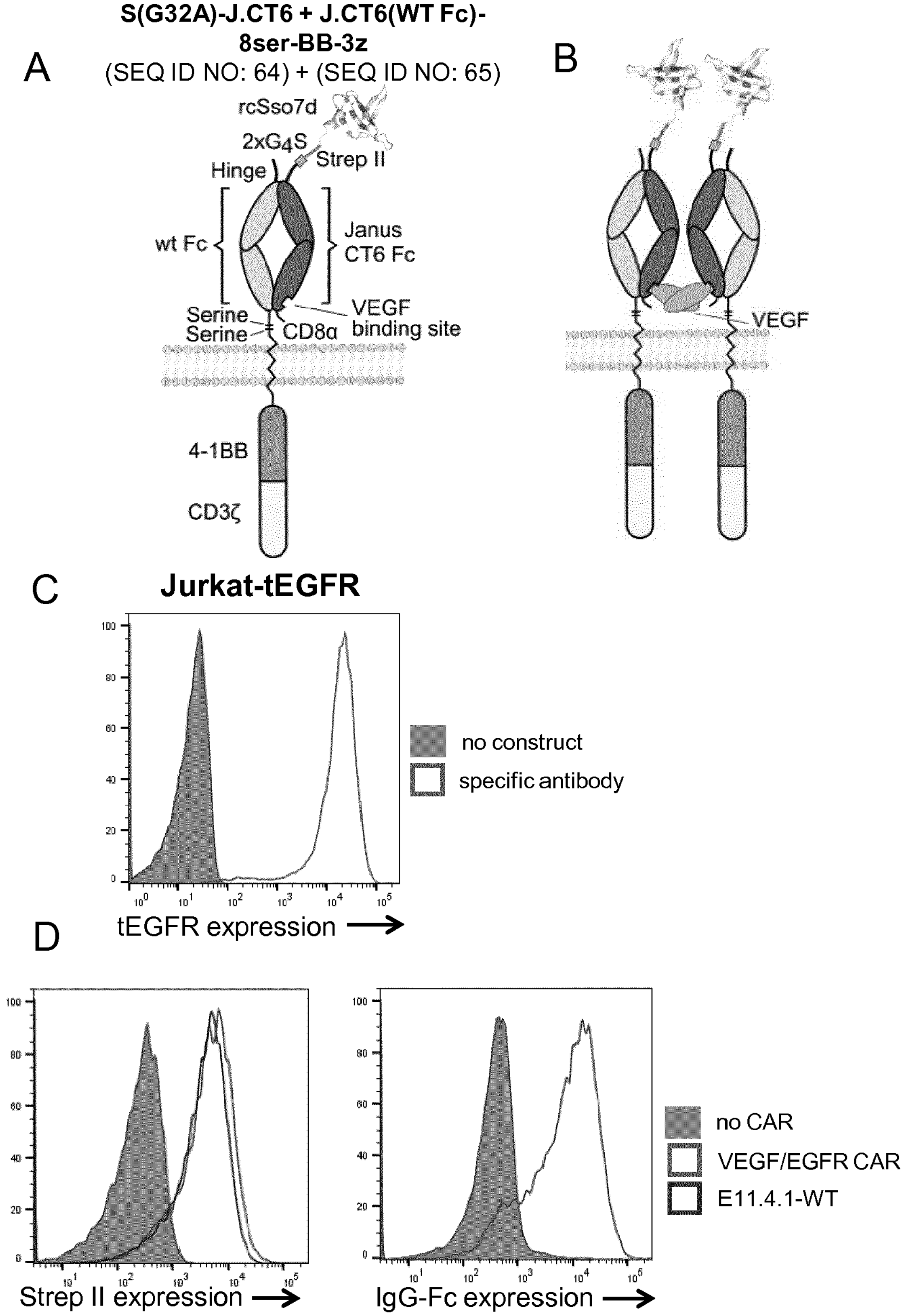
A
S(G32A)-J.CT6 + J.CT6(WT Fc)-
8ser-BB-3z
(SEQ ID NO: 64) + (SEQ ID NO: 65)
rcSso7d
2xG4S
Strep II
Hinge
wt Fc
Janus
CT6 Fc
Serine
Serine
CD8α
VEGF
binding site
4-1BB
CD3ζ
B
VEGF
C
Jurkat-tEGFR
no construct
specific antibody
tEGFR expression
D
no CAR
VEGF/EGFR CAR
E11.4.1-WT
Strep II expression
IgG-Fc expression

Fig. 9

E)

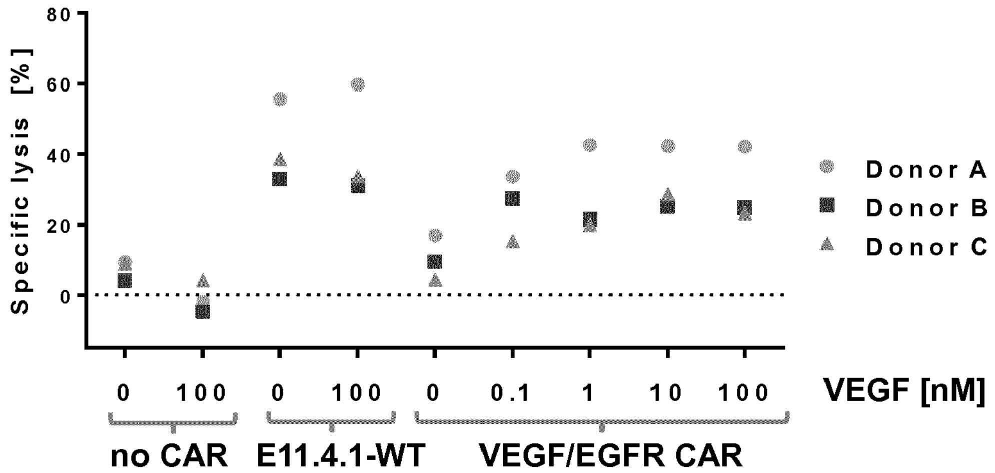
80
60
40
20
0
Specific lysis [%]
Donor A
Donor B
Donor C
0
100
0
100
0
0.1
1
10
100
VEGF [nM]
no CAR
E11.4.1-WT
VEGF/EGFR CAR

Fig. 10

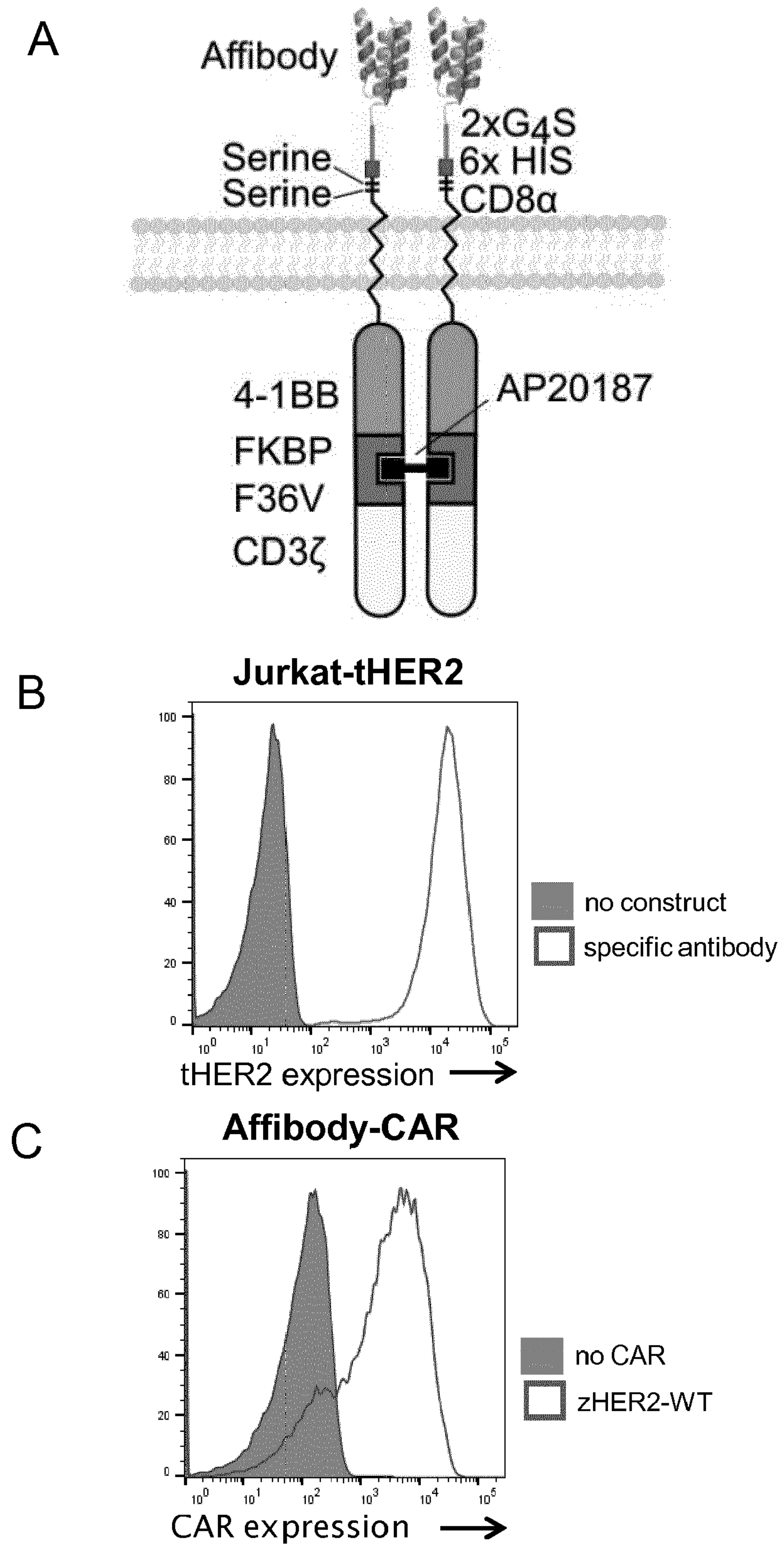
A
Affibody
2xG4S
6x HIS
CD8α
Serine
Serine
4-1BB
FKBP
F36V
CD3ζ
AP20187
B
Jurkat-tHER2
no construct
specific antibody
tHER2 expression
C
Affibody-CAR
no CAR
zHER2-WT
CAR expression

Fig. 10

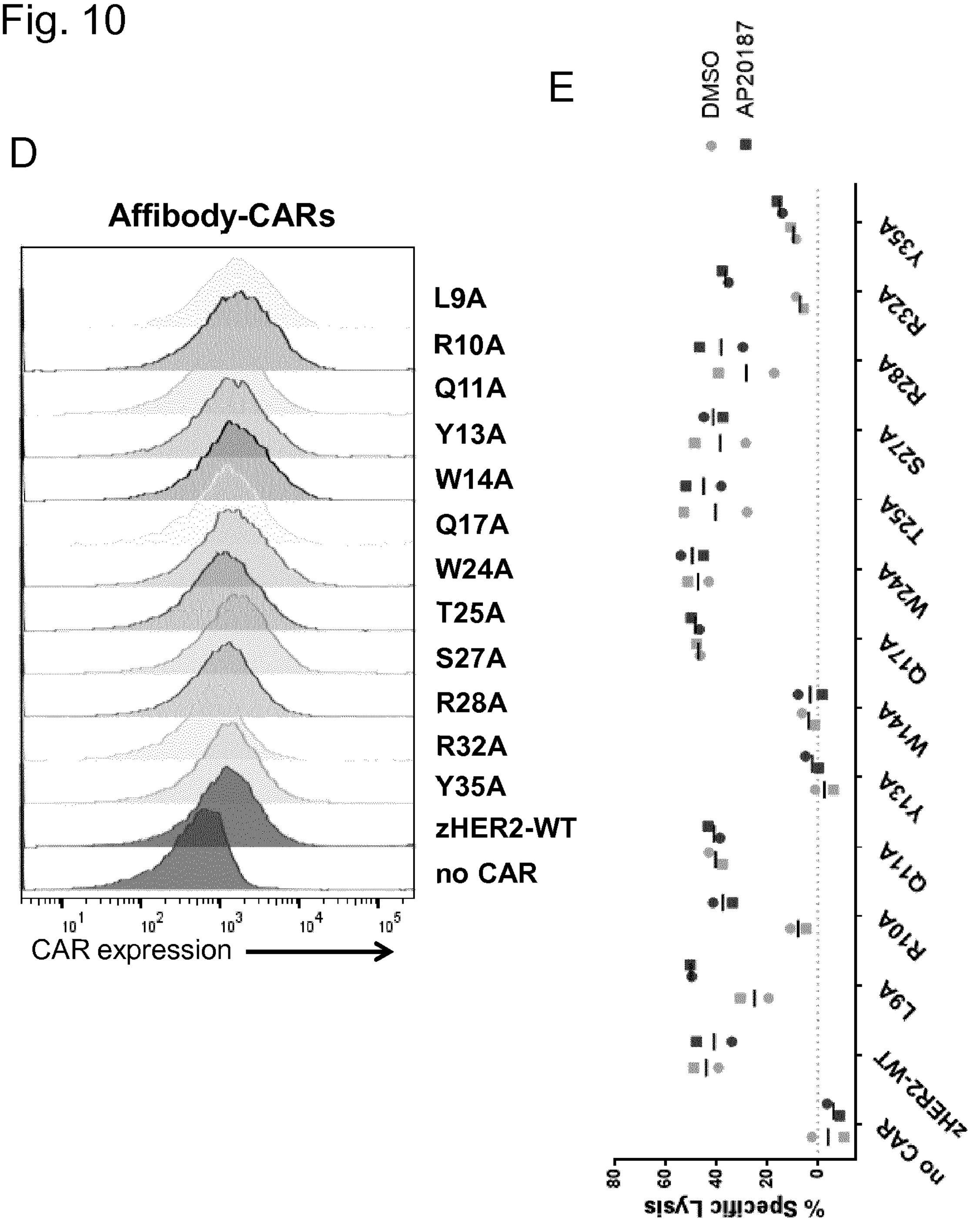
D
Affibody-CARs
L9A
R10A
Q11A
Y13A
W14A
Q17A
W24A
T25A
S27A
R28A
R32A
Y35A
zHER2-WT
no CAR
$10^1$
$10^2$
$10^3$
$10^4$
$10^5$
CAR expression
E
DMSO
AP20187
% Specific Lysis
80
60
40
20
0
Y35A
R32A
R28A
S27A
T25A
W24A
Q17A
W14A
Y13A
Q11A
R10A
L9A
zHER2-WT
no CAR

Fig. 10

F

| | Kinetic analysis mode – SPR |
|---|---|
| Ligand | HER2-Fc (immobilized on Protein A sensor chip) |
| Analyte | Mean $K_d$ [nM] |
| zHER2-WT | 22.9 (n = 4) |
| zHER2-R10A | 639.0 (n = 4) |
| zHER2-R32A | 285.0 (n = 4) |

Fig. 11

A

**S(G32A)-8ser-BB-FRB-FRB +**
**S(G32A)-8ser-BB-FKBP-3z**
(SEQ ID NO: 67) + (SEQ ID NO: 48)

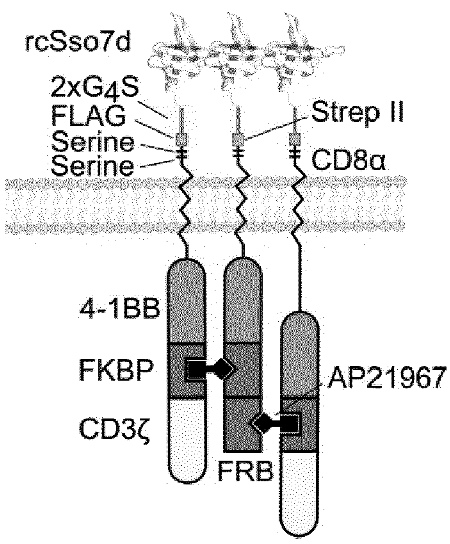


B

**S(G32A)-8ser-BB-FRB-FKBP(36V) +**
**S(G32A)-8ser-BB-FKBP-3z**
(SEQ ID NO: 68) + (SEQ ID NO: 48)

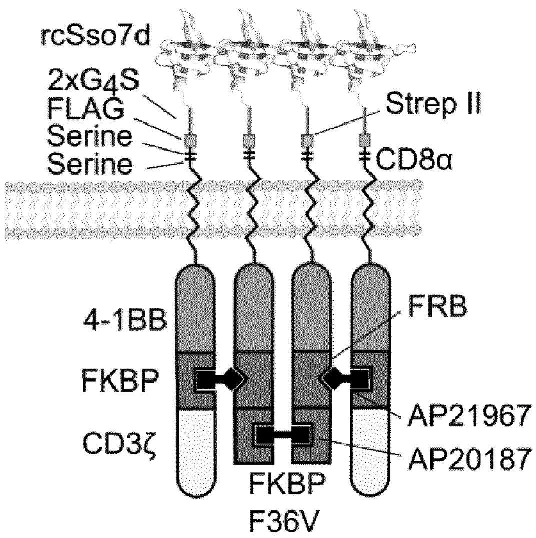


C

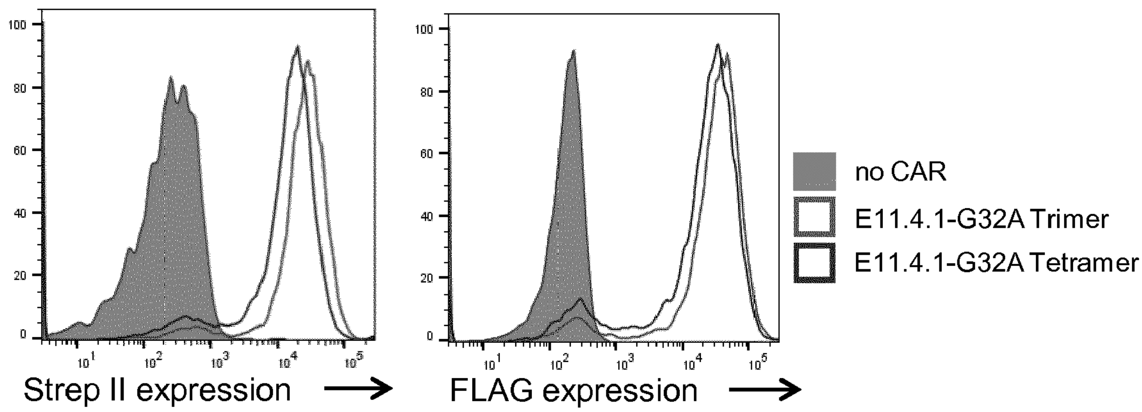

Fig. 11

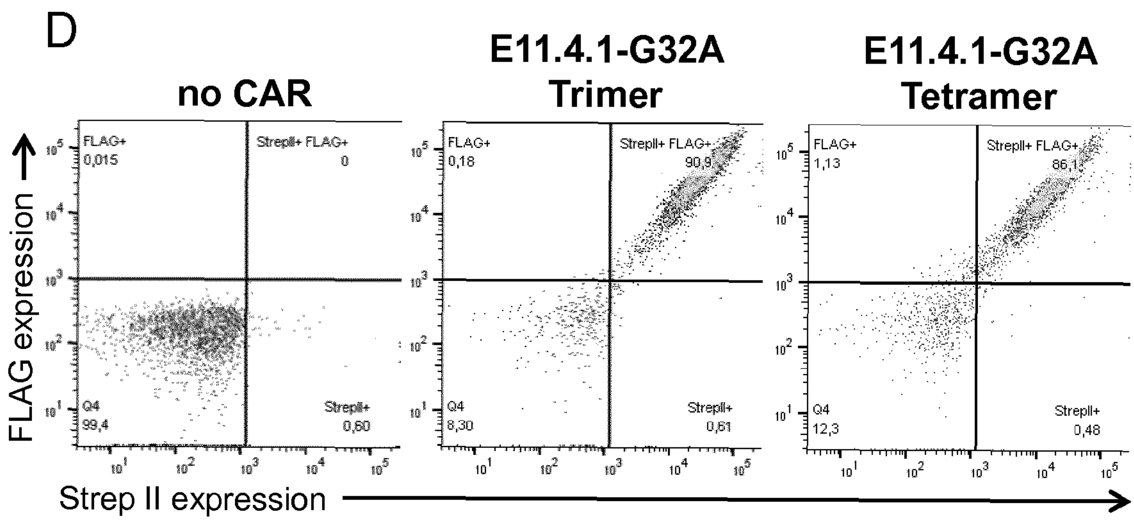
D
no CAR
E11.4.1-G32A Trimer
E11.4.1-G32A Tetramer
FLAG expression
Strep II expression
FLAG+ 0,015
StrepII+ FLAG+ 0
Q4 99,4
StrepII+ 0,60
FLAG+ 0,18
StrepII+ FLAG+ 90,9
Q4 8,30
StrepII+ 0,61
FLAG+ 1,13
StrepII+ FLAG+ 86,1
Q4 12,3
StrepII+ 0,48

Fig. 12

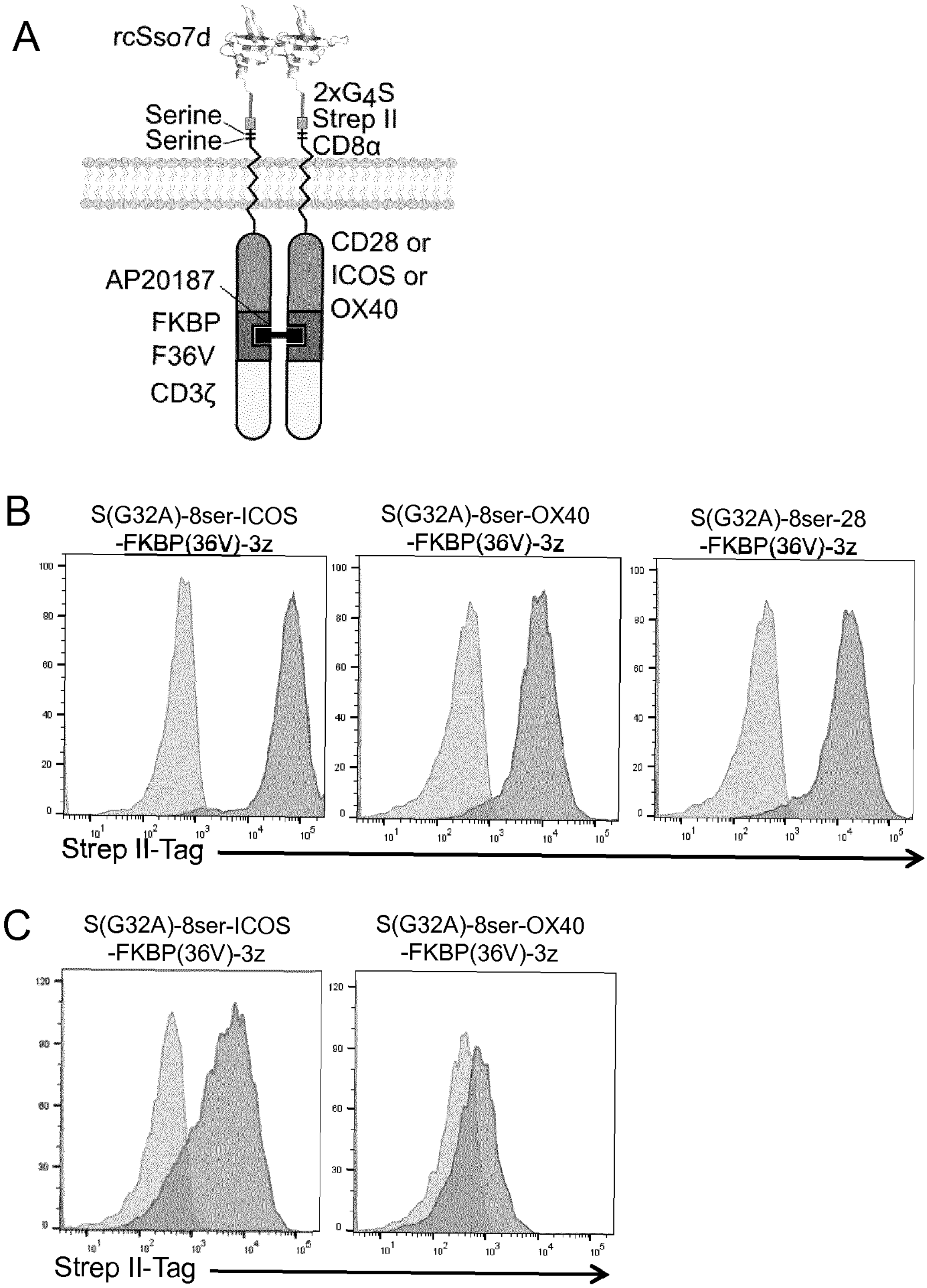
A
rcSso7d
2xG4S
Strep II
CD8α
Serine
Serine
CD28 or
ICOS or
OX40
AP20187
FKBP
F36V
CD3ζ
B
S(G32A)-8ser-ICOS
-FKBP(36V)-3z
S(G32A)-8ser-OX40
-FKBP(36V)-3z
S(G32A)-8ser-28
-FKBP(36V)-3z
Strep II-Tag
C
S(G32A)-8ser-ICOS
-FKBP(36V)-3z
S(G32A)-8ser-OX40
-FKBP(36V)-3z
Strep II-Tag

Fig. 12

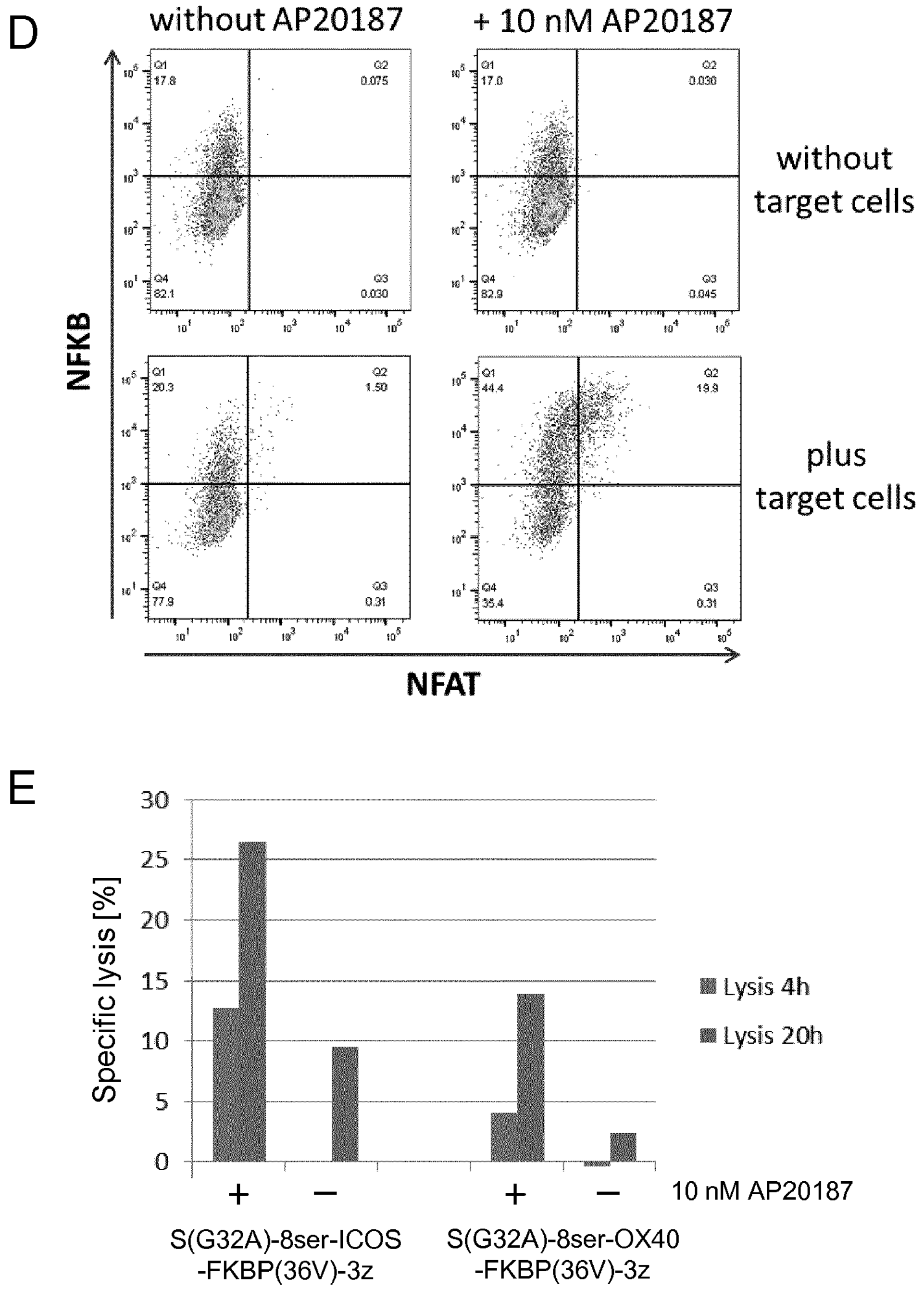
D
without AP20187
+ 10 nM AP20187
Q1 17.8
Q2 0.075
Q4 82.1
Q3 0.030
Q1 17.0
Q2 0.030
Q4 82.9
Q3 0.045
without target cells
Q1 20.3
Q2 1.50
Q4 77.9
Q3 0.31
Q1 44.4
Q2 19.9
Q4 35.4
Q3 0.31
plus target cells
NFKB
NFAT
E
Specific lysis [%]
30
25
20
15
10
5
0
Lysis 4h
Lysis 20h
+
−
+
−
10 nM AP20187
S(G32A)-8ser-ICOS -FKBP(36V)-3z
S(G32A)-8ser-OX40 -FKBP(36V)-3z

Fig. 13

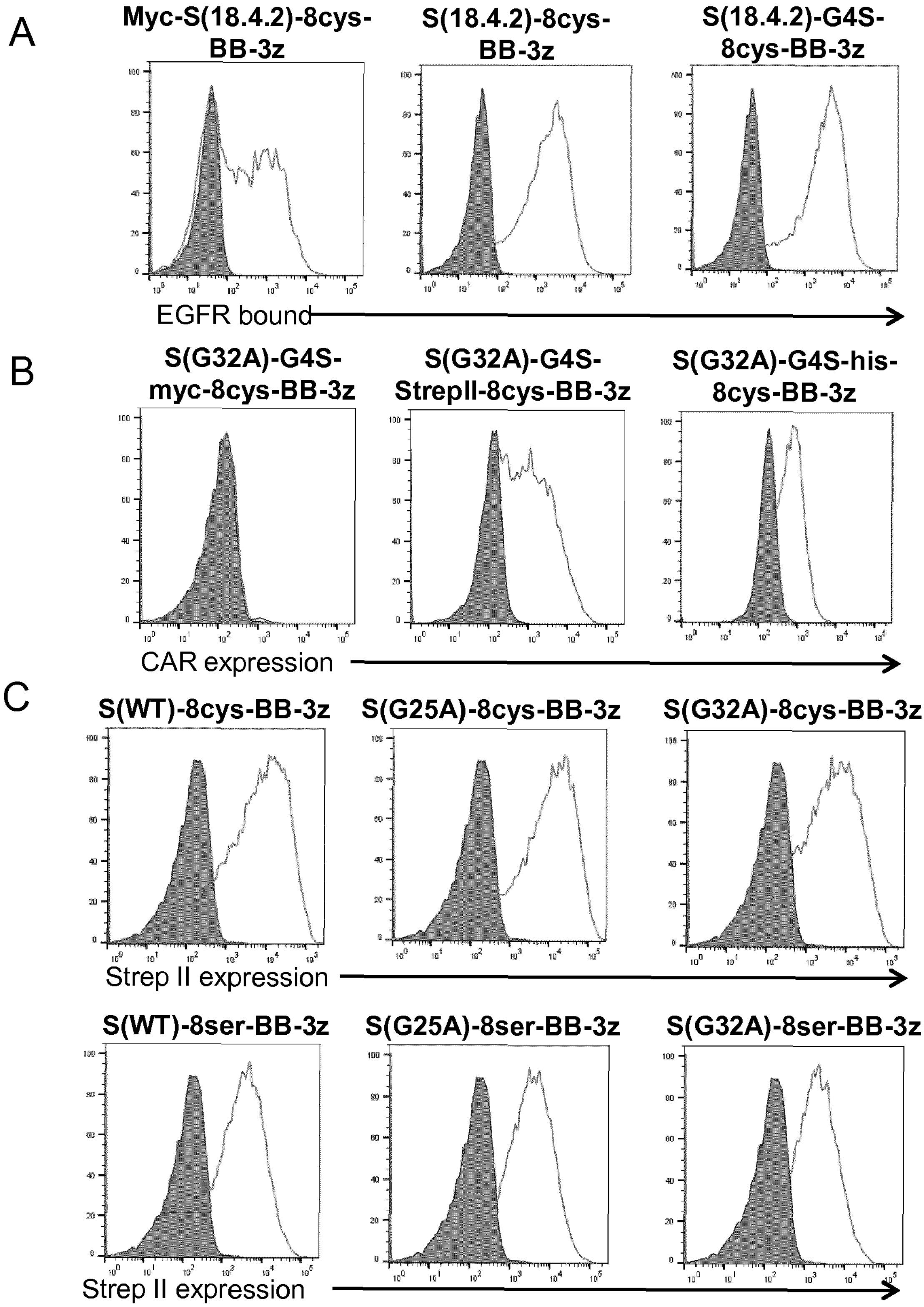
A
Myc-S(18.4.2)-8cys-BB-3z
S(18.4.2)-8cys-BB-3z
S(18.4.2)-G4S-8cys-BB-3z
EGFR bound
B
S(G32A)-G4S-myc-8cys-BB-3z
S(G32A)-G4S-StrepII-8cys-BB-3z
S(G32A)-G4S-his-8cys-BB-3z
CAR expression
C
S(WT)-8cys-BB-3z
S(G25A)-8cys-BB-3z
S(G32A)-8cys-BB-3z
Strep II expression
S(WT)-8ser-BB-3z
S(G25A)-8ser-BB-3z
S(G32A)-8ser-BB-3z
Strep II expression

Fig. 13
D

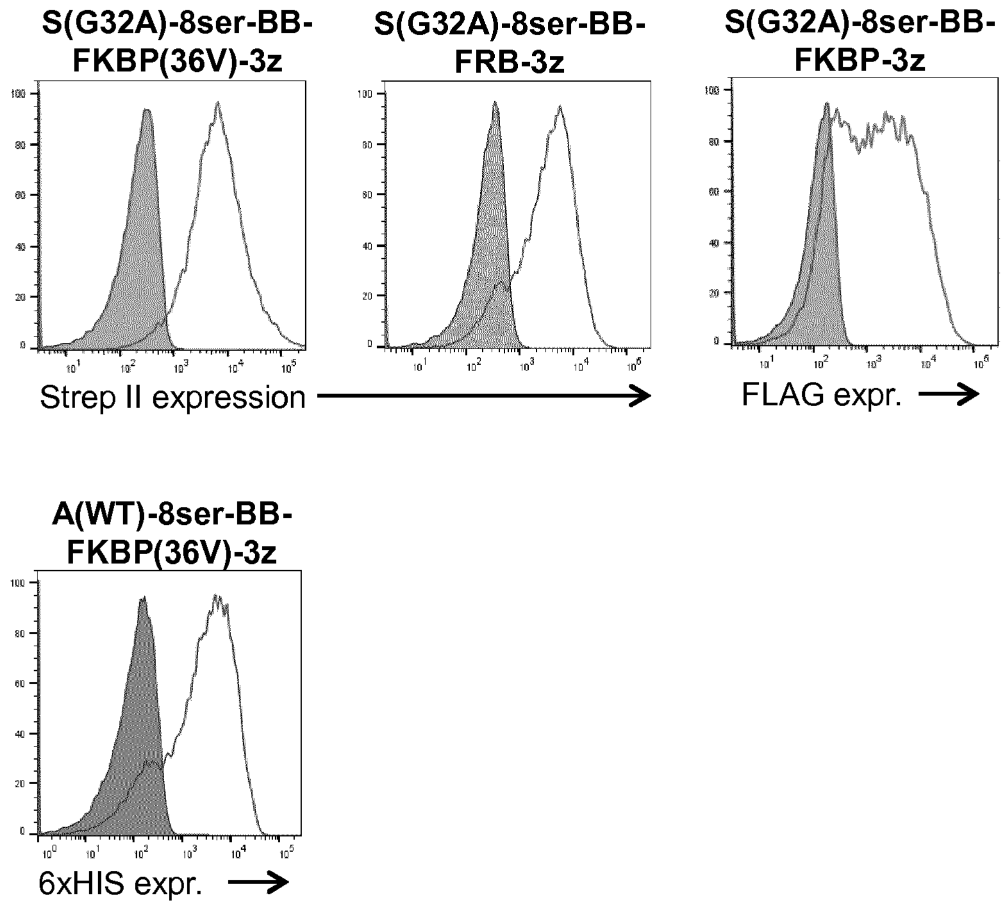
S(G32A)-8ser-BB-FKBP(36V)-3z
S(G32A)-8ser-BB-FRB-3z
S(G32A)-8ser-BB-FKBP-3z
Strep II expression
FLAG expr.
A(WT)-8ser-BB-FKBP(36V)-3z
6xHIS expr.

Fig. 14
A

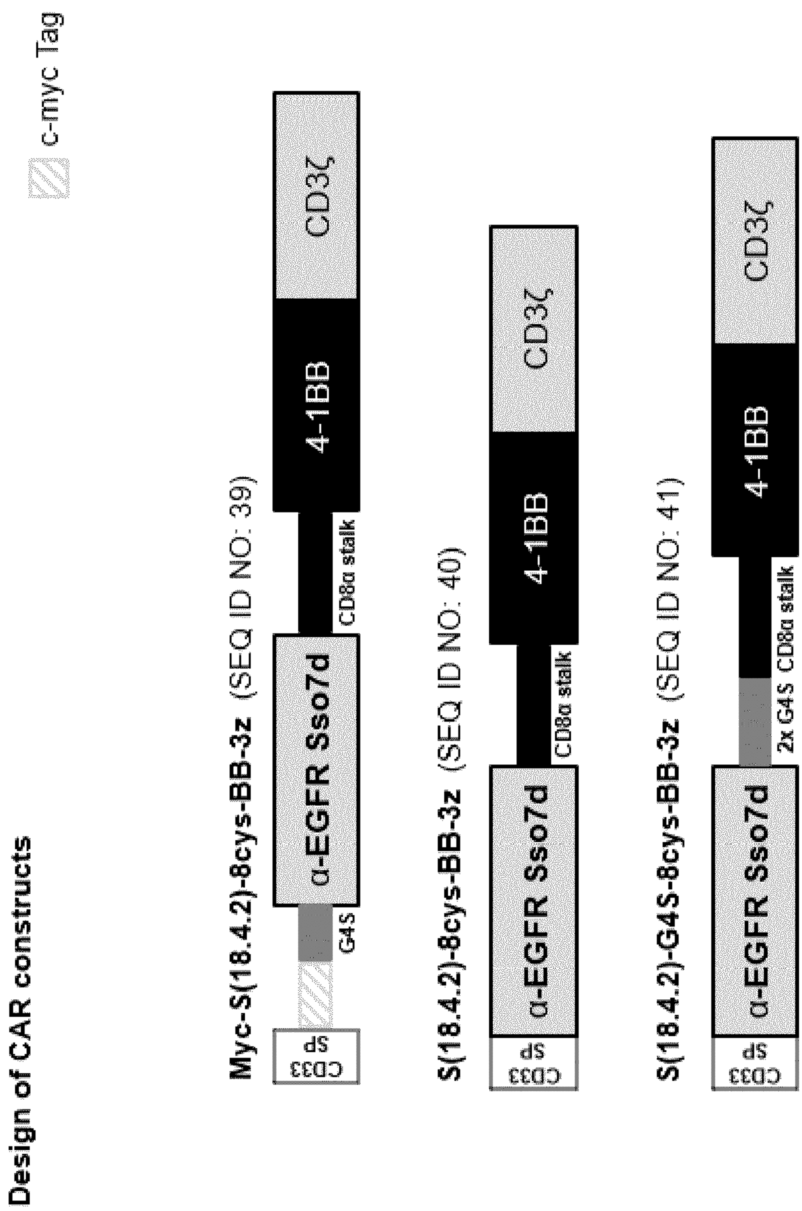
Design of CAR constructs
c-myc Tag
Myc-S(18.4.2)-8cys-BB-3z (SEQ ID NO: 39)
CD33 SP
G4S
α-EGFR Sso7d
CD8α stalk
4-1BB
CD3ζ
S(18.4.2)-8cys-BB-3z (SEQ ID NO: 40)
CD33 SP
α-EGFR Sso7d
CD8α stalk
4-1BB
CD3ζ
S(18.4.2)-G4S-8cys-BB-3z (SEQ ID NO: 41)
CD33 SP
α-EGFR Sso7d
2x G4S
CD8α stalk
4-1BB
CD3ζ

Fig. 14
B

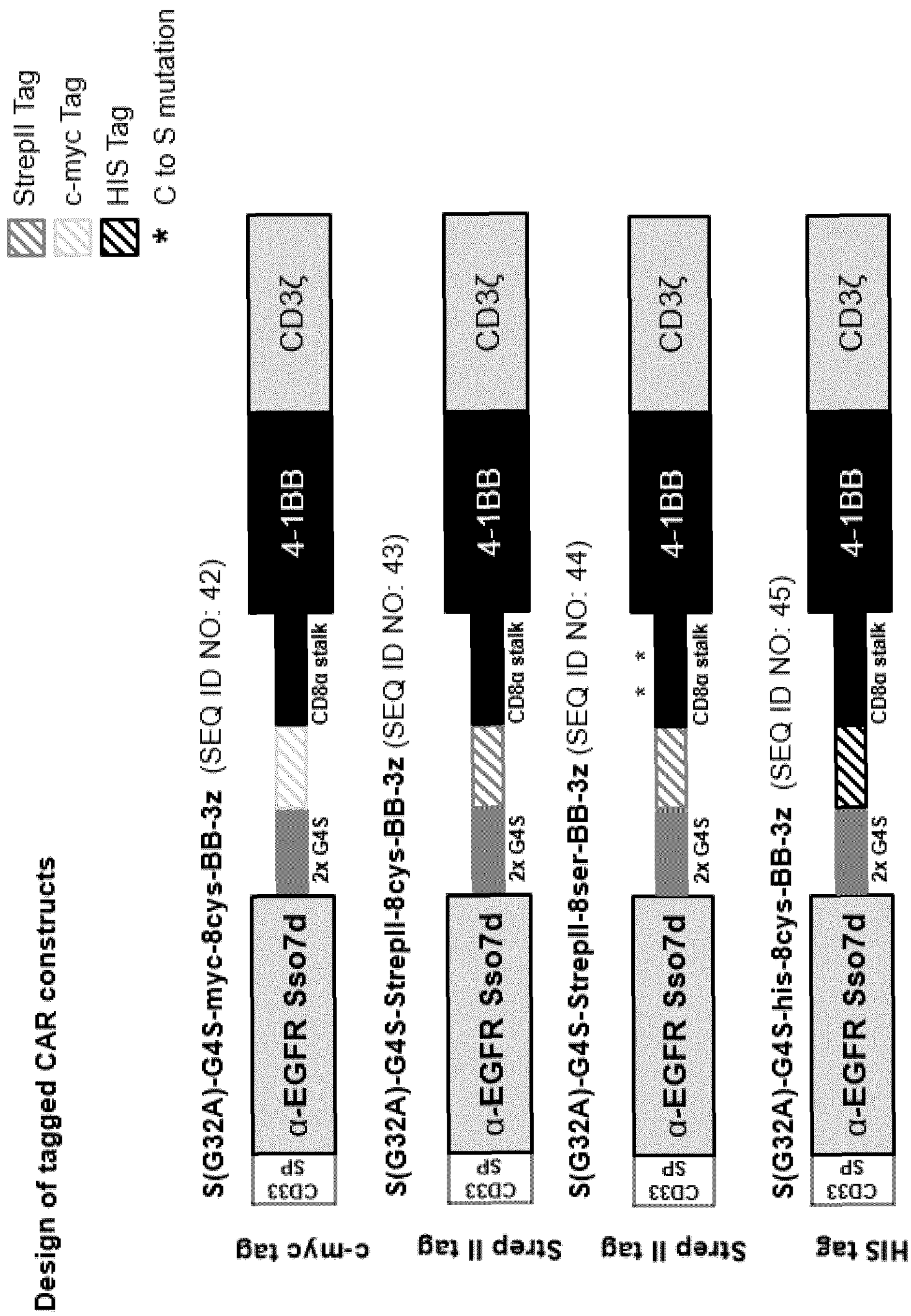
Design of tagged CAR constructs
StrepII Tag
c-myc Tag
HIS Tag
* C to S mutation
c-myc tag
S(G32A)-G4S-myc-8cys-BB-3z (SEQ ID NO: 42)
Strep II tag
S(G32A)-G4S-StrepII-8cys-BB-3z (SEQ ID NO: 43)
Strep II tag
S(G32A)-G4S-StrepII-8ser-BB-3z (SEQ ID NO: 44)
HIS tag
S(G32A)-G4S-his-8cys-BB-3z (SEQ ID NO: 45)
CD33 SP
α-EGFR Sso7d
2x G4S
CD8α stalk
4-1BB
CD3ζ

Fig. 14
C

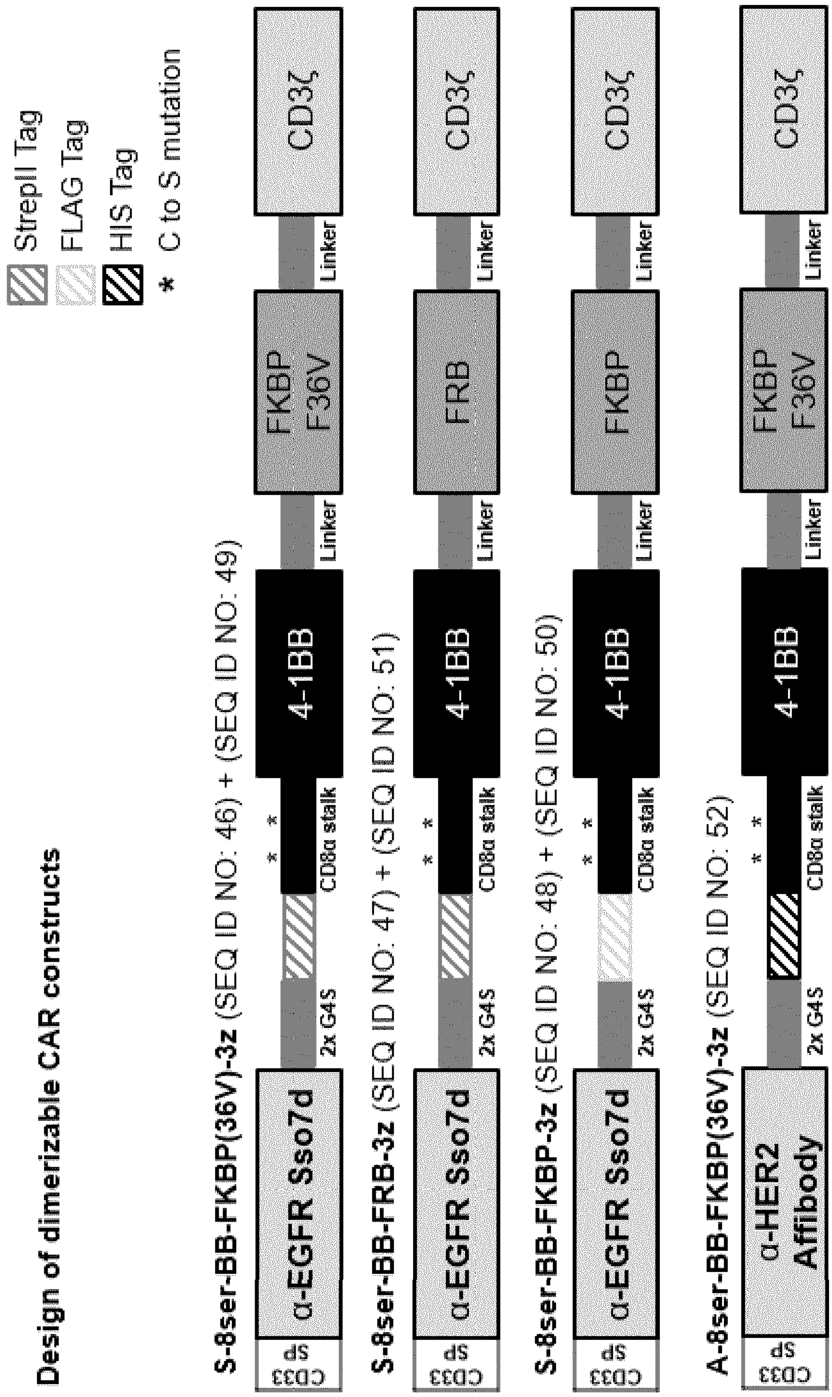
Design of dimerizable CAR constructs
StrepII Tag
FLAG Tag
HIS Tag
* C to S mutation
S-8ser-BB-FKBP(36V)-3z (SEQ ID NO: 46) + (SEQ ID NO: 49)
CD33 SP
α-EGFR Sso7d
2x G4S
CD8α stalk
4-1BB
Linker
FKBP F36V
Linker
CD3ζ
S-8ser-BB-FRB-3z (SEQ ID NO: 47) + (SEQ ID NO: 51)
CD33 SP
α-EGFR Sso7d
2x G4S
CD8α stalk
4-1BB
Linker
FRB
Linker
CD3ζ
S-8ser-BB-FKBP-3z (SEQ ID NO: 48) + (SEQ ID NO: 50)
CD33 SP
α-EGFR Sso7d
2x G4S
CD8α stalk
4-1BB
Linker
FKBP
Linker
CD3ζ
A-8ser-BB-FKBP(36V)-3z (SEQ ID NO: 52)
CD33 SP
α-HER2 Affibody
2x G4S
CD8α stalk
4-1BB
Linker
FKBP F36V
Linker
CD3ζ

Fig. 14
D

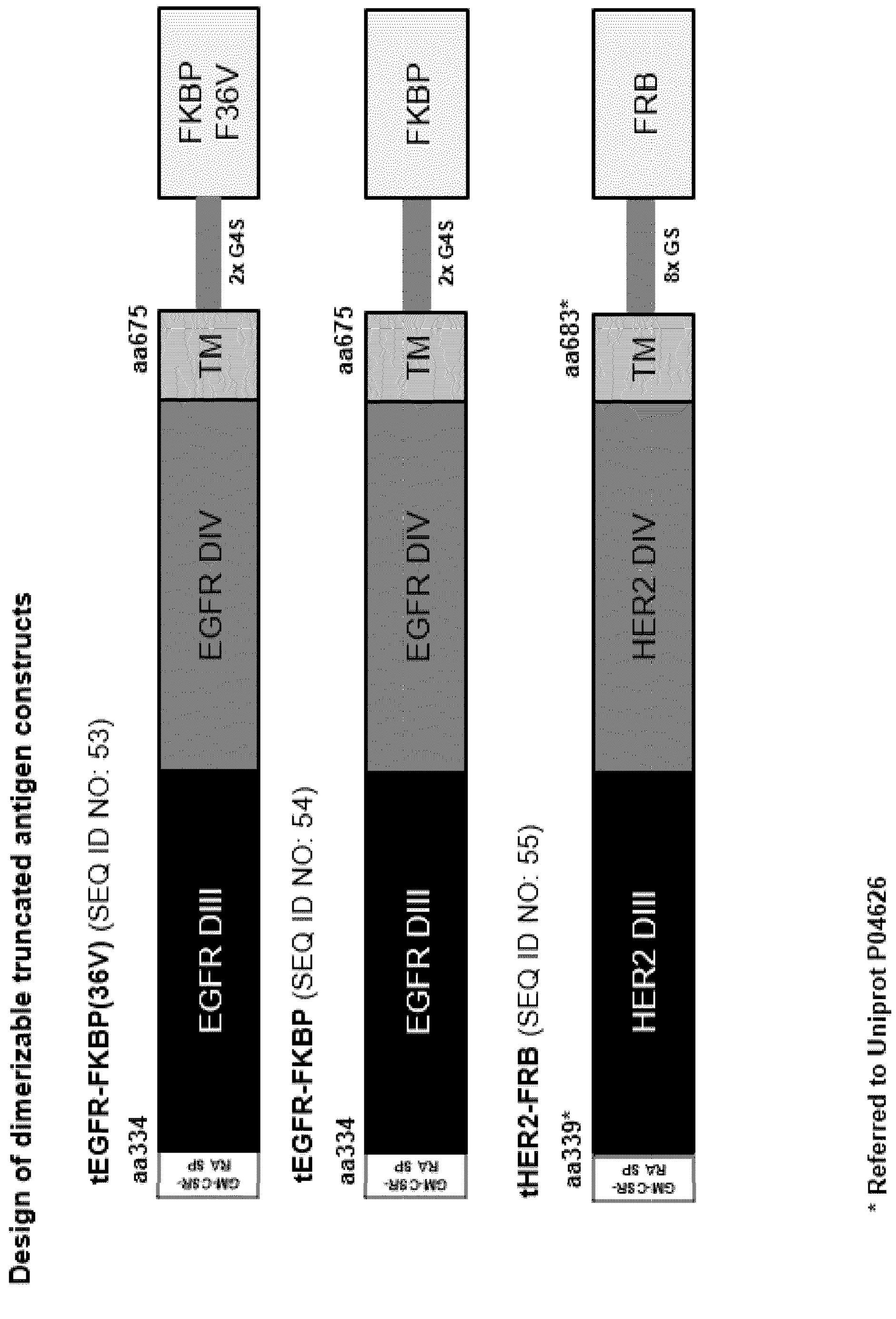
Design of dimerizable truncated antigen constructs
tEGFR-FKBP(36V) (SEQ ID NO: 53)
aa334
aa675
GM-CSR-RA SP
EGFR DIII
EGFR DIV
TM
2x G4S
FKBP F36V
tEGFR-FKBP (SEQ ID NO: 54)
aa334
aa675
GM-CSR-RA SP
EGFR DIII
EGFR DIV
TM
2x G4S
FKBP
tHER2-FRB (SEQ ID NO: 55)
aa339*
aa683*
GM-CSR-RA SP
HER2 DIII
HER2 DIV
TM
8x GS
FRB
* Referred to Uniprot P04626

Fig. 14
E

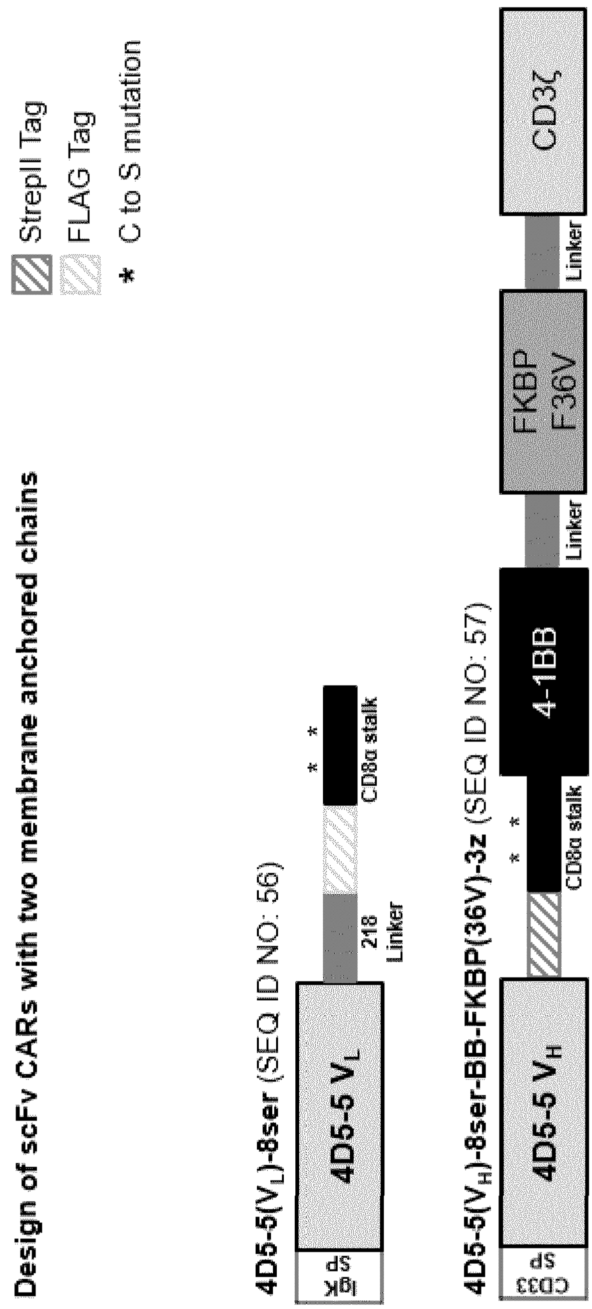
Design of scFv CARs with two membrane anchored chains
StrepII Tag
FLAG Tag
* C to S mutation
4D5-5(V_L)-8ser (SEQ ID NO: 56)
IgK SP
4D5-5 V_L
218 Linker
CD8α stalk
4D5-5(V_H)-8ser-BB-FKBP(36V)-3z (SEQ ID NO: 57)
CD33 SP
4D5-5 V_H
CD8α stalk
4-1BB
Linker
FKBP F36V
Linker
CD3ζ

Fig. 14
F

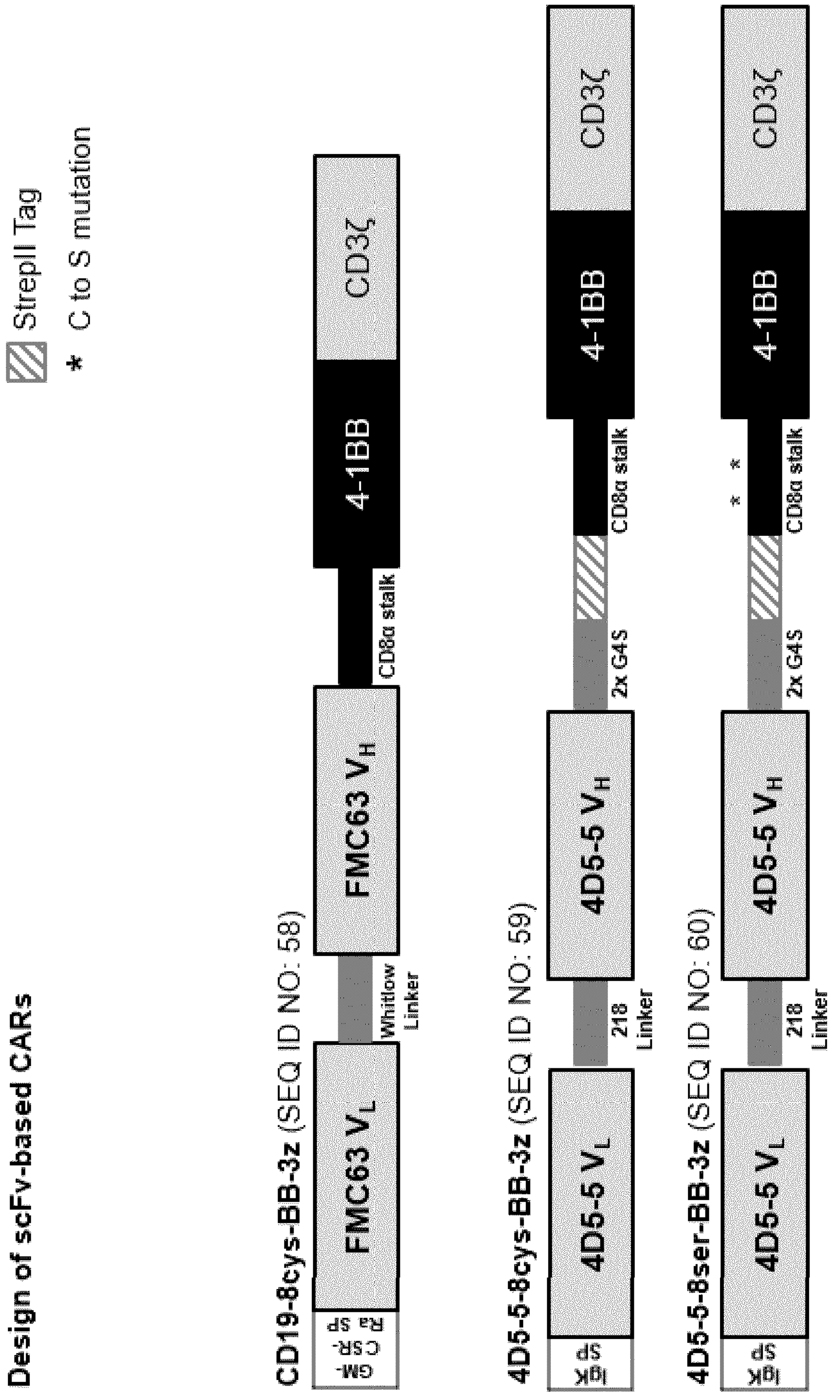
Design of scFv-based CARs
StrepII Tag
* C to S mutation
CD19-8cys-BB-3z (SEQ ID NO: 58)
GM-CSR-Ra SP
FMC63 V$_L$
Whitlow Linker
FMC63 V$_H$
CD8α stalk
4-1BB
CD3ζ
4D5-5-8cys-BB-3z (SEQ ID NO: 59)
IgK SP
4D5-5 V$_L$
218 Linker
4D5-5 V$_H$
2x G4S
CD8α stalk
4-1BB
CD3ζ
4D5-5-8ser-BB-3z (SEQ ID NO: 60)
IgK SP
4D5-5 V$_L$
218 Linker
4D5-5 V$_H$
2x G4S
* *
CD8α stalk
4-1BB
CD3ζ

Fig. 14
G

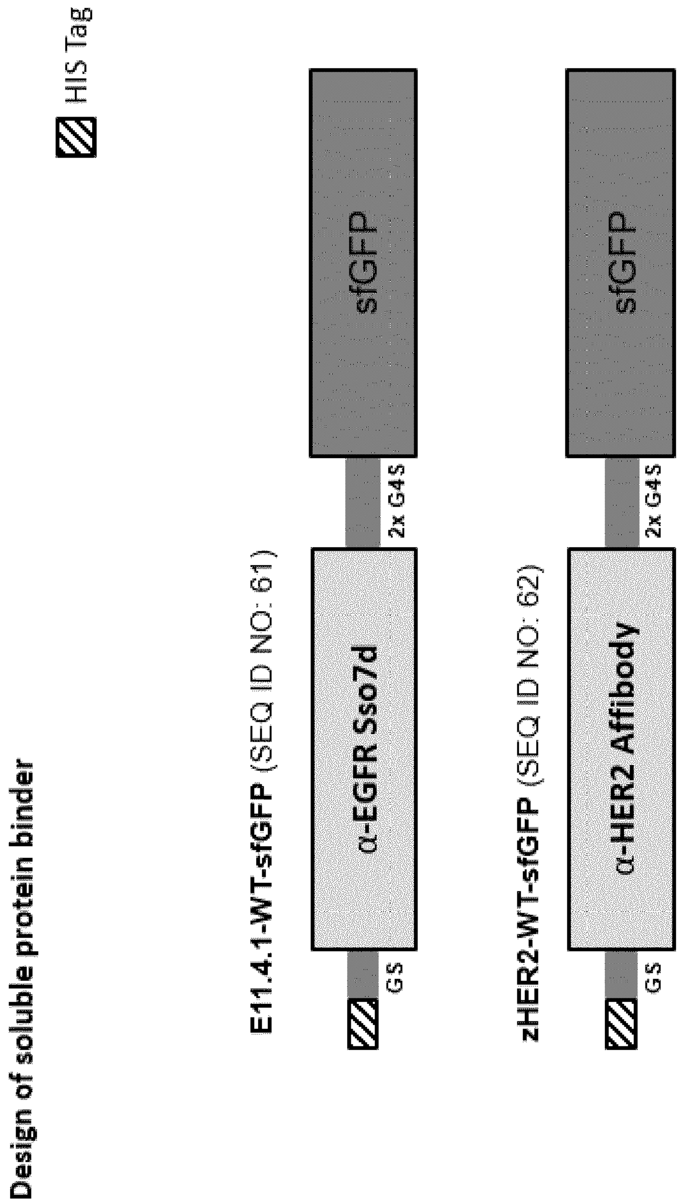
Design of soluble protein binder
HIS Tag
E11.4.1-WT-sfGFP (SEQ ID NO: 61)
GS
α-EGFR Sso7d
2x G4S
sfGFP
zHER2-WT-sfGFP (SEQ ID NO: 62)
GS
α-HER2 Affibody
2x G4S
sfGFP

Fig. 14
H

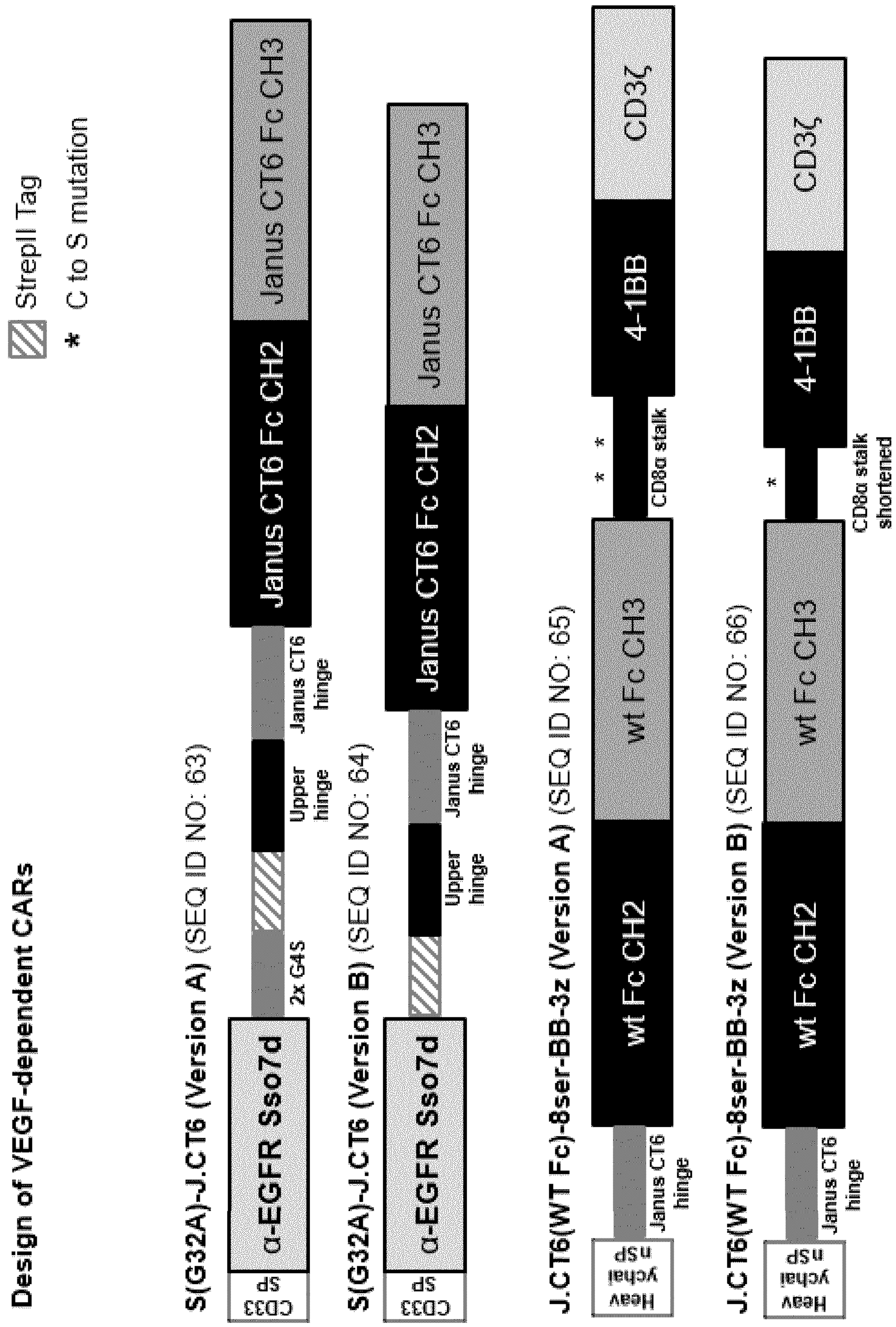
Design of VEGF-dependent CARs
StrepII Tag
* C to S mutation
S(G32A)-J.CT6 (Version A) (SEQ ID NO: 63)
CD33 SP
α-EGFR Sso7d
2x G4S
Upper hinge
Janus CT6 hinge
Janus CT6 Fc CH2
Janus CT6 Fc CH3
S(G32A)-J.CT6 (Version B) (SEQ ID NO: 64)
CD33 SP
α-EGFR Sso7d
Upper hinge
Janus CT6 hinge
Janus CT6 Fc CH2
Janus CT6 Fc CH3
J.CT6(WT Fc)-8ser-BB-3z (Version A) (SEQ ID NO: 65)
Heavychain SP
Janus CT6 hinge
wt Fc CH2
wt Fc CH3
CD8α stalk
4-1BB
CD3ζ
J.CT6(WT Fc)-8ser-BB-3z (Version B) (SEQ ID NO: 66)
Heavychain SP
Janus CT6 hinge
wt Fc CH2
wt Fc CH3
CD8α stalk shortened
4-1BB
CD3ζ

Fig. 14
I

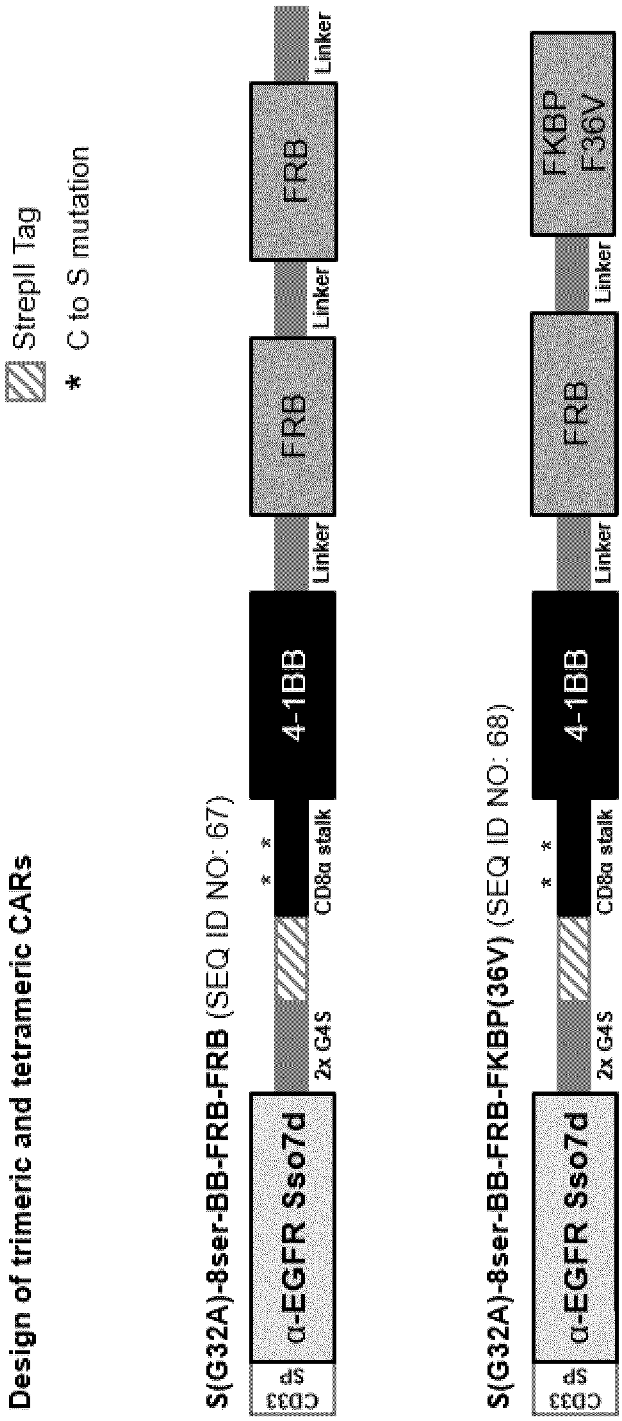
Design of trimeric and tetrameric CARs
StrepII Tag
* C to S mutation
S(G32A)-8ser-BB-FRB-FRB (SEQ ID NO: 67)
CD33 SP
α-EGFR Sso7d
2x G4S
CD8α stalk
4-1BB
Linker
FRB
Linker
FRB
Linker
S(G32A)-8ser-BB-FRB-FKBP(36V) (SEQ ID NO: 68)
CD33 SP
α-EGFR Sso7d
2x G4S
CD8α stalk
4-1BB
Linker
FRB
Linker
FKBP F36V

Fig. 14 J

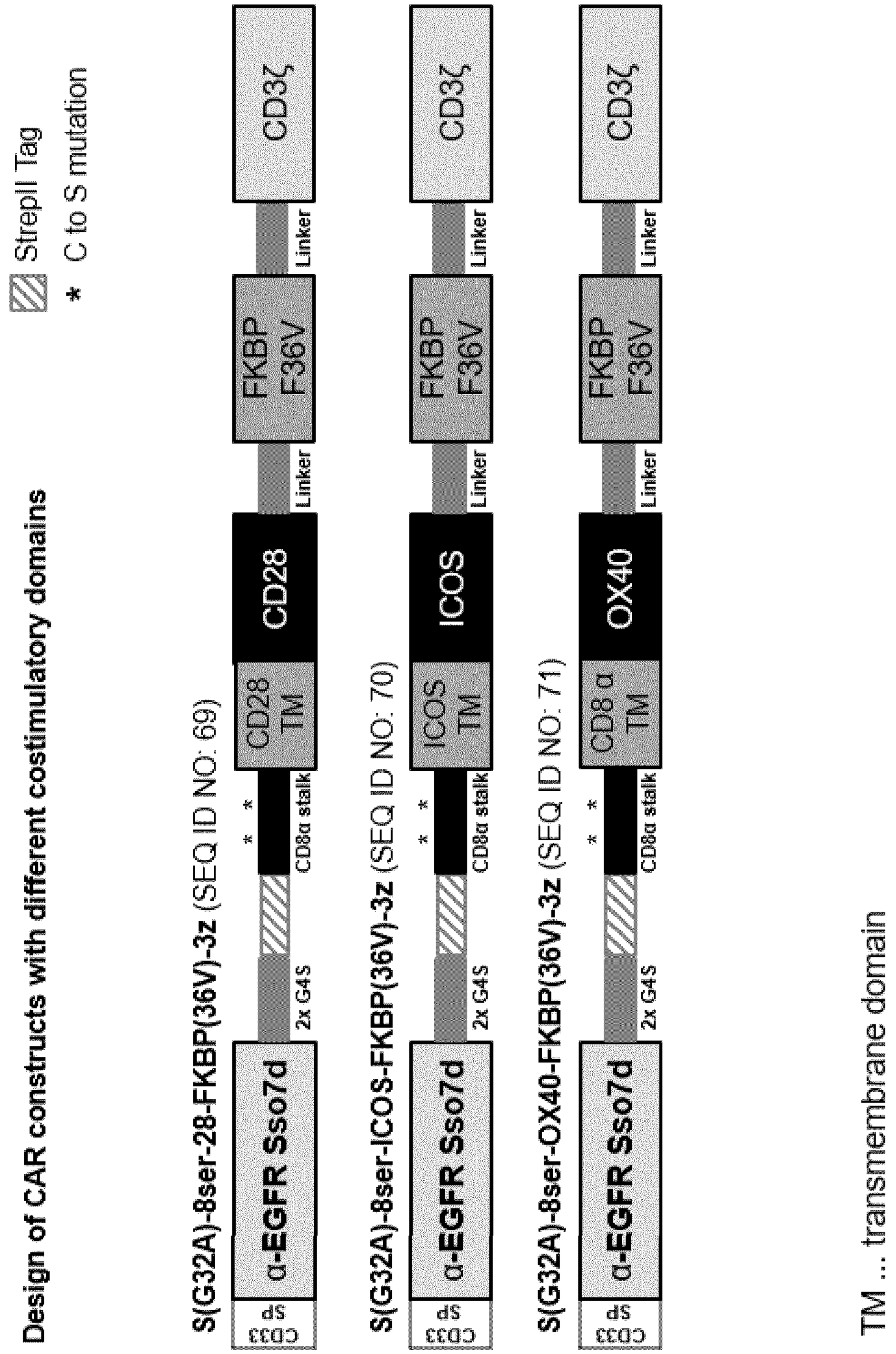
StrepII Tag
* C to S mutation
Design of CAR constructs with different costimulatory domains
S(G32A)-8ser-28-FKBP(36V)-3z (SEQ ID NO: 69)
CD33 SP
α-EGFR Sso7d
2x G4S
* *
CD8α stalk
CD28 TM
CD28
Linker
FKBP F36V
Linker
CD3ζ
S(G32A)-8ser-ICOS-FKBP(36V)-3z (SEQ ID NO: 70)
CD33 SP
α-EGFR Sso7d
2x G4S
* *
CD8α stalk
ICOS TM
ICOS
Linker
FKBP F36V
Linker
CD3ζ
S(G32A)-8ser-OX40-FKBP(36V)-3z (SEQ ID NO: 71)
CD33 SP
α-EGFR Sso7d
2x G4S
* *
CD8α stalk
CD8 α TM
OX40
Linker
FKBP F36V
Linker
CD3ζ
TM ... transmembrane domain

Fig. 15
A

**Flexible linker**

SEQ ID NO: 1

GGSG

SEQ ID NO: 2

GGSGG

SEQ ID NO: 3

GSGSG

SEQ ID NO: 4

GSGGG

SEQ ID NO: 5

GGGSG

SEQ ID NO: 6

GSSSG

**Epitope tags**

SEQ ID NO: 7

YPYDVPDYA

SEQ ID NO: 8

DYKDDDDK

SEQ ID NO: 9

EQKLISEEDL

**Homodimerization domain**

SEQ ID NO: 10

MASRGVQVETISPGDGRTFPKRGQTCVVHYTGMLEDGKKVDSSRDRNKPFKFMLGKQEVI
RGWEEGVAQMSVGQRAKLTISPDYAYGATGHPGIIPPHATLVFDVELLKLE

# Fig. 15
B

**Co-regulator peptides**

SEQ ID NO: 11

DAFQLRQLILRGLQDD

SEQ ID NO: 12

SPGSREWFKDMLS

SEQ ID NO: 13

PRQGSILYSMLTSAKQT

SEQ ID NO: 14

PKKENNALLRYLLDRDDPSDV

SEQ ID NO: 15

SSKGVLWRMLAEPVSR

SEQ ID NO: 16

SRTLQLDWGTLYWSR

SEQ ID NO: 17

SSNHQSSRLIELLSR

SEQ ID NO: 18

RLTKTNPILYYMLQKGGNSVA

SEQ ID NO: 19

NLLERRTVLQLLLGNPTKGRV

**Single domain protein binders**

rcSso7d (SEQ ID NO: 20)

ATVKFTYQGEEKQVDISKIKWVIRWGQHIAFKYDEGGGAAGYGWVSEKDAPKELLQMLEKQ

Fig. 15
C

E18.4.2-WT (SEQ ID NO: 21)
AAVKLTYQGEEKQVDISKIKYVDRAGQFIWFEYDEGGGALGTGWVSEKDAPKELLQMLEKQ

E11.4.1-WT (SEQ ID NO: 22)
ATVKFTYQGEEKQVDISKIMYVIRGGQRIAFGYDEGDGAWGDGIVSEKDAPKELLQMLEKQ

E11.4.1-G25A (SEQ ID NO: 23)
ATVKFTYQGEEKQVDISKIMYVIRAGQRIAFGYDEGDGAWGDGIVSEKDAPKELLQMLEKQ

E11.4.1-R28A (SEQ ID NO: 24)
ATVKFTYQGEEKQVDISKIMYVIRGGQAIAFGYDEGDGAWGDGIVSEKDAPKELLQMLEKQ

E11.4.1-G32A (SEQ ID NO: 25)
ATVKFTYQGEEKQVDISKIMYVIRGGQRIAFAYDEGDGAWGDGIVSEKDAPKELLQMLEKQ

Affibody zHER2-WT (SEQ ID NO: 26)
VDNKFNKELRQAYWEIQALPNLAWTQSRAFIRKLYDDPSQSANLLAEAKKLNDAQAPK

zHER2-L9A (SEQ ID NO: 27)
VDNKFNKEARQAYWEIQALPNLAWTQSRAFIRKLYDDPSQSANLLAEAKKLNDAQAPK

zHER2-R10A (SEQ ID NO: 28)
VDNKFNKELAQAYWEIQALPNLAWTQSRAFIRKLYDDPSQSANLLAEAKKLNDAQAPK

zHER2-Q11A (SEQ ID NO: 29)
VDNKFNKELRAAYWEIQALPNLAWTQSRAFIRKLYDDPSQSANLLAEAKKLNDAQAPK

zHER2-Y13A (SEQ ID NO: 30)
VDNKFNKELRQAAWEIQALPNLAWTQSRAFIRKLYDDPSQSANLLAEAKKLNDAQAPK

zHER2-W14A (SEQ ID NO: 31)
VDNKFNKELRQAYAEIQALPNLAWTQSRAFIRKLYDDPSQSANLLAEAKKLNDAQAPK

Fig. 15
D

zHER2-Q17A (SEQ ID NO: 32)
VDNKFNKELRQAYWEIAALPNLAWTQSRAFIRKLYDDPSQSANLLAEAKKLNDAQAPK

zHER2-W24A (SEQ ID NO: 33)
VDNKFNKELRQAYWEIQALPNLAATQSRAFIRKLYDDPSQSANLLAEAKKLNDAQAPK

zHER2-T25A (SEQ ID NO: 34)
VDNKFNKELRQAYWEIQALPNLAWAQSRAFIRKLYDDPSQSANLLAEAKKLNDAQAPK

zHER2-S27A (SEQ ID NO: 35)
VDNKFNKELRQAYWEIQALPNLAWTQARAFIRKLYDDPSQSANLLAEAKKLNDAQAPK

zHER2-R28A (SEQ ID NO: 36)
VDNKFNKELRQAYWEIQALPNLAWTQSAAFIRKLYDDPSQSANLLAEAKKLNDAQAPK

zHER2-R32A (SEQ ID NO: 37)
VDNKFNKELRQAYWEIQALPNLAWTQSRAFIAKLYDDPSQSANLLAEAKKLNDAQAPK

zHER2-Y35A (SEQ ID NO: 38)
VDNKFNKELRQAYWEIQALPNLAWTQSRAFIRKLADDPSQSANLLAEAKKLNDAQAPK

# Fig. 15 E

Underlined text = signal peptide

**Underlined (bold) text** = rcSso7d or Affibody

*Italic text* = truncated form of EGFR or HER2

**Underlined (bold) text** = $V_H$ or $V_L$ of scFvs

Underlined text = StrepII Tag

*Underlined (italic) text* = FLAG Tag

Underlined (dotted) text = HIS Tag

**Underlined (dotted and bold) test** = c-myc Tag

Dark grey text = flexible linker

**Bold text** = CD8 stalk and/or transmembrane domain

Underlined (dotted) text = 4-1BB domain

Underlined (double) text = CD3 zeta signaling domain

*Underlined (double) (italic) text* = dimerization domain

***Bold and italic text*** = hinge region

*Underlined (italic) text* = Fc domain

*Underlined (dotted) (italic) text* = sfGFP reporter gene

**Underlined (double) (bold) text** = CD28/ICOS/OX40 transmembrane and/or costimulatory domain

**Sso7d-based CAR constructs**

Myc-S(18.4.2)-8cys-BB-3z (SEQ ID NO: 39)

MPLLLLLPLLWAGALAMEQKLISEEDLGGGGS**AAVKLTYQGEEKQVDISKIKYVDRAGQFIWFEYDEGGGALGTGWVSEKDAPKELLQMLEKQTTTPAPRPPTPAPTIASQPLSLRPEACRPAAGGAVHTRGLDFACDIYIWAPLAGTCGVLLLSLVITLYC**KRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR*

S(18.4.2)-8cys-BB-3z (SEQ ID NO: 40)

MPLLLLLPLLWAGALAM**AAVKLTYQGEEKQVDISKIKYVDRAGQFIWFEYDEGGGALGTGWVSEKDAPKELLQMLEKQTTTPAPRPPTPAPTIASQPLSLRPEACRPAAGGAVHTRGLDFACDIYIWAPLAGTCGVLLLSLVITLYC**KRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR*

S(18.4.2)-G4S-8cys-BB-3z (SEQ ID NO: 41)

MPLLLLLPLLWAGALAM**AAVKLTYQGEEKQVDISKIKYVDRAGQFIWFEYDEGGGALGTGWVSEKDAPKELLQMLEKQ**GGGGSGGGGS**TTTPAPRPPTPAPTIASQPLSLRPEACRPAAGGAVHTRGLDFACDIYIWAPLAGTCGVLLLSLVITLYC**KRGRKKLLYIFKQPFMRPVQTTQEEDGCSCRFPEEEEGGCELRVKFSRSADAPAYKQGQNQLYNELNLGRREEYDVLDKRRGR

# Fig. 15 F

DPEMGGKPRRKNPQEGLYNELQKDKMAEAYSEIGMKGERRRGKGHDGLYQGLSTATKDT
YDALHMQALPPR*

S(G32A)-G4S-myc-8cys-BB-3z (SEQ ID NO: 42)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSEQKLISEEDL**TTTPAPRPPTPAPTIASQPLSLRP
EACRPAAGGAVHTRGLDFACDIYIWAPLAGTCGVLLLSLVITLYC**KRGRKKLLYIFKQPFMR
PVQTTQEEDGCSCRFPEEEEGGCELRVKFSRSADAPAYKQGQNQLYNELNLGRREEYDVL
DKRRGRDPEMGGKPRRKNPQEGLYNELQKDKMAEAYSEIGMKGERRRGKGHDGLYQGL
STATKDTYDALHMQALPPR*

S(G32A)-G4S-StrepII-8cys-BB-3z (= S(G32A)-8cys-BB-3z) (SEQ ID NO: 43)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSNWSHPQFEK**TTTPAPRPPTPAPTIASQPLSLR
PEACRPAAGGAVHTRGLDFACDIYIWAPLAGTCGVLLLSLVITLYC**KRGRKKLLYIFKQPFM
RPVQTTQEEDGCSCRFPEEEEGGCELRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

S(G32A)-G4S-StrepII-8ser-BB-3z (= S(G32A)-8ser-BB-3z) (SEQ ID NO: 44)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSNWSHPQFEK**TTTPAPRPPTPAPTIASQPLSLR
PEASRPAAGGAVHTRGLDFASDIYIWAPLAGTCGVLLLSLVITLYC**KRGRKKLLYIFKQPFM
RPVQTTQEEDGCSCRFPEEEEGGCELRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

S(G32A)-G4S-his-8cys-BB-3z (SEQ ID NO: 45)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSHHHHHH**TTTPAPRPPTPAPTIASQPLSLRPEA
CRPAAGGAVHTRGLDFACDIYIWAPLAGTCGVLLLSLVITLYC**KRGRKKLLYIFKQPFMRPV
QTTQEEDGCSCRFPEEEEGGCELRVKFSRSADAPAYKQGQNQLYNELNLGRREEYDVLDK
RRGRDPEMGGKPRRKNPQEGLYNELQKDKMAEAYSEIGMKGERRRGKGHDGLYQGLSTA
TKDTYDALHMQALPPR*

**Dimerizable CAR constructs**

S(G32A)-8ser-BB-FKBP(36V)-3z (SEQ ID NO: 46)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSNWSHPQFEK**TTTPAPRPPTPAPTIASQPLSLR
PEASRPAAGGAVHTRGLDFASDIYIWAPLAGTCGVLLLSLVITLYC**KRGRKKLLYIFKQPFM
RPVQTTQEEDGCSCRFPEEEEGGCELSRGSGSGSGS*MGVQVETISPGDGRTFPKRGQTC
VVHYTGMLEDGKKVDSSRDRNKPFKFMLGKQEVIRGWEEGVAQMSVGQRAKLTISPDYAY
GATGHPGIIPPHATLVFDVELLKLE*GSGSGSGSSLRVKFSRSADAPAYKQGQNQLYNELNL
GRREEYDVLDKRRGRDPEMGGKPRRKNPQEGLYNELQKDKMAEAYSEIGMKGERRRGKG
HDGLYQGLSTATKDTYDALHMQALPPR*

S(G32A)-8ser-BB-FRB-3z (SEQ ID NO: 47)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSNWSHPQFEK**TTTPAPRPPTPAPTIASQPLSLR**

Fig. 15
G

**PEASRPAAGGAVHTRGLDFASDIYIWAPLAGTCGVLLLSLVITLYC**KRGRKKLLYIFKQPFM
RPVQTTQEEDGCSCRFPEEEEGGCELGSGSGSGSGSGSGSGS*ILWHEMWHEGLEEASRL
YFGERNVKGMFEVLEPLHAMMERGPQTLKETSFNQAYGRDLMEAQEWCRKYMKSGNVKD
LLQAWDLYYHVFRRISK*GSGSGSGSSLRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

S(G32A)-8ser-BB-FKBP-3z (SEQ ID NO: 48)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGS*DYKDDDDK***TTTPAPRPPTPAPTIASQPLSLRP
EASRPAAGGAVHTRGLDFASDIYIWAPLAGTCGVLLLSLVITLYC**KRGRKKLLYIFKQPFMR
PVQTTQEEDGCSCRFPEEEEGGCELSRGSGSGSGS*MGVQVETISPGDGRTFPKRGQTCV
VHYTGMLEDGKKFDSSRDRNKPFKFMLGKQEVIRGWEEGVAQMSVGQRAKLTISPDYAYG
ATGHPGIIPPHATLVFDVELLKLE*GSGSGSGSSLRVKFSRSADAPAYKQGQNQLYNELNLGR
REEYDVLDKRRGRDPEMGGKPRRKNPQEGLYNELQKDKMAEAYSEIGMKGERRRGKGHD
GLYQGLSTATKDTYDALHMQALPPR*

S(WT)-8ser-BB-FKBP(36V)-3z (SEQ ID NO: 49)

MPLLLLLPLLWAGALAM**ATVKFTYQGEEKQVDISKIMYVIRGGQRIAFGYDEGDGAWGDGI
VSEKDAPKELLQMLEKQ**GGGGSGGGGSNWSHPQFEK**TTTPAPRPPTPAPTIASQPLSLR
PEASRPAAGGAVHTRGLDFASDIYIWAPLAGTCGVLLLSLVITLYC**KRGRKKLLYIFKQPFM
RPVQTTQEEDGCSCRFPEEEEGGCELSRGSGSGSGS*MGVQVETISPGDGRTFPKRGQTC
VVHYTGMLEDGKKVDSSRDRNKPFKFMLGKQEVIRGWEEGVAQMSVGQRAKLTISPDYAY
GATGHPGIIPPHATLVFDVELLKLE*GSGSGSGSSLRVKFSRSADAPAYKQGQNQLYNELNL
GRREEYDVLDKRRGRDPEMGGKPRRKNPQEGLYNELQKDKMAEAYSEIGMKGERRRGKG
HDGLYQGLSTATKDTYDALHMQALPPR*

S(WT)-8ser-BB-FKBP-3z (SEQ ID NO: 50)

MPLLLLLPLLWAGALAM**ATVKFTYQGEEKQVDISKIMYVIRGGQRIAFGYDEGDGAWGDGI
VSEKDAPKELLQMLEKQ**GGGGSGGGGS*DYKDDDDK***TTTPAPRPPTPAPTIASQPLSLRP
EASRPAAGGAVHTRGLDFASDIYIWAPLAGTCGVLLLSLVITLYC**KRGRKKLLYIFKQPFMR
PVQTTQEEDGCSCRFPEEEEGGCELSRGSGSGSGS*MGVQVETISPGDGRTFPKRGQTCV
VHYTGMLEDGKKFDSSRDRNKPFKFMLGKQEVIRGWEEGVAQMSVGQRAKLTISPDYAYG
ATGHPGIIPPHATLVFDVELLKLE*GSGSGSGSSLRVKFSRSADAPAYKQGQNQLYNELNLGR
REEYDVLDKRRGRDPEMGGKPRRKNPQEGLYNELQKDKMAEAYSEIGMKGERRRGKGHD
GLYQGLSTATKDTYDALHMQALPPR*

S(WT)-8ser-BB-FRB-3z (SEQ ID NO: 51)

MPLLLLLPLLWAGALAM**ATVKFTYQGEEKQVDISKIMYVIRGGQRIAFGYDEGDGAWGDGI
VSEKDAPKELLQMLEKQ**GGGGSGGGGSNWSHPQFEK**TTTPAPRPPTPAPTIASQPLSLR
PEASRPAAGGAVHTRGLDFASDIYIWAPLAGTCGVLLLSLVITLYC**KRGRKKLLYIFKQPFM
RPVQTTQEEDGCSCRFPEEEEGGCELGSGSGSGSGSGSGSGS*ILWHEMWHEGLEEASRL
YFGERNVKGMFEVLEPLHAMMERGPQTLKETSFNQAYGRDLMEAQEWCRKYMKSGNVKD
LLQAWDLYYHVFRRISK*GSGSGSGSSLRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

A(WT)-8ser-BB-FKBP(36V)-3z (SEQ ID NO: 52)

MPLLLLLPLLWAGALAM**VDNKFNKELRQAYWEIQALPNLAWTQSRAFIRKLYDDPSQSAN
LLAEAKKLNDAQAPK**GGGGSGGGGSHHHHHH**TTTPAPRPPTPAPTIASQPLSLRPEACR
PAAGGAVHTRGLDFACDIYIWAPLAGTCGVLLLSLVITLYC**KRGRKKLLYIFKQPFMRPVQT

Fig. 15
H

TQEEDGCSCRFPEEEEGGCELSRGSGSGSGSMGVQVETISPGDGRTFPKRGQTCVVHYT
GMLEDGKKVDSSRDRNKPFKFMLGKQEVIRGWEEGVAQMSVGQRAKLTISPDYAYGATGH
PGIIPPHATLVFDVELLKLEGSGSGSGSSLRVKFSRSADAPAYKQGQNQLYNELNLGRREEY
DVLDKRRGRDPEMGGKPRRKNPQEGLYNELQKDKMAEAYSEIGMKGERRRGKGHDGLYQ
GLSTATKDTYDALHMQALPPR*

**Dimerizable antigen constructs**

tEGFR-FKBP(36V) (SEQ ID NO: 53)

MLLLVTSLLLCELPHPAFLLIPRKVCNGIGIGEFKDSLSINATNIKHFKNCTSISGDLHILPVAFR
GDSFTHTPPLDPQELDILKTVKEITGFLLIQAWPENRTDLHAFENLEIIRGRTKQHGQFSLAVV
SLNITSLGLRSLKEISDGDVIISGNKNLCYANTINWKKLFGTSGQKTKIISNRGENSCKATGQV
CHALCSPEGCWGPEPRDCVSCRNVSRGRECVDKCKLLEGEPREFVENSECIQCHPECLPQ
AMNITCTGRGPDNCIQCAHYIDGPHCVKTCPAGVMGENNTLVWKYADAGHVCHLCHPNCT
YGCTGPGLEGCPTNGPKIPSIATGMVGALLLLLVVALGIGLFMRRRHIVRGGGGSGGGGSM
GVQVETISPGDGRTFPKRGQTCVVHYTGMLEDGKKVDSSRDRNKPFKFMLGKQEVIRGWE
EGVAQMSVGQRAKLTISPDYAYGATGHPGIIPPHATLVFDVELLKLE*

tEGFR-FKBP (SEQ ID NO: 54)

MLLLVTSLLLCELPHPAFLLIPRKVCNGIGIGEFKDSLSINATNIKHFKNCTSISGDLHILPVAFR
GDSFTHTPPLDPQELDILKTVKEITGFLLIQAWPENRTDLHAFENLEIIRGRTKQHGQFSLAVV
SLNITSLGLRSLKEISDGDVIISGNKNLCYANTINWKKLFGTSGQKTKIISNRGENSCKATGQV
CHALCSPEGCWGPEPRDCVSCRNVSRGRECVDKCKLLEGEPREFVENSECIQCHPECLPQ
AMNITCTGRGPDNCIQCAHYIDGPHCVKTCPAGVMGENNTLVWKYADAGHVCHLCHPNCT
YGCTGPGLEGCPTNGPKIPSIATGMVGALLLLLVVALGIGLFMRRRHIVRGGGGSGGGGSR
GVQVETISPGDGRTFPKRGQTCVVHYTGMLEDGKKFDSSRDRNKPFKFMLGKQEVIRGWE
EGVAQMSVGQRAKLTISPDYAYGATGHPGIIPPHATLVFDVELLKLE*

tHER2-FRB (SEQ ID NO: 55)

MLLLVTSLLLCELPHPAFLLIPARVCYGLGMEHLREVRAVTSANIQEFAGCKKIFGSLAFLPES
FDGDPASNTAPLQPEQLQVFETLEEITGYLYISAWPDSLPDLSVFQNLQVIRGRILHNGAYSL
TLQGLGISWLGLRSLRELGSGLALIHHNTHLCFVHTVPWDQLFRNPHQALLHTANRPEDEC
VGEGLACHQLCARGHCWGPGPTQCVNCSQFLRGQECVEECRVLQGLPREYVNARHCLPC
HPECQPQNGSVTCFGPEADQCVACAHYKDPPFCVARCPSGVKPDLSYMPIWKFPDEEGA
CQPCPINCTHSCVDLDDKGCPAEQRASPLTSIISAVVGILLVVVLGVVFGILIKRRQQKIRGSG
SGSGSGSGSGSGSILWHEMWHEGLEEASRLYFGERNVKGMFEVLEPLHAMMERGPQTLK
ETSFNQAYGRDLMEAQEWCRKYMKSGNVKDLLQAWDLYYHVFRRISK*

**Split scFv CAR constructs**

4D5-5($V_L$)-8ser (SEQ ID NO: 56)

MDFQVQIFSFLLISASVIMSRGDIQMTQSPSSLSASVGDRVTITCRASQDVNTAVAWYQQK
PGKAPKLLIYSASFLESGVPSRFSGSRSGTDFTLTISSLQPEDFATYYCQQHYTTPPTFGQ
GTKVEIKRTGSTSGSGKPGSGEGSDYKDDDDKTTTPAPRPPTPAPTIASQPLSLRPEASRP
AAGGAVHTRGLDFASDIYIWAPLAGTCGVLLLSLVITLYC*

4D5-5($V_H$)-8ser-BB-FKBP(36V)-3z (SEQ ID NO: 57)

MPLLLLLPLLWAGALAMEVQLVESGGGLVQPGGSLRLSCAASGFNIKDTYIHWVRQAPGK
GLEWVARIYPTNGYTRYADSVKGRFTISADTSKNTAYLQMNSLRAEDTAVYYCSRWGGD

# Fig. 15

I

GFVAMDVWGQGTLVTVSSGSNWSHPQFEKTTTPAPRPPTPAPTIASQPLSLRPEASRPAA
GGAVHTRGLDFASDIYIWAPLAGTCGVLLLSLVITLYCKRGRKKLLYIFKQPFMRPVQTTQE
EDGCSCRFPEEEEGGCELSRGSGSGSGSMGVQVETISPGDGRTFPKRGQTCVVHYTGML
EDGKKVDSSRDRNKPFKFMLGKQEVIRGWEEGVAQMSVGQRAKLTISPDYAYGATGHPGII
PPHATLVFDVELLKLEGSGSGSGSSLRVKFSRSADAPAYKQGQNQLYNELNLGRREEYDVL
DKRRGRDPEMGGKPRRKNPQEGLYNELQKDKMAEAYSEIGMKGERRRGKGHDGLYQGL
STATKDTYDALHMQALPPR*

CD19-8cys-BB-3z (SEQ ID NO: 58)

MLLLVTSLLLCELPHPAFLLIPDIQMTQTTSSLSASLGDRVTISCRASQDISKYLNWYQQKPD
GTVKLLIYHTSRLHSGVPSRFSGSGSGTDYSLTISNLEQEDIATYFCQQGNTLPYTFGGGTK
LEITGSTSGSGKPGSGEGSTKGEVKLQESGPGLVAPSQSLSVTCTVSGVSLPDYGVSWIR
QPPRKGLEWLGVIWGSETTYYNSALKSRLTIIKDNSKSQVFLKMNSLQTDDTAIYYCAKHY
YYGGSYAMDYWGQGTSVTVSSAAATTTPAPRPPTPAPTIASQPLSLRPEACRPAAGGAVH
TRGLDFACDIYIWAPLAGTCGVLLLSLVITLYCKRGRKKLLYIFKQPFMRPVQTTQEEDGCS
CRFPEEEEGGCELRVKFSRSADAPAYKQGQNQLYNELNLGRREEYDVLDKRRGRDPEMG
GKPRRKNPQEGLYNELQKDKMAEAYSEIGMKGERRRGKGHDGLYQGLSTATKDTYDALH
MQALPPR*

4D5-5-8cys-BB-3z (SEQ ID NO: 59)

MDFQVQIFSFLLISASVIMSRGDIQMTQSPSSLSASVGDRVTITCRASQDVNTAVAWYQQK
PGKAPKLLIYSASFLESGVPSRFSGSRSGTDFTLTISSLQPEDFATYYCQQHYTTPPTFGQ
GTKVEIKRTGSTSGSGKPGSGEGSEVQLVESGGGLVQPGGSLRLSCAASGFNIKDTYIHW
VRQAPGKGLEWVARIYPTNGYTRYADSVKGRFTISADTSKNTAYLQMNSLRAEDTAVYYC
SRWGGDGFVAMDVWGQGTLVTVSSGSGGGGSGGGGSNWSHPQFEKTTTPAPRPPTPA
PTIASQPLSLRPEACRPAAGGAVHTRGLDFACDIYIWAPLAGTCGVLLLSLVITLYCKRGRK
KLLYIFKQPFMRPVQTTQEEDGCSCRFPEEEEGGCELRVKFSRSADAPAYKQGQNQLYNE
LNLGRREEYDVLDKRRGRDPEMGGKPRRKNPQEGLYNELQKDKMAEAYSEIGMKGERRR
GKGHDGLYQGLSTATKDTYDALHMQALPPR*

4D5-5-8ser-BB-3z (SEQ ID NO: 60)

MDFQVQIFSFLLISASVIMSRGDIQMTQSPSSLSASVGDRVTITCRASQDVNTAVAWYQQK
PGKAPKLLIYSASFLESGVPSRFSGSRSGTDFTLTISSLQPEDFATYYCQQHYTTPPTFGQ
GTKVEIKRTGSTSGSGKPGSGEGSEVQLVESGGGLVQPGGSLRLSCAASGFNIKDTYIHW
VRQAPGKGLEWVARIYPTNGYTRYADSVKGRFTISADTSKNTAYLQMNSLRAEDTAVYYC
SRWGGDGFVAMDVWGQGTLVTVSSGSGGGGSGGGGSNWSHPQFEKTTTPAPRPPTPA
PTIASQPLSLRPEASRPAAGGAVHTRGLDFASDIYIWAPLAGTCGVLLLSLVITLYCKRGRK
KLLYIFKQPFMRPVQTTQEEDGCSCRFPEEEEGGCELRVKFSRSADAPAYKQGQNQLYNE
LNLGRREEYDVLDKRRGRDPEMGGKPRRKNPQEGLYNELQKDKMAEAYSEIGMKGERRR
GKGHDGLYQGLSTATKDTYDALHMQALPPR*

**rcSso7d-sfGFP fusion proteins**

E11.4.1-WT-sfGFP (SEQ ID NO: 61)

MSHHHHHHGSATVKFTYQGEEKQVDISKIMYVIRGGQRIAFGYDEGDGAWGDGIVSEKDA
PKELLQMLEKQGGGGSGGGGSMVSKGEELFTGVVPILVELDGDVNGHKFSVRGEGEGDA
TNGKLTLKFICTTGKLPVPWPTLVTTLTYGVQCFSRYPDHMKRHDFFKSAMPEGYVQERTIS
FKDDGTYKTRAEVKFEGDTLVNRIELKGIDFKEDGNILGHKLEYNFNSHNVYITADKQKNGIK
ANFKIRHNVEDGSVQLADHYQQNTPIGDGPVLLPDNHYLSTQSVLSKDPNEKRDHMVLLEF
VTAAGITHGMDELYK*

# Fig. 15 J

**Affibody-sfGFP fusion proteins**

zHER2-WT-sfGFP (SEQ ID NO: 62)

MGHHHHHHGS**VDNKFNKELRQAYWEIQALPNLAWTQSRAFIRKLYDDPSQSANLLAEAK
KLNDAQAPK**GGGGSGGGGS*MVSKGEELFTGVVPILVELDGDVNGHKFSVRGEGEGDATN
GKLTLKFICTTGKLPVPWPTLVTTLTYGVQCFSRYPDHMKRHDFFKSAMPEGYVQERTISFK
DDGTYKTRAEVKFEGDTLVNRIELKGIDFKEDGNILGHKLEYNFNSHNVYITADKQKNGIKAN
FKIRHNVEDGSVQLADHYQQNTPIGDGPVLLPDNHYLSTQSVLSKDPNEKRDHMVLLEFVT
AAGITHGMDELYK**

**VEGF CAR constructs**

S(G32A)-J.CT6 (Version A) (SEQ ID NO: 63)

MPLLLLLPLLWAGALAM**ATVKFTYQGEEKQVDISKIMYVIRGGQRIAFAYDEGDGAWGDGI
VSEKDAPKELLQMLEKQ**GGGGSGGGGSNWSHPQFEK***EPKSPDKTHTCPPCPAPELL***_GG
PSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYVYPPSRDELRF
YQVSLTCLVKGFYPSDIAVEWESNGQPDIFPNGLNYKTTPPVLDSDGSFALVSKLTVPYPSW
LMGTRFSCSVMHEALHNHYTQKHLEYQWPT*_

S(G32A)-J.CT6 (Version B) (SEQ ID NO: 64)

MPLLLLLPLLWAGALAM**ATVKFTYQGEEKQVDISKIMYVIRGGQRIAFAYDEGDGAWGDGI
VSEKDAPKELLQMLEKQ**NWSHPQFEK***EPKSPDKTHTCPPCPAPELL***_GGPSVFLFPPKPKD
TLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYVYPPSRDELRFYQVSLTCLVKG
FYPSDIAVEWESNGQPDIFPNGLNYKTTPPVLDSDGSFALVSKLTVPYPSWLMGTRFSCSV
MHEALHNHYTQKHLEYQWPT*_

J.CT6(WT Fc)-8ser-BB-3z (Version A) (SEQ ID NO: 65)

MELGLSWIFLLAILKGVQC***TCPPCPAPELL***_GGPSVFLFPPKPKDTLMISRTPEVTCVVVDVSH
EDPEVKFNWYVDGVEVHNAKTKPREEQYNSTYRVVSVLTVLHQDWLNGKEYKCKVSNKAL
PAPIEKTISKAKGQPREPQVYVLPPSRDELTKNQVSLLCLVKGFYPSDIAVEWESNGQPENN
YLTWPPVLDSDGSFFLYSKLTVDKSRWQQGNVFSCSVMHEALHNHYTQKSLSLSPGK_**LRP
EASRPAAGGAVHTRGLDFASDIYIWAPLAGTCGVLLLSLVITLYC**KRGRKKLLYIFKQPFMR
PVQTTQEEDGCSCRFPEEEEGGCELRVKFSRSADAPAYKQGQNQLYNELNLGRREEYDVL
DKRRGRDPEMGGKPRRKNPQEGLYNELQKDKMAEAYSEIGMKGERRRGKGHDGLYQGL
STATKDTYDALHMQALPPR*

J.CT6(WT Fc)-8ser-BB-3z (Version B) (SEQ ID NO: 66)

MELGLSWIFLLAILKGVQC***TCPPCPAPELL***_GGPSVFLFPPKPKDTLMISRTPEVTCVVVDVSH
EDPEVKFNWYVDGVEVHNAKTKPREEQYNSTYRVVSVLTVLHQDWLNGKEYKCKVSNKAL
PAPIEKTISKAKGQPREPQVYVLPPSRDELTKNQVSLLCLVKGFYPSDIAVEWESNGQPENN
YLTWPPVLDSDGSFFLYSKLTVDKSRWQQGNVFSCSVMHEALHNHYTQKSLSLSPGK_**AVH
TRGLDFASDIYIWAPLAGTCGVLLLSLVITLYC**KRGRKKLLYIFKQPFMRPVQTTQEEDGCS
CRFPEEEEGGCELRVKFSRSADAPAYKQGQNQLYNELNLGRREEYDVLDKRRGRDPEMG
GKPRRKNPQEGLYNELQKDKMAEAYSEIGMKGERRRGKGHDGLYQGLSTATKDTYDALH
MQALPPR*

# Fig. 15 K

**Trimeric and tetrameric CAR constructs**

S(G32A)-8ser-BB-FRB-FRB (SEQ ID NO: 67)

MPLLLLLPLLWAGALAMATVKFTYQGEEKQVDISKIMYVIRGGQRIAFAYDEGDGAWGDGI VSEKDAPKELLQMLEKQGGGGSGGGGSNWSHPQFEKTTTPAPRPPTPAPTIASQPLSLR PEASRPAAGGAVHTRGLDFASDIYIWAPLAGTCGVLLLSLVITLYCKRGRKKLLYIFKQPFM RPVQTTQEEDGCSCRFPEEEEGGCELGSGSGSGSGSGSGSGSILWHEMWHEGLEEASRL YFGERNVKGMFEVLEPLHAMMERGPQTLKETSFNQAYGRDLMEAQEWCRKYMKSGNVKD LLQAWDLYYHVFRRISKGSGSGSGSGSGSGSGSILWHEMWHEGLEEASRLYFGERNVKG MFEVLEPLHAMMERGPQTLKETSFNQAYGRDLMEAQEWCRKYMKSGNVKDLLQAWDLYY HVFRRISKGSGSGSGSSL*

S(G32A)-8ser-BB-FRB-FKBP(36V) (SEQ ID NO: 68)

MPLLLLLPLLWAGALAMATVKFTYQGEEKQVDISKIMYVIRGGQRIAFAYDEGDGAWGDGI VSEKDAPKELLQMLEKQGGGGSGGGGSNWSHPQFEKTTTPAPRPPTPAPTIASQPLSLR PEASRPAAGGAVHTRGLDFASDIYIWAPLAGTCGVLLLSLVITLYCKRGRKKLLYIFKQPFM RPVQTTQEEDGCSCRFPEEEEGGCELGSGSGSGSGSGSGSGSILWHEMWHEGLEEASRL YFGERNVKGMFEVLEPLHAMMERGPQTLKETSFNQAYGRDLMEAQEWCRKYMKSGNVKD LLQAWDLYYHVFRRISKGSGSGSGSSLMGVQVETISPGDGRTFPKRGQTCVVHYTGMLED GKKVDSSRDRNKPFKFMLGKQEVIRGWEEGVAQMSVGQRAKLTISPDYAYGATGHPGIIPP HATLVFDVELLKLE*

**Costimulatory CAR constructs**

S(G32A)-8ser-28-FKBP(36V)-3z (SEQ ID NO: 69)

MPLLLLLPLLWAGALAMATVKFTYQGEEKQVDISKIMYVIRGGQRIAFAYDEGDGAWGDGI VSEKDAPKELLQMLEKQGGGGSGGGGSNWSHPQFEKTTTPAPRPPTPAPTIASQPLSLR PEASRPAAGGAVHTRGLDFASDFWVLVVVGGVLACYSLLVTVAFIIFWVRSKRSRLLHSD YMNMTPRRPGPTRKHYQPYAPPRDFAAYRSSRGSGSGSGSMGVQVETISPGDGRTFPKR GQTCVVHYTGMLEDGKKVDSSRDRNKPFKFMLGKQEVIRGWEEGVAQMSVGQRAKLTISP DYAYGATGHPGIIPPHATLVFDVELLKLEGSGSGSGSSLRVKFSRSADAPAYKQGQNQLYN ELNLGRREEYDVLDKRRGRDPEMGGKPRRKNPQEGLYNELQKDKMAEAYSEIGMKGERR RGKGHDGLYQGLSTATKDTYDALHMQALPPR*

S(G32A)-8ser-ICOS-FKBP(36V)-3z (SEQ ID NO: 70)

MPLLLLLPLLWAGALAMATVKFTYQGEEKQVDISKIMYVIRGGQRIAFAYDEGDGAWGDGI VSEKDAPKELLQMLEKQGGGGSGGGGSNWSHPQFEKTTTPAPRPPTPAPTIASQPLSLR PEASRPAAGGAVHTRGLDFASDFEFWLPIGCAAFVVVCILGCILICWLTKKKYSSSVHDPN GEYMFMRAVNTAKKSRLTDVTLTSSRGSGSGSGSMGVQVETISPGDGRTFPKRGQTCVV HYTGMLEDGKKVDSSRDRNKPFKFMLGKQEVIRGWEEGVAQMSVGQRAKLTISPDYAYGA TGHPGIIPPHATLVFDVELLKLEGSGSGSGSSLRVKFSRSADAPAYKQGQNQLYNELNLGR REEYDVLDKRRGRDPEMGGKPRRKNPQEGLYNELQKDKMAEAYSEIGMKGERRRGKGHD GLYQGLSTATKDTYDALHMQALPPR*

S(G32A)-8ser-OX40-FKBP(36V)-3z (SEQ ID NO: 71)

MPLLLLLPLLWAGALAMATVKFTYQGEEKQVDISKIMYVIRGGQRIAFAYDEGDGAWGDGI VSEKDAPKELLQMLEKQGGGGSGGGGSNWSHPQFEKTTTPAPRPPTPAPTIASQPLSLR PEASRPAAGGAVHTRGLDFASDIYIWAPLAGTCGVLLLSLVITLYCRRDQRLPPDAHKPPG GGSFRTPIQEEQADAHSTLAKISRGSGSGSGSMGVQVETISPGDGRTFPKRGQTCVVHYT GMLEDGKKVDSSRDRNKPFKFMLGKQEVIRGWEEGVAQMSVGQRAKLTISPDYAYGATGH

Fig. 15
L

*PGIIPPHATLVFDVELLKLE*GSGSGSGSSLRVKFSRSADAPAYKQGQNQLYNELNLGRREEY
DVLDKRRGRDPEMGGKPRRKNPQEGLYNELQKDKMAEAYSEIGMKGERRRGKGHDGLYQ
GLSTATKDTYDALHMQALPPR*

**Sso7d-based CAR constructs**

S(G25A)-8cys-BB-3z (SEQ ID NO: 72)

MPLLLLLPLLWAGALAM**ATVKFTYQGEEKQVDISKIMYVIRAGQRIAFGYDEGDGAWGDGI**
**VSEKDAPKELLQMLEKQ**GGGGSGGGGSNWSHPQFEK**TTTPAPRPPTPAPTIASQPLSLR**
**PEACRPAAGGAVHTRGLDFACDIYIWAPLAGTCGVLLLSLVITLYC**KRGRKKLLYIFKQPFM
RPVQTTQEEDGCSCRFPEEEEGGCELRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

S(G25A)-8ser-BB-3z (SEQ ID NO: 73)

MPLLLLLPLLWAGALAM**ATVKFTYQGEEKQVDISKIMYVIRAGQRIAFGYDEGDGAWGDGI**
**VSEKDAPKELLQMLEKQ**GGGGSGGGGSNWSHPQFEK**TTTPAPRPPTPAPTIASQPLSLR**
**PEASRPAAGGAVHTRGLDFASDIYIWAPLAGTCGVLLLSLVITLYC**KRGRKKLLYIFKQPFM
RPVQTTQEEDGCSCRFPEEEEGGCELRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

S(WT)-8cys-BB-3z (SEQ ID NO: 74)

MPLLLLLPLLWAGALAM**ATVKFTYQGEEKQVDISKIMYVIRGGQRIAFGYDEGDGAWGDGI**
**VSEKDAPKELLQMLEKQ**GGGGSGGGGSNWSHPQFEK**TTTPAPRPPTPAPTIASQPLSLR**
**PEACRPAAGGAVHTRGLDFACDIYIWAPLAGTCGVLLLSLVITLYC**KRGRKKLLYIFKQPFM
RPVQTTQEEDGCSCRFPEEEEGGCELRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

S(WT)-8ser-BB-3z (SEQ ID NO: 75)

MPLLLLLPLLWAGALAM**ATVKFTYQGEEKQVDISKIMYVIRGGQRIAFGYDEGDGAWGDGI**
**VSEKDAPKELLQMLEKQ**GGGGSGGGGSNWSHPQFEK**TTTPAPRPPTPAPTIASQPLSLR**
**PEASRPAAGGAVHTRGLDFASDIYIWAPLAGTCGVLLLSLVITLYC**KRGRKKLLYIFKQPFM
RPVQTTQEEDGCSCRFPEEEEGGCELRVKFSRSADAPAYKQGQNQLYNELNLGRREEYD
VLDKRRGRDPEMGGKPRRKNPQEGLYNELQKDKMAEAYSEIGMKGERRRGKGHDGLYQ
GLSTATKDTYDALHMQALPPR*

SEQ ID NO: 76

NWSHPQFEK

SEQ ID NO: 77

HHHHHH

Europäisches Patentamt
European Patent Office
Office européen des brevets

# EUROPEAN SEARCH REPORT

Application Number
EP 18 19 8850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/180993 A1 (BLUEBIRD BIO INC [US]) 19 October 2017 (2017-10-19)<br>* the whole document *<br>----- | 1-15 | INV.<br>A61K39/00 |
| A | E. LANITIS ET AL: "Chimeric Antigen Receptor T Cells with Dissociated Signaling Domains Exhibit Focused Antitumor Activity with Reduced Potential for Toxicity In Vivo",<br>CANCER IMMUNOLOGY RESEARCH,<br>vol. 1, no. 1, 7 April 2013 (2013-04-07), pages 43-53, XP055170367,<br>ISSN: 2326-6066, DOI: 10.1158/2326-6066.CIR-13-0008<br>* the whole document *<br>----- | 1-15 | |
| A | CHRISTOPHER C KLOSS ET AL: "Combinatorial antigen recognition with balanced signaling promotes selective tumor eradication by engineered T cells",<br>NATURE BIOTECHNOLOGY,<br>vol. 31, no. 1,<br>16 December 2012 (2012-12-16), pages 71-75, XP055130697,<br>New York<br>ISSN: 1087-0156, DOI: 10.1038/nbt.2459<br>* the whole document *<br>----- | 1-15 | TECHNICAL FIELDS SEARCHED (IPC)<br>A61K<br>C07K |
| A | WO 2015/075468 A1 (UCL BUSINESS PLC [GB]) 28 May 2015 (2015-05-28)<br>* the whole document *<br>-----<br>-/-- | 1-15 | |

The present search report has been drawn up for all claims

2

| Place of search | Date of completion of the search | Examiner |
|---|---|---|
| The Hague | 1 April 2019 | Manu, Dominique |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 19 8850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | C.-Y. WU ET AL: "Remote control of therapeutic T cells through a small molecule-gated chimeric receptor", SCIENCE, vol. 350, no. 6258, 16 October 2015 (2015-10-16), pages aab4077-aab4077, XP055260068, US ISSN: 0036-8075, DOI: 10.1126/science.aab4077 * the whole document * ----- | 1-15 | |
| A | ADAM AJINA ET AL: "Strategies to Address Chimeric Antigen Receptor Tonic Signaling", MOLECULAR CANCER THERAPEUTICS, vol. 17, no. 9, 1 September 2018 (2018-09-01), pages 1795-1815, XP055575347, US ISSN: 1535-7163, DOI: 10.1158/1535-7163.MCT-17-1097 * the whole document * ----- | 1-15 | TECHNICAL FIELDS SEARCHED (IPC) |

The present search report has been drawn up for all claims

2

| Place of search | Date of completion of the search | Examiner |
|---|---|---|
| The Hague | 1 April 2019 | Manu, Dominique |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 18 19 8850

This annex lists the patent family members relating to the patent documents cited in the above-mentioned European search report. The members are as contained in the European Patent Office EDP file on
The European Patent Office is in no way liable for these particulars which are merely given for the purpose of information.

01-04-2019

| Patent document cited in search report | Publication date | Patent family member(s) | Publication date |
|---|---|---|---|
| WO 2017180993 A1 | 19-10-2017 | AU 2017250773 A1 | 08-11-2018 |
| | | CA 3020993 A1 | 19-10-2017 |
| | | CN 109311963 A | 05-02-2019 |
| | | EP 3443002 A1 | 20-02-2019 |
| | | WO 2017180993 A1 | 19-10-2017 |
| WO 2015075468 A1 | 28-05-2015 | AU 2014351557 A1 | 26-05-2016 |
| | | AU 2014351558 A1 | 26-05-2016 |
| | | BR 112016011459 A2 | 26-09-2017 |
| | | BR 112016011460 A2 | 26-09-2017 |
| | | CA 2929984 A1 | 28-05-2015 |
| | | CA 2930215 A1 | 28-05-2015 |
| | | CL 2016001134 A1 | 19-05-2017 |
| | | CL 2016001135 A1 | 23-12-2016 |
| | | CN 105792840 A | 20-07-2016 |
| | | CN 105848673 A | 10-08-2016 |
| | | EP 3071221 A1 | 28-09-2016 |
| | | EP 3071222 A1 | 28-09-2016 |
| | | EP 3071223 A1 | 28-09-2016 |
| | | HK 1223553 A1 | 04-08-2017 |
| | | HK 1223554 A1 | 04-08-2017 |
| | | IL 245572 A | 28-02-2019 |
| | | IL 245573 A | 29-11-2018 |
| | | JP 6433498 B2 | 05-12-2018 |
| | | JP 2016538854 A | 15-12-2016 |
| | | JP 2016538855 A | 15-12-2016 |
| | | JP 2019041775 A | 22-03-2019 |
| | | KR 20160085347 A | 15-07-2016 |
| | | KR 20160085348 A | 15-07-2016 |
| | | RU 2016124278 A | 22-12-2017 |
| | | RU 2016124280 A | 26-12-2017 |
| | | SG 11201603479T A | 30-05-2016 |
| | | SG 11201603484P A | 30-05-2016 |
| | | US 2016289293 A1 | 06-10-2016 |
| | | US 2016289294 A1 | 06-10-2016 |
| | | US 2016296562 A1 | 13-10-2016 |
| | | US 2019038672 A1 | 07-02-2019 |
| | | WO 2015075468 A1 | 28-05-2015 |
| | | WO 2015075469 A1 | 28-05-2015 |
| | | WO 2015075470 A1 | 28-05-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2956175 B1
- US 20170081411 A1
- WO 2017032777 A1
- US 20180094044 A1
- US 20180044399 A1
- US 20170260268 A1
- WO 2014127261 A
- US 6916846 B
- US 8236925 B
- US 20170306303 A1
- EP 17208924 A
- WO 9641865 A
- WO 9936553 A
- WO 0114387 A
- US 5525610 A
- US 5310903 A
- US 5362718 A
- US 5527907 A
- US 20120130076 A
- US 7067526 B1


### Non-patent literature cited in the description


- **LIM ; JUNE.** *Cell,* 2017, vol. 168 (4), 724
- **WU et al.** *Science,* 2015, vol. 350 (6258
- **JUILLERAT et al.** *Sci Rep.,* 2016, vol. 6, 18950
- **MA et al.** *Proc Natl Acad Sci U S A.,* 2016, vol. 113 (4), E450
- **URBANSKA et al.** *J Transl Med.,* 2014, vol. 12, 347
- **URBANSKA et al.** *Cancer Res.,* 2012, vol. 72 (7), 1844
- **CARTELLIERI et al.** *Blood Cancer J.,* 2016, vol. 6 (8), e458
- **HUDSON et al.** *J Immunol Methods.,* 1999, vol. 231 (1-2), 177-89
- **LONG et al.** *Nat Med.,* 2015, vol. 21 (6), 581-90
- **CHO et al.** *Cell,* 2018, vol. 173 (6), 1426-1438
- **MA et al.** *Proc Natl Acad Sci U S A.,* 2016, vol. 113 (4), E450-458
- **URBANSKA et al.** *Cancer Res.,* 2012, vol. 72 (7), 1844-1852
- **LABANIEH et al.** *Nat Biomed Eng.,* 2018, vol. 2, 377-391
- **SIMEON et al.** *Protein Cell,* 2017
- **GILBRETH et al.** *Curr Opin Struct Biol.,* 2012, vol. 22 (4), 413-420
- **KOIDE et al.** *ACS Chem Biol.,* 2009, vol. 4 (5), 325-334
- **TRAXLMAYR et al.** *J Biol Chem.,* 2016, vol. 291 (43), 22496-22508
- **PLÜCKTHUN.** Alternative Scaffolds: Expanding the options of antibodies. Cambridge University Press, 2009, 244-271
- **CHAPMAN et al.** *Cell Chem Biol.,* 2016, vol. 23 (5), 543-553
- **BINZ et al.** *Nat Biotechnol.,* 2005, vol. 23 (10), 1257-1268
- **VAZQUEZ-LOMBARDI et al.** *Drug Discov Today.,* 2015, vol. 20 (10), 1271-1283
- **THAYAPARAN et al.** *Oncoimmunology,* 2014, vol. 6 (12), e1363137
- **PARK et al.** *Sci Rep.,* 2017, vol. 7 (1), 14366
- **SCHERAG.** *Rev. Computational Chem.,* 1992, 11173-11142
- **JAMES.** *Sci Signal.,* 2018, vol. 11 (531
- **RAVETCH ; LANIER.** *Science,* 2000, vol. 290 (5489), 84
- **BARROW ; TROWSDALE.** *Eur J Immunol.,* 2006, vol. 36, 1646
- **SHANER et al.** *Nat. Methods,* 2005, vol. 2, 905-909
- **MATZ et al.** *Nature Biotechnol.,* 1999, vol. 17, 969-973
- **RUTKOWSKA et al.** *Angew Chem Int Ed Engl.,* 2012, vol. 51 (33), 8166
- **AMARA et al.** *PNAS,* 1997, vol. 94 (20), 10618
- **FARRAR et al.** *Nature,* 1996, vol. 383, 178
- **SORENSEN et al.** *Skin Pharmacol.,* 1997, vol. 10, 144
- **SCHIEFNER et al.** *Acc Chem Res.,* 2015, vol. 48 (4), 976-985
- **YE et al.** *Science,* 1999, vol. 283, 88-91
- **YOUNG et al.** *J Biol Chem.,* 2004, vol. 279 (46), 47633-42
- **DOTOR et al.** *Cytokine,* 2007, vol. 39 (2), 106-15
- **LOBNER et al.** *MAbs,* 2017, vol. 9 (7), 1088-1104

- **MILONE et al.** *Mol Ther.,* 2009, vol. 17 (8), 1453-1464
- **GAJ et al.** *Trends Biotechnol.,* 2013, vol. 31 (7), 397-405
- **REN et al.** *Protein Cell,* 2017, vol. 8 (9), 634-643
- **EYQUEM et al.** *Nature,* 2017, vol. 543 (7643), 113-117
- **PROFF et al.** *Front Micro-biol.,* 2016, vol. 7, 844
- Remington's Pharmaceutical Sciences. Mack Publishing Company, 1985
- **CHEN.** *Drug Dev Ind Pharm,* 1992, vol. 18, 1311-54
- **WANG et al.** *Blood,* 2011, vol. 118 (5), 1255-1263
- **LIU et al.** *Cancer Res.,* 2015, vol. 75 (17), 3596-3607
- **ATWELL et al.** *Protein Eng.,* 1999, vol. 12 (7), 597-604
- **LONG et al.** *Nat Med.,* 2015, vol. 21 (6), 581-590
- **WIKMAN et al.** *Protein Eng Des Sel.,* 2004, vol. 17 (5), 455-462
- **FELDWISCH et al.** *J. Mol. Biol.,* 2010, vol. 398 (2), 232-47
- **CIOFFI et al.** *J Med Chem.,* 2014, vol. 57 (18), 7731-7757
- **CIOFFI et al.** *J Med Chem.,* 2015, vol. 58 (15), 5863-5888

SEQUENCE LISTING

<110> ST. ANNA KINDERKREBSFORSCHUNG
UNIVERSITÄT FÜR BODENKULTUR WIEN

<120> A group of chimeric antigen receptors (CARs)

<130> R 72067

<160> 77

<170> PatentIn version 3.5

<210> 1
<211> 4
<212> PRT
<213> Artificial Sequence

<220>
<223> synthetic polypeptide

<400> 1

Gly Gly Ser Gly
1

<210> 2
<211> 5
<212> PRT
<213> Artificial Sequence

<220>
<223> synthetic polypeptide

<400> 2

Gly Gly Ser Gly Gly
1 5

<210> 3
<211> 5
<212> PRT
<213> Artificial Sequence

<220>
<223> synthetic polypeptide

<400> 3

Gly Ser Gly Ser Gly
1 5

<210> 4
<211> 5
<212> PRT
<213> Artificial Sequence

<220>
<223> synthetic polypeptide

<400> 4

Gly Ser Gly Gly Gly
1 5

<210> 5
<211> 5
<212> PRT
<213> Artificial Sequence

<220>
<223> synthetic polypeptide

<400> 5

Gly Gly Gly Ser Gly
1 5

<210> 6
<211> 5
<212> PRT
<213> Artificial Sequence

<220>
<223> synthetic polypeptide

<400> 6

Gly Ser Ser Ser Gly
1 5

<210> 7
<211> 9
<212> PRT
<213> Artificial Sequence

<220>
<223> synthetic polypeptide

<400> 7

Tyr Pro Tyr Asp Val Pro Asp Tyr Ala
1 5

<210> 8
<211> 8
<212> PRT
<213> Artificial Sequence

<220>
<223> synthetic polypeptide

<400> 8

Asp Tyr Lys Asp Asp Asp Asp Lys
1 5

<210> 9

<211> 10
<212> PRT
<213> Artificial Sequence

<220>
<223> synthetic polypeptide

<400> 9

Glu Gln Lys Leu Ile Ser Glu Glu Asp Leu
1 5 10

<210> 10
<211> 111
<212> PRT
<213> Artificial Sequence

<220>
<223> synthetic polypeptide

<400> 10

Met Ala Ser Arg Gly Val Gln Val Glu Thr Ile Ser Pro Gly Asp Gly
1 5 10 15

Arg Thr Phe Pro Lys Arg Gly Gln Thr Cys Val Val His Tyr Thr Gly
20 25 30

Met Leu Glu Asp Gly Lys Lys Val Asp Ser Ser Arg Asp Arg Asn Lys
35 40 45

Pro Phe Lys Phe Met Leu Gly Lys Gln Glu Val Ile Arg Gly Trp Glu
50 55 60

Glu Gly Val Ala Gln Met Ser Val Gly Gln Arg Ala Lys Leu Thr Ile
65 70 75 80

Ser Pro Asp Tyr Ala Tyr Gly Ala Thr Gly His Pro Gly Ile Ile Pro
85 90 95

Pro His Ala Thr Leu Val Phe Asp Val Glu Leu Leu Lys Leu Glu
100 105 110

<210> 11
<211> 16
<212> PRT
<213> Artificial Sequence

<220>
<223> synthetic polypeptide

<400> 11

Asp Ala Phe Gln Leu Arg Gln Leu Ile Leu Arg Gly Leu Gln Asp Asp
1 5 10 15

<210> 12
<211> 13
<212> PRT
<213> Artificial Sequence

<220>
<223> synthetic polypeptide

<400> 12

Ser Pro Gly Ser Arg Glu Trp Phe Lys Asp Met Leu Ser
1 5 10

<210> 13
<211> 17
<212> PRT
<213> Artificial Sequence

<220>
<223> synthetic polypeptide

<400> 13

Pro Arg Gln Gly Ser Ile Leu Tyr Ser Met Leu Thr Ser Ala Lys Gln
1 5 10 15

Thr

<210> 14
<211> 21
<212> PRT
<213> Artificial Sequence

<220>
<223> synthetic polypeptide

<400> 14

Pro Lys Lys Glu Asn Asn Ala Leu Leu Arg Tyr Leu Leu Asp Arg Asp
1 5 10 15

Asp Pro Ser Asp Val
20

<210> 15
<211> 16
<212> PRT
<213> Artificial Sequence

<220>
<223> synthetic polypeptide

<400> 15

Ser Ser Lys Gly Val Leu Trp Arg Met Leu Ala Glu Pro Val Ser Arg

1 5 10 15

<210> 16
<211> 15
<212> PRT
<213> Artificial Sequence

<220>
<223> synthetic polypeptide

<400> 16

Ser Arg Thr Leu Gln Leu Asp Trp Gly Thr Leu Tyr Trp Ser Arg
1 5 10 15

<210> 17
<211> 15
<212> PRT
<213> Artificial Sequence

<220>
<223> synthetic polypeptide

<400> 17

Ser Ser Asn His Gln Ser Ser Arg Leu Ile Glu Leu Leu Ser Arg
1 5 10 15

<210> 18
<211> 21
<212> PRT
<213> Artificial Sequence

<220>
<223> synthetic polypeptide

<400> 18

Arg Leu Thr Lys Thr Asn Pro Ile Leu Tyr Tyr Met Leu Gln Lys Gly
1 5 10 15

Gly Asn Ser Val Ala
20

<210> 19
<211> 21
<212> PRT
<213> Artificial Sequence

<220>
<223> synthetic polypeptide

<400> 19

Asn Leu Leu Glu Arg Arg Thr Val Leu Gln Leu Leu Leu Gly Asn Pro
1 5 10 15

Thr Lys Gly Arg Val
20

<210> 20
<211> 61
<212> PRT
<213> Artificial Sequence

<220>
<223> synthetic polypeptide

<400> 20

Ala Thr Val Lys Phe Thr Tyr Gln Gly Glu Glu Lys Gln Val Asp Ile
1 5 10 15

Ser Lys Ile Lys Trp Val Ile Arg Trp Gly Gln His Ile Ala Phe Lys
20 25 30

Tyr Asp Glu Gly Gly Gly Ala Ala Gly Tyr Gly Trp Val Ser Glu Lys
35 40 45

Asp Ala Pro Lys Glu Leu Leu Gln Met Leu Glu Lys Gln
50 55 60

<210> 21
<211> 61
<212> PRT
<213> Artificial Sequence

<220>
<223> synthetic polypeptide

<400> 21

Ala Ala Val Lys Leu Thr Tyr Gln Gly Glu Glu Lys Gln Val Asp Ile
1 5 10 15

Ser Lys Ile Lys Tyr Val Asp Arg Ala Gly Gln Phe Ile Trp Phe Glu
20 25 30

Tyr Asp Glu Gly Gly Gly Ala Leu Gly Thr Gly Trp Val Ser Glu Lys
35 40 45

Asp Ala Pro Lys Glu Leu Leu Gln Met Leu Glu Lys Gln
50 55 60

<210> 22
<211> 61
<212> PRT
<213> Artificial Sequence

<220>
<223> synthetic polypeptide

<400> 22

Ala Thr Val Lys Phe Thr Tyr Gln Gly Glu Glu Lys Gln Val Asp Ile
1               5                   10                  15

Ser Lys Ile Met Tyr Val Ile Arg Gly Gly Gln Arg Ile Ala Phe Gly
            20                  25                  30

Tyr Asp Glu Gly Asp Gly Ala Trp Gly Asp Gly Ile Val Ser Glu Lys
        35                  40                  45

Asp Ala Pro Lys Glu Leu Leu Gln Met Leu Glu Lys Gln
    50                  55                  60

<210> 23
<211> 61
<212> PRT
<213> Artificial Sequence

<220>
<223> synthetic polypeptide

<400> 23

Ala Thr Val Lys Phe Thr Tyr Gln Gly Glu Glu Lys Gln Val Asp Ile
1               5                   10                  15

Ser Lys Ile Met Tyr Val Ile Arg Ala Gly Gln Arg Ile Ala Phe Gly
            20                  25                  30

Tyr Asp Glu Gly Asp Gly Ala Trp Gly Asp Gly Ile Val Ser Glu Lys
        35                  40                  45

Asp Ala Pro Lys Glu Leu Leu Gln Met Leu Glu Lys Gln
    50                  55                  60

<210> 24
<211> 61
<212> PRT
<213> Artificial Sequence

<220>
<223> synthetic polypeptide

<400> 24

Ala Thr Val Lys Phe Thr Tyr Gln Gly Glu Glu Lys Gln Val Asp Ile
1               5                   10                  15

Ser Lys Ile Met Tyr Val Ile Arg Gly Gly Gln Ala Ile Ala Phe Gly
            20                  25                  30

```
Tyr Asp Glu Gly Asp Gly Ala Trp Gly Asp Gly Ile Val Ser Glu Lys
        35                  40                  45

Asp Ala Pro Lys Glu Leu Leu Gln Met Leu Glu Lys Gln
    50                  55                  60
```

<210> 25
<211> 61
<212> PRT
<213> Artificial Sequence

<220>
<223> synthetic polypeptide

<400> 25

```
Ala Thr Val Lys Phe Thr Tyr Gln Gly Glu Glu Lys Gln Val Asp Ile
1               5                   10                  15

Ser Lys Ile Met Tyr Val Ile Arg Gly Gly Gln Arg Ile Ala Phe Ala
            20                  25                  30

Tyr Asp Glu Gly Asp Gly Ala Trp Gly Asp Gly Ile Val Ser Glu Lys
        35                  40                  45

Asp Ala Pro Lys Glu Leu Leu Gln Met Leu Glu Lys Gln
    50                  55                  60
```

<210> 26
<211> 58
<212> PRT
<213> Artificial Sequence

<220>
<223> synthetic polypeptide

<400> 26

```
Val Asp Asn Lys Phe Asn Lys Glu Leu Arg Gln Ala Tyr Trp Glu Ile
1               5                   10                  15

Gln Ala Leu Pro Asn Leu Ala Trp Thr Gln Ser Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 27
<211> 58
<212> PRT

<213> Artificial Sequence

<220>
<223> synthetic polypeptide

<400> 27

```
Val Asp Asn Lys Phe Asn Lys Glu Ala Arg Gln Ala Tyr Trp Glu Ile
1               5                   10                  15

Gln Ala Leu Pro Asn Leu Ala Trp Thr Gln Ser Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 28
<211> 58
<212> PRT
<213> Artificial Sequence

<220>
<223> synthetic polypeptide

<400> 28

```
Val Asp Asn Lys Phe Asn Lys Glu Leu Ala Gln Ala Tyr Trp Glu Ile
1               5                   10                  15

Gln Ala Leu Pro Asn Leu Ala Trp Thr Gln Ser Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 29
<211> 58
<212> PRT
<213> Artificial Sequence

<220>
<223> synthetic polypeptide

<400> 29

```
Val Asp Asn Lys Phe Asn Lys Glu Leu Arg Ala Ala Tyr Trp Glu Ile
1               5                   10                  15
```

Gln Ala Leu Pro Asn Leu Ala Trp Thr Gln Ser Arg Ala Phe Ile Arg
20 25 30

Lys Leu Tyr Asp Asp Pro Ser Gln Ser Ala Asn Leu Leu Ala Glu Ala
35 40 45

Lys Lys Leu Asn Asp Ala Gln Ala Pro Lys
50 55

<210> 30
<211> 58
<212> PRT
<213> Artificial Sequence

<220>
<223> synthetic polypeptide

<400> 30

Val Asp Asn Lys Phe Asn Lys Glu Leu Arg Gln Ala Ala Trp Glu Ile
1 5 10 15

Gln Ala Leu Pro Asn Leu Ala Trp Thr Gln Ser Arg Ala Phe Ile Arg
20 25 30

Lys Leu Tyr Asp Asp Pro Ser Gln Ser Ala Asn Leu Leu Ala Glu Ala
35 40 45

Lys Lys Leu Asn Asp Ala Gln Ala Pro Lys
50 55

<210> 31
<211> 58
<212> PRT
<213> Artificial Sequence

<220>
<223> synthetic polypeptide

<400> 31

Val Asp Asn Lys Phe Asn Lys Glu Leu Arg Gln Ala Tyr Ala Glu Ile
1 5 10 15

Gln Ala Leu Pro Asn Leu Ala Trp Thr Gln Ser Arg Ala Phe Ile Arg
20 25 30

Lys Leu Tyr Asp Asp Pro Ser Gln Ser Ala Asn Leu Leu Ala Glu Ala
35 40 45

Lys Lys Leu Asn Asp Ala Gln Ala Pro Lys
50 55

<210> 32
<211> 58
<212> PRT
<213> Artificial Sequence

<220>
<223> synthetic polypeptide

<400> 32

```
Val Asp Asn Lys Phe Asn Lys Glu Leu Arg Gln Ala Tyr Trp Glu Ile
1               5                   10                  15

Ala Ala Leu Pro Asn Leu Ala Trp Thr Gln Ser Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 33
<211> 58
<212> PRT
<213> Artificial Sequence

<220>
<223> synthetic polypeptide

<400> 33

```
Val Asp Asn Lys Phe Asn Lys Glu Leu Arg Gln Ala Tyr Trp Glu Ile
1               5                   10                  15

Gln Ala Leu Pro Asn Leu Ala Ala Thr Gln Ser Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 34
<211> 58
<212> PRT
<213> Artificial Sequence

<220>
<223> synthetic polypeptide

<400> 34

Val Asp Asn Lys Phe Asn Lys Glu Leu Arg Gln Ala Tyr Trp Glu Ile
1               5                   10                  15

Gln Ala Leu Pro Asn Leu Ala Trp Ala Gln Ser Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55

<210> 35
<211> 58
<212> PRT
<213> Artificial Sequence

<220>
<223> synthetic polypeptide

<400> 35

Val Asp Asn Lys Phe Asn Lys Glu Leu Arg Gln Ala Tyr Trp Glu Ile
1               5                   10                  15

Gln Ala Leu Pro Asn Leu Ala Trp Thr Gln Ala Arg Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
    50                  55

<210> 36
<211> 58
<212> PRT
<213> Artificial Sequence

<220>
<223> synthetic polypeptide

<400> 36

Val Asp Asn Lys Phe Asn Lys Glu Leu Arg Gln Ala Tyr Trp Glu Ile
1               5                   10                  15

Gln Ala Leu Pro Asn Leu Ala Trp Thr Gln Ser Ala Ala Phe Ile Arg
            20                  25                  30

Lys Leu Tyr Asp Asp Pro Ser Gln Ser Ala Asn Leu Leu Ala Glu Ala
        35                  40                  45

Lys Lys Leu Asn Asp Ala Gln Ala Pro Lys
50 55

<210> 37
<211> 58
<212> PRT
<213> Artificial Sequence

<220>
<223> synthetic polypeptide

<400> 37

Val Asp Asn Lys Phe Asn Lys Glu Leu Arg Gln Ala Tyr Trp Glu Ile
1 5 10 15

Gln Ala Leu Pro Asn Leu Ala Trp Thr Gln Ser Arg Ala Phe Ile Ala
20 25 30

Lys Leu Tyr Asp Asp Pro Ser Gln Ser Ala Asn Leu Leu Ala Glu Ala
35 40 45

Lys Lys Leu Asn Asp Ala Gln Ala Pro Lys
50 55

<210> 38
<211> 58
<212> PRT
<213> Artificial Sequence

<220>
<223> synthetic polypeptide

<400> 38

Val Asp Asn Lys Phe Asn Lys Glu Leu Arg Gln Ala Tyr Trp Glu Ile
1 5 10 15

Gln Ala Leu Pro Asn Leu Ala Trp Thr Gln Ser Arg Ala Phe Ile Arg
20 25 30

Lys Leu Ala Asp Asp Pro Ser Gln Ser Ala Asn Leu Leu Ala Glu Ala
35 40 45

Lys Lys Leu Asn Asp Ala Gln Ala Pro Lys
50 55

<210> 39
<211> 316
<212> PRT
<213> Artificial Sequence

<220>

<223> synthetic polypeptide

<400> 39

Met Pro Leu Leu Leu Leu Leu Pro Leu Leu Trp Ala Gly Ala Leu Ala
1               5                   10                  15

Met Glu Gln Lys Leu Ile Ser Glu Glu Asp Leu Gly Gly Gly Gly Ser
            20                  25                  30

Ala Ala Val Lys Leu Thr Tyr Gln Gly Glu Glu Lys Gln Val Asp Ile
        35                  40                  45

Ser Lys Ile Lys Tyr Val Asp Arg Ala Gly Gln Phe Ile Trp Phe Glu
    50                  55                  60

Tyr Asp Glu Gly Gly Gly Ala Leu Gly Thr Gly Trp Val Ser Glu Lys
65                  70                  75                  80

Asp Ala Pro Lys Glu Leu Leu Gln Met Leu Glu Lys Gln Thr Thr Thr
                85                  90                  95

Pro Ala Pro Arg Pro Pro Thr Pro Ala Pro Thr Ile Ala Ser Gln Pro
            100                 105                 110

Leu Ser Leu Arg Pro Glu Ala Cys Arg Pro Ala Ala Gly Gly Ala Val
        115                 120                 125

His Thr Arg Gly Leu Asp Phe Ala Cys Asp Ile Tyr Ile Trp Ala Pro
    130                 135                 140

Leu Ala Gly Thr Cys Gly Val Leu Leu Leu Ser Leu Val Ile Thr Leu
145                 150                 155                 160

Tyr Cys Lys Arg Gly Arg Lys Lys Leu Leu Tyr Ile Phe Lys Gln Pro
                165                 170                 175

Phe Met Arg Pro Val Gln Thr Thr Gln Glu Glu Asp Gly Cys Ser Cys
            180                 185                 190

Arg Phe Pro Glu Glu Glu Glu Gly Gly Cys Glu Leu Arg Val Lys Phe
        195                 200                 205

Ser Arg Ser Ala Asp Ala Pro Ala Tyr Lys Gln Gly Gln Asn Gln Leu
    210                 215                 220

Tyr Asn Glu Leu Asn Leu Gly Arg Arg Glu Glu Tyr Asp Val Leu Asp
225                 230                 235                 240

```
Lys Arg Arg Gly Arg Asp Pro Glu Met Gly Gly Lys Pro Arg Arg Lys
                245                 250                 255

Asn Pro Gln Glu Gly Leu Tyr Asn Glu Leu Gln Lys Asp Lys Met Ala
            260                 265                 270

Glu Ala Tyr Ser Glu Ile Gly Met Lys Gly Glu Arg Arg Arg Gly Lys
        275                 280                 285

Gly His Asp Gly Leu Tyr Gln Gly Leu Ser Thr Ala Thr Lys Asp Thr
    290                 295                 300

Tyr Asp Ala Leu His Met Gln Ala Leu Pro Pro Arg
305                 310                 315


<210>  40
<211>  301
<212>  PRT
<213>  Artificial Sequence

<220>
<223>  synthetic polypeptide

<400>  40

Met Pro Leu Leu Leu Leu Leu Pro Leu Leu Trp Ala Gly Ala Leu Ala
1               5                   10                  15

Met Ala Ala Val Lys Leu Thr Tyr Gln Gly Glu Glu Lys Gln Val Asp
            20                  25                  30

Ile Ser Lys Ile Lys Tyr Val Asp Arg Ala Gly Gln Phe Ile Trp Phe
        35                  40                  45

Glu Tyr Asp Glu Gly Gly Gly Ala Leu Gly Thr Gly Trp Val Ser Glu
    50                  55                  60

Lys Asp Ala Pro Lys Glu Leu Leu Gln Met Leu Glu Lys Gln Thr Thr
65                  70                  75                  80

Thr Pro Ala Pro Arg Pro Pro Thr Pro Ala Pro Thr Ile Ala Ser Gln
                85                  90                  95

Pro Leu Ser Leu Arg Pro Glu Ala Cys Arg Pro Ala Ala Gly Gly Ala
            100                 105                 110

Val His Thr Arg Gly Leu Asp Phe Ala Cys Asp Ile Tyr Ile Trp Ala
        115                 120                 125
```

Pro Leu Ala Gly Thr Cys Gly Val Leu Leu Leu Ser Leu Val Ile Thr
    130                 135                 140

Leu Tyr Cys Lys Arg Gly Arg Lys Lys Leu Leu Tyr Ile Phe Lys Gln
145                 150                 155                 160

Pro Phe Met Arg Pro Val Gln Thr Thr Gln Glu Glu Asp Gly Cys Ser
                165                 170                 175

Cys Arg Phe Pro Glu Glu Glu Glu Gly Gly Cys Glu Leu Arg Val Lys
            180                 185                 190

Phe Ser Arg Ser Ala Asp Ala Pro Ala Tyr Lys Gln Gly Gln Asn Gln
        195                 200                 205

Leu Tyr Asn Glu Leu Asn Leu Gly Arg Arg Glu Glu Tyr Asp Val Leu
    210                 215                 220

Asp Lys Arg Arg Gly Arg Asp Pro Glu Met Gly Gly Lys Pro Arg Arg
225                 230                 235                 240

Lys Asn Pro Gln Glu Gly Leu Tyr Asn Glu Leu Gln Lys Asp Lys Met
                245                 250                 255

Ala Glu Ala Tyr Ser Glu Ile Gly Met Lys Gly Glu Arg Arg Arg Gly
            260                 265                 270

Lys Gly His Asp Gly Leu Tyr Gln Gly Leu Ser Thr Ala Thr Lys Asp
        275                 280                 285

Thr Tyr Asp Ala Leu His Met Gln Ala Leu Pro Pro Arg
    290                 295                 300

<210> 41
<211> 311
<212> PRT
<213> Artificial Sequence

<220>
<223> synthetic polypeptide

<400> 41

Met Pro Leu Leu Leu Leu Leu Pro Leu Leu Trp Ala Gly Ala Leu Ala
1               5                   10                  15

Met Ala Ala Val Lys Leu Thr Tyr Gln Gly Glu Glu Lys Gln Val Asp
            20                  25                  30

Ile Ser Lys Ile Lys Tyr Val Asp Arg Ala Gly Gln Phe Ile Trp Phe

35 40 45

Glu Tyr Asp Glu Gly Gly Gly Ala Leu Gly Thr Gly Trp Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Thr Thr Thr Pro Ala Pro Arg Pro
85 90 95

Pro Thr Pro Ala Pro Thr Ile Ala Ser Gln Pro Leu Ser Leu Arg Pro
100 105 110

Glu Ala Cys Arg Pro Ala Ala Gly Gly Ala Val His Thr Arg Gly Leu
115 120 125

Asp Phe Ala Cys Asp Ile Tyr Ile Trp Ala Pro Leu Ala Gly Thr Cys
130 135 140

Gly Val Leu Leu Leu Ser Leu Val Ile Thr Leu Tyr Cys Lys Arg Gly
145 150 155 160

Arg Lys Lys Leu Leu Tyr Ile Phe Lys Gln Pro Phe Met Arg Pro Val
165 170 175

Gln Thr Thr Gln Glu Glu Asp Gly Cys Ser Cys Arg Phe Pro Glu Glu
180 185 190

Glu Glu Gly Gly Cys Glu Leu Arg Val Lys Phe Ser Arg Ser Ala Asp
195 200 205

Ala Pro Ala Tyr Lys Gln Gly Gln Asn Gln Leu Tyr Asn Glu Leu Asn
210 215 220

Leu Gly Arg Arg Glu Glu Tyr Asp Val Leu Asp Lys Arg Arg Gly Arg
225 230 235 240

Asp Pro Glu Met Gly Gly Lys Pro Arg Arg Lys Asn Pro Gln Glu Gly
245 250 255

Leu Tyr Asn Glu Leu Gln Lys Asp Lys Met Ala Glu Ala Tyr Ser Glu
260 265 270

Ile Gly Met Lys Gly Glu Arg Arg Arg Gly Lys Gly His Asp Gly Leu
275 280 285

Tyr Gln Gly Leu Ser Thr Ala Thr Lys Asp Thr Tyr Asp Ala Leu His
290 295 300

Met Gln Ala Leu Pro Pro Arg
305 310

<210> 42
<211> 321
<212> PRT
<213> Artificial Sequence

<220>
<223> synthetic polypeptide

<400> 42

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Glu Gln Lys Leu Ile Ser Glu Glu
85 90 95

Asp Leu Thr Thr Thr Pro Ala Pro Arg Pro Pro Thr Pro Ala Pro Thr
100 105 110

Ile Ala Ser Gln Pro Leu Ser Leu Arg Pro Glu Ala Cys Arg Pro Ala
115 120 125

Ala Gly Gly Ala Val His Thr Arg Gly Leu Asp Phe Ala Cys Asp Ile
130 135 140

Tyr Ile Trp Ala Pro Leu Ala Gly Thr Cys Gly Val Leu Leu Leu Ser
145 150 155 160

Leu Val Ile Thr Leu Tyr Cys Lys Arg Gly Arg Lys Lys Leu Leu Tyr
165 170 175

Ile Phe Lys Gln Pro Phe Met Arg Pro Val Gln Thr Thr Gln Glu Glu

180 185 190

Asp Gly Cys Ser Cys Arg Phe Pro Glu Glu Glu Glu Gly Gly Cys Glu
195 200 205

Leu Arg Val Lys Phe Ser Arg Ser Ala Asp Ala Pro Ala Tyr Lys Gln
210 215 220

Gly Gln Asn Gln Leu Tyr Asn Glu Leu Asn Leu Gly Arg Arg Glu Glu
225 230 235 240

Tyr Asp Val Leu Asp Lys Arg Arg Gly Arg Asp Pro Glu Met Gly Gly
245 250 255

Lys Pro Arg Arg Lys Asn Pro Gln Glu Gly Leu Tyr Asn Glu Leu Gln
260 265 270

Lys Asp Lys Met Ala Glu Ala Tyr Ser Glu Ile Gly Met Lys Gly Glu
275 280 285

Arg Arg Arg Gly Lys Gly His Asp Gly Leu Tyr Gln Gly Leu Ser Thr
290 295 300

Ala Thr Lys Asp Thr Tyr Asp Ala Leu His Met Gln Ala Leu Pro Pro
305 310 315 320

Arg

<210> 43
<211> 320
<212> PRT
<213> Artificial Sequence

<220>
<223> synthetic polypeptide

<400> 43

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

```
Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Cys Arg Pro Ala Ala
        115                 120                 125

Gly Gly Ala Val His Thr Arg Gly Leu Asp Phe Ala Cys Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Arg Val Lys Phe Ser Arg Ser Ala Asp Ala Pro Ala Tyr Lys Gln Gly
    210                 215                 220

Gln Asn Gln Leu Tyr Asn Glu Leu Asn Leu Gly Arg Arg Glu Glu Tyr
225                 230                 235                 240

Asp Val Leu Asp Lys Arg Arg Gly Arg Asp Pro Glu Met Gly Gly Lys
                245                 250                 255

Pro Arg Arg Lys Asn Pro Gln Glu Gly Leu Tyr Asn Glu Leu Gln Lys
            260                 265                 270

Asp Lys Met Ala Glu Ala Tyr Ser Glu Ile Gly Met Lys Gly Glu Arg
        275                 280                 285

Arg Arg Gly Lys Gly His Asp Gly Leu Tyr Gln Gly Leu Ser Thr Ala
    290                 295                 300

Thr Lys Asp Thr Tyr Asp Ala Leu His Met Gln Ala Leu Pro Pro Arg
```

305 310 315 320

<210> 44
<211> 320
<212> PRT
<213> Artificial Sequence

<220>
<223> synthetic polypeptide

<400> 44

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asn Trp Ser His Pro Gln Phe Glu
85 90 95

Lys Thr Thr Thr Pro Ala Pro Arg Pro Pro Thr Pro Ala Pro Thr Ile
100 105 110

Ala Ser Gln Pro Leu Ser Leu Arg Pro Glu Ala Ser Arg Pro Ala Ala
115 120 125

Gly Gly Ala Val His Thr Arg Gly Leu Asp Phe Ala Ser Asp Ile Tyr
130 135 140

Ile Trp Ala Pro Leu Ala Gly Thr Cys Gly Val Leu Leu Leu Ser Leu
145 150 155 160

Val Ile Thr Leu Tyr Cys Lys Arg Gly Arg Lys Lys Leu Leu Tyr Ile
165 170 175

Phe Lys Gln Pro Phe Met Arg Pro Val Gln Thr Thr Gln Glu Glu Asp
180 185 190

Gly Cys Ser Cys Arg Phe Pro Glu Glu Glu Glu Gly Gly Cys Glu Leu
195 200 205

```
Arg Val Lys Phe Ser Arg Ser Ala Asp Ala Pro Ala Tyr Lys Gln Gly
    210                 215                 220

Gln Asn Gln Leu Tyr Asn Glu Leu Asn Leu Gly Arg Arg Glu Glu Tyr
225                 230                 235                 240

Asp Val Leu Asp Lys Arg Arg Gly Arg Asp Pro Glu Met Gly Gly Lys
                245                 250                 255

Pro Arg Arg Lys Asn Pro Gln Glu Gly Leu Tyr Asn Glu Leu Gln Lys
            260                 265                 270

Asp Lys Met Ala Glu Ala Tyr Ser Glu Ile Gly Met Lys Gly Glu Arg
        275                 280                 285

Arg Arg Gly Lys Gly His Asp Gly Leu Tyr Gln Gly Leu Ser Thr Ala
    290                 295                 300

Thr Lys Asp Thr Tyr Asp Ala Leu His Met Gln Ala Leu Pro Pro Arg
305                 310                 315                 320
```

<210> 45
<211> 317
<212> PRT
<213> Artificial Sequence

<220>
<223> synthetic polypeptide

<400> 45

```
Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser His His His His His His Thr Thr
                85                  90                  95
```

Thr Pro Ala Pro Arg Pro Pro Thr Pro Ala Pro Thr Ile Ala Ser Gln
100 105 110

Pro Leu Ser Leu Arg Pro Glu Ala Cys Arg Pro Ala Ala Gly Gly Ala
115 120 125

Val His Thr Arg Gly Leu Asp Phe Ala Cys Asp Ile Tyr Ile Trp Ala
130 135 140

Pro Leu Ala Gly Thr Cys Gly Val Leu Leu Leu Ser Leu Val Ile Thr
145 150 155 160

Leu Tyr Cys Lys Arg Gly Arg Lys Lys Leu Leu Tyr Ile Phe Lys Gln
165 170 175

Pro Phe Met Arg Pro Val Gln Thr Thr Gln Glu Glu Asp Gly Cys Ser
180 185 190

Cys Arg Phe Pro Glu Glu Glu Glu Gly Gly Cys Glu Leu Arg Val Lys
195 200 205

Phe Ser Arg Ser Ala Asp Ala Pro Ala Tyr Lys Gln Gly Gln Asn Gln
210 215 220

Leu Tyr Asn Glu Leu Asn Leu Gly Arg Arg Glu Glu Tyr Asp Val Leu
225 230 235 240

Asp Lys Arg Arg Gly Arg Asp Pro Glu Met Gly Gly Lys Pro Arg Arg
245 250 255

Lys Asn Pro Gln Glu Gly Leu Tyr Asn Glu Leu Gln Lys Asp Lys Met
260 265 270

Ala Glu Ala Tyr Ser Glu Ile Gly Met Lys Gly Glu Arg Arg Arg Gly
275 280 285

Lys Gly His Asp Gly Leu Tyr Gln Gly Leu Ser Thr Ala Thr Lys Asp
290 295 300

Thr Tyr Asp Ala Leu His Met Gln Ala Leu Pro Pro Arg
305 310 315

<210> 46
<211> 448
<212> PRT
<213> Artificial Sequence

<220>

<223> synthetic polypeptide

<400> 46

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Ser Arg Gly Ser Gly Ser Gly Ser Gly Ser Met Gly Val Gln Val Glu
    210                 215                 220

Thr Ile Ser Pro Gly Asp Gly Arg Thr Phe Pro Lys Arg Gly Gln Thr
225                 230                 235                 240

Cys Val Val His Tyr Thr Gly Met Leu Glu Asp Gly Lys Lys Val Asp
245 250 255

Ser Ser Arg Asp Arg Asn Lys Pro Phe Lys Phe Met Leu Gly Lys Gln
260 265 270

Glu Val Ile Arg Gly Trp Glu Glu Gly Val Ala Gln Met Ser Val Gly
275 280 285

Gln Arg Ala Lys Leu Thr Ile Ser Pro Asp Tyr Ala Tyr Gly Ala Thr
290 295 300

Gly His Pro Gly Ile Ile Pro Pro His Ala Thr Leu Val Phe Asp Val
305 310 315 320

Glu Leu Leu Lys Leu Glu Gly Ser Gly Ser Gly Ser Gly Ser Ser Leu
325 330 335

Arg Val Lys Phe Ser Arg Ser Ala Asp Ala Pro Ala Tyr Lys Gln Gly
340 345 350

Gln Asn Gln Leu Tyr Asn Glu Leu Asn Leu Gly Arg Arg Glu Glu Tyr
355 360 365

Asp Val Leu Asp Lys Arg Arg Gly Arg Asp Pro Glu Met Gly Gly Lys
370 375 380

Pro Arg Arg Lys Asn Pro Gln Glu Gly Leu Tyr Asn Glu Leu Gln Lys
385 390 395 400

Asp Lys Met Ala Glu Ala Tyr Ser Glu Ile Gly Met Lys Gly Glu Arg
405 410 415

Arg Arg Gly Lys Gly His Asp Gly Leu Tyr Gln Gly Leu Ser Thr Ala
420 425 430

Thr Lys Asp Thr Tyr Asp Ala Leu His Met Gln Ala Leu Pro Pro Arg
435 440 445

<210> 47
<211> 439
<212> PRT
<213> Artificial Sequence

<220>
<223> synthetic polypeptide

<400> 47

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Gly Ser Gly Ser Gly Ser Gly Ser Gly Ser Gly Ser Gly Ser Gly Ser
    210                 215                 220

Ile Leu Trp His Glu Met Trp His Glu Gly Leu Glu Glu Ala Ser Arg
225                 230                 235                 240

Leu Tyr Phe Gly Glu Arg Asn Val Lys Gly Met Phe Glu Val Leu Glu
                245                 250                 255

Pro Leu His Ala Met Met Glu Arg Gly Pro Gln Thr Leu Lys Glu Thr
260 265 270

Ser Phe Asn Gln Ala Tyr Gly Arg Asp Leu Met Glu Ala Gln Glu Trp
275 280 285

Cys Arg Lys Tyr Met Lys Ser Gly Asn Val Lys Asp Leu Leu Gln Ala
290 295 300

Trp Asp Leu Tyr Tyr His Val Phe Arg Arg Ile Ser Lys Gly Ser Gly
305 310 315 320

Ser Gly Ser Gly Ser Ser Leu Arg Val Lys Phe Ser Arg Ser Ala Asp
325 330 335

Ala Pro Ala Tyr Lys Gln Gly Gln Asn Gln Leu Tyr Asn Glu Leu Asn
340 345 350

Leu Gly Arg Arg Glu Glu Tyr Asp Val Leu Asp Lys Arg Arg Gly Arg
355 360 365

Asp Pro Glu Met Gly Gly Lys Pro Arg Arg Lys Asn Pro Gln Glu Gly
370 375 380

Leu Tyr Asn Glu Leu Gln Lys Asp Lys Met Ala Glu Ala Tyr Ser Glu
385 390 395 400

Ile Gly Met Lys Gly Glu Arg Arg Arg Gly Lys Gly His Asp Gly Leu
405 410 415

Tyr Gln Gly Leu Ser Thr Ala Thr Lys Asp Thr Tyr Asp Ala Leu His
420 425 430

Met Gln Ala Leu Pro Pro Arg
435

<210> 48
<211> 447
<212> PRT
<213> Artificial Sequence

<220>
<223> synthetic polypeptide

<400> 48

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asp Tyr Lys Asp Asp Asp Asp Lys
85 90 95

Thr Thr Thr Pro Ala Pro Arg Pro Pro Thr Pro Ala Pro Thr Ile Ala
100 105 110

Ser Gln Pro Leu Ser Leu Arg Pro Glu Ala Ser Arg Pro Ala Ala Gly
115 120 125

Gly Ala Val His Thr Arg Gly Leu Asp Phe Ala Ser Asp Ile Tyr Ile
130 135 140

Trp Ala Pro Leu Ala Gly Thr Cys Gly Val Leu Leu Leu Ser Leu Val
145 150 155 160

Ile Thr Leu Tyr Cys Lys Arg Gly Arg Lys Lys Leu Leu Tyr Ile Phe
165 170 175

Lys Gln Pro Phe Met Arg Pro Val Gln Thr Thr Gln Glu Glu Asp Gly
180 185 190

Cys Ser Cys Arg Phe Pro Glu Glu Glu Glu Gly Gly Cys Glu Leu Ser
195 200 205

Arg Gly Ser Gly Ser Gly Ser Gly Ser Met Gly Val Gln Val Glu Thr
210 215 220

Ile Ser Pro Gly Asp Gly Arg Thr Phe Pro Lys Arg Gly Gln Thr Cys
225 230 235 240

Val Val His Tyr Thr Gly Met Leu Glu Asp Gly Lys Lys Phe Asp Ser
245 250 255

Ser Arg Asp Arg Asn Lys Pro Phe Lys Phe Met Leu Gly Lys Gln Glu
260 265 270

Val Ile Arg Gly Trp Glu Glu Gly Val Ala Gln Met Ser Val Gly Gln
        275                 280                 285

Arg Ala Lys Leu Thr Ile Ser Pro Asp Tyr Ala Tyr Gly Ala Thr Gly
    290                 295                 300

His Pro Gly Ile Ile Pro Pro His Ala Thr Leu Val Phe Asp Val Glu
305                 310                 315                 320

Leu Leu Lys Leu Glu Gly Ser Gly Ser Gly Ser Gly Ser Ser Leu Arg
                325                 330                 335

Val Lys Phe Ser Arg Ser Ala Asp Ala Pro Ala Tyr Lys Gln Gly Gln
            340                 345                 350

Asn Gln Leu Tyr Asn Glu Leu Asn Leu Gly Arg Arg Glu Glu Tyr Asp
        355                 360                 365

Val Leu Asp Lys Arg Arg Gly Arg Asp Pro Glu Met Gly Gly Lys Pro
    370                 375                 380

Arg Arg Lys Asn Pro Gln Glu Gly Leu Tyr Asn Glu Leu Gln Lys Asp
385                 390                 395                 400

Lys Met Ala Glu Ala Tyr Ser Glu Ile Gly Met Lys Gly Glu Arg Arg
                405                 410                 415

Arg Gly Lys Gly His Asp Gly Leu Tyr Gln Gly Leu Ser Thr Ala Thr
            420                 425                 430

Lys Asp Thr Tyr Asp Ala Leu His Met Gln Ala Leu Pro Pro Arg
        435                 440                 445

<210> 49
<211> 448
<212> PRT
<213> Artificial Sequence

<220>
<223> synthetic polypeptide

<400> 49

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

```
Ile Ser Lys Ile Met Tyr Val Ile Arg Gly Gly Gln Arg Ile Ala Phe
        35                  40                  45

Gly Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Ser Arg Gly Ser Gly Ser Gly Ser Gly Ser Met Gly Val Gln Val Glu
    210                 215                 220

Thr Ile Ser Pro Gly Asp Gly Arg Thr Phe Pro Lys Arg Gly Gln Thr
225                 230                 235                 240

Cys Val Val His Tyr Thr Gly Met Leu Glu Asp Gly Lys Lys Val Asp
                245                 250                 255

Ser Ser Arg Asp Arg Asn Lys Pro Phe Lys Phe Met Leu Gly Lys Gln
            260                 265                 270

Glu Val Ile Arg Gly Trp Glu Glu Gly Val Ala Gln Met Ser Val Gly
        275                 280                 285
```

Gln Arg Ala Lys Leu Thr Ile Ser Pro Asp Tyr Ala Tyr Gly Ala Thr
    290                 295                 300

Gly His Pro Gly Ile Ile Pro Pro His Ala Thr Leu Val Phe Asp Val
305                 310                 315                 320

Glu Leu Leu Lys Leu Glu Gly Ser Gly Ser Gly Ser Gly Ser Ser Leu
                325                 330                 335

Arg Val Lys Phe Ser Arg Ser Ala Asp Ala Pro Ala Tyr Lys Gln Gly
            340                 345                 350

Gln Asn Gln Leu Tyr Asn Glu Leu Asn Leu Gly Arg Arg Glu Glu Tyr
        355                 360                 365

Asp Val Leu Asp Lys Arg Arg Gly Arg Asp Pro Glu Met Gly Gly Lys
    370                 375                 380

Pro Arg Arg Lys Asn Pro Gln Glu Gly Leu Tyr Asn Glu Leu Gln Lys
385                 390                 395                 400

Asp Lys Met Ala Glu Ala Tyr Ser Glu Ile Gly Met Lys Gly Glu Arg
                405                 410                 415

Arg Arg Gly Lys Gly His Asp Gly Leu Tyr Gln Gly Leu Ser Thr Ala
            420                 425                 430

Thr Lys Asp Thr Tyr Asp Ala Leu His Met Gln Ala Leu Pro Pro Arg
        435                 440                 445

<210> 50
<211> 447
<212> PRT
<213> Artificial Sequence

<220>
<223> synthetic polypeptide

<400> 50

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

```
Gly Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asp Tyr Lys Asp Asp Asp Asp Lys
                85                  90                  95

Thr Thr Thr Pro Ala Pro Arg Pro Pro Thr Pro Ala Pro Thr Ile Ala
            100                 105                 110

Ser Gln Pro Leu Ser Leu Arg Pro Glu Ala Ser Arg Pro Ala Ala Gly
        115                 120                 125

Gly Ala Val His Thr Arg Gly Leu Asp Phe Ala Ser Asp Ile Tyr Ile
    130                 135                 140

Trp Ala Pro Leu Ala Gly Thr Cys Gly Val Leu Leu Leu Ser Leu Val
145                 150                 155                 160

Ile Thr Leu Tyr Cys Lys Arg Gly Arg Lys Lys Leu Leu Tyr Ile Phe
                165                 170                 175

Lys Gln Pro Phe Met Arg Pro Val Gln Thr Thr Gln Glu Glu Asp Gly
            180                 185                 190

Cys Ser Cys Arg Phe Pro Glu Glu Glu Glu Gly Gly Cys Glu Leu Ser
        195                 200                 205

Arg Gly Ser Gly Ser Gly Ser Gly Ser Met Gly Val Gln Val Glu Thr
    210                 215                 220

Ile Ser Pro Gly Asp Gly Arg Thr Phe Pro Lys Arg Gly Gln Thr Cys
225                 230                 235                 240

Val Val His Tyr Thr Gly Met Leu Glu Asp Gly Lys Lys Phe Asp Ser
                245                 250                 255

Ser Arg Asp Arg Asn Lys Pro Phe Lys Phe Met Leu Gly Lys Gln Glu
            260                 265                 270

Val Ile Arg Gly Trp Glu Glu Gly Val Ala Gln Met Ser Val Gly Gln
        275                 280                 285

Arg Ala Lys Leu Thr Ile Ser Pro Asp Tyr Ala Tyr Gly Ala Thr Gly
    290                 295                 300
```

His Pro Gly Ile Ile Pro Pro His Ala Thr Leu Val Phe Asp Val Glu
305 310 315 320

Leu Leu Lys Leu Glu Gly Ser Gly Ser Gly Ser Gly Ser Ser Leu Arg
325 330 335

Val Lys Phe Ser Arg Ser Ala Asp Ala Pro Ala Tyr Lys Gln Gly Gln
340 345 350

Asn Gln Leu Tyr Asn Glu Leu Asn Leu Gly Arg Arg Glu Glu Tyr Asp
355 360 365

Val Leu Asp Lys Arg Arg Gly Arg Asp Pro Glu Met Gly Gly Lys Pro
370 375 380

Arg Arg Lys Asn Pro Gln Glu Gly Leu Tyr Asn Glu Leu Gln Lys Asp
385 390 395 400

Lys Met Ala Glu Ala Tyr Ser Glu Ile Gly Met Lys Gly Glu Arg Arg
405 410 415

Arg Gly Lys Gly His Asp Gly Leu Tyr Gln Gly Leu Ser Thr Ala Thr
420 425 430

Lys Asp Thr Tyr Asp Ala Leu His Met Gln Ala Leu Pro Pro Arg
435 440 445

<210> 51
<211> 439
<212> PRT
<213> Artificial Sequence

<220>
<223> synthetic polypeptide

<400> 51

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Gly Tyr Asp Glu Gly Asp Gly Ala Trp Gly Asp Gly Ile Val Ser Glu
50 55 60

```
Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Gly Ser Gly Ser Gly Ser Gly Ser Gly Ser Gly Ser Gly Ser Gly Ser
    210                 215                 220

Ile Leu Trp His Glu Met Trp His Glu Gly Leu Glu Glu Ala Ser Arg
225                 230                 235                 240

Leu Tyr Phe Gly Glu Arg Asn Val Lys Gly Met Phe Glu Val Leu Glu
                245                 250                 255

Pro Leu His Ala Met Met Glu Arg Gly Pro Gln Thr Leu Lys Glu Thr
            260                 265                 270

Ser Phe Asn Gln Ala Tyr Gly Arg Asp Leu Met Glu Ala Gln Glu Trp
        275                 280                 285

Cys Arg Lys Tyr Met Lys Ser Gly Asn Val Lys Asp Leu Leu Gln Ala
    290                 295                 300

Trp Asp Leu Tyr Tyr His Val Phe Arg Arg Ile Ser Lys Gly Ser Gly
305                 310                 315                 320
```

Ser Gly Ser Gly Ser Ser Leu Arg Val Lys Phe Ser Arg Ser Ala Asp
325 330 335

Ala Pro Ala Tyr Lys Gln Gly Gln Asn Gln Leu Tyr Asn Glu Leu Asn
340 345 350

Leu Gly Arg Arg Glu Glu Tyr Asp Val Leu Asp Lys Arg Arg Gly Arg
355 360 365

Asp Pro Glu Met Gly Gly Lys Pro Arg Arg Lys Asn Pro Gln Glu Gly
370 375 380

Leu Tyr Asn Glu Leu Gln Lys Asp Lys Met Ala Glu Ala Tyr Ser Glu
385 390 395 400

Ile Gly Met Lys Gly Glu Arg Arg Arg Gly Lys Gly His Asp Gly Leu
405 410 415

Tyr Gln Gly Leu Ser Thr Ala Thr Lys Asp Thr Tyr Asp Ala Leu His
420 425 430

Met Gln Ala Leu Pro Pro Arg
435

<210> 52
<211> 442
<212> PRT
<213> Artificial Sequence

<220>
<223> synthetic polypeptide

<400> 52

Met Pro Leu Leu Leu Leu Leu Pro Leu Leu Trp Ala Gly Ala Leu Ala
1 5 10 15

Met Val Asp Asn Lys Phe Asn Lys Glu Leu Arg Gln Ala Tyr Trp Glu
20 25 30

Ile Gln Ala Leu Pro Asn Leu Ala Trp Thr Gln Ser Arg Ala Phe Ile
35 40 45

Arg Lys Leu Tyr Asp Asp Pro Ser Gln Ser Ala Asn Leu Leu Ala Glu
50 55 60

Ala Lys Lys Leu Asn Asp Ala Gln Ala Pro Lys Gly Gly Gly Gly Ser
65 70 75 80

```
Gly Gly Gly Gly Ser His His His His His His Thr Thr Thr Pro Ala
                85                  90                  95

Pro Arg Pro Pro Thr Pro Ala Pro Thr Ile Ala Ser Gln Pro Leu Ser
            100                 105                 110

Leu Arg Pro Glu Ala Ser Arg Pro Ala Ala Gly Gly Ala Val His Thr
        115                 120                 125

Arg Gly Leu Asp Phe Ala Ser Asp Ile Tyr Ile Trp Ala Pro Leu Ala
    130                 135                 140

Gly Thr Cys Gly Val Leu Leu Leu Ser Leu Val Ile Thr Leu Tyr Cys
145                 150                 155                 160

Lys Arg Gly Arg Lys Lys Leu Leu Tyr Ile Phe Lys Gln Pro Phe Met
                165                 170                 175

Arg Pro Val Gln Thr Thr Gln Glu Glu Asp Gly Cys Ser Cys Arg Phe
            180                 185                 190

Pro Glu Glu Glu Glu Gly Gly Cys Glu Leu Ser Arg Gly Ser Gly Ser
        195                 200                 205

Gly Ser Gly Ser Met Gly Val Gln Val Glu Thr Ile Ser Pro Gly Asp
    210                 215                 220

Gly Arg Thr Phe Pro Lys Arg Gly Gln Thr Cys Val Val His Tyr Thr
225                 230                 235                 240

Gly Met Leu Glu Asp Gly Lys Lys Val Asp Ser Ser Arg Asp Arg Asn
                245                 250                 255

Lys Pro Phe Lys Phe Met Leu Gly Lys Gln Glu Val Ile Arg Gly Trp
            260                 265                 270

Glu Glu Gly Val Ala Gln Met Ser Val Gly Gln Arg Ala Lys Leu Thr
        275                 280                 285

Ile Ser Pro Asp Tyr Ala Tyr Gly Ala Thr Gly His Pro Gly Ile Ile
    290                 295                 300

Pro Pro His Ala Thr Leu Val Phe Asp Val Glu Leu Leu Lys Leu Glu
305                 310                 315                 320

Gly Ser Gly Ser Gly Ser Gly Ser Ser Leu Arg Val Lys Phe Ser Arg
                325                 330                 335
```

Ser Ala Asp Ala Pro Ala Tyr Lys Gln Gly Gln Asn Gln Leu Tyr Asn
340 345 350

Glu Leu Asn Leu Gly Arg Arg Glu Glu Tyr Asp Val Leu Asp Lys Arg
355 360 365

Arg Gly Arg Asp Pro Glu Met Gly Gly Lys Pro Arg Arg Lys Asn Pro
370 375 380

Gln Glu Gly Leu Tyr Asn Glu Leu Gln Lys Asp Lys Met Ala Glu Ala
385 390 395 400

Tyr Ser Glu Ile Gly Met Lys Gly Glu Arg Arg Arg Gly Lys Gly His
405 410 415

Asp Gly Leu Tyr Gln Gly Leu Ser Thr Ala Thr Lys Asp Thr Tyr Asp
420 425 430

Ala Leu His Met Gln Ala Leu Pro Pro Arg
435 440

<210> 53
<211> 482
<212> PRT
<213> Artificial Sequence

<220>
<223> synthetic polypeptide

<400> 53

Met Leu Leu Leu Val Thr Ser Leu Leu Leu Cys Glu Leu Pro His Pro
1 5 10 15

Ala Phe Leu Leu Ile Pro Arg Lys Val Cys Asn Gly Ile Gly Ile Gly
20 25 30

Glu Phe Lys Asp Ser Leu Ser Ile Asn Ala Thr Asn Ile Lys His Phe
35 40 45

Lys Asn Cys Thr Ser Ile Ser Gly Asp Leu His Ile Leu Pro Val Ala
50 55 60

Phe Arg Gly Asp Ser Phe Thr His Thr Pro Pro Leu Asp Pro Gln Glu
65 70 75 80

Leu Asp Ile Leu Lys Thr Val Lys Glu Ile Thr Gly Phe Leu Leu Ile
85 90 95

```
Gln Ala Trp Pro Glu Asn Arg Thr Asp Leu His Ala Phe Glu Asn Leu
            100                 105                 110

Glu Ile Ile Arg Gly Arg Thr Lys Gln His Gly Gln Phe Ser Leu Ala
        115                 120                 125

Val Val Ser Leu Asn Ile Thr Ser Leu Gly Leu Arg Ser Leu Lys Glu
    130                 135                 140

Ile Ser Asp Gly Asp Val Ile Ile Ser Gly Asn Lys Asn Leu Cys Tyr
145                 150                 155                 160

Ala Asn Thr Ile Asn Trp Lys Lys Leu Phe Gly Thr Ser Gly Gln Lys
                165                 170                 175

Thr Lys Ile Ile Ser Asn Arg Gly Glu Asn Ser Cys Lys Ala Thr Gly
            180                 185                 190

Gln Val Cys His Ala Leu Cys Ser Pro Glu Gly Cys Trp Gly Pro Glu
        195                 200                 205

Pro Arg Asp Cys Val Ser Cys Arg Asn Val Ser Arg Gly Arg Glu Cys
    210                 215                 220

Val Asp Lys Cys Lys Leu Leu Glu Gly Glu Pro Arg Glu Phe Val Glu
225                 230                 235                 240

Asn Ser Glu Cys Ile Gln Cys His Pro Glu Cys Leu Pro Gln Ala Met
                245                 250                 255

Asn Ile Thr Cys Thr Gly Arg Gly Pro Asp Asn Cys Ile Gln Cys Ala
            260                 265                 270

His Tyr Ile Asp Gly Pro His Cys Val Lys Thr Cys Pro Ala Gly Val
        275                 280                 285

Met Gly Glu Asn Asn Thr Leu Val Trp Lys Tyr Ala Asp Ala Gly His
    290                 295                 300

Val Cys His Leu Cys His Pro Asn Cys Thr Tyr Gly Cys Thr Gly Pro
305                 310                 315                 320

Gly Leu Glu Gly Cys Pro Thr Asn Gly Pro Lys Ile Pro Ser Ile Ala
                325                 330                 335

Thr Gly Met Val Gly Ala Leu Leu Leu Leu Leu Val Val Ala Leu Gly
            340                 345                 350
```

Ile Gly Leu Phe Met Arg Arg Arg His Ile Val Arg Gly Gly Gly Gly
355 360 365

Ser Gly Gly Gly Gly Ser Met Gly Val Gln Val Glu Thr Ile Ser Pro
370 375 380

Gly Asp Gly Arg Thr Phe Pro Lys Arg Gly Gln Thr Cys Val Val His
385 390 395 400

Tyr Thr Gly Met Leu Glu Asp Gly Lys Lys Val Asp Ser Ser Arg Asp
405 410 415

Arg Asn Lys Pro Phe Lys Phe Met Leu Gly Lys Gln Glu Val Ile Arg
420 425 430

Gly Trp Glu Glu Gly Val Ala Gln Met Ser Val Gly Gln Arg Ala Lys
435 440 445

Leu Thr Ile Ser Pro Asp Tyr Ala Tyr Gly Ala Thr Gly His Pro Gly
450 455 460

Ile Ile Pro Pro His Ala Thr Leu Val Phe Asp Val Glu Leu Leu Lys
465 470 475 480

Leu Glu

<210> 54
<211> 482
<212> PRT
<213> Artificial Sequence

<220>
<223> synthetic polypeptide

<400> 54

Met Leu Leu Leu Val Thr Ser Leu Leu Leu Cys Glu Leu Pro His Pro
1 5 10 15

Ala Phe Leu Leu Ile Pro Arg Lys Val Cys Asn Gly Ile Gly Ile Gly
20 25 30

Glu Phe Lys Asp Ser Leu Ser Ile Asn Ala Thr Asn Ile Lys His Phe
35 40 45

Lys Asn Cys Thr Ser Ile Ser Gly Asp Leu His Ile Leu Pro Val Ala
50 55 60

```
Phe Arg Gly Asp Ser Phe Thr His Thr Pro Pro Leu Asp Pro Gln Glu
65                  70                  75                  80

Leu Asp Ile Leu Lys Thr Val Lys Glu Ile Thr Gly Phe Leu Leu Ile
                85                  90                  95

Gln Ala Trp Pro Glu Asn Arg Thr Asp Leu His Ala Phe Glu Asn Leu
            100                 105                 110

Glu Ile Ile Arg Gly Arg Thr Lys Gln His Gly Gln Phe Ser Leu Ala
        115                 120                 125

Val Val Ser Leu Asn Ile Thr Ser Leu Gly Leu Arg Ser Leu Lys Glu
    130                 135                 140

Ile Ser Asp Gly Asp Val Ile Ile Ser Gly Asn Lys Asn Leu Cys Tyr
145                 150                 155                 160

Ala Asn Thr Ile Asn Trp Lys Lys Leu Phe Gly Thr Ser Gly Gln Lys
                165                 170                 175

Thr Lys Ile Ile Ser Asn Arg Gly Glu Asn Ser Cys Lys Ala Thr Gly
            180                 185                 190

Gln Val Cys His Ala Leu Cys Ser Pro Glu Gly Cys Trp Gly Pro Glu
        195                 200                 205

Pro Arg Asp Cys Val Ser Cys Arg Asn Val Ser Arg Gly Arg Glu Cys
    210                 215                 220

Val Asp Lys Cys Lys Leu Leu Glu Gly Glu Pro Arg Glu Phe Val Glu
225                 230                 235                 240

Asn Ser Glu Cys Ile Gln Cys His Pro Glu Cys Leu Pro Gln Ala Met
                245                 250                 255

Asn Ile Thr Cys Thr Gly Arg Gly Pro Asp Asn Cys Ile Gln Cys Ala
            260                 265                 270

His Tyr Ile Asp Gly Pro His Cys Val Lys Thr Cys Pro Ala Gly Val
        275                 280                 285

Met Gly Glu Asn Asn Thr Leu Val Trp Lys Tyr Ala Asp Ala Gly His
    290                 295                 300

Val Cys His Leu Cys His Pro Asn Cys Thr Tyr Gly Cys Thr Gly Pro
305                 310                 315                 320
```

Gly Leu Glu Gly Cys Pro Thr Asn Gly Pro Lys Ile Pro Ser Ile Ala
325 330 335

Thr Gly Met Val Gly Ala Leu Leu Leu Leu Leu Val Val Ala Leu Gly
340 345 350

Ile Gly Leu Phe Met Arg Arg Arg His Ile Val Arg Gly Gly Gly Gly
355 360 365

Ser Gly Gly Gly Gly Ser Arg Gly Val Gln Val Glu Thr Ile Ser Pro
370 375 380

Gly Asp Gly Arg Thr Phe Pro Lys Arg Gly Gln Thr Cys Val Val His
385 390 395 400

Tyr Thr Gly Met Leu Glu Asp Gly Lys Lys Phe Asp Ser Ser Arg Asp
405 410 415

Arg Asn Lys Pro Phe Lys Phe Met Leu Gly Lys Gln Glu Val Ile Arg
420 425 430

Gly Trp Glu Glu Gly Val Ala Gln Met Ser Val Gly Gln Arg Ala Lys
435 440 445

Leu Thr Ile Ser Pro Asp Tyr Ala Tyr Gly Ala Thr Gly His Pro Gly
450 455 460

Ile Ile Pro Pro His Ala Thr Leu Val Phe Asp Val Glu Leu Leu Lys
465 470 475 480

Leu Glu

<210> 55
<211> 476
<212> PRT
<213> Artificial Sequence

<220>
<223> synthetic polypeptide

<400> 55

Met Leu Leu Leu Val Thr Ser Leu Leu Leu Cys Glu Leu Pro His Pro
1 5 10 15

Ala Phe Leu Leu Ile Pro Ala Arg Val Cys Tyr Gly Leu Gly Met Glu
20 25 30

```
His Leu Arg Glu Val Arg Ala Val Thr Ser Ala Asn Ile Gln Glu Phe
        35                  40                  45

Ala Gly Cys Lys Lys Ile Phe Gly Ser Leu Ala Phe Leu Pro Glu Ser
    50                  55                  60

Phe Asp Gly Asp Pro Ala Ser Asn Thr Ala Pro Leu Gln Pro Glu Gln
65                  70                  75                  80

Leu Gln Val Phe Glu Thr Leu Glu Glu Ile Thr Gly Tyr Leu Tyr Ile
                85                  90                  95

Ser Ala Trp Pro Asp Ser Leu Pro Asp Leu Ser Val Phe Gln Asn Leu
            100                 105                 110

Gln Val Ile Arg Gly Arg Ile Leu His Asn Gly Ala Tyr Ser Leu Thr
        115                 120                 125

Leu Gln Gly Leu Gly Ile Ser Trp Leu Gly Leu Arg Ser Leu Arg Glu
    130                 135                 140

Leu Gly Ser Gly Leu Ala Leu Ile His His Asn Thr His Leu Cys Phe
145                 150                 155                 160

Val His Thr Val Pro Trp Asp Gln Leu Phe Arg Asn Pro His Gln Ala
                165                 170                 175

Leu Leu His Thr Ala Asn Arg Pro Glu Asp Glu Cys Val Gly Glu Gly
            180                 185                 190

Leu Ala Cys His Gln Leu Cys Ala Arg Gly His Cys Trp Gly Pro Gly
        195                 200                 205

Pro Thr Gln Cys Val Asn Cys Ser Gln Phe Leu Arg Gly Gln Glu Cys
    210                 215                 220

Val Glu Glu Cys Arg Val Leu Gln Gly Leu Pro Arg Glu Tyr Val Asn
225                 230                 235                 240

Ala Arg His Cys Leu Pro Cys His Pro Glu Cys Gln Pro Gln Asn Gly
                245                 250                 255

Ser Val Thr Cys Phe Gly Pro Glu Ala Asp Gln Cys Val Ala Cys Ala
            260                 265                 270

His Tyr Lys Asp Pro Pro Phe Cys Val Ala Arg Cys Pro Ser Gly Val
        275                 280                 285
```

Lys Pro Asp Leu Ser Tyr Met Pro Ile Trp Lys Phe Pro Asp Glu Glu
    290                 295                 300

Gly Ala Cys Gln Pro Cys Pro Ile Asn Cys Thr His Ser Cys Val Asp
305                 310                 315                 320

Leu Asp Asp Lys Gly Cys Pro Ala Glu Gln Arg Ala Ser Pro Leu Thr
                325                 330                 335

Ser Ile Ile Ser Ala Val Val Gly Ile Leu Leu Val Val Val Leu Gly
            340                 345                 350

Val Val Phe Gly Ile Leu Ile Lys Arg Arg Gln Gln Lys Ile Arg Gly
        355                 360                 365

Ser Gly Ser Gly Ser Gly Ser Gly Ser Gly Ser Gly Ser Gly Ser Ile
    370                 375                 380

Leu Trp His Glu Met Trp His Glu Gly Leu Glu Glu Ala Ser Arg Leu
385                 390                 395                 400

Tyr Phe Gly Glu Arg Asn Val Lys Gly Met Phe Glu Val Leu Glu Pro
                405                 410                 415

Leu His Ala Met Met Glu Arg Gly Pro Gln Thr Leu Lys Glu Thr Ser
            420                 425                 430

Phe Asn Gln Ala Tyr Gly Arg Asp Leu Met Glu Ala Gln Glu Trp Cys
        435                 440                 445

Arg Lys Tyr Met Lys Ser Gly Asn Val Lys Asp Leu Leu Gln Ala Trp
    450                 455                 460

Asp Leu Tyr Tyr His Val Phe Arg Arg Ile Ser Lys
465                 470                 475

<210> 56
<211> 223
<212> PRT
<213> Artificial Sequence

<220>
<223> synthetic polypeptide

<400> 56

Met Asp Phe Gln Val Gln Ile Phe Ser Phe Leu Leu Ile Ser Ala Ser
1               5                   10                  15

```
Val Ile Met Ser Arg Gly Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Asp Val Asn Thr Ala Val Ala Trp Tyr Gln Gln Lys Pro Gly Lys
    50                  55                  60

Ala Pro Lys Leu Leu Ile Tyr Ser Ala Ser Phe Leu Glu Ser Gly Val
65                  70                  75                  80

Pro Ser Arg Phe Ser Gly Ser Arg Ser Gly Thr Asp Phe Thr Leu Thr
                85                  90                  95

Ile Ser Ser Leu Gln Pro Glu Asp Phe Ala Thr Tyr Tyr Cys Gln Gln
            100                 105                 110

His Tyr Thr Thr Pro Pro Thr Phe Gly Gln Gly Thr Lys Val Glu Ile
        115                 120                 125

Lys Arg Thr Gly Ser Thr Ser Gly Ser Gly Lys Pro Gly Ser Gly Glu
    130                 135                 140

Gly Ser Asp Tyr Lys Asp Asp Asp Asp Lys Thr Thr Thr Pro Ala Pro
145                 150                 155                 160

Arg Pro Pro Thr Pro Ala Pro Thr Ile Ala Ser Gln Pro Leu Ser Leu
                165                 170                 175

Arg Pro Glu Ala Ser Arg Pro Ala Ala Gly Gly Ala Val His Thr Arg
            180                 185                 190

Gly Leu Asp Phe Ala Ser Asp Ile Tyr Ile Trp Ala Pro Leu Ala Gly
        195                 200                 205

Thr Cys Gly Val Leu Leu Leu Ser Leu Val Ile Thr Leu Tyr Cys
    210                 215                 220
```

<210> 57
<211> 499
<212> PRT
<213> Artificial Sequence

<220>
<223> synthetic polypeptide

<400> 57

```
Met Pro Leu Leu Leu Leu Leu Pro Leu Leu Trp Ala Gly Ala Leu Ala
```

```
1               5                   10                  15

Met Glu Val Gln Leu Val Glu Ser Gly Gly Gly Leu Val Gln Pro Gly
            20                  25                  30

Gly Ser Leu Arg Leu Ser Cys Ala Ala Ser Gly Phe Asn Ile Lys Asp
        35                  40                  45

Thr Tyr Ile His Trp Val Arg Gln Ala Pro Gly Lys Gly Leu Glu Trp
    50                  55                  60

Val Ala Arg Ile Tyr Pro Thr Asn Gly Tyr Thr Arg Tyr Ala Asp Ser
65                  70                  75                  80

Val Lys Gly Arg Phe Thr Ile Ser Ala Asp Thr Ser Lys Asn Thr Ala
                85                  90                  95

Tyr Leu Gln Met Asn Ser Leu Arg Ala Glu Asp Thr Ala Val Tyr Tyr
            100                 105                 110

Cys Ser Arg Trp Gly Gly Asp Gly Phe Val Ala Met Asp Val Trp Gly
        115                 120                 125

Gln Gly Thr Leu Val Thr Val Ser Ser Gly Ser Asn Trp Ser His Pro
    130                 135                 140

Gln Phe Glu Lys Thr Thr Thr Pro Ala Pro Arg Pro Pro Thr Pro Ala
145                 150                 155                 160

Pro Thr Ile Ala Ser Gln Pro Leu Ser Leu Arg Pro Glu Ala Ser Arg
                165                 170                 175

Pro Ala Ala Gly Gly Ala Val His Thr Arg Gly Leu Asp Phe Ala Ser
            180                 185                 190

Asp Ile Tyr Ile Trp Ala Pro Leu Ala Gly Thr Cys Gly Val Leu Leu
        195                 200                 205

Leu Ser Leu Val Ile Thr Leu Tyr Cys Lys Arg Gly Arg Lys Lys Leu
    210                 215                 220

Leu Tyr Ile Phe Lys Gln Pro Phe Met Arg Pro Val Gln Thr Thr Gln
225                 230                 235                 240

Glu Glu Asp Gly Cys Ser Cys Arg Phe Pro Glu Glu Glu Glu Gly Gly
                245                 250                 255
```

```
Cys Glu Leu Ser Arg Gly Ser Gly Ser Gly Ser Gly Ser Met Gly Val
            260                 265                 270

Gln Val Glu Thr Ile Ser Pro Gly Asp Gly Arg Thr Phe Pro Lys Arg
        275                 280                 285

Gly Gln Thr Cys Val Val His Tyr Thr Gly Met Leu Glu Asp Gly Lys
    290                 295                 300

Lys Val Asp Ser Ser Arg Asp Arg Asn Lys Pro Phe Lys Phe Met Leu
305                 310                 315                 320

Gly Lys Gln Glu Val Ile Arg Gly Trp Glu Glu Gly Val Ala Gln Met
                325                 330                 335

Ser Val Gly Gln Arg Ala Lys Leu Thr Ile Ser Pro Asp Tyr Ala Tyr
            340                 345                 350

Gly Ala Thr Gly His Pro Gly Ile Ile Pro Pro His Ala Thr Leu Val
        355                 360                 365

Phe Asp Val Glu Leu Leu Lys Leu Glu Gly Ser Gly Ser Gly Ser Gly
    370                 375                 380

Ser Ser Leu Arg Val Lys Phe Ser Arg Ser Ala Asp Ala Pro Ala Tyr
385                 390                 395                 400

Lys Gln Gly Gln Asn Gln Leu Tyr Asn Glu Leu Asn Leu Gly Arg Arg
                405                 410                 415

Glu Glu Tyr Asp Val Leu Asp Lys Arg Arg Gly Arg Asp Pro Glu Met
            420                 425                 430

Gly Gly Lys Pro Arg Arg Lys Asn Pro Gln Glu Gly Leu Tyr Asn Glu
        435                 440                 445

Leu Gln Lys Asp Lys Met Ala Glu Ala Tyr Ser Glu Ile Gly Met Lys
    450                 455                 460

Gly Glu Arg Arg Arg Gly Lys Gly His Asp Gly Leu Tyr Gln Gly Leu
465                 470                 475                 480

Ser Thr Ala Thr Lys Asp Thr Tyr Asp Ala Leu His Met Gln Ala Leu
                485                 490                 495

Pro Pro Arg
```

<210> 58
<211> 493
<212> PRT
<213> Artificial Sequence

<220>
<223> synthetic polypeptide

<400> 58

Met Leu Leu Leu Val Thr Ser Leu Leu Leu Cys Glu Leu Pro His Pro
1 5 10 15

Ala Phe Leu Leu Ile Pro Asp Ile Gln Met Thr Gln Thr Thr Ser Ser
20 25 30

Leu Ser Ala Ser Leu Gly Asp Arg Val Thr Ile Ser Cys Arg Ala Ser
35 40 45

Gln Asp Ile Ser Lys Tyr Leu Asn Trp Tyr Gln Gln Lys Pro Asp Gly
50 55 60

Thr Val Lys Leu Leu Ile Tyr His Thr Ser Arg Leu His Ser Gly Val
65 70 75 80

Pro Ser Arg Phe Ser Gly Ser Gly Ser Gly Thr Asp Tyr Ser Leu Thr
85 90 95

Ile Ser Asn Leu Glu Gln Glu Asp Ile Ala Thr Tyr Phe Cys Gln Gln
100 105 110

Gly Asn Thr Leu Pro Tyr Thr Phe Gly Gly Gly Thr Lys Leu Glu Ile
115 120 125

Thr Gly Ser Thr Ser Gly Ser Gly Lys Pro Gly Ser Gly Glu Gly Ser
130 135 140

Thr Lys Gly Glu Val Lys Leu Gln Glu Ser Gly Pro Gly Leu Val Ala
145 150 155 160

Pro Ser Gln Ser Leu Ser Val Thr Cys Thr Val Ser Gly Val Ser Leu
165 170 175

Pro Asp Tyr Gly Val Ser Trp Ile Arg Gln Pro Pro Arg Lys Gly Leu
180 185 190

Glu Trp Leu Gly Val Ile Trp Gly Ser Glu Thr Thr Tyr Tyr Asn Ser
195 200 205

```
Ala Leu Lys Ser Arg Leu Thr Ile Ile Lys Asp Asn Ser Lys Ser Gln
    210                 215                 220

Val Phe Leu Lys Met Asn Ser Leu Gln Thr Asp Asp Thr Ala Ile Tyr
225                 230                 235                 240

Tyr Cys Ala Lys His Tyr Tyr Tyr Gly Gly Ser Tyr Ala Met Asp Tyr
                245                 250                 255

Trp Gly Gln Gly Thr Ser Val Thr Val Ser Ser Ala Ala Ala Thr Thr
            260                 265                 270

Thr Pro Ala Pro Arg Pro Pro Thr Pro Ala Pro Thr Ile Ala Ser Gln
        275                 280                 285

Pro Leu Ser Leu Arg Pro Glu Ala Cys Arg Pro Ala Ala Gly Gly Ala
    290                 295                 300

Val His Thr Arg Gly Leu Asp Phe Ala Cys Asp Ile Tyr Ile Trp Ala
305                 310                 315                 320

Pro Leu Ala Gly Thr Cys Gly Val Leu Leu Leu Ser Leu Val Ile Thr
                325                 330                 335

Leu Tyr Cys Lys Arg Gly Arg Lys Lys Leu Leu Tyr Ile Phe Lys Gln
            340                 345                 350

Pro Phe Met Arg Pro Val Gln Thr Thr Gln Glu Glu Asp Gly Cys Ser
        355                 360                 365

Cys Arg Phe Pro Glu Glu Glu Glu Gly Gly Cys Glu Leu Arg Val Lys
    370                 375                 380

Phe Ser Arg Ser Ala Asp Ala Pro Ala Tyr Lys Gln Gly Gln Asn Gln
385                 390                 395                 400

Leu Tyr Asn Glu Leu Asn Leu Gly Arg Arg Glu Glu Tyr Asp Val Leu
                405                 410                 415

Asp Lys Arg Arg Gly Arg Asp Pro Glu Met Gly Gly Lys Pro Arg Arg
            420                 425                 430

Lys Asn Pro Gln Glu Gly Leu Tyr Asn Glu Leu Gln Lys Asp Lys Met
        435                 440                 445

Ala Glu Ala Tyr Ser Glu Ile Gly Met Lys Gly Glu Arg Arg Arg Gly
    450                 455                 460
```

Lys Gly His Asp Gly Leu Tyr Gln Gly Leu Ser Thr Ala Thr Lys Asp
465 470 475 480

Thr Tyr Asp Ala Leu His Met Gln Ala Leu Pro Pro Arg
485 490

<210> 59
<211> 510
<212> PRT
<213> Artificial Sequence

<220>
<223> synthetic polypeptide

<400> 59

Met Asp Phe Gln Val Gln Ile Phe Ser Phe Leu Leu Ile Ser Ala Ser
1 5 10 15

Val Ile Met Ser Arg Gly Asp Ile Gln Met Thr Gln Ser Pro Ser Ser
20 25 30

Leu Ser Ala Ser Val Gly Asp Arg Val Thr Ile Thr Cys Arg Ala Ser
35 40 45

Gln Asp Val Asn Thr Ala Val Ala Trp Tyr Gln Gln Lys Pro Gly Lys
50 55 60

Ala Pro Lys Leu Leu Ile Tyr Ser Ala Ser Phe Leu Glu Ser Gly Val
65 70 75 80

Pro Ser Arg Phe Ser Gly Ser Arg Ser Gly Thr Asp Phe Thr Leu Thr
85 90 95

Ile Ser Ser Leu Gln Pro Glu Asp Phe Ala Thr Tyr Tyr Cys Gln Gln
100 105 110

His Tyr Thr Thr Pro Pro Thr Phe Gly Gln Gly Thr Lys Val Glu Ile
115 120 125

Lys Arg Thr Gly Ser Thr Ser Gly Ser Gly Lys Pro Gly Ser Gly Glu
130 135 140

Gly Ser Glu Val Gln Leu Val Glu Ser Gly Gly Gly Leu Val Gln Pro
145 150 155 160

Gly Gly Ser Leu Arg Leu Ser Cys Ala Ala Ser Gly Phe Asn Ile Lys
165 170 175

Asp Thr Tyr Ile His Trp Val Arg Gln Ala Pro Gly Lys Gly Leu Glu
180 185 190

Trp Val Ala Arg Ile Tyr Pro Thr Asn Gly Tyr Thr Arg Tyr Ala Asp
195 200 205

Ser Val Lys Gly Arg Phe Thr Ile Ser Ala Asp Thr Ser Lys Asn Thr
210 215 220

Ala Tyr Leu Gln Met Asn Ser Leu Arg Ala Glu Asp Thr Ala Val Tyr
225 230 235 240

Tyr Cys Ser Arg Trp Gly Gly Asp Gly Phe Val Ala Met Asp Val Trp
245 250 255

Gly Gln Gly Thr Leu Val Thr Val Ser Ser Gly Ser Gly Gly Gly Gly
260 265 270

Ser Gly Gly Gly Gly Ser Asn Trp Ser His Pro Gln Phe Glu Lys Thr
275 280 285

Thr Thr Pro Ala Pro Arg Pro Pro Thr Pro Ala Pro Thr Ile Ala Ser
290 295 300

Gln Pro Leu Ser Leu Arg Pro Glu Ala Cys Arg Pro Ala Ala Gly Gly
305 310 315 320

Ala Val His Thr Arg Gly Leu Asp Phe Ala Cys Asp Ile Tyr Ile Trp
325 330 335

Ala Pro Leu Ala Gly Thr Cys Gly Val Leu Leu Leu Ser Leu Val Ile
340 345 350

Thr Leu Tyr Cys Lys Arg Gly Arg Lys Lys Leu Leu Tyr Ile Phe Lys
355 360 365

Gln Pro Phe Met Arg Pro Val Gln Thr Thr Gln Glu Glu Asp Gly Cys
370 375 380

Ser Cys Arg Phe Pro Glu Glu Glu Glu Gly Gly Cys Glu Leu Arg Val
385 390 395 400

Lys Phe Ser Arg Ser Ala Asp Ala Pro Ala Tyr Lys Gln Gly Gln Asn
405 410 415

Gln Leu Tyr Asn Glu Leu Asn Leu Gly Arg Arg Glu Glu Tyr Asp Val
420 425 430

Leu Asp Lys Arg Arg Gly Arg Asp Pro Glu Met Gly Gly Lys Pro Arg
435 440 445

Arg Lys Asn Pro Gln Glu Gly Leu Tyr Asn Glu Leu Gln Lys Asp Lys
450 455 460

Met Ala Glu Ala Tyr Ser Glu Ile Gly Met Lys Gly Glu Arg Arg Arg
465 470 475 480

Gly Lys Gly His Asp Gly Leu Tyr Gln Gly Leu Ser Thr Ala Thr Lys
485 490 495

Asp Thr Tyr Asp Ala Leu His Met Gln Ala Leu Pro Pro Arg
500 505 510

<210> 60
<211> 510
<212> PRT
<213> Artificial Sequence

<220>
<223> synthetic polypeptide

<400> 60

Met Asp Phe Gln Val Gln Ile Phe Ser Phe Leu Leu Ile Ser Ala Ser
1 5 10 15

Val Ile Met Ser Arg Gly Asp Ile Gln Met Thr Gln Ser Pro Ser Ser
20 25 30

Leu Ser Ala Ser Val Gly Asp Arg Val Thr Ile Thr Cys Arg Ala Ser
35 40 45

Gln Asp Val Asn Thr Ala Val Ala Trp Tyr Gln Gln Lys Pro Gly Lys
50 55 60

Ala Pro Lys Leu Leu Ile Tyr Ser Ala Ser Phe Leu Glu Ser Gly Val
65 70 75 80

Pro Ser Arg Phe Ser Gly Ser Arg Ser Gly Thr Asp Phe Thr Leu Thr
85 90 95

Ile Ser Ser Leu Gln Pro Glu Asp Phe Ala Thr Tyr Tyr Cys Gln Gln
100 105 110

His Tyr Thr Thr Pro Pro Thr Phe Gly Gln Gly Thr Lys Val Glu Ile
115 120 125

Lys Arg Thr Gly Ser Thr Ser Gly Ser Gly Lys Pro Gly Ser Gly Glu
    130                 135                 140

Gly Ser Glu Val Gln Leu Val Glu Ser Gly Gly Gly Leu Val Gln Pro
145                 150                 155                 160

Gly Gly Ser Leu Arg Leu Ser Cys Ala Ala Ser Gly Phe Asn Ile Lys
                165                 170                 175

Asp Thr Tyr Ile His Trp Val Arg Gln Ala Pro Gly Lys Gly Leu Glu
            180                 185                 190

Trp Val Ala Arg Ile Tyr Pro Thr Asn Gly Tyr Thr Arg Tyr Ala Asp
        195                 200                 205

Ser Val Lys Gly Arg Phe Thr Ile Ser Ala Asp Thr Ser Lys Asn Thr
    210                 215                 220

Ala Tyr Leu Gln Met Asn Ser Leu Arg Ala Glu Asp Thr Ala Val Tyr
225                 230                 235                 240

Tyr Cys Ser Arg Trp Gly Gly Asp Gly Phe Val Ala Met Asp Val Trp
                245                 250                 255

Gly Gln Gly Thr Leu Val Thr Val Ser Ser Gly Ser Gly Gly Gly Gly
            260                 265                 270

Ser Gly Gly Gly Gly Ser Asn Trp Ser His Pro Gln Phe Glu Lys Thr
        275                 280                 285

Thr Thr Pro Ala Pro Arg Pro Pro Thr Pro Ala Pro Thr Ile Ala Ser
    290                 295                 300

Gln Pro Leu Ser Leu Arg Pro Glu Ala Ser Arg Pro Ala Ala Gly Gly
305                 310                 315                 320

Ala Val His Thr Arg Gly Leu Asp Phe Ala Ser Asp Ile Tyr Ile Trp
                325                 330                 335

Ala Pro Leu Ala Gly Thr Cys Gly Val Leu Leu Leu Ser Leu Val Ile
            340                 345                 350

Thr Leu Tyr Cys Lys Arg Gly Arg Lys Lys Leu Leu Tyr Ile Phe Lys
        355                 360                 365

Gln Pro Phe Met Arg Pro Val Gln Thr Thr Gln Glu Glu Asp Gly Cys
    370                 375                 380

Ser Cys Arg Phe Pro Glu Glu Glu Glu Gly Gly Cys Glu Leu Arg Val
385 390 395 400

Lys Phe Ser Arg Ser Ala Asp Ala Pro Ala Tyr Lys Gln Gly Gln Asn
405 410 415

Gln Leu Tyr Asn Glu Leu Asn Leu Gly Arg Arg Glu Glu Tyr Asp Val
420 425 430

Leu Asp Lys Arg Arg Gly Arg Asp Pro Glu Met Gly Gly Lys Pro Arg
435 440 445

Arg Lys Asn Pro Gln Glu Gly Leu Tyr Asn Glu Leu Gln Lys Asp Lys
450 455 460

Met Ala Glu Ala Tyr Ser Glu Ile Gly Met Lys Gly Glu Arg Arg Arg
465 470 475 480

Gly Lys Gly His Asp Gly Leu Tyr Gln Gly Leu Ser Thr Ala Thr Lys
485 490 495

Asp Thr Tyr Asp Ala Leu His Met Gln Ala Leu Pro Pro Arg
500 505 510

<210> 61
<211> 320
<212> PRT
<213> Artificial Sequence

<220>
<223> synthetic polypeptide

<400> 61

Met Ser His His His His His His Gly Ser Ala Thr Val Lys Phe Thr
1 5 10 15

Tyr Gln Gly Glu Glu Lys Gln Val Asp Ile Ser Lys Ile Met Tyr Val
20 25 30

Ile Arg Gly Gly Gln Arg Ile Ala Phe Gly Tyr Asp Glu Gly Asp Gly
35 40 45

Ala Trp Gly Asp Gly Ile Val Ser Glu Lys Asp Ala Pro Lys Glu Leu
50 55 60

Leu Gln Met Leu Glu Lys Gln Gly Gly Gly Gly Ser Gly Gly Gly Gly
65 70 75 80

Ser Met Val Ser Lys Gly Glu Glu Leu Phe Thr Gly Val Val Pro Ile
85 90 95

Leu Val Glu Leu Asp Gly Asp Val Asn Gly His Lys Phe Ser Val Arg
100 105 110

Gly Glu Gly Glu Gly Asp Ala Thr Asn Gly Lys Leu Thr Leu Lys Phe
115 120 125

Ile Cys Thr Thr Gly Lys Leu Pro Val Pro Trp Pro Thr Leu Val Thr
130 135 140

Thr Leu Thr Tyr Gly Val Gln Cys Phe Ser Arg Tyr Pro Asp His Met
145 150 155 160

Lys Arg His Asp Phe Phe Lys Ser Ala Met Pro Glu Gly Tyr Val Gln
165 170 175

Glu Arg Thr Ile Ser Phe Lys Asp Asp Gly Thr Tyr Lys Thr Arg Ala
180 185 190

Glu Val Lys Phe Glu Gly Asp Thr Leu Val Asn Arg Ile Glu Leu Lys
195 200 205

Gly Ile Asp Phe Lys Glu Asp Gly Asn Ile Leu Gly His Lys Leu Glu
210 215 220

Tyr Asn Phe Asn Ser His Asn Val Tyr Ile Thr Ala Asp Lys Gln Lys
225 230 235 240

Asn Gly Ile Lys Ala Asn Phe Lys Ile Arg His Asn Val Glu Asp Gly
245 250 255

Ser Val Gln Leu Ala Asp His Tyr Gln Gln Asn Thr Pro Ile Gly Asp
260 265 270

Gly Pro Val Leu Leu Pro Asp Asn His Tyr Leu Ser Thr Gln Ser Val
275 280 285

Leu Ser Lys Asp Pro Asn Glu Lys Arg Asp His Met Val Leu Leu Glu
290 295 300

Phe Val Thr Ala Ala Gly Ile Thr His Gly Met Asp Glu Leu Tyr Lys
305 310 315 320

<210> 62
<211> 317
<212> PRT

<213> Artificial Sequence

<220>
<223> synthetic polypeptide

<400> 62

```
Met Gly His His His His His His Gly Ser Val Asp Asn Lys Phe Asn
1               5                   10                  15

Lys Glu Leu Arg Gln Ala Tyr Trp Glu Ile Gln Ala Leu Pro Asn Leu
            20                  25                  30

Ala Trp Thr Gln Ser Arg Ala Phe Ile Arg Lys Leu Tyr Asp Asp Pro
        35                  40                  45

Ser Gln Ser Ala Asn Leu Leu Ala Glu Ala Lys Lys Leu Asn Asp Ala
    50                  55                  60

Gln Ala Pro Lys Gly Gly Gly Gly Ser Gly Gly Gly Gly Ser Met Val
65                  70                  75                  80

Ser Lys Gly Glu Glu Leu Phe Thr Gly Val Val Pro Ile Leu Val Glu
                85                  90                  95

Leu Asp Gly Asp Val Asn Gly His Lys Phe Ser Val Arg Gly Glu Gly
            100                 105                 110

Glu Gly Asp Ala Thr Asn Gly Lys Leu Thr Leu Lys Phe Ile Cys Thr
        115                 120                 125

Thr Gly Lys Leu Pro Val Pro Trp Pro Thr Leu Val Thr Thr Leu Thr
    130                 135                 140

Tyr Gly Val Gln Cys Phe Ser Arg Tyr Pro Asp His Met Lys Arg His
145                 150                 155                 160

Asp Phe Phe Lys Ser Ala Met Pro Glu Gly Tyr Val Gln Glu Arg Thr
                165                 170                 175

Ile Ser Phe Lys Asp Asp Gly Thr Tyr Lys Thr Arg Ala Glu Val Lys
            180                 185                 190

Phe Glu Gly Asp Thr Leu Val Asn Arg Ile Glu Leu Lys Gly Ile Asp
        195                 200                 205

Phe Lys Glu Asp Gly Asn Ile Leu Gly His Lys Leu Glu Tyr Asn Phe
    210                 215                 220
```

Asn Ser His Asn Val Tyr Ile Thr Ala Asp Lys Gln Lys Asn Gly Ile
225 230 235 240

Lys Ala Asn Phe Lys Ile Arg His Asn Val Glu Asp Gly Ser Val Gln
245 250 255

Leu Ala Asp His Tyr Gln Gln Asn Thr Pro Ile Gly Asp Gly Pro Val
260 265 270

Leu Leu Pro Asp Asn His Tyr Leu Ser Thr Gln Ser Val Leu Ser Lys
275 280 285

Asp Pro Asn Glu Lys Arg Asp His Met Val Leu Leu Glu Phe Val Thr
290 295 300

Ala Ala Gly Ile Thr His Gly Met Asp Glu Leu Tyr Lys
305 310 315

<210> 63
<211> 334
<212> PRT
<213> Artificial Sequence

<220>
<223> synthetic polypeptide

<400> 63

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asn Trp Ser His Pro Gln Phe Glu
85 90 95

Lys Glu Pro Lys Ser Pro Asp Lys Thr His Thr Cys Pro Pro Cys Pro
100 105 110

Ala Pro Glu Leu Leu Gly Gly Pro Ser Val Phe Leu Phe Pro Pro Lys

115 120 125

Pro Lys Asp Thr Leu Met Ile Ser Arg Thr Pro Glu Val Thr Cys Val
130 135 140

Val Val Asp Val Ser His Glu Asp Pro Glu Val Lys Phe Asn Trp Tyr
145 150 155 160

Val Asp Gly Val Glu Val His Asn Ala Lys Thr Lys Pro Arg Glu Glu
165 170 175

Gln Tyr Asn Ser Thr Tyr Arg Val Val Ser Val Leu Thr Val Leu His
180 185 190

Gln Asp Trp Leu Asn Gly Lys Glu Tyr Lys Cys Lys Val Ser Asn Lys
195 200 205

Ala Leu Pro Ala Pro Ile Glu Lys Thr Ile Ser Lys Ala Lys Gly Gln
210 215 220

Pro Arg Glu Pro Gln Val Tyr Val Tyr Pro Pro Ser Arg Asp Glu Leu
225 230 235 240

Arg Phe Tyr Gln Val Ser Leu Thr Cys Leu Val Lys Gly Phe Tyr Pro
245 250 255

Ser Asp Ile Ala Val Glu Trp Glu Ser Asn Gly Gln Pro Asp Ile Phe
260 265 270

Pro Asn Gly Leu Asn Tyr Lys Thr Thr Pro Pro Val Leu Asp Ser Asp
275 280 285

Gly Ser Phe Ala Leu Val Ser Lys Leu Thr Val Pro Tyr Pro Ser Trp
290 295 300

Leu Met Gly Thr Arg Phe Ser Cys Ser Val Met His Glu Ala Leu His
305 310 315 320

Asn His Tyr Thr Gln Lys His Leu Glu Tyr Gln Trp Pro Thr
325 330

<210> 64
<211> 324
<212> PRT
<213> Artificial Sequence

<220>
<223> synthetic polypeptide

<400> 64

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Asn Trp
65                  70                  75                  80

Ser His Pro Gln Phe Glu Lys Glu Pro Lys Ser Pro Asp Lys Thr His
                85                  90                  95

Thr Cys Pro Pro Cys Pro Ala Pro Glu Leu Leu Gly Gly Pro Ser Val
            100                 105                 110

Phe Leu Phe Pro Pro Lys Pro Lys Asp Thr Leu Met Ile Ser Arg Thr
        115                 120                 125

Pro Glu Val Thr Cys Val Val Val Asp Val Ser His Glu Asp Pro Glu
    130                 135                 140

Val Lys Phe Asn Trp Tyr Val Asp Gly Val Glu Val His Asn Ala Lys
145                 150                 155                 160

Thr Lys Pro Arg Glu Glu Gln Tyr Asn Ser Thr Tyr Arg Val Val Ser
                165                 170                 175

Val Leu Thr Val Leu His Gln Asp Trp Leu Asn Gly Lys Glu Tyr Lys
            180                 185                 190

Cys Lys Val Ser Asn Lys Ala Leu Pro Ala Pro Ile Glu Lys Thr Ile
        195                 200                 205

Ser Lys Ala Lys Gly Gln Pro Arg Glu Pro Gln Val Tyr Val Tyr Pro
    210                 215                 220

Pro Ser Arg Asp Glu Leu Arg Phe Tyr Gln Val Ser Leu Thr Cys Leu
225                 230                 235                 240

Val Lys Gly Phe Tyr Pro Ser Asp Ile Ala Val Glu Trp Glu Ser Asn

245 250 255

Gly Gln Pro Asp Ile Phe Pro Asn Gly Leu Asn Tyr Lys Thr Thr Pro
260 265 270

Pro Val Leu Asp Ser Asp Gly Ser Phe Ala Leu Val Ser Lys Leu Thr
275 280 285

Val Pro Tyr Pro Ser Trp Leu Met Gly Thr Arg Phe Ser Cys Ser Val
290 295 300

Met His Glu Ala Leu His Asn His Tyr Thr Gln Lys His Leu Glu Tyr
305 310 315 320

Gln Trp Pro Thr

<210> 65
<211> 444
<212> PRT
<213> Artificial Sequence

<220>
<223> synthetic polypeptide

<400> 65

Met Glu Leu Gly Leu Ser Trp Ile Phe Leu Leu Ala Ile Leu Lys Gly
1 5 10 15

Val Gln Cys Thr Cys Pro Pro Cys Pro Ala Pro Glu Leu Leu Gly Gly
20 25 30

Pro Ser Val Phe Leu Phe Pro Pro Lys Pro Lys Asp Thr Leu Met Ile
35 40 45

Ser Arg Thr Pro Glu Val Thr Cys Val Val Val Asp Val Ser His Glu
50 55 60

Asp Pro Glu Val Lys Phe Asn Trp Tyr Val Asp Gly Val Glu Val His
65 70 75 80

Asn Ala Lys Thr Lys Pro Arg Glu Glu Gln Tyr Asn Ser Thr Tyr Arg
85 90 95

Val Val Ser Val Leu Thr Val Leu His Gln Asp Trp Leu Asn Gly Lys
100 105 110

Glu Tyr Lys Cys Lys Val Ser Asn Lys Ala Leu Pro Ala Pro Ile Glu
115 120 125

```
Lys Thr Ile Ser Lys Ala Lys Gly Gln Pro Arg Glu Pro Gln Val Tyr
    130                 135                 140

Val Leu Pro Pro Ser Arg Asp Glu Leu Thr Lys Asn Gln Val Ser Leu
145                 150                 155                 160

Leu Cys Leu Val Lys Gly Phe Tyr Pro Ser Asp Ile Ala Val Glu Trp
                165                 170                 175

Glu Ser Asn Gly Gln Pro Glu Asn Asn Tyr Leu Thr Trp Pro Pro Val
            180                 185                 190

Leu Asp Ser Asp Gly Ser Phe Phe Leu Tyr Ser Lys Leu Thr Val Asp
        195                 200                 205

Lys Ser Arg Trp Gln Gln Gly Asn Val Phe Ser Cys Ser Val Met His
    210                 215                 220

Glu Ala Leu His Asn His Tyr Thr Gln Lys Ser Leu Ser Leu Ser Pro
225                 230                 235                 240

Gly Lys Leu Arg Pro Glu Ala Ser Arg Pro Ala Ala Gly Gly Ala Val
                245                 250                 255

His Thr Arg Gly Leu Asp Phe Ala Ser Asp Ile Tyr Ile Trp Ala Pro
            260                 265                 270

Leu Ala Gly Thr Cys Gly Val Leu Leu Leu Ser Leu Val Ile Thr Leu
        275                 280                 285

Tyr Cys Lys Arg Gly Arg Lys Lys Leu Leu Tyr Ile Phe Lys Gln Pro
    290                 295                 300

Phe Met Arg Pro Val Gln Thr Thr Gln Glu Glu Asp Gly Cys Ser Cys
305                 310                 315                 320

Arg Phe Pro Glu Glu Glu Glu Gly Gly Cys Glu Leu Arg Val Lys Phe
                325                 330                 335

Ser Arg Ser Ala Asp Ala Pro Ala Tyr Lys Gln Gly Gln Asn Gln Leu
            340                 345                 350

Tyr Asn Glu Leu Asn Leu Gly Arg Arg Glu Glu Tyr Asp Val Leu Asp
        355                 360                 365

Lys Arg Arg Gly Arg Asp Pro Glu Met Gly Gly Lys Pro Arg Arg Lys
```

370 375 380

Asn Pro Gln Glu Gly Leu Tyr Asn Glu Leu Gln Lys Asp Lys Met Ala
385 390 395 400

Glu Ala Tyr Ser Glu Ile Gly Met Lys Gly Glu Arg Arg Arg Gly Lys
405 410 415

Gly His Asp Gly Leu Tyr Gln Gly Leu Ser Thr Ala Thr Lys Asp Thr
420 425 430

Tyr Asp Ala Leu His Met Gln Ala Leu Pro Pro Arg
435 440

<210> 66
<211> 432
<212> PRT
<213> Artificial Sequence

<220>
<223> synthetic polypeptide

<400> 66

Met Glu Leu Gly Leu Ser Trp Ile Phe Leu Leu Ala Ile Leu Lys Gly
1 5 10 15

Val Gln Cys Thr Cys Pro Pro Cys Pro Ala Pro Glu Leu Leu Gly Gly
20 25 30

Pro Ser Val Phe Leu Phe Pro Pro Lys Pro Lys Asp Thr Leu Met Ile
35 40 45

Ser Arg Thr Pro Glu Val Thr Cys Val Val Val Asp Val Ser His Glu
50 55 60

Asp Pro Glu Val Lys Phe Asn Trp Tyr Val Asp Gly Val Glu Val His
65 70 75 80

Asn Ala Lys Thr Lys Pro Arg Glu Glu Gln Tyr Asn Ser Thr Tyr Arg
85 90 95

Val Val Ser Val Leu Thr Val Leu His Gln Asp Trp Leu Asn Gly Lys
100 105 110

Glu Tyr Lys Cys Lys Val Ser Asn Lys Ala Leu Pro Ala Pro Ile Glu
115 120 125

Lys Thr Ile Ser Lys Ala Lys Gly Gln Pro Arg Glu Pro Gln Val Tyr
130 135 140

Val Leu Pro Pro Ser Arg Asp Glu Leu Thr Lys Asn Gln Val Ser Leu
145 150 155 160

Leu Cys Leu Val Lys Gly Phe Tyr Pro Ser Asp Ile Ala Val Glu Trp
165 170 175

Glu Ser Asn Gly Gln Pro Glu Asn Asn Tyr Leu Thr Trp Pro Pro Val
180 185 190

Leu Asp Ser Asp Gly Ser Phe Phe Leu Tyr Ser Lys Leu Thr Val Asp
195 200 205

Lys Ser Arg Trp Gln Gln Gly Asn Val Phe Ser Cys Ser Val Met His
210 215 220

Glu Ala Leu His Asn His Tyr Thr Gln Lys Ser Leu Ser Leu Ser Pro
225 230 235 240

Gly Lys Ala Val His Thr Arg Gly Leu Asp Phe Ala Ser Asp Ile Tyr
245 250 255

Ile Trp Ala Pro Leu Ala Gly Thr Cys Gly Val Leu Leu Leu Ser Leu
260 265 270

Val Ile Thr Leu Tyr Cys Lys Arg Gly Arg Lys Lys Leu Leu Tyr Ile
275 280 285

Phe Lys Gln Pro Phe Met Arg Pro Val Gln Thr Thr Gln Glu Glu Asp
290 295 300

Gly Cys Ser Cys Arg Phe Pro Glu Glu Glu Glu Gly Gly Cys Glu Leu
305 310 315 320

Arg Val Lys Phe Ser Arg Ser Ala Asp Ala Pro Ala Tyr Lys Gln Gly
325 330 335

Gln Asn Gln Leu Tyr Asn Glu Leu Asn Leu Gly Arg Arg Glu Glu Tyr
340 345 350

Asp Val Leu Asp Lys Arg Arg Gly Arg Asp Pro Glu Met Gly Gly Lys
355 360 365

Pro Arg Arg Lys Asn Pro Gln Glu Gly Leu Tyr Asn Glu Leu Gln Lys
370 375 380

Asp Lys Met Ala Glu Ala Tyr Ser Glu Ile Gly Met Lys Gly Glu Arg

385 390 395 400

Arg Arg Gly Lys Gly His Asp Gly Leu Tyr Gln Gly Leu Ser Thr Ala
405 410 415

Thr Lys Asp Thr Tyr Asp Ala Leu His Met Gln Ala Leu Pro Pro Arg
420 425 430

<210> 67
<211> 436
<212> PRT
<213> Artificial Sequence

<220>
<223> synthetic polypeptide

<400> 67

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asn Trp Ser His Pro Gln Phe Glu
85 90 95

Lys Thr Thr Thr Pro Ala Pro Arg Pro Pro Thr Pro Ala Pro Thr Ile
100 105 110

Ala Ser Gln Pro Leu Ser Leu Arg Pro Glu Ala Ser Arg Pro Ala Ala
115 120 125

Gly Gly Ala Val His Thr Arg Gly Leu Asp Phe Ala Ser Asp Ile Tyr
130 135 140

Ile Trp Ala Pro Leu Ala Gly Thr Cys Gly Val Leu Leu Leu Ser Leu
145 150 155 160

Val Ile Thr Leu Tyr Cys Lys Arg Gly Arg Lys Lys Leu Leu Tyr Ile
165 170 175

```
Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Gly Ser Gly Ser Gly Ser Gly Ser Gly Ser Gly Ser Gly Ser Gly Ser
    210                 215                 220

Ile Leu Trp His Glu Met Trp His Glu Gly Leu Glu Glu Ala Ser Arg
225                 230                 235                 240

Leu Tyr Phe Gly Glu Arg Asn Val Lys Gly Met Phe Glu Val Leu Glu
                245                 250                 255

Pro Leu His Ala Met Met Glu Arg Gly Pro Gln Thr Leu Lys Glu Thr
            260                 265                 270

Ser Phe Asn Gln Ala Tyr Gly Arg Asp Leu Met Glu Ala Gln Glu Trp
        275                 280                 285

Cys Arg Lys Tyr Met Lys Ser Gly Asn Val Lys Asp Leu Leu Gln Ala
    290                 295                 300

Trp Asp Leu Tyr Tyr His Val Phe Arg Arg Ile Ser Lys Gly Ser Gly
305                 310                 315                 320

Ser Gly Ser Gly Ser Gly Ser Gly Ser Gly Ser Gly Ser Ile Leu Trp
                325                 330                 335

His Glu Met Trp His Glu Gly Leu Glu Glu Ala Ser Arg Leu Tyr Phe
            340                 345                 350

Gly Glu Arg Asn Val Lys Gly Met Phe Glu Val Leu Glu Pro Leu His
        355                 360                 365

Ala Met Met Glu Arg Gly Pro Gln Thr Leu Lys Glu Thr Ser Phe Asn
    370                 375                 380

Gln Ala Tyr Gly Arg Asp Leu Met Glu Ala Gln Glu Trp Cys Arg Lys
385                 390                 395                 400

Tyr Met Lys Ser Gly Asn Val Lys Asp Leu Leu Gln Ala Trp Asp Leu
                405                 410                 415

Tyr Tyr His Val Phe Arg Arg Ile Ser Lys Gly Ser Gly Ser Gly Ser
```

420 425 430

Gly Ser Ser Leu
435

<210> 68
<211> 435
<212> PRT
<213> Artificial Sequence

<220>
<223> synthetic polypeptide

<400> 68

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Ala Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asn Trp Ser His Pro Gln Phe Glu
85 90 95

Lys Thr Thr Thr Pro Ala Pro Arg Pro Pro Thr Pro Ala Pro Thr Ile
100 105 110

Ala Ser Gln Pro Leu Ser Leu Arg Pro Glu Ala Ser Arg Pro Ala Ala
115 120 125

Gly Gly Ala Val His Thr Arg Gly Leu Asp Phe Ala Ser Asp Ile Tyr
130 135 140

Ile Trp Ala Pro Leu Ala Gly Thr Cys Gly Val Leu Leu Leu Ser Leu
145 150 155 160

Val Ile Thr Leu Tyr Cys Lys Arg Gly Arg Lys Lys Leu Leu Tyr Ile
165 170 175

Phe Lys Gln Pro Phe Met Arg Pro Val Gln Thr Thr Gln Glu Glu Asp
180 185 190

```
Gly Cys Ser Cys Arg Phe Pro Glu Glu Glu Glu Gly Gly Cys Glu Leu
        195                 200                 205

Gly Ser Gly Ser Gly Ser Gly Ser Gly Ser Gly Ser Gly Ser Gly Ser
    210                 215                 220

Ile Leu Trp His Glu Met Trp His Glu Gly Leu Glu Glu Ala Ser Arg
225                 230                 235                 240

Leu Tyr Phe Gly Glu Arg Asn Val Lys Gly Met Phe Glu Val Leu Glu
                245                 250                 255

Pro Leu His Ala Met Met Glu Arg Gly Pro Gln Thr Leu Lys Glu Thr
            260                 265                 270

Ser Phe Asn Gln Ala Tyr Gly Arg Asp Leu Met Glu Ala Gln Glu Trp
        275                 280                 285

Cys Arg Lys Tyr Met Lys Ser Gly Asn Val Lys Asp Leu Leu Gln Ala
    290                 295                 300

Trp Asp Leu Tyr Tyr His Val Phe Arg Arg Ile Ser Lys Gly Ser Gly
305                 310                 315                 320

Ser Gly Ser Gly Ser Ser Leu Met Gly Val Gln Val Glu Thr Ile Ser
                325                 330                 335

Pro Gly Asp Gly Arg Thr Phe Pro Lys Arg Gly Gln Thr Cys Val Val
            340                 345                 350

His Tyr Thr Gly Met Leu Glu Asp Gly Lys Lys Val Asp Ser Ser Arg
        355                 360                 365

Asp Arg Asn Lys Pro Phe Lys Phe Met Leu Gly Lys Gln Glu Val Ile
    370                 375                 380

Arg Gly Trp Glu Glu Gly Val Ala Gln Met Ser Val Gly Gln Arg Ala
385                 390                 395                 400

Lys Leu Thr Ile Ser Pro Asp Tyr Ala Tyr Gly Ala Thr Gly His Pro
                405                 410                 415

Gly Ile Ile Pro Pro His Ala Thr Leu Val Phe Asp Val Glu Leu Leu
            420                 425                 430

Lys Leu Glu
```

435

<210> 69
<211> 450
<212> PRT
<213> Artificial Sequence

<220>
<223> synthetic polypeptide

<400> 69

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Phe Trp
    130                 135                 140

Val Leu Val Val Val Gly Gly Val Leu Ala Cys Tyr Ser Leu Leu Val
145                 150                 155                 160

Thr Val Ala Phe Ile Ile Phe Trp Val Arg Ser Lys Arg Ser Arg Leu
                165                 170                 175

Leu His Ser Asp Tyr Met Asn Met Thr Pro Arg Arg Pro Gly Pro Thr
            180                 185                 190

Arg Lys His Tyr Gln Pro Tyr Ala Pro Pro Arg Asp Phe Ala Ala Tyr
        195                 200                 205

```
Arg Ser Ser Arg Gly Ser Gly Ser Gly Ser Gly Ser Met Gly Val Gln
    210                 215                 220

Val Glu Thr Ile Ser Pro Gly Asp Gly Arg Thr Phe Pro Lys Arg Gly
225                 230                 235                 240

Gln Thr Cys Val Val His Tyr Thr Gly Met Leu Glu Asp Gly Lys Lys
                245                 250                 255

Val Asp Ser Ser Arg Asp Arg Asn Lys Pro Phe Lys Phe Met Leu Gly
            260                 265                 270

Lys Gln Glu Val Ile Arg Gly Trp Glu Glu Gly Val Ala Gln Met Ser
        275                 280                 285

Val Gly Gln Arg Ala Lys Leu Thr Ile Ser Pro Asp Tyr Ala Tyr Gly
    290                 295                 300

Ala Thr Gly His Pro Gly Ile Ile Pro Pro His Ala Thr Leu Val Phe
305                 310                 315                 320

Asp Val Glu Leu Leu Lys Leu Glu Gly Ser Gly Ser Gly Ser Gly Ser
                325                 330                 335

Ser Leu Arg Val Lys Phe Ser Arg Ser Ala Asp Ala Pro Ala Tyr Lys
            340                 345                 350

Gln Gly Gln Asn Gln Leu Tyr Asn Glu Leu Asn Leu Gly Arg Arg Glu
        355                 360                 365

Glu Tyr Asp Val Leu Asp Lys Arg Arg Gly Arg Asp Pro Glu Met Gly
    370                 375                 380

Gly Lys Pro Arg Arg Lys Asn Pro Gln Glu Gly Leu Tyr Asn Glu Leu
385                 390                 395                 400

Gln Lys Asp Lys Met Ala Glu Ala Tyr Ser Glu Ile Gly Met Lys Gly
                405                 410                 415

Glu Arg Arg Arg Gly Lys Gly His Asp Gly Leu Tyr Gln Gly Leu Ser
            420                 425                 430

Thr Ala Thr Lys Asp Thr Tyr Asp Ala Leu His Met Gln Ala Leu Pro
        435                 440                 445

Pro Arg
```

450

<210> 70
<211> 445
<212> PRT
<213> Artificial Sequence

<220>
<223> synthetic polypeptide

<400> 70

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Phe Glu
    130                 135                 140

Phe Trp Leu Pro Ile Gly Cys Ala Ala Phe Val Val Val Cys Ile Leu
145                 150                 155                 160

Gly Cys Ile Leu Ile Cys Trp Leu Thr Lys Lys Lys Tyr Ser Ser Ser
                165                 170                 175

Val His Asp Pro Asn Gly Glu Tyr Met Phe Met Arg Ala Val Asn Thr
            180                 185                 190

Ala Lys Lys Ser Arg Leu Thr Asp Val Thr Leu Thr Ser Ser Arg Gly
        195                 200                 205

Ser Gly Ser Gly Ser Gly Ser Met Gly Val Gln Val Glu Thr Ile Ser
    210                 215                 220

Pro Gly Asp Gly Arg Thr Phe Pro Lys Arg Gly Gln Thr Cys Val Val
225                 230                 235                 240

His Tyr Thr Gly Met Leu Glu Asp Gly Lys Lys Val Asp Ser Ser Arg
                245                 250                 255

Asp Arg Asn Lys Pro Phe Lys Phe Met Leu Gly Lys Gln Glu Val Ile
            260                 265                 270

Arg Gly Trp Glu Glu Gly Val Ala Gln Met Ser Val Gly Gln Arg Ala
        275                 280                 285

Lys Leu Thr Ile Ser Pro Asp Tyr Ala Tyr Gly Ala Thr Gly His Pro
    290                 295                 300

Gly Ile Ile Pro Pro His Ala Thr Leu Val Phe Asp Val Glu Leu Leu
305                 310                 315                 320

Lys Leu Glu Gly Ser Gly Ser Gly Ser Gly Ser Ser Leu Arg Val Lys
                325                 330                 335

Phe Ser Arg Ser Ala Asp Ala Pro Ala Tyr Lys Gln Gly Gln Asn Gln
            340                 345                 350

Leu Tyr Asn Glu Leu Asn Leu Gly Arg Arg Glu Glu Tyr Asp Val Leu
        355                 360                 365

Asp Lys Arg Arg Gly Arg Asp Pro Glu Met Gly Gly Lys Pro Arg Arg
    370                 375                 380

Lys Asn Pro Gln Glu Gly Leu Tyr Asn Glu Leu Gln Lys Asp Lys Met
385                 390                 395                 400

Ala Glu Ala Tyr Ser Glu Ile Gly Met Lys Gly Glu Arg Arg Arg Gly
                405                 410                 415

Lys Gly His Asp Gly Leu Tyr Gln Gly Leu Ser Thr Ala Thr Lys Asp
            420                 425                 430

Thr Tyr Asp Ala Leu His Met Gln Ala Leu Pro Pro Arg
        435                 440                 445

<210> 71

<211> 443
<212> PRT
<213> Artificial Sequence

<220>
<223> synthetic polypeptide

<400> 71

```
Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Ala Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125

Gly Gly Ala Val His Thr Arg Gly Leu Asp Phe Ala Ser Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Arg Arg Asp Gln Arg Leu Pro Pro Asp Ala
                165                 170                 175

His Lys Pro Pro Gly Gly Gly Ser Phe Arg Thr Pro Ile Gln Glu Glu
            180                 185                 190

Gln Ala Asp Ala His Ser Thr Leu Ala Lys Ile Ser Arg Gly Ser Gly
        195                 200                 205

Ser Gly Ser Gly Ser Met Gly Val Gln Val Glu Thr Ile Ser Pro Gly
    210                 215                 220
```

Asp Gly Arg Thr Phe Pro Lys Arg Gly Gln Thr Cys Val Val His Tyr
225 230 235 240

Thr Gly Met Leu Glu Asp Gly Lys Lys Val Asp Ser Ser Arg Asp Arg
245 250 255

Asn Lys Pro Phe Lys Phe Met Leu Gly Lys Gln Glu Val Ile Arg Gly
260 265 270

Trp Glu Glu Gly Val Ala Gln Met Ser Val Gly Gln Arg Ala Lys Leu
275 280 285

Thr Ile Ser Pro Asp Tyr Ala Tyr Gly Ala Thr Gly His Pro Gly Ile
290 295 300

Ile Pro Pro His Ala Thr Leu Val Phe Asp Val Glu Leu Leu Lys Leu
305 310 315 320

Glu Gly Ser Gly Ser Gly Ser Gly Ser Ser Leu Arg Val Lys Phe Ser
325 330 335

Arg Ser Ala Asp Ala Pro Ala Tyr Lys Gln Gly Gln Asn Gln Leu Tyr
340 345 350

Asn Glu Leu Asn Leu Gly Arg Arg Glu Glu Tyr Asp Val Leu Asp Lys
355 360 365

Arg Arg Gly Arg Asp Pro Glu Met Gly Gly Lys Pro Arg Arg Lys Asn
370 375 380

Pro Gln Glu Gly Leu Tyr Asn Glu Leu Gln Lys Asp Lys Met Ala Glu
385 390 395 400

Ala Tyr Ser Glu Ile Gly Met Lys Gly Glu Arg Arg Arg Gly Lys Gly
405 410 415

His Asp Gly Leu Tyr Gln Gly Leu Ser Thr Ala Thr Lys Asp Thr Tyr
420 425 430

Asp Ala Leu His Met Gln Ala Leu Pro Pro Arg
435 440

<210> 72
<211> 320
<212> PRT
<213> Artificial Sequence

<220>
<223> synthetic polypeptide

<400> 72

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Ala Gly Gln Arg Ile Ala Phe
35 40 45

Gly Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asn Trp Ser His Pro Gln Phe Glu
85 90 95

Lys Thr Thr Thr Pro Ala Pro Arg Pro Pro Thr Pro Ala Pro Thr Ile
100 105 110

Ala Ser Gln Pro Leu Ser Leu Arg Pro Glu Ala Cys Arg Pro Ala Ala
115 120 125

Gly Gly Ala Val His Thr Arg Gly Leu Asp Phe Ala Cys Asp Ile Tyr
130 135 140

Ile Trp Ala Pro Leu Ala Gly Thr Cys Gly Val Leu Leu Leu Ser Leu
145 150 155 160

Val Ile Thr Leu Tyr Cys Lys Arg Gly Arg Lys Lys Leu Leu Tyr Ile
165 170 175

Phe Lys Gln Pro Phe Met Arg Pro Val Gln Thr Thr Gln Glu Glu Asp
180 185 190

Gly Cys Ser Cys Arg Phe Pro Glu Glu Glu Glu Gly Gly Cys Glu Leu
195 200 205

Arg Val Lys Phe Ser Arg Ser Ala Asp Ala Pro Ala Tyr Lys Gln Gly
210 215 220

Gln Asn Gln Leu Tyr Asn Glu Leu Asn Leu Gly Arg Arg Glu Glu Tyr
225 230 235 240

```
Asp Val Leu Asp Lys Arg Arg Gly Arg Asp Pro Glu Met Gly Gly Lys
                245                 250                 255

Pro Arg Arg Lys Asn Pro Gln Glu Gly Leu Tyr Asn Glu Leu Gln Lys
            260                 265                 270

Asp Lys Met Ala Glu Ala Tyr Ser Glu Ile Gly Met Lys Gly Glu Arg
        275                 280                 285

Arg Arg Gly Lys Gly His Asp Gly Leu Tyr Gln Gly Leu Ser Thr Ala
    290                 295                 300

Thr Lys Asp Thr Tyr Asp Ala Leu His Met Gln Ala Leu Pro Pro Arg
305                 310                 315                 320
```

<210> 73
<211> 320
<212> PRT
<213> Artificial Sequence

<220>
<223> synthetic polypeptide

<400> 73

```
Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Met Tyr Val Ile Arg Ala Gly Gln Arg Ile Ala Phe
        35                  40                  45

Gly Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Ser Arg Pro Ala Ala
        115                 120                 125
```

Gly Gly Ala Val His Thr Arg Gly Leu Asp Phe Ala Ser Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Arg Val Lys Phe Ser Arg Ser Ala Asp Ala Pro Ala Tyr Lys Gln Gly
    210                 215                 220

Gln Asn Gln Leu Tyr Asn Glu Leu Asn Leu Gly Arg Arg Glu Glu Tyr
225                 230                 235                 240

Asp Val Leu Asp Lys Arg Arg Gly Arg Asp Pro Glu Met Gly Gly Lys
                245                 250                 255

Pro Arg Arg Lys Asn Pro Gln Glu Gly Leu Tyr Asn Glu Leu Gln Lys
            260                 265                 270

Asp Lys Met Ala Glu Ala Tyr Ser Glu Ile Gly Met Lys Gly Glu Arg
        275                 280                 285

Arg Arg Gly Lys Gly His Asp Gly Leu Tyr Gln Gly Leu Ser Thr Ala
    290                 295                 300

Thr Lys Asp Thr Tyr Asp Ala Leu His Met Gln Ala Leu Pro Pro Arg
305                 310                 315                 320

<210> 74
<211> 320
<212> PRT
<213> Artificial Sequence

<220>
<223> synthetic polypeptide

<400> 74

Met Pro Leu Leu Leu Leu Leu Pro Leu Leu Trp Ala Gly Ala Leu Ala
1               5                   10                  15

```
Met Ala Thr Val Lys Phe Thr Tyr Gln Gly Glu Glu Lys Gln Val Asp
            20                  25                  30

Ile Ser Lys Ile Met Tyr Val Ile Arg Gly Gly Gln Arg Ile Ala Phe
        35                  40                  45

Gly Tyr Asp Glu Gly Asp Gly Ala Trp Gly Asp Gly Ile Val Ser Glu
    50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Cys Arg Pro Ala Ala
        115                 120                 125

Gly Gly Ala Val His Thr Arg Gly Leu Asp Phe Ala Cys Asp Ile Tyr
    130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
        195                 200                 205

Arg Val Lys Phe Ser Arg Ser Ala Asp Ala Pro Ala Tyr Lys Gln Gly
    210                 215                 220

Gln Asn Gln Leu Tyr Asn Glu Leu Asn Leu Gly Arg Arg Glu Glu Tyr
225                 230                 235                 240

Asp Val Leu Asp Lys Arg Arg Gly Arg Asp Pro Glu Met Gly Gly Lys
                245                 250                 255

Pro Arg Arg Lys Asn Pro Gln Glu Gly Leu Tyr Asn Glu Leu Gln Lys
            260                 265                 270
```

Asp Lys Met Ala Glu Ala Tyr Ser Glu Ile Gly Met Lys Gly Glu Arg
275 280 285

Arg Arg Gly Lys Gly His Asp Gly Leu Tyr Gln Gly Leu Ser Thr Ala
290 295 300

Thr Lys Asp Thr Tyr Asp Ala Leu His Met Gln Ala Leu Pro Pro Arg
305 310 315 320

<210> 75
<211> 320
<212> PRT
<213> Artificial Sequence

<220>
<223> synthetic polypeptide

<400> 75

Met Pro Leu Leu Leu Leu Leu Pro Leu Leu Trp Ala Gly Ala Leu Ala
1 5 10 15

Met Ala Thr Val Lys Phe Thr Tyr Gln Gly Glu Glu Lys Gln Val Asp
20 25 30

Ile Ser Lys Ile Met Tyr Val Ile Arg Gly Gly Gln Arg Ile Ala Phe
35 40 45

Gly Tyr Asp Glu Gly Asp Gly Ala Trp Gly Asp Gly Ile Val Ser Glu
50 55 60

Lys Asp Ala Pro Lys Glu Leu Leu Gln Met Leu Glu Lys Gln Gly Gly
65 70 75 80

Gly Gly Ser Gly Gly Gly Gly Ser Asn Trp Ser His Pro Gln Phe Glu
85 90 95

Lys Thr Thr Thr Pro Ala Pro Arg Pro Pro Thr Pro Ala Pro Thr Ile
100 105 110

Ala Ser Gln Pro Leu Ser Leu Arg Pro Glu Ala Ser Arg Pro Ala Ala
115 120 125

Gly Gly Ala Val His Thr Arg Gly Leu Asp Phe Ala Ser Asp Ile Tyr
130 135 140

Ile Trp Ala Pro Leu Ala Gly Thr Cys Gly Val Leu Leu Leu Ser Leu
145 150 155 160

Val Ile Thr Leu Tyr Cys Lys Arg Gly Arg Lys Lys Leu Leu Tyr Ile
165 170 175

Phe Lys Gln Pro Phe Met Arg Pro Val Gln Thr Thr Gln Glu Glu Asp
180 185 190

Gly Cys Ser Cys Arg Phe Pro Glu Glu Glu Glu Gly Gly Cys Glu Leu
195 200 205

Arg Val Lys Phe Ser Arg Ser Ala Asp Ala Pro Ala Tyr Lys Gln Gly
210 215 220

Gln Asn Gln Leu Tyr Asn Glu Leu Asn Leu Gly Arg Arg Glu Glu Tyr
225 230 235 240

Asp Val Leu Asp Lys Arg Arg Gly Arg Asp Pro Glu Met Gly Gly Lys
245 250 255

Pro Arg Arg Lys Asn Pro Gln Glu Gly Leu Tyr Asn Glu Leu Gln Lys
260 265 270

Asp Lys Met Ala Glu Ala Tyr Ser Glu Ile Gly Met Lys Gly Glu Arg
275 280 285

Arg Arg Gly Lys Gly His Asp Gly Leu Tyr Gln Gly Leu Ser Thr Ala
290 295 300

Thr Lys Asp Thr Tyr Asp Ala Leu His Met Gln Ala Leu Pro Pro Arg
305 310 315 320

<210> 76
<211> 9
<212> PRT
<213> Artificial Sequence

<220>
<223> synthetic polypeptide

<400> 76

Asn Trp Ser His Pro Gln Phe Glu Lys
1 5

<210> 77
<211> 6
<212> PRT
<213> Artificial Sequence

<220>
<223> synthetic polypeptide

<400> 77

```
His His His His His His
1               5
```